# EUROPEAN PATENT APPLICATION

(11) **EP 2 103 665 A1**
(43) Date of publication of application: **23.09.2009**
(21) Application number: 09154747.1
(22) Date of filing: 10.03.2009
(51) Int. Cl.: C09K 11/06, H05B 33/14, C07D 221/08, C07F 15/00, H01L 51/50, H01L 51/00

(54) **Novel organic electroluminescent compounds and organic electroluminescent device using the same**

(30) Priority: 19.03.2008 KR 20080025361
(71) Applicant: Gracel Display Inc., Seoul 133-833 (KR)
(72) Inventor: Cho, Young Jun, 136-060, Seoul (KR); Kwon, Hyuck Joo, 130-100, Seoul (KR); Kim, Bong Ok, 135-090, Seoul (KR); Kim, Sung Min, 157-886, Seoul (KR); Yoon, Seung Soo, 135-884, Seoul (KR)
(74) Representative: Kent, Venetia Katherine

(57) **Abstract**

The present invention relates to novel organic electroluminescent compounds, and organic electroluminescent devices comprising the same. Specifically, the novel organic electroluminescent compounds according to the invention are **characterized in that** they are represented by Chemical Formula (1) :

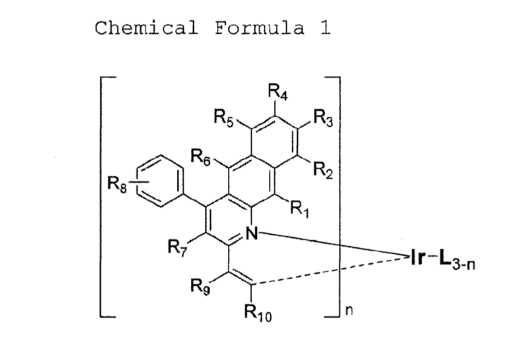

## Description

### FIELD OF THE INVENTION

The present invention relates to novel organic electroluminescent compounds, and organic electroluminescent devices employing the same as electroluminescent dopant. More specifically, the invention relates to novel iridium compounds having red phosphorescent property of high efficiency, which can be used for a constituent of an electroluminescent layer in an electroluminescent device; and organic electroluminescent devices employing the compounds as electroluminescent dopant.

### BACKGROUND OF THE INVENTION

The most important factor to determine luminous efficiency in an OLED (organic light-emitting diode) is electroluminescent material. Though fluorescent materials has been widely used as electroluminescent material up to the present, development of phosphorescent materials is one of the best methods to improve the luminous efficiency theoretically up to four (4) times, in view of electroluminescent mechanism.

Up to now, iridium (III) complexes have been widely known as phosphorescent material, including (acac)Ir(btp)₂, Ir(ppy)₃ and Firpic, as the red, green and blue one, respectively. In particular, a lot of phosphorescent materials have been recently investigated in Japan, Europe and America.

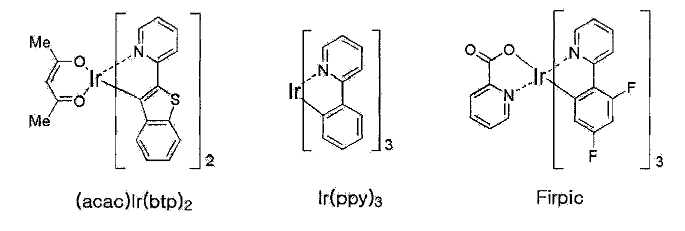

Among conventional red phosphorescent materials, several materials have been reported to have good EL (electroluminescence) properties. However, very rare materials among them have reached the level of commercialization. As the most preferable material, an iridium complex of 1-phenyl isoquinoline may be mentioned, which is known to have excellent EL property and to exhibit color purity of dark red with high luminous efficiency. [See A. Tsuboyama et al., J. Am. Chem. Soc. 2003, 125(42), 12971-12979.]

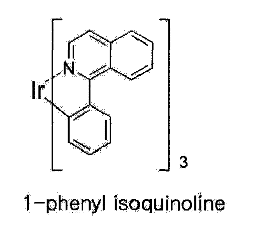

Moreover, the red materials, having no significant problem of lifetime, have tendency of easy commercialization if they have good color purity or luminous efficiency. Thus, the above-mentioned iridium complexes are a material having noticeable viability of commercialization due to their excellent color purity and luminous efficiency.

However, the iridium complex is still construed as a material which is merely applicable to small displays (thereby having limitation to be applied to medium to large sized OLED panels) because they cannot provide pure red color and high luminous efficiency at the same time, while higher levels of EL properties than those of known materials are practically required for an OLED panel of medium to large size.

### SUMMARY OF THE INVENTION

With intensive efforts to overcome the problems of conventional techniques as described above, the present inventors have researched for developing novel organic red electroluminescent compounds to realize an organic EL device having excellent luminous efficiency and surprisingly improved lifetime. Eventually, the inventors found that excellent luminous efficiency and life property with pure red color could be obtained when using an iridium complex, which was synthesized by introducing 4-phenylbenzo[g]quinoline derivative as a primary ligand instead of pyridine (as was for conventional iridium complex), and completed the present invention.

Thus, the object of the invention is to provide novel red phosphorescent compounds having the backbone to give more excellent electroluminescent properties as compared to those of conventional red phosphorescent materials, with overcoming disadvantages of them.

Another object of the invention is to provide novel phosphorescent compounds which are applicable to OLED panels of medium to large size, and organic electroluminescent devices employing the same as an electroluminescent dopant.

Thus, the present invention relates to organic electroluminescent compounds and organic electroluminescent devices employing the same in an electroluminescent layer. Specifically, the novel organic electroluminescent compounds according to the invention are represented by Chemical Formula (1) : 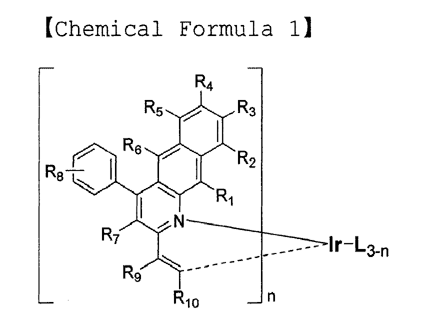 wherein, L is an organic ligand;
R₁ through R₇ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁ through R₇ may be linked to an adjacent substituent via (C3-C60)alkylene or (C3-C60)alkenylene with or without a fused ring to form an alicyclic ring, or a monocyclic or polycyclic aromatic ring;
R₈ represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
R₉ and R₁₀ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉ and R₁₀ may be linked to an adjacent substituent via (C3-C60)alkylene or (C3-C60)alkenylene with or without a fused ring to form an alicyclic ring, or a monocyclic or polycyclic aromatic ring;
the alkyl, alkenyl, alkynyl, cycloalkyl, heterocycloalkyl, aryl, heteroaryl, arylsilyl, alkylsilyl, alkylamino, arylamino of R₁ through R₁₀, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and
n is an integer from 1 to 3.

### BRIEF DESCRIPTION OF THE DRAWINGS

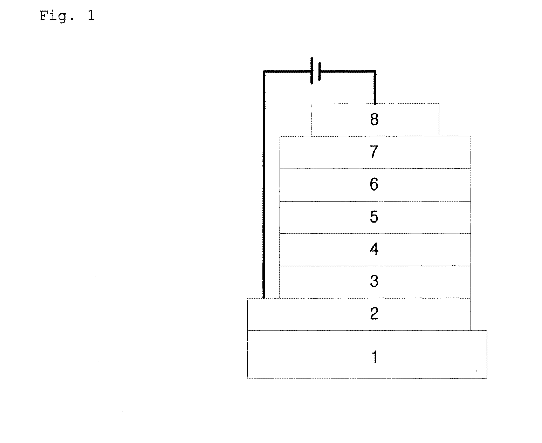

Fig. 1 is a cross-sectional view of an OLED.

### DETAILED DESCRIPTION OF THE INVENTION

Referring now to the Drawings, Fig. 1 illustrates a cross-sectional view of an OLED of the present invention comprising a Glass 1, Transparent electrode 2, Hole injecting layer 3, Hole transport layer 4, Electroluminescent layer 5, Electron transport layer 6, Electron injecting layer 7 and Al cathode 8.

The term "alkyl" includes saturated linear or branched monovalent hydrocarbon radicals consisting only of carbon atoms and hydrogen atoms, or combinations thereof. The term "alkoxy" means -O-alkyl groups, wherein the "alkyl" is defined as above.

The term "aryl" described herein means an organic radical derived from aromatic hydrocarbon via elimination of one hydrogen atom. Each ring suitably comprises a monocyclic or fused ring system containing from 4 to 7, preferably from 5 to 6 cyclic atoms. Further, "aryl" includes the structures wherein more than one aryls are bonded via chemical bond(s). Specific examples include phenyl, naphthyl, biphenyl, anthryl, indenyl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O and S for the aromatic cyclic backbone atoms, and carbon atom(s) for remaining aromatic cyclic backbone atoms. The heteroaryl may be a 5- or 6-membered monocyclic heteroaryl or a polycyclic heteroaryl which is fused with one or more benzene ring(s), and may be partially saturated. The heteroaryl groups may include divalent aryl groups of which the heteroatoms are oxidized or quarternized to form N-oxides, quaternary salts, or the like. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenanthridinyl and benzodioxolyl; and corresponding N-oxides (for example, pyridyl N-oxide, quinolyl N-oxide) and quaternary salts thereof; but they are not restricted thereto.

The compound within the square bracket ([]) in Chemical Formula (1) according to the present invention serves as a primary ligand of iridium, and L serves as a subsidiary ligand. The organic phosphorescent compounds according to the present invention also include the complex with the ratio of primary ligand: subsidiary ligand = 2:1 (n=2) and the complex with the ratio of primary ligand: subsidiary ligand = 1:2 (n=1), as well as tris-chelated complexes without subsidiary ligand (L) (n=3).

The organic electroluminescent compound according to the invention may be exemplified by the compounds represented by one of Chemical Formulas (2) to (7): 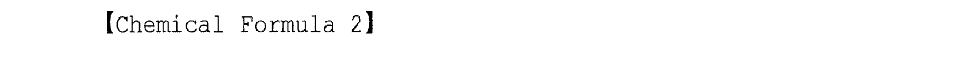 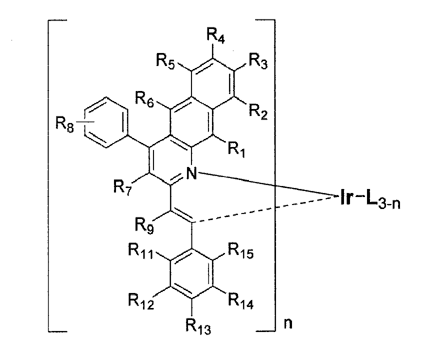 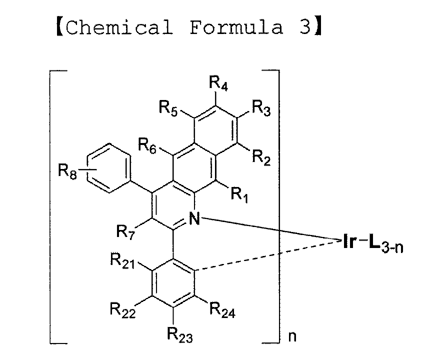 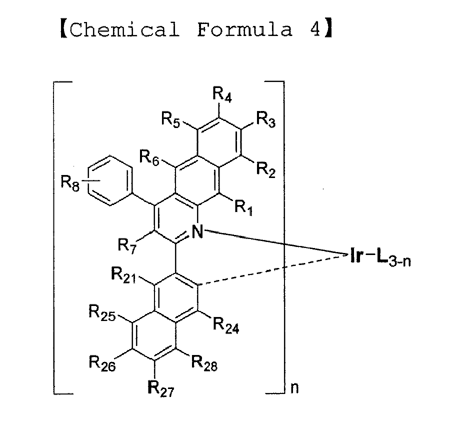 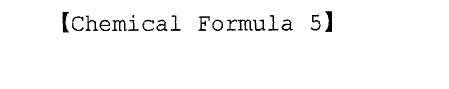 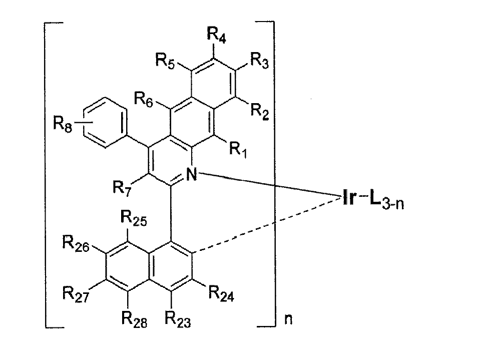 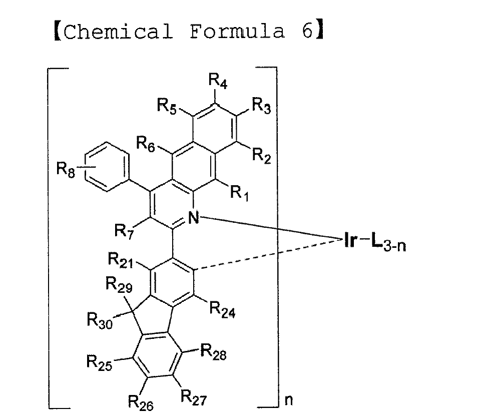 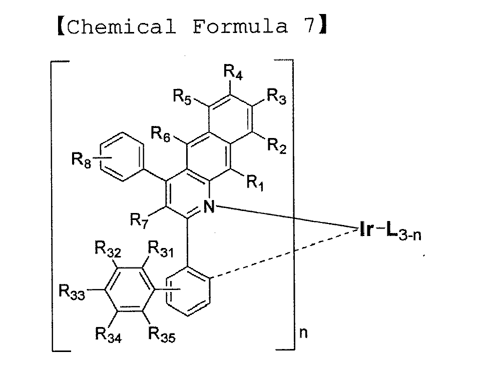 wherein, L, R₁, R₂, R₃, R₄, R₅, R₆, R₇, R₈, R₉ and n are defined as in Chemical Formula (1);
R₁₁ through R₂₈ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁₁ through R₂₈ may be linked to an adjacent substituent via (C3-C60)alkylene or (C3-C60)alkenylene with or without a fused ring to form an alicyclic ring, or a monocyclic or polycyclic aromatic ring;
R₂₉ and R₃₀ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₂₉ and R₃₀ may be linked via (C3-C60)alkylene or (C3-C60)alkenylene with or without a fused ring to form an alicyclic ring, or a monocyclic or polycyclic aromatic ring;
R₃₁ through R₃₅ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamin (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₃₁ through R₃₅ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkylamino, arylamino of R₁₁ through R₃₅, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamin (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

In the Chemical Formula (1), R₁ through R₁₀ are independently represent hydrogen, deuterium, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, fluoro, cyano, methoxy, ethoxy, n-propoxy, i-propoxy, n-butoxy, i-butoxy, t-butoxy, n-pentoxy, n-hexyloxy, n-heptoxy, cyclopropyl, cyclobutyl, cyclopentyl, cyclohexyl, cycloheptyl, cyclooctyl, cyclononyl, cyclodecyl, trifluoromethyl, perfluorethyl, trifluorethyl, perfluoropropyl, perfluorobutyl, phenyl, naphthyl, biphenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, pyrazinyl, pyrimidinyl, pyridazinyl, quinolyl, triazinyl, benzofuranyl, benzothiophenyl, pyrazolyl, indolyl, carbazolyl, thiazolyl, oxazolyl, benzothiazolyl, benzoxazolyl, trimethylsilyl, triethylsilyl, tripropylsilyl, tri(t-butyl)silyl, t-butyldimethylsilyl, dimethylphenylsilyl, triphenylsilyl or benzyl; and the phenyl, naphthyl, biphenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, pyrazinyl, pyrimidinyl, pyridazinyl, quinolyl, triazinyl, benzofuranyl, benzothiophenyl, pyrazolyl, indolyl, carbazolyl, thiazolyl, oxazolyl, benzothiazolyl or benzoxazolyl of R₁ through R₁₀ may be further substituted by one or more substituent(s) selected from deuterium, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluorethyl, trifluorethyl, perfluoropropyl, perfluorobutyl, methoxy, ethoxy, butoxy, hexyloxy, cyclopropyl, cyclopentyl, cyclohexyl, fluoro, cyano, phenyl, naphthyl, anthryl, trimethylsilyl, triethylsilyl, tripropylsilyl, tri(t-butyl)silyl, t-butyldimethylsilyl, dimethylphenylsilyl and triphenylsilyl.

The organic electroluminescent compounds according to the present invention can be specifically exemplified by the following compounds, but they are not restricted thereto: 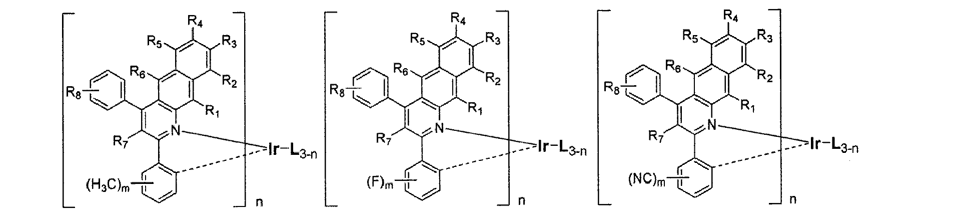 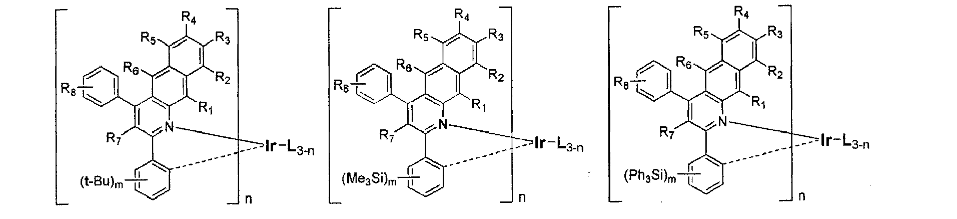 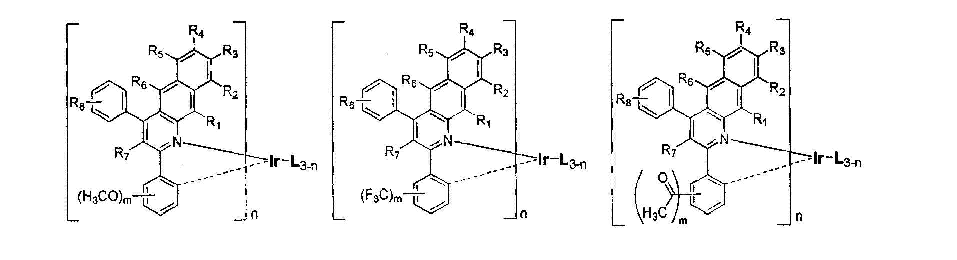 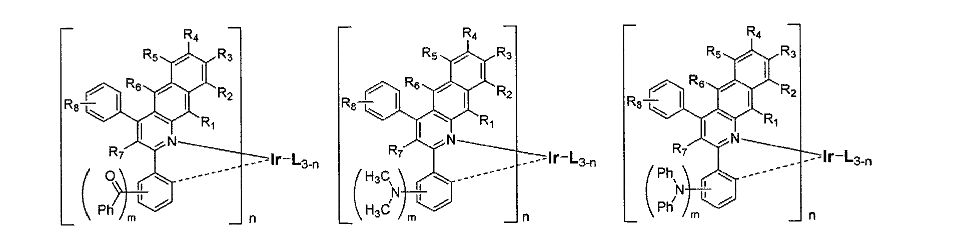 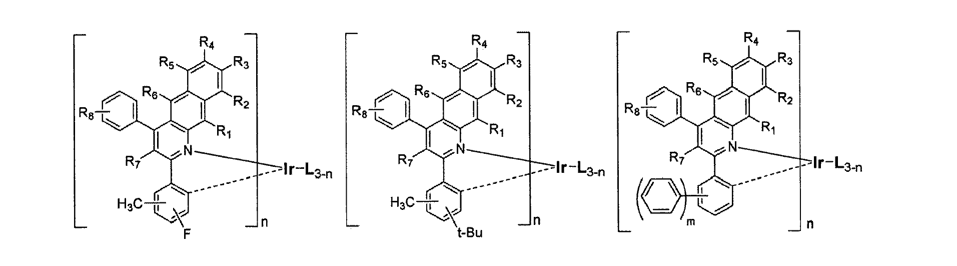 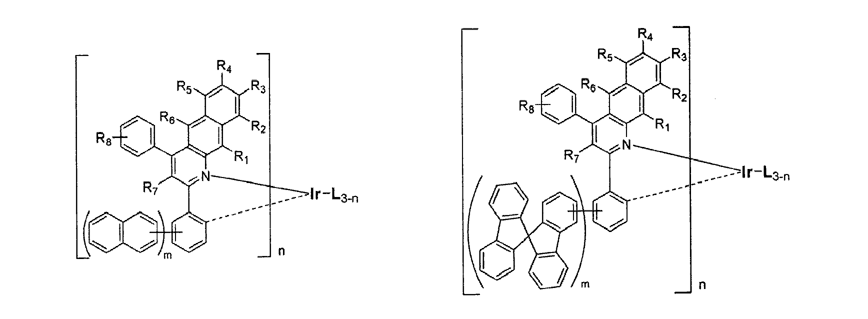 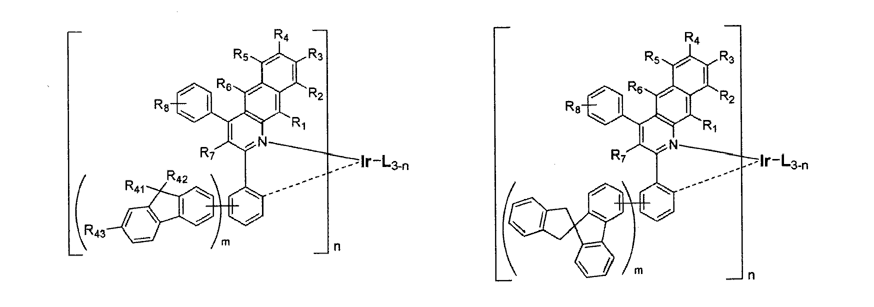 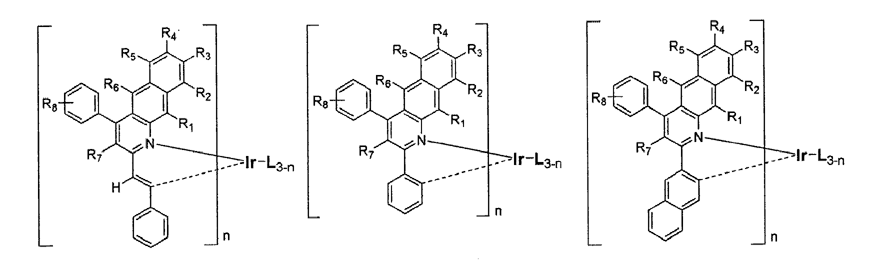 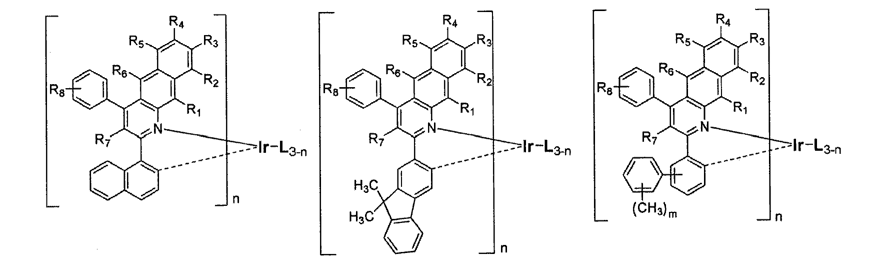 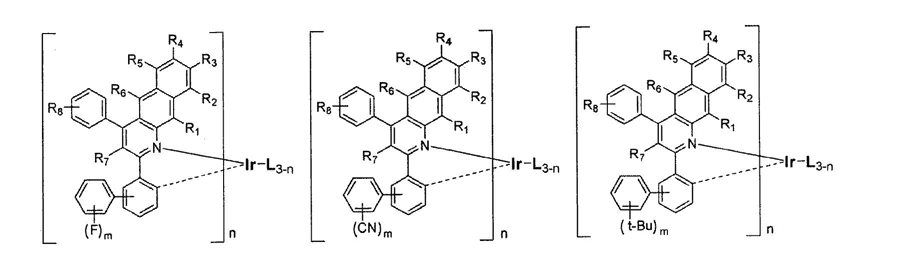 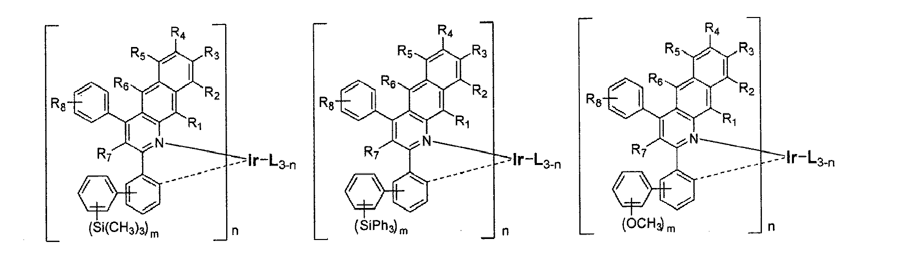 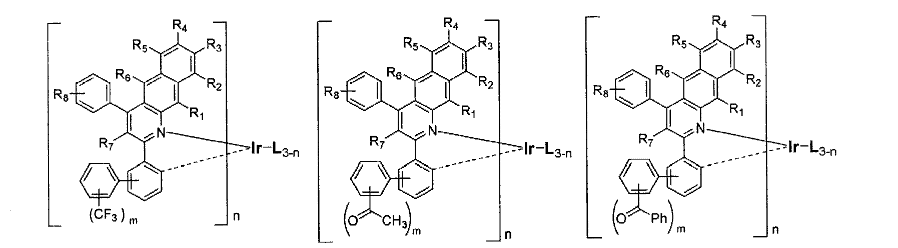 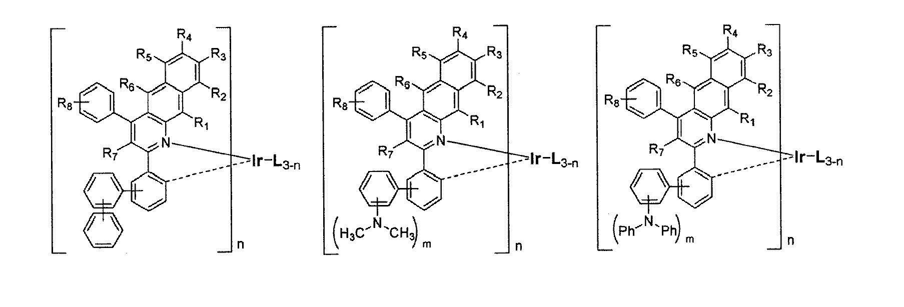 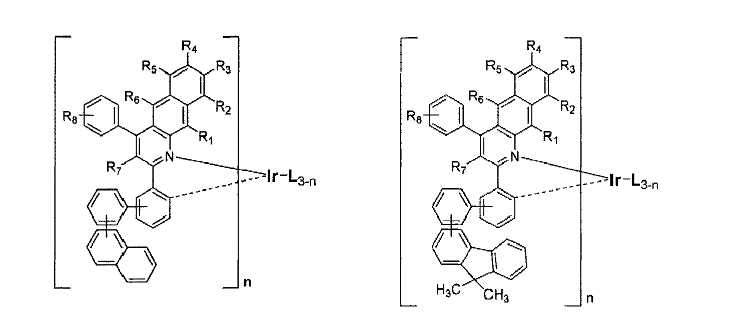 wherein, L, R₁, R₂, R₃, R₄, R₅, R₆, R₇, R₈ and n are defined as in Chemical Formula (1);
R₄₁ and R₄₂ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₄₁ and R₄₂ may be linked to an adjacent substituent via (C3-C60)alkylene or (C3-C60)alkenylene with or without a fused ring to form an alicyclic ring, or a monocyclic or polycyclic aromatic ring;
R₄₃ represents hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
the alkyl, alkenyl, alkynyl, cycloalkyl, heterocycloalkyl, aryl, heteroaryl, arylsilyl, alkylsilyl, alkylamino, arylamino of R₄₁ through R₄₃, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and
m is an integer from 1 to 5.

The subsidiary ligands (L) of the organic electroluminescent compounds according to the present invention include the following structures: 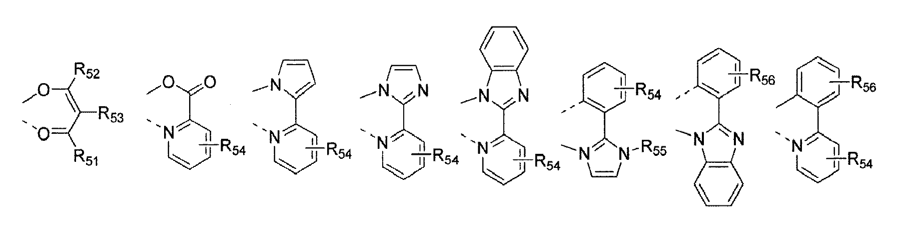 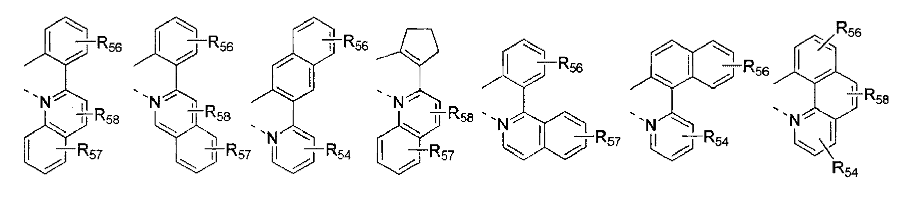 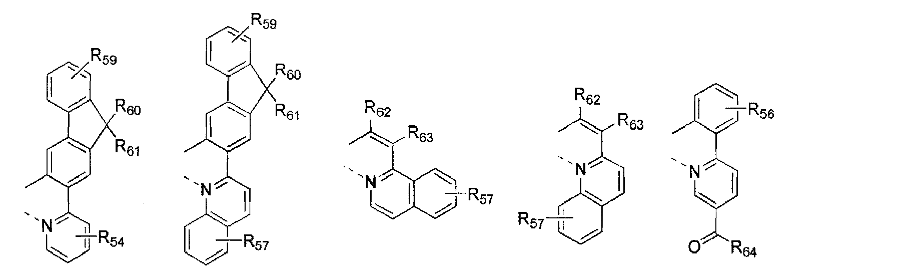 wherein, R₅₁ through R₆₄ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₅₁ through R₆₄ may be linked to an adjacent substituent via (C3-C60)alkylene or (C3-C60)alkenylene with or without a fused ring to form an alicyclic ring, or a monocyclic or polycyclic aromatic ring; and the alkyl, alkenyl, alkynyl, cycloalkyl, heterocycloalkyl, aryl, heteroaryl, arylsilyl, alkylsilyl, alkylamino, arylamino of R₅₁ through R₆₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The subsidiary ligands (L) of the organic electroluminescent compounds according to the present invention can be exemplified by the following structures, but they are not restricted thereto:

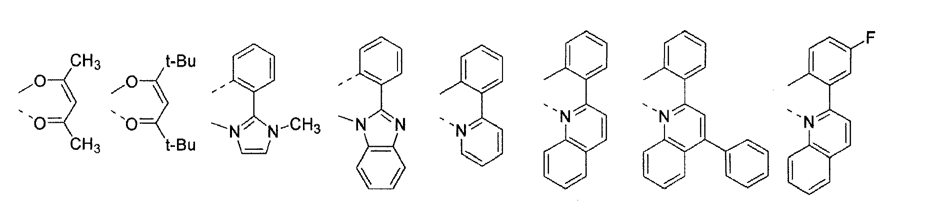

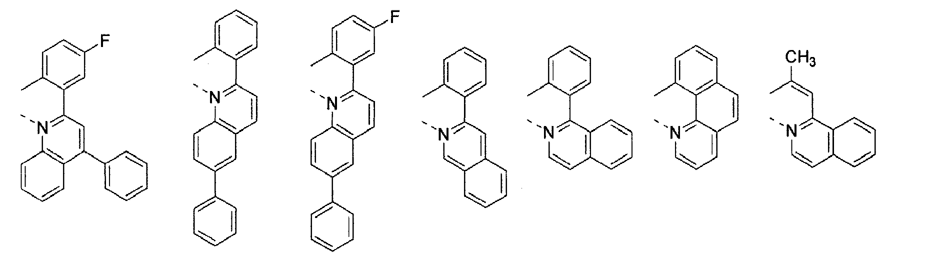

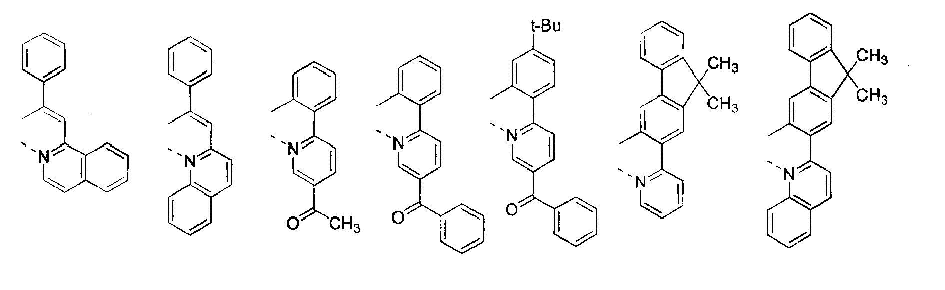

The processes for preparing the organic electroluminescent compounds according to the present invention are described by referring to Reaction Schemes (1) to (3) shown below: 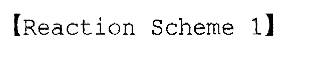 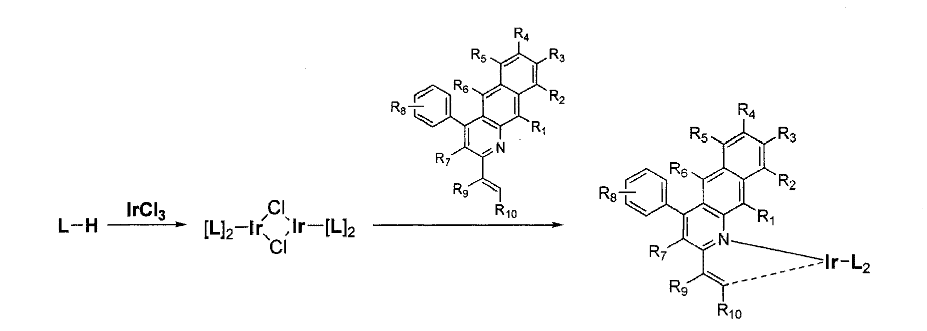 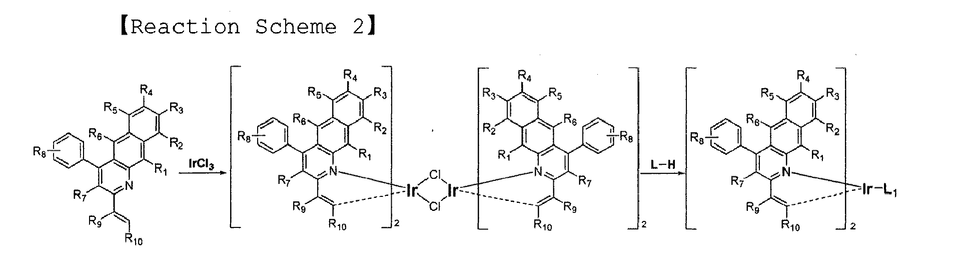 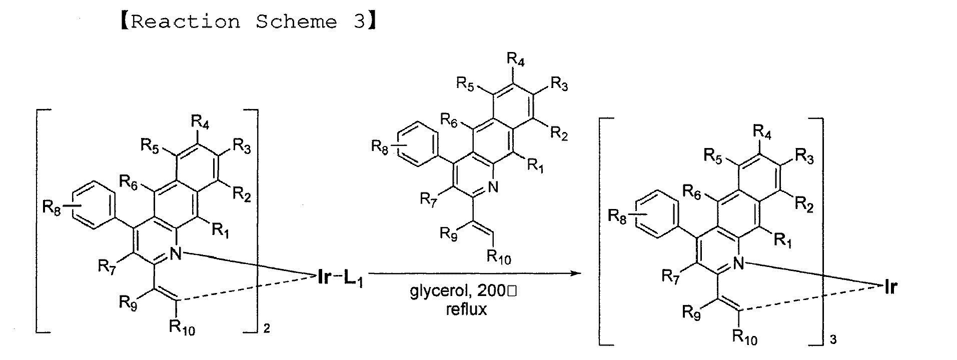 wherein, R₁, R₂, R₃, R₄, R₅, R₆, R₇, R₈, R₉, R₁₀ and L are defined as in Chemical Formula (1).

Reaction Scheme (1) provides a compound of Chemical Formula (1) with n=1, in which iridium trichloride (IrCl₃) and subsidiary ligand compound (L-H) are mixed in a solvent at a molar ratio of 1:2∼3, and the mixture is heated under reflux before isolating diiridium dimer. In the reaction stage, preferable solvent is alcohol or a mixed solvent of alcohol/water, such as 2-ethoxyethanol, and 2-ethoxyethanol/water mixtures. The isolated diiridium dimer is then heated with a primary ligand compound in organic solvent to provide an organic phosphorescent iridium compound having the ratio of primary ligand: subsidiary ligand of 1:2 as the final product. The reaction is carried out with AgCF₃SO₃, Na₂CO₃ or NaOH being admixed with organic solvent such as 2-ethoxyethanol and 2-methoxyethylether.

Reaction Scheme (2) provides a compound of Chemical Formula (1) with n=2, in which iridium trichloride (IrCl₃) and a primary ligand compound are mixed in a solvent at a molar ratio of 1:2∼3, and the mixture is heated under reflux before isolating diiridium dimer. In the reaction stage, preferable solvent is alcohol or a mixed solvent of alcohol/water, such as 2-ethoxyethanol, and 2-ethoxyethanol/water mixtures. The isolated diiridium dimer is then heated with the subsidiary ligand compound (L-H) in organic solvent to provide an organic phosphorescent iridium compound having the ratio of primary ligand: subsidiary ligand of 2:1 as the final product.

The molar ratio of the primary ligand compound to the subsidiary ligand (L) in the final product is determined by appropriate molar ratio of the reactant depending on the composition. The reaction may be carried out with AgCF₃SO₃, Na₂CO₃ or NaOH being admixed with organic solvent such as 2-ethoxyethanol, 2-methoxyethylether and 1,2-dichloroethane.

Reaction Scheme (3) provides a compound of Chemical Formula (1) with n=3, in which iridium complex prepared according to Reaction Scheme (2) and the primary ligand compound are mixed in glycerol at a molar ratio of 1:2∼3, and the mixture is heated under reflux to obtain organic phosphorescent iridium complex coordinated with three primary ligands.

The compounds employed as a primary ligand in the present invention can be prepared, without limitation, according to the process illustrated by Reaction Scheme (4), on the basis of conventional processes. 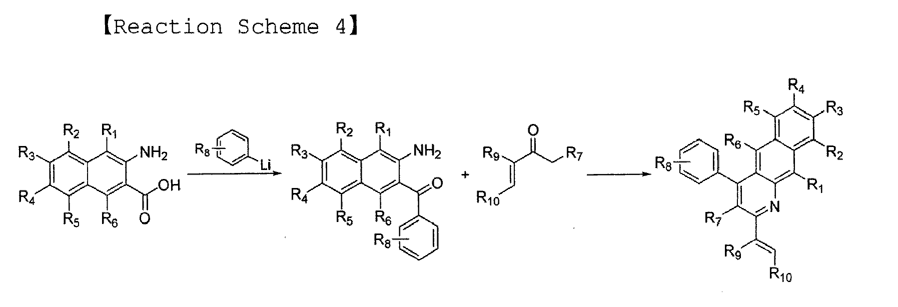 wherein, R₁ through R₁₀ are defined as in Chemical Formula (1).

The present invention also provides organic solar cells, which comprises one or more organic electroluminescent compound(s) represented by Chemical Formula (1).

The present invention also provides an organic electroluminescent device which is comprised of a first electrode; a second electrode; and at least one organic layer(s) interposed between the first electrode and the second electrode; wherein the organic layer comprises one or more compound(s) represented by Chemical Formula (1).

The organic electroluminescent device according to the present invention is characterized in that the organic layer comprises an electroluminescent region, which comprises one or more compound(s) represented by Chemical Formula (1) as electroluminescent dopant in an amount of 0.01 to 10% by weight, and one or more host(s). The host applied to the organic electroluminescent device according to the invention is not particularly restricted, but may be exemplified by 1,3,5-tricarbazolylbenzene, polyvinylcarbazole, m-biscarbazolylphenyl, 4,4'4"-tri(N-carbazolyl)triphenylamine, 1,3,5-tri(2-carbazolylphenyl)benzene, 1,3,5-tris(2-carbazolyl-5-methoxyphenyl)benzene, bis(4-carbazolylphenyl)silane or the compounds represented by one of Chemical Formulas (8) to (11):

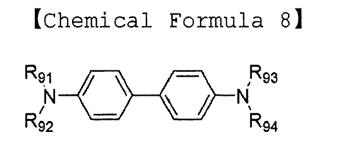

In Chemical Formula (8), R₉₁ through R₉₄ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉₁ through R₉₄ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkylamino, or arylamino of R₉₁ through R₉₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

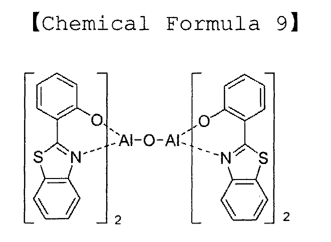

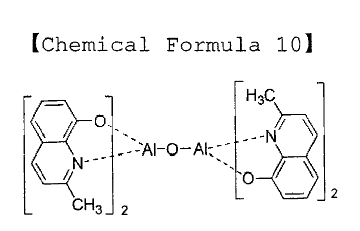

[Chemical Formula 11] L¹L²M¹(Q)_{y}

In Chemical Formula (11), the ligands, L¹ and L² are independently selected from the following structures: 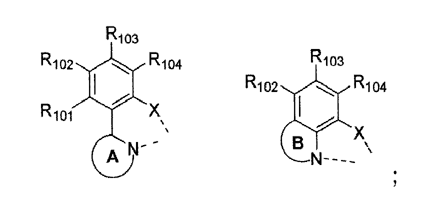
M¹ is a bivalent or trivalent metal;
y is 0 when M¹ is a bivalent metal, while y is 1 when M¹ is a trivalent metal;
Q represents (C6-C60)aryloxy or tri(C6-C60)arylsilyl, and the aryloxy and triarylsilyl of Q may be further substituted by (C1-C60)alkyl or (C6-C60)aryl;
X represents O, S or Se;
ring A represents oxazole, thiazole, imidazole, oxadiazole, thiadiazole, benzoxazole, benzothiazole, benzimidazole, pyridine or quinoline;
ring B represents pyridine or quinoline, and ring B may be further substituted by (C1-C60)alkyl, or phenyl or naphthyl with or without (C1-C60)alkyl substituent(s);
R₁₀₁ through R₁₀₄ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁₀₁ through R₁₀₄ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkylamino, or arylamino of ring A and R₁₀₁ through R₁₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The ligands, L¹ and L² are independently selected from the following structures: 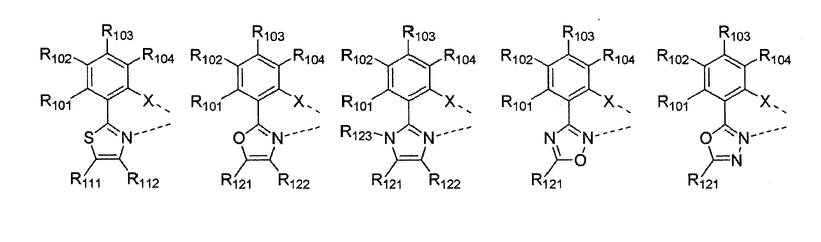 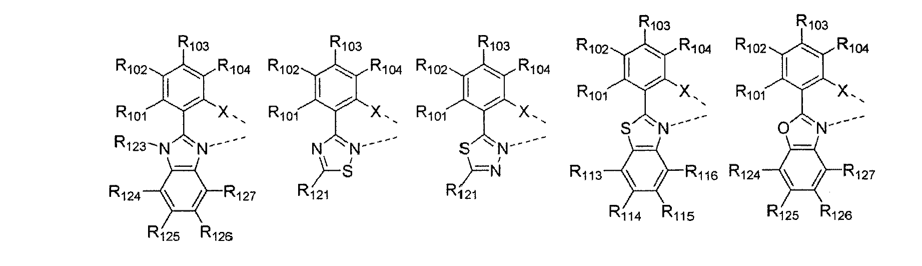 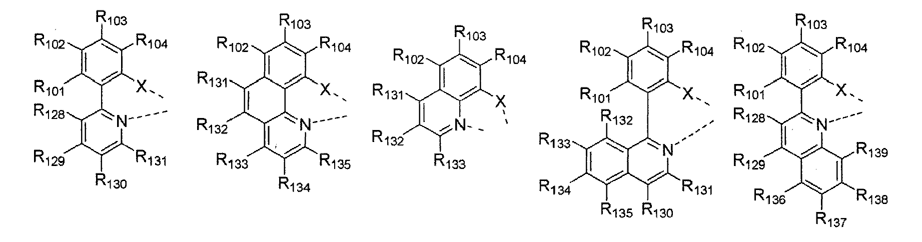 wherein, X represents O, S or Se;
R₁₀₁ through R₁₀₄ independently represent hydrogen, deuterium, halogen, (C1-C60)alkyl, (C6-C60) aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁₀₁ through R₁₀₄ may be linked to an adjacent substituent via (C3-C60)alkylene or (C3-C60)alkenylene with or without a fused ring to form an alicyclic ring, or a monocyclic or polycyclic aromatic ring;
R₁₁₁ through R₁₃₉ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁₁₁ through R₁₃₉ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkylamino, or arylamino of R₁₀₁ through R₁₀₄ and R₁₁₁ through R₁₃₉, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C1-C60)alkyloxy, (C1-C60)alkylthio, (C1-C60)alkoxycarbonyl, carboxyl, nitro and hydroxyl.

In Chemical Formula (11), M¹ is a bivalent metal selected from Be, Zn, Mg, Cu and Ni, or a trivalent metal selected from Al, Ga, In and B, and Q is selected from the following structures.

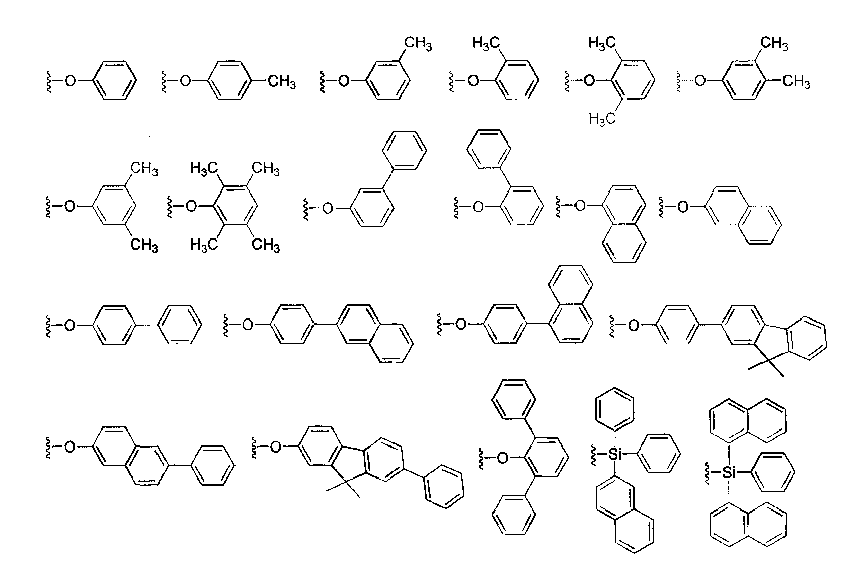

The compounds of Chemical Formula (8) may be specifically exemplified by the compounds represented by the following structures, but they are not restricted thereto.

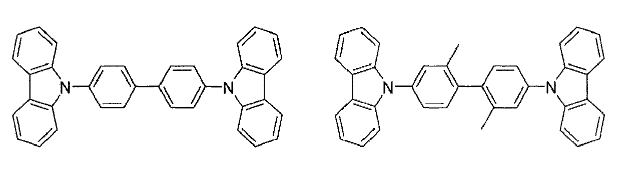

The compounds represented by one of Chemical Formula (11) may be specifically exemplified by the compounds having one of the following structures, but they are not restricted thereto.

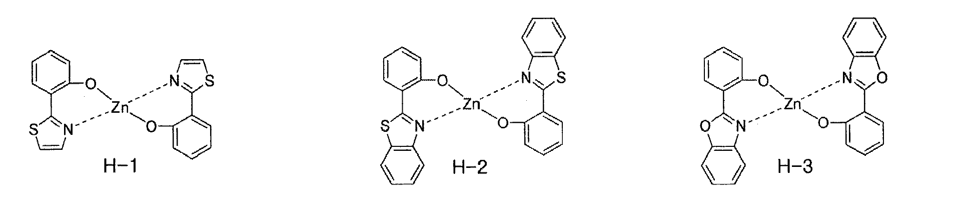

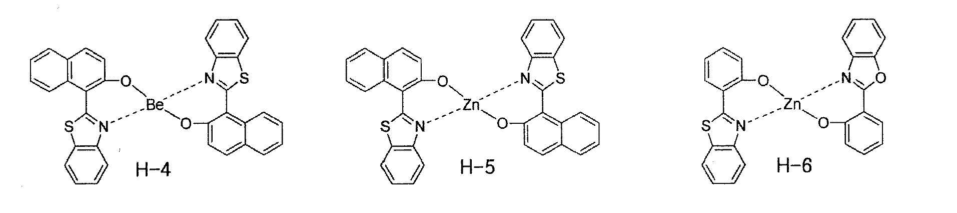

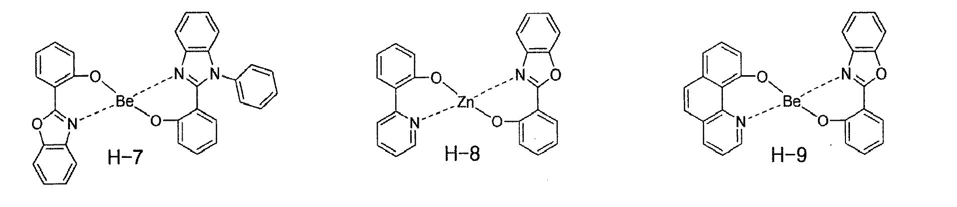

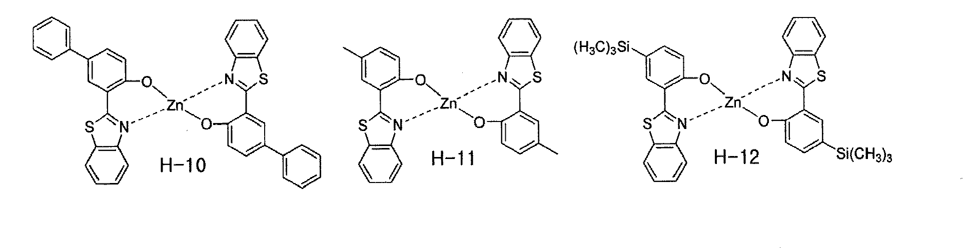

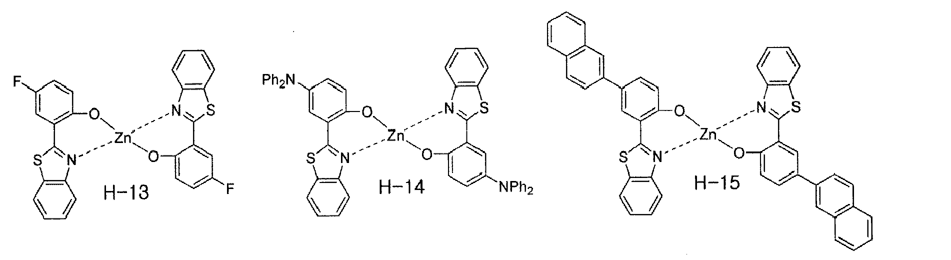

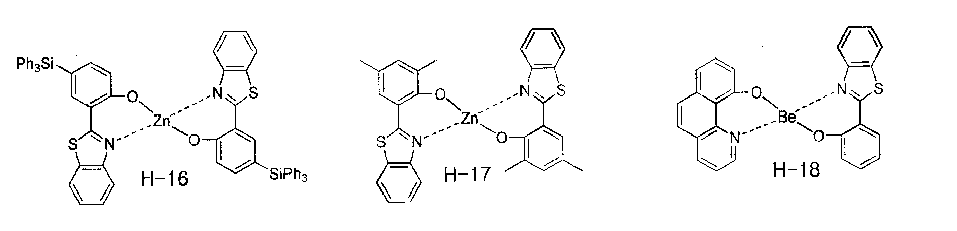

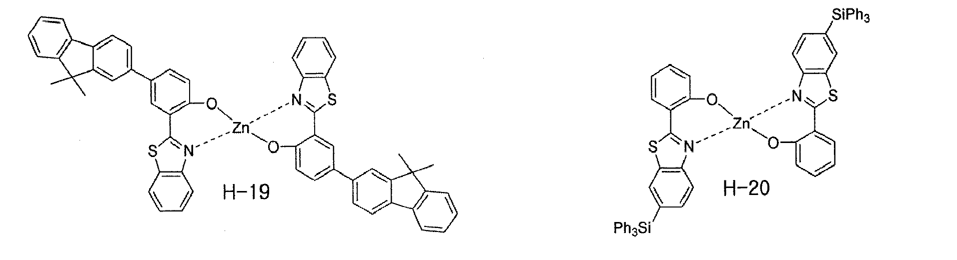

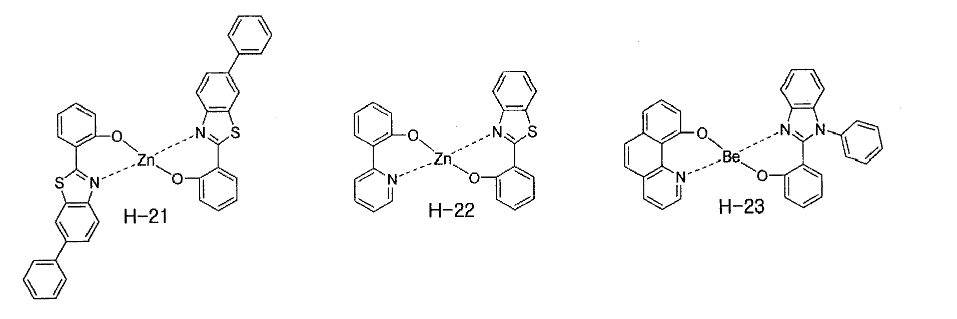

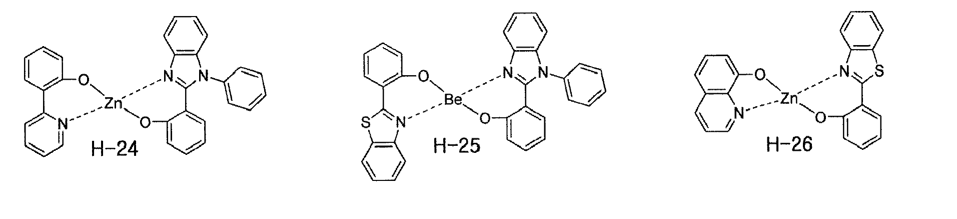

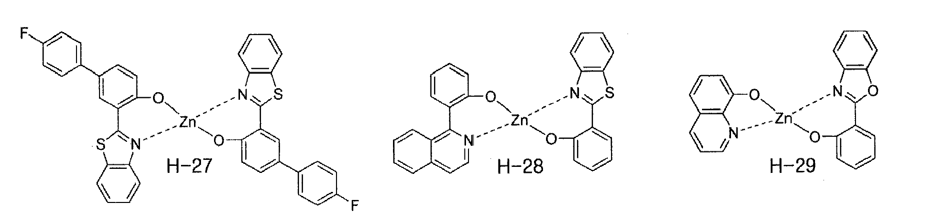

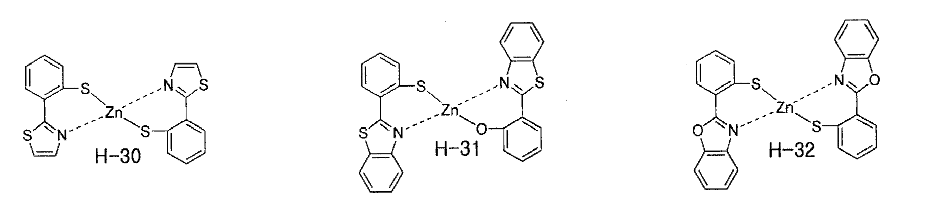

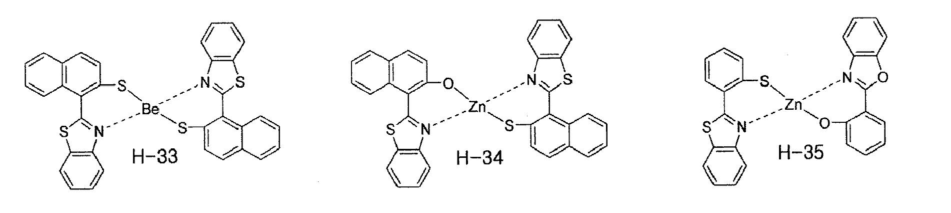

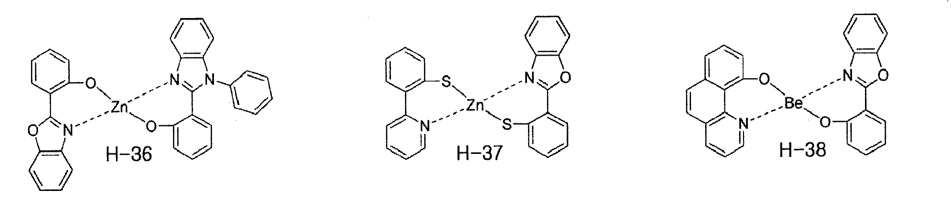

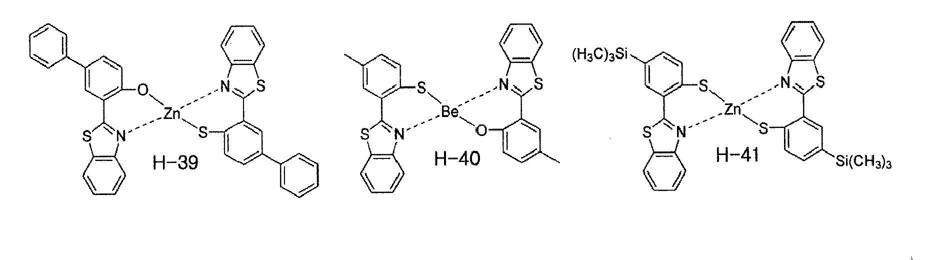

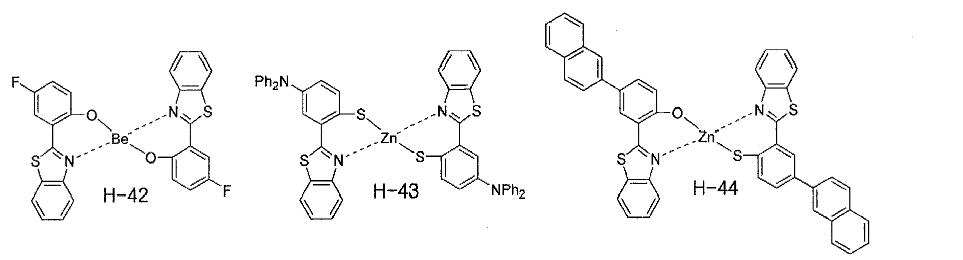

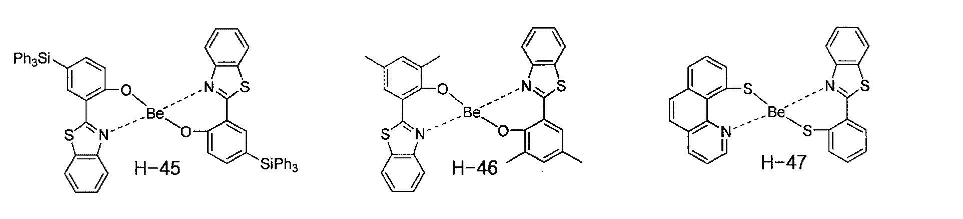

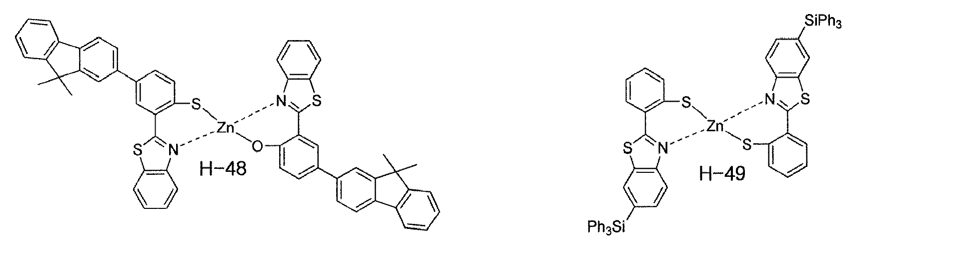

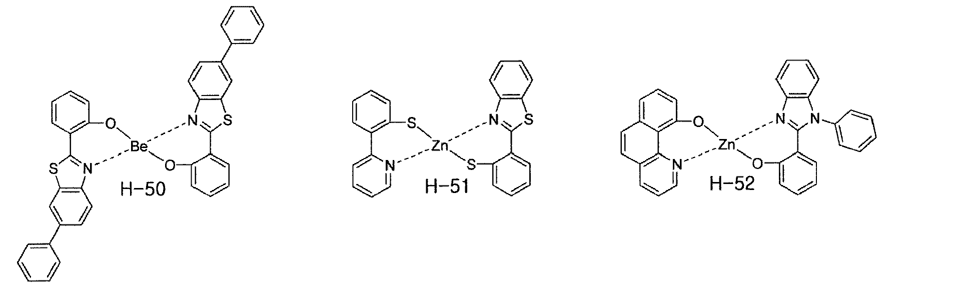

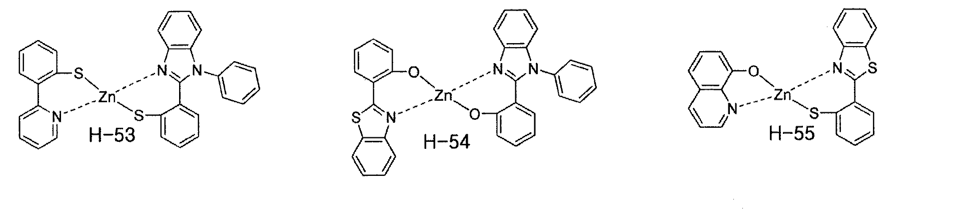

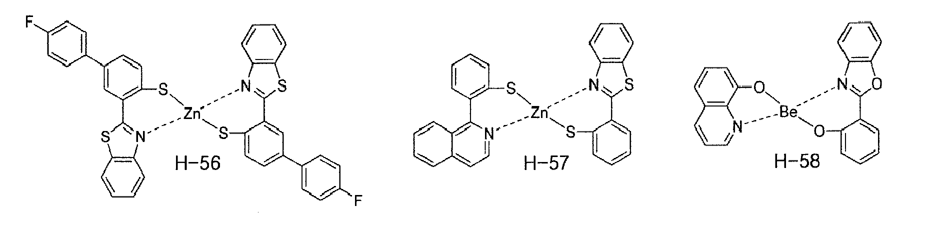

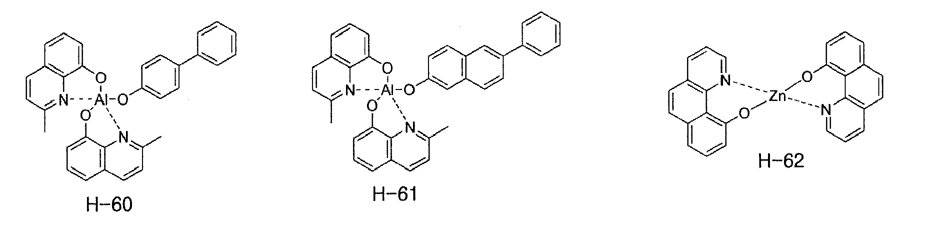

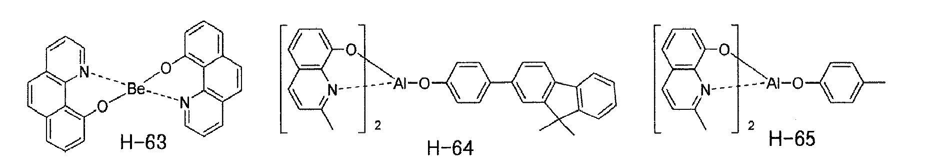

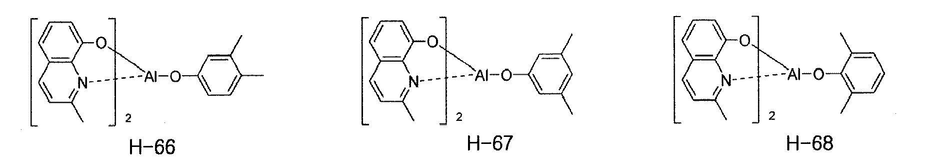

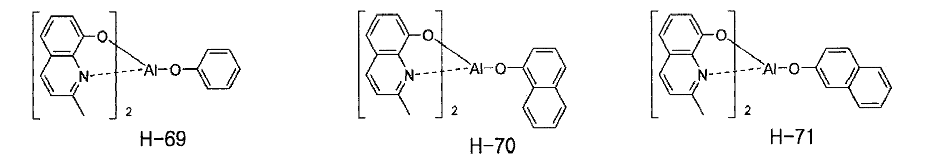

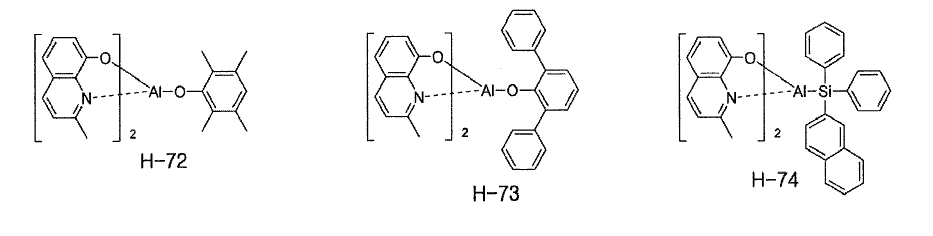

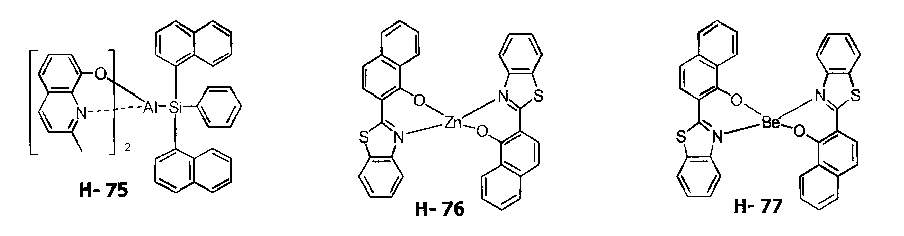

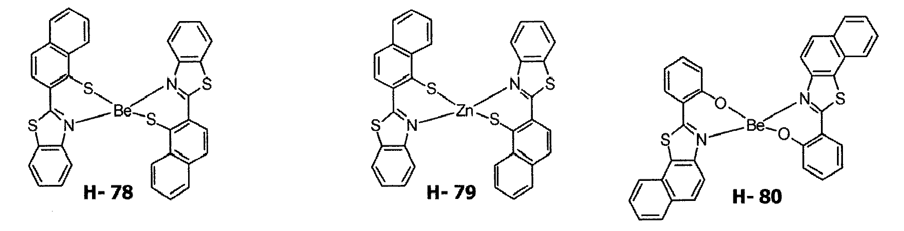

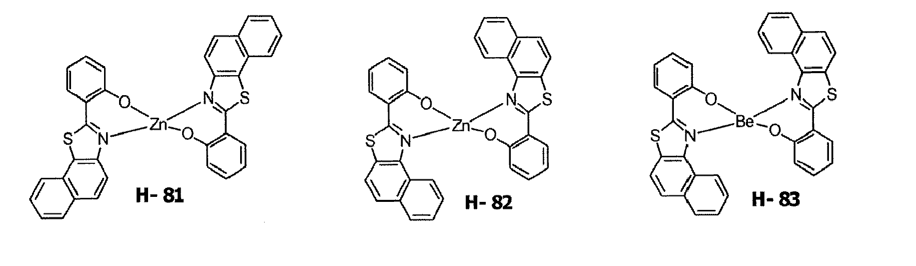

The electroluminescent layer means the layer where electroluminescence occurs, and it may be a single layer or a multi-layer consisting of two or more layers laminated. When a mixture of host-dopant is used according to the constitution of the present invention, noticeable improvement in device life as well as in luminous efficiency could be confirmed.

The organic electroluminescent device according to the invention may further comprise one or more compound(s) selected from a group consisting of arylamine compounds and styrylarylamine compounds, as well as the organic electroluminescent compound represented by Chemical Formula (1). Examples of the arylamine or styrylarylamine compounds include the compounds represented by Chemical Formula (12), but they are not restricted thereto: 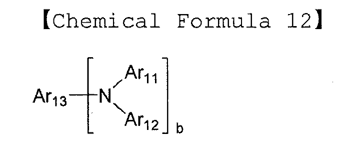 wherein, Ar₁₁ and Ar₁₂ independently represent hydrogen, deuterium, halogen, (C1-C60)alkyl, (C6-C60)aryl, (C4-C60)heteroaryl, mono or di-(C6-C60)arylamino, mono or di-(C1-C60)alkylamino, a 5- or 6-membered heterocycloalkyl containing one or more heteroatom(s) selected from N, O and S, or (C3-C60)cycloalkyl, or Ar₁₁ and Ar₁₂ may be linked via (C3-C60)alkylene or (C3-C60)alkenylene with or without a fused ring to form an alicyclic ring, or a monocyclic or polycyclic aromatic ring;
when b is 1, Ar₁₃ represents (C6-C60)aryl, (C4-C60)heteroaryl, or aryl represented by one of the following structural formulas: 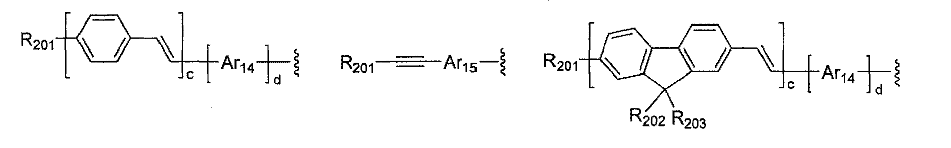 when b is 2, Ar₁₃ represents (C6-C60)arylene, (C4-C60)heteroarylene, or arylene represented by one of the following structural formulas: 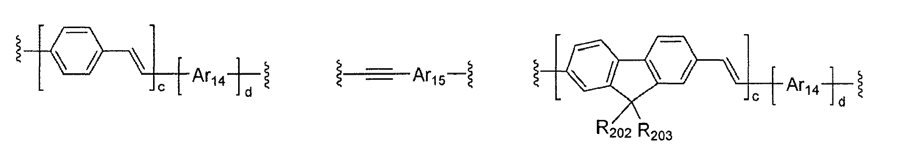 wherein Ar₁₄ and Ar₁₅ independently represent (C6-C60)arylene or (C4-C60)heteroarylene;
R₂₀₁ through R₂₀₃ independently represent hydrogen, deuterium, (C1-C60)alkyl or (C6-C60)aryl;
c is an integer from 1 to 4, d is an integer of 0 or 1; and
the alkyl, aryl, heteroaryl, arylamino, alkylamino, cycloalkyl or heterocycloalkyl of Ar₁₁ and Ar₁₂; or the alicyclic ring, or the monocyclic or polycyclic aromatic ring formed from Ar₁₁ and Ar₁₂ by linkage via alkylene or alkenylene; aryl, heteroaryl, arylene or heteroarylene of Ar₁₃; or the arylene or heteroarylene of Ar₁₄ and Ar₁₅; or the alkyl or aryl of R₂₀₁ through R₂₀₃ may be further substituted by one or more substituent(s) selected from a group consisting of halogen, deuterium, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono or di-(C1-C60)alkylamino, mono or di-(C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, (C1-C60)alkoxycarbonyloxy, (C1-C60)alkylcarbonyloxy, (C6-C60)arylcarbonyloxy, (C6-C60)aryloxycarbonyloxy, carboxyl, nitro and hydroxyl.

The arylamine compounds and styrylarylamine compounds may be more specifically exemplified by the following compounds, but are not restricted thereto.

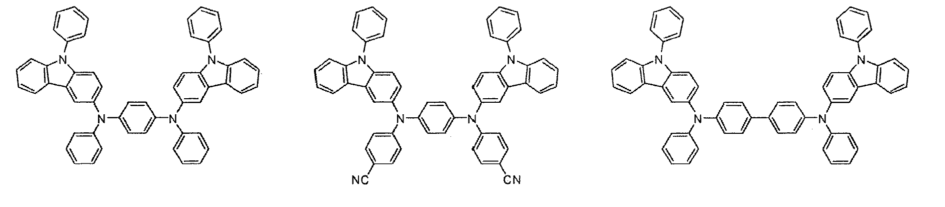

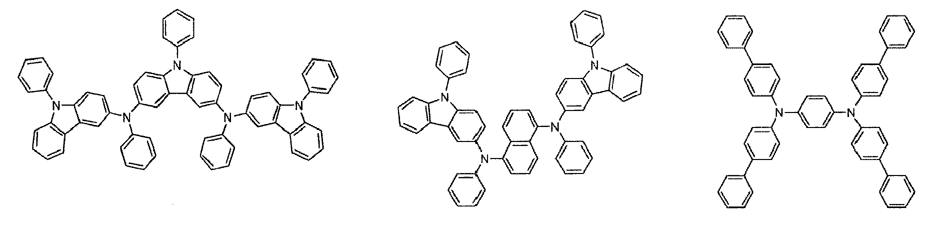

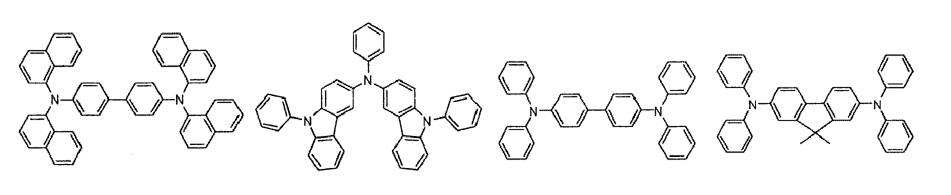

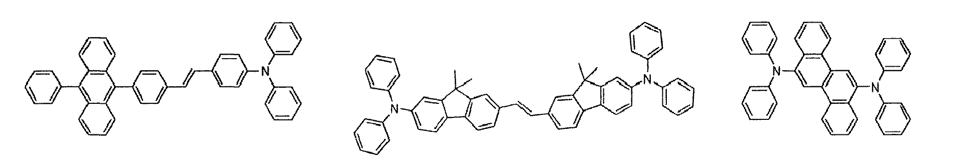

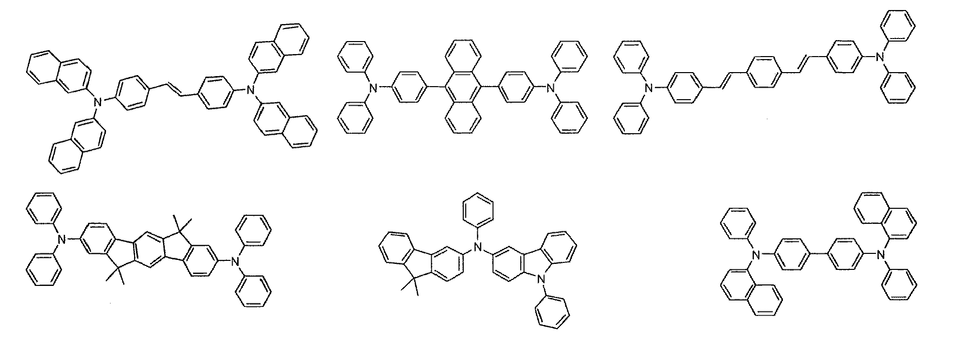

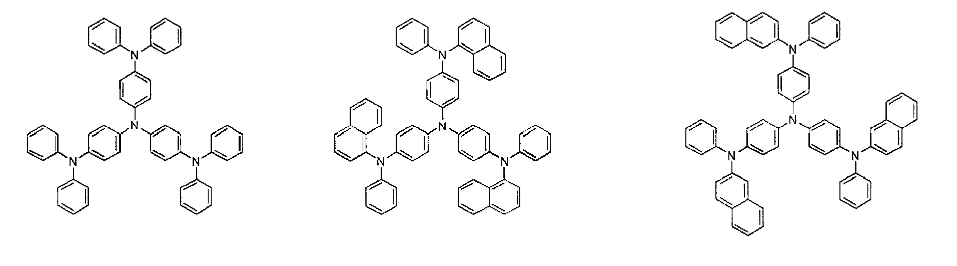

In an organic electroluminescent device according to the present invention, the organic layer may further comprise one or more metal(s) selected from a group consisting of organic metals of Group 1, Group 2, 4^{th} period and 5^{th} period transition metals, lanthanide metals and d-transition elements, as well as the organic electroluminescent compound represented by Chemical Formula (1). The organic layer may comprise a charge generating layer in addition to the electroluminescent layer.

The present invention can realize an organic electroluminescent device having a pixel structure of independent light-emitting mode, which comprises an organic electroluminescent device containing the compound of Chemical Formula (1) as a sub-pixel and one or more sub-pixel(s) comprising one or more compound(s) selected from a group consisting of arylamine compounds and styrylarylamine compounds, patterned in parallel at the same time.

Further, the organic electroluminescent device is an organic electroluminescent display which comprises one or more compound(s) selected from compounds having electroluminescent peak of wavelength of blue or green, at the same time. The compounds having electroluminescent peak of wavelength of blue or green may be exemplified by the compounds represented by one of Chemical Formulas (13) to (18), but they are not restricted thereto.

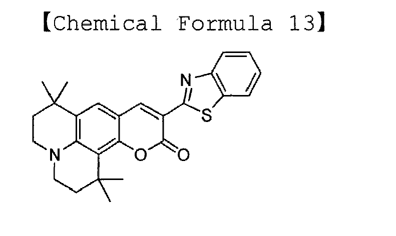

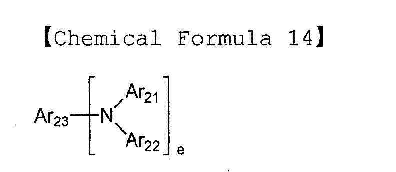

In Chemical Formula (14), Ar₂₁ and Ar₂₂ independently represent hydrogen, deuterium, halogen, (C1-C60)alkyl, (C6-C60)aryl, (C4-C60)heteroaryl, mono or di-(C6-C60)arylamino, mono or di-(C1-C60)alkylamino, a 5- or 6-membered heterocycloalkyl containing one or more heteroatom(s) selected from N, O and S, or Ar₂₁ and Ar₂₂ may be linked via (C3-C60)alkylene or (C3-C60)alkenylene with or without a fused ring to form an alicyclic ring, or a monocyclic or polycyclic aromatic ring;
when e is 1, Ar₂₃ represents (C6-C60)aryl, (C4-C60)heteroaryl, or a substituent represented by one of the following structural formulas: 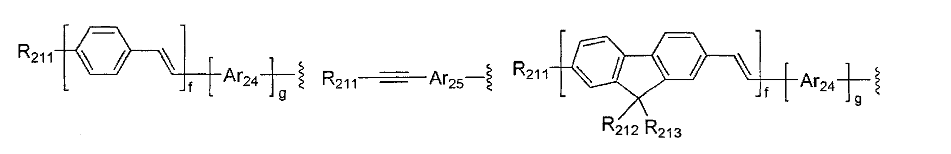 when e is 2, Ar₂₃ represents (C6-C60)arylene, (C4-C60)heteroarylene, or a substituent represented by one of the following structural formulas: 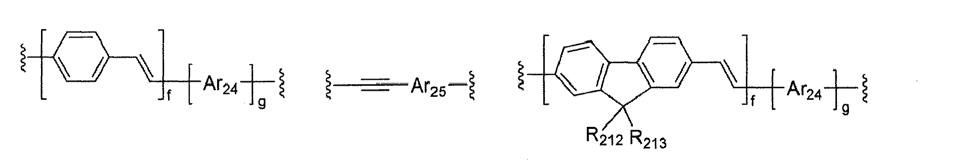 wherein Ar₂₄ and Ar₂₅ independently represent (C6-C60)arylene or (C4-C60)heteroarylene;
R₂₁₁ through R₂₁₃ independently represent hydrogen, halogen, deuterium, (C1-C60)alkyl or (C6-C60)aryl;
f is an integer from 1 to 4, g is an integer of 0 or 1; and
the alkyl, aryl, heteroaryl, arylamino, alkylamino, cycloalkyl or heterocycloalkyl of Ar₂₁ and Ar₂₂, or the aryl, heteroaryl, arylene or heteroarylene of Ar₂₃, or the arylene or heteroarylene of Ar₂₄ and Ar₂₅, or the alkyl or aryl of R₂₁₁ through R₂₁₃ may be further substituted by one or more substituent(s) selected from a group consisting of deuterium, halogen, (C1-C60)alkyl, (C6-C60)aryl, (C4-C60)heteroaryl, a 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono or di-(C1-C60)alkylamino, mono or di-(C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

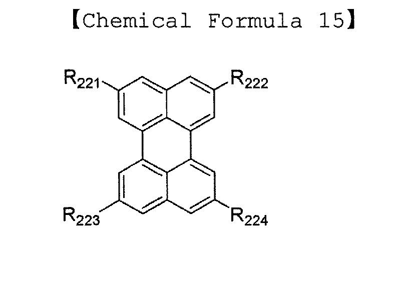

In Chemical Formula (15), R₂₂₁ through R₂₂₄ independently represents hydrogen, deuterium, halogen, (C1-C60)alkyl, (C6-C60)aryl, (C4-C60)heteroaryl containing one or more heteroatom(s) selected from N, O and S, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, mono or di-(C1-C60)alkylamino, mono or di-(C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₂₁ through R₂₂₄ may be linked to an adjacent substituent via (C3-C60)alkylene or (C3-C60)alkenylene with or without a fused ring to form an alicyclic ring, or a monocyclic or polycyclic aromatic ring;
the alkyl, alkenyl, alkynyl, cycloalkyl, heterocycloalkyl, aryl, heteroaryl, arylsilyl, alkylsilyl, alkylamino or arylamino of R₂₂₁ through R₂₂₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl containing one or more heteroatom(s) selected from N, O and S, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, mono or di-(C1-C60)alkylamino, mono or di-(C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

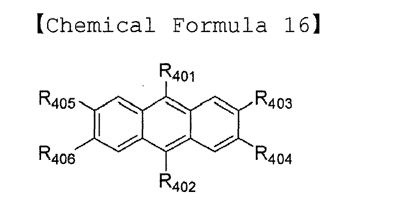

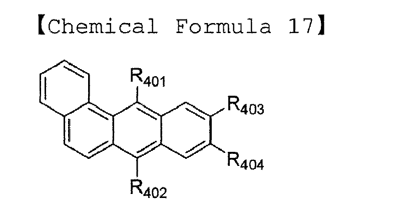

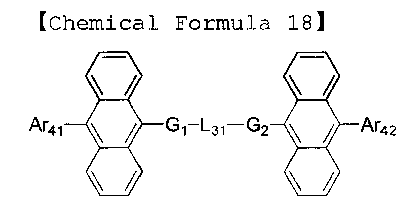

In Chemical Formulas (16) through (18),
R₄₀₁ and R₄₀₂ independently represent (C6-C60)aryl, (C4-C60)heteroaryl, a 5- or 6-membered heterocycloalkyl containing one or more heteroatom(s) selected from N, O and S, or (C3-C60)cycloalkyl, and the aryl or heteroaryl of R₄₀₁ and R₄₀₂ may be further substituted by one or more substituent(s) selected from a group consisting of deuterium, (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₄₀₃ through R₄₀₆ independently represent hydrogen, deuterium, (C1-C60)alkyl, (C1-C60)alkoxy, halogen, (C4-C60)heteroaryl, (C5-C60)cycloalkyl or (C6-C60)aryl, and the heteroayl, cycloalkyl or aryl of R₄₀₃ through R₄₀₆ may be further substituted by one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
G₁ and G₂ independently represent a chemical bond, or (C6-C60)arylene with or without one or more substituent(s) selected from (C1-C60)alkyl, (C1-C60)alkoxy, (C6-C60) aryl, (C4-C60)heteroaryl and halogen;
Ar₄₁ and Ar₄₂ represent (C4-C60)heteroaryl or aryl selected from the following structures: 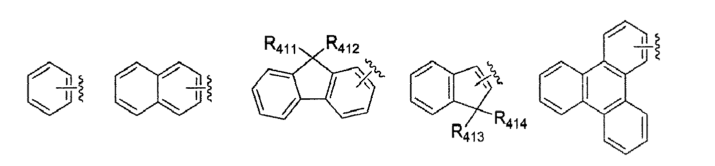 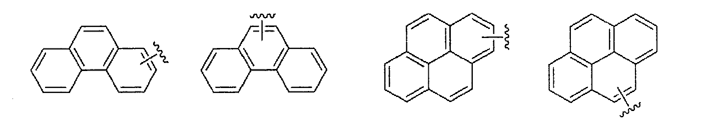 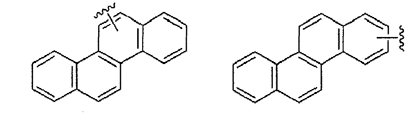
the aryl or heteroaryl of Ar₄₁ and Ar₄₂ may be substituted by one or more substituent(s) selected from deuterium, (C1-C60)alkyl, (Cl-C60)alkoxy, (C6-C60)aryl and (C4-C60)heteroaryl;
L₃₁ represents (C6-C60)arylene, (C4-C60)heteroarylene or a compound represented by the following structural formula: 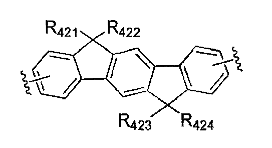
the arylene or heteroarylene of L₃₁ may be substituted by one or more substituent(s) selected from deuterium, (C1-C60)alkyl, (C1-C60)alkoxy, (C6-C60)aryl, (C4-C60)heteroaryl and halogen;
R₄₁₁ through R₄₁₄ independently represent hydrogen, deuterium, halogen, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and
R₄₂₁ through R₄₂₄ independently represent hydrogen, deuterium, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring.

The organic compounds and organometallic compounds with green or blue electroluminescence can be more specifically exemplified by the following compounds, but they are not restricted thereto.

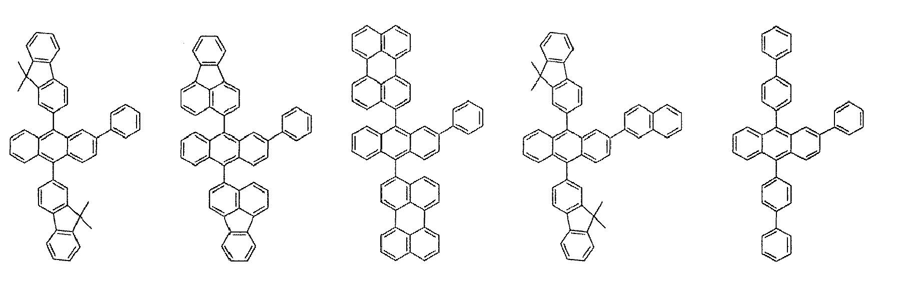

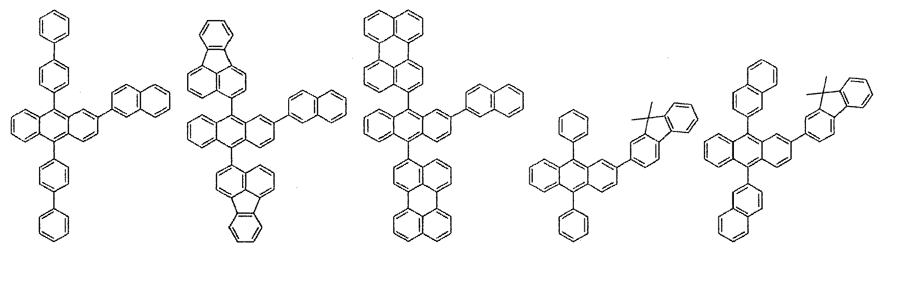

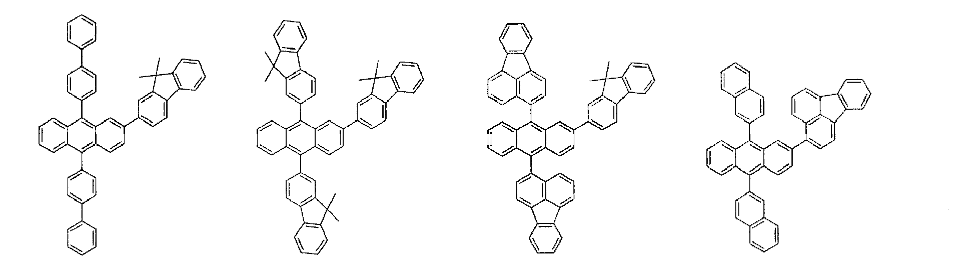

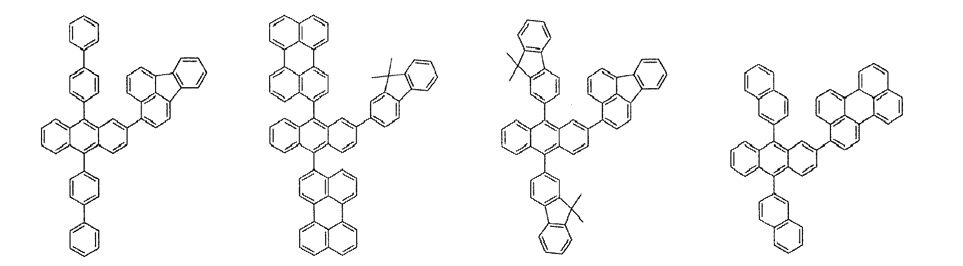

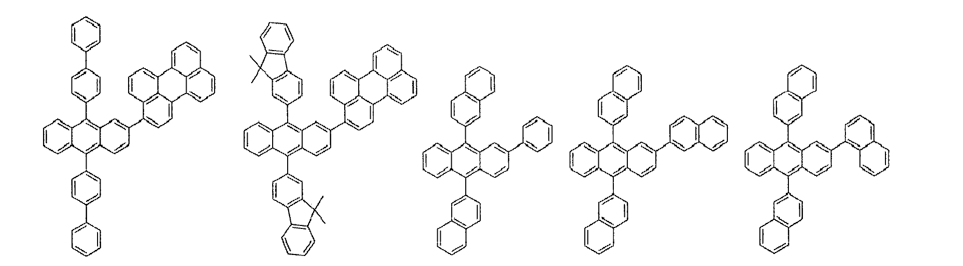

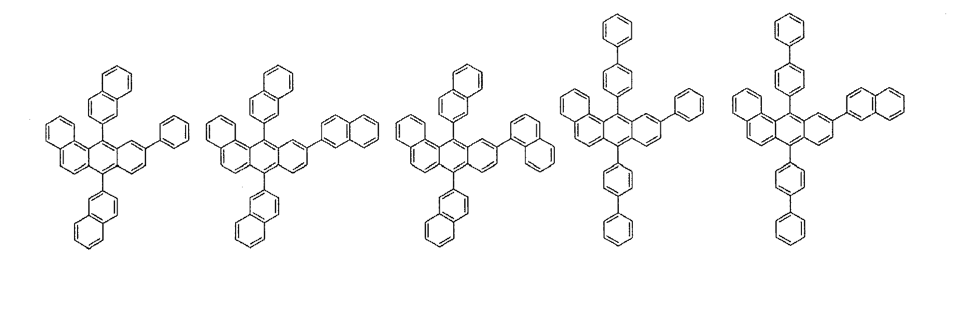

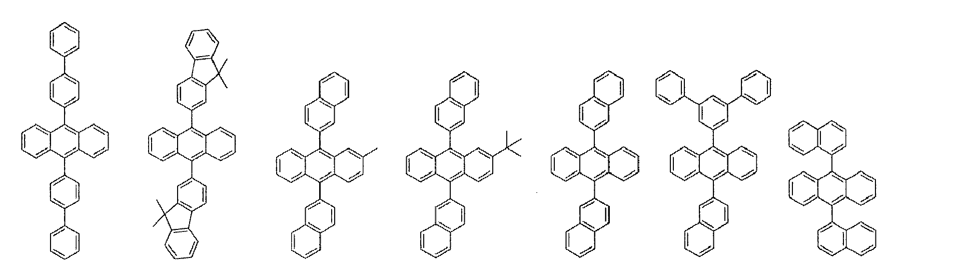

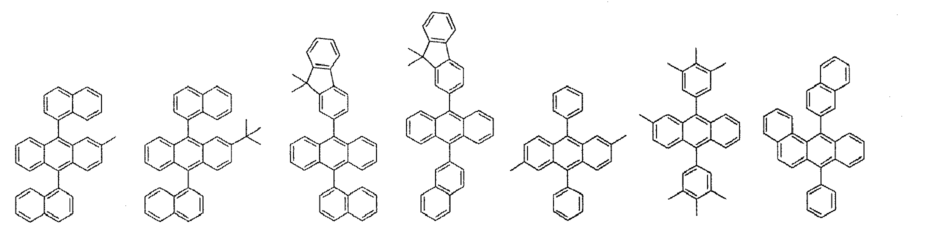

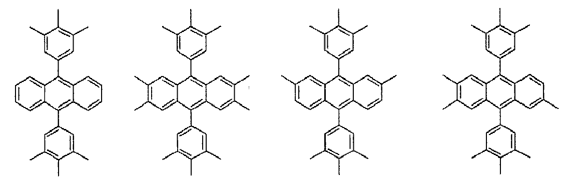

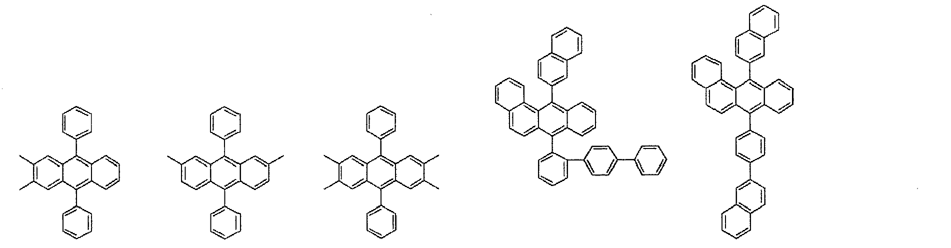

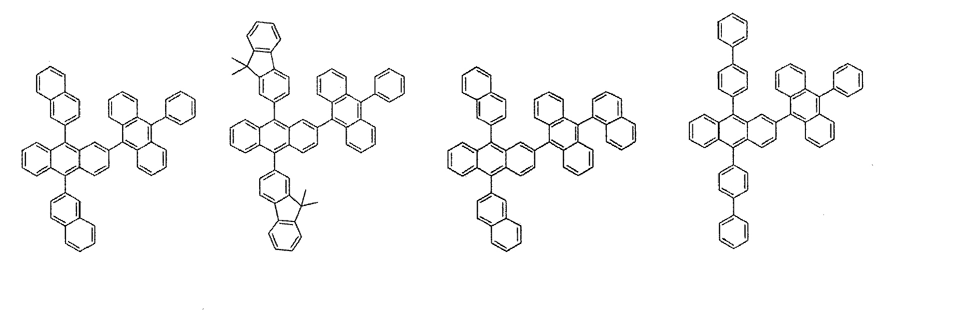

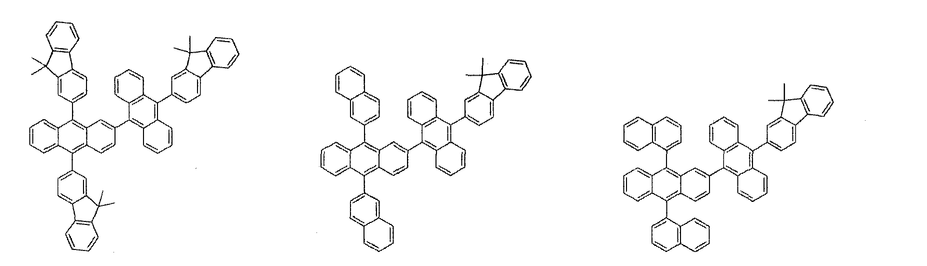

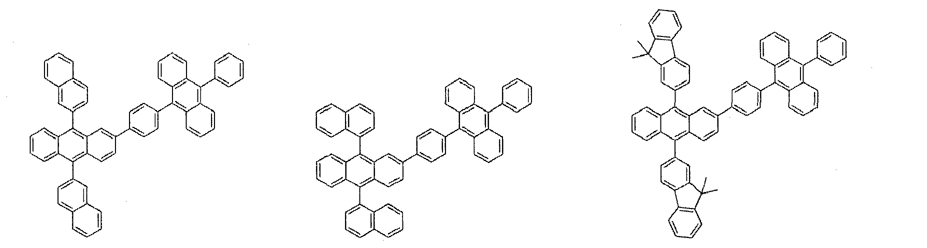

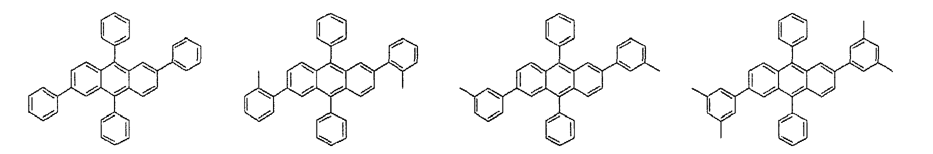

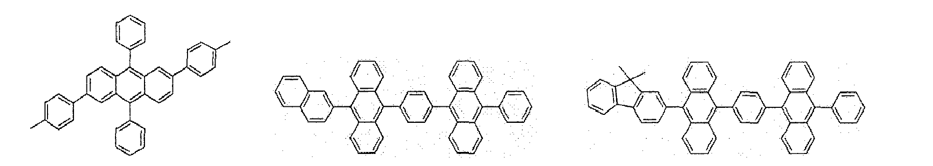

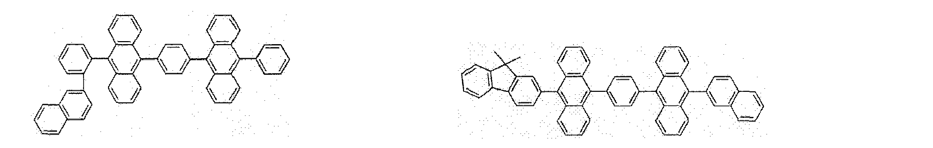

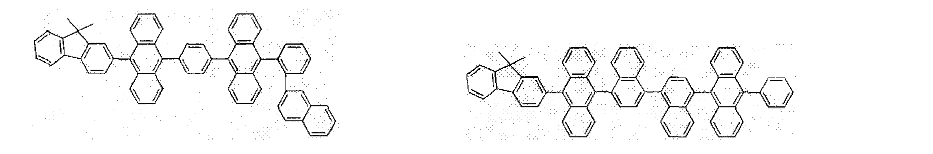

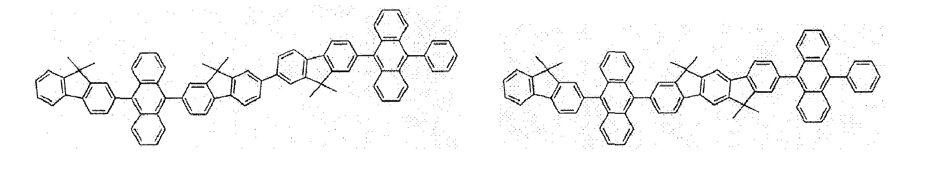

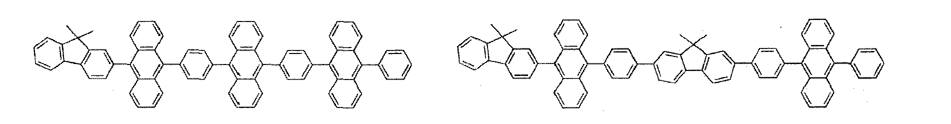

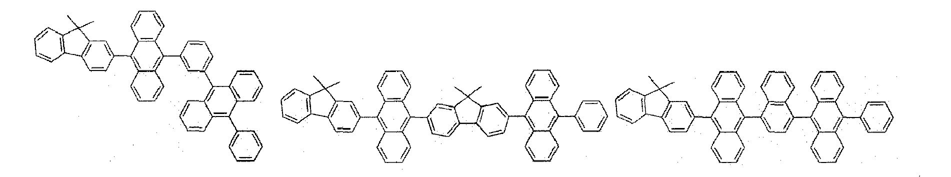

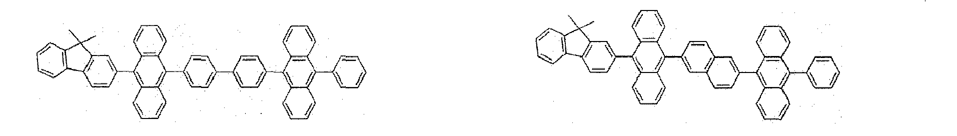

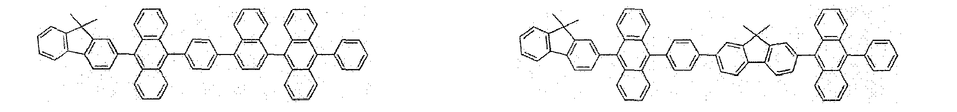

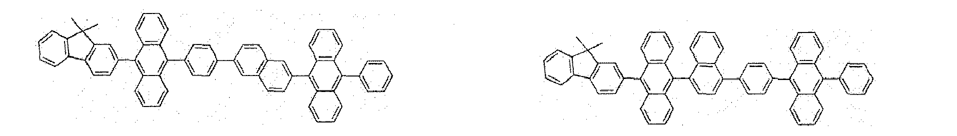

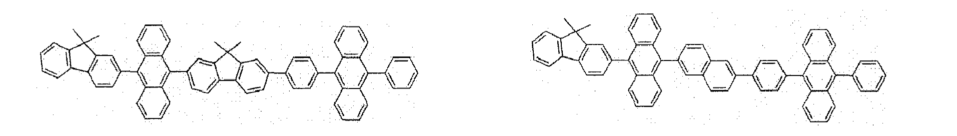

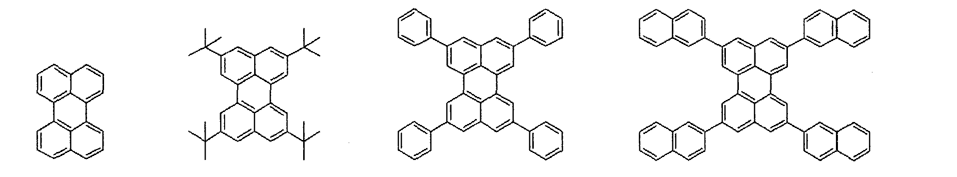

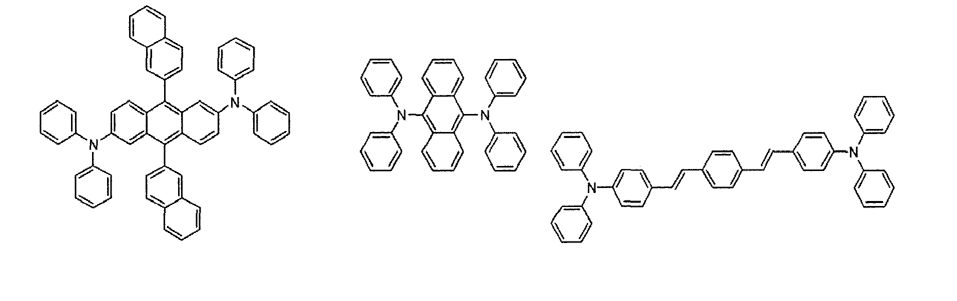

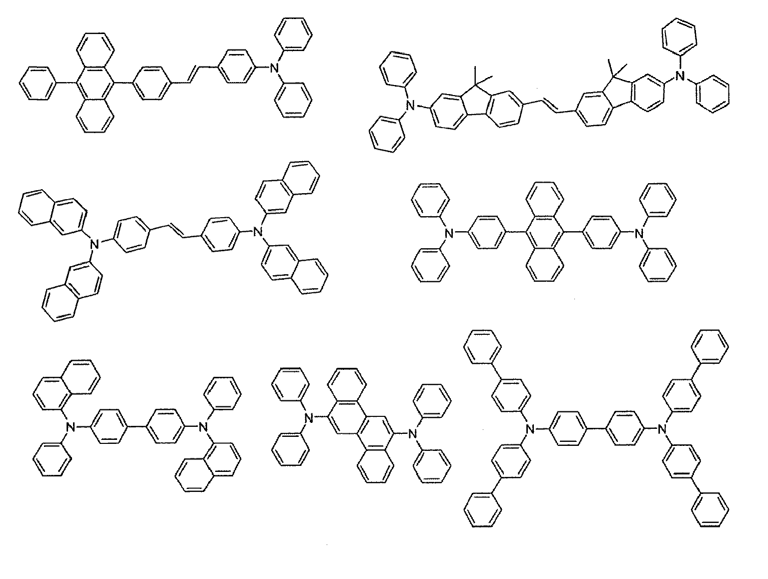

In an organic electroluminescent device according to the present invention, it is preferable to place one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2). AlOₓ (I≤X≤1.5), SiON, SiAlON, or the like. Examples of metal halides preferably include LiF, MgF₂, CaF₂, fluorides of rare earth metal, or the like. Examples of metal oxides preferably include Cs₂O, Li₂O, MgO, SrO, BaO, CaO, or the like.

In an organic electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The organic compounds according to the invention can be advantageously employed for manufacturing OLED's with high luminous efficiency, good color purity and decreased operation voltage.

### [Best Mode]

The present invention is further described with respect to the representative compounds of the invention, by describing the organic phosphorescent compounds, the processes for preparing the same, and luminescent properties of the device manufactured therefrom in the Examples below, which are provided for illustration of the embodiments only but are not intended to limit the scope of the invention by any means.

### [Preparation Examples]

### [Preparation Example 1] Preparation of Compound (1)

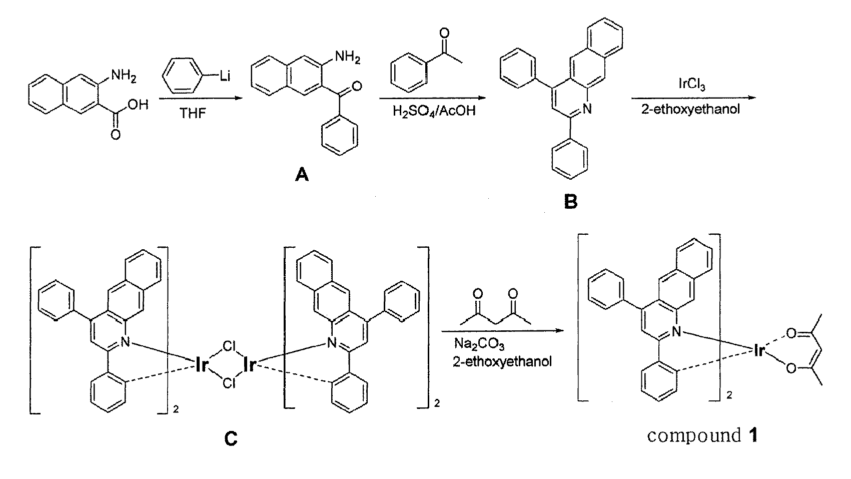

### Preparation of Compound (A)

In THF (50 mL), dissolved was 3-aminonaphthalene-2-carboxylic acid (1.0 g, 5.4 mmol), and temperature of the solution was lowered to 0°C. Phenyllithium (11.9 mL, 21.4 mmol) was slowly added thereto. After stirring for 2 hours, aqueous ammonium chloride solution was added to the reaction mixture to quench the reaction. The resultant mixture was extracted with diethyl ether, and the extract filtered under reduced pressure. Purification via silica gel column chromatography gave Compound (A) (0.79 g, 60%).

### Preparation of Compound (B)

Compound (A) (1.29 g, 5.24 mmol), acetophenone (0.55 mL, 4.76 mmol), acetic acid (7.13 mL) and sulfuric acid (0.04 mL) were charged to a reaction vessel, and stirred under reflux in the presence of argon atmosphere. When the reaction was completed, the reaction mixture was cooled to room temperature, and an excess amount of aqueous ammonium hydroxide was added thereto. Solid precipitate was filtered, washed with distilled water, and purified via silica gel column chromatography to obtain Compound (B) (1.27 g, 80%).

### Preparation of Compound (C)

Compound (B) (0.6 g, 1.81 mmol) and iridium chloride (0.25 g, 0.82 mmol) were dissolved in 2-ethoxyethanol (9 mL) and distilled water (3 mL), and the solution was stirred under reflux in the presence of argon atmosphere for 24 hours. When the reaction was completed, the reaction mixture was cooled to room temperature. Solid produced was filtered and dried to obtain Compound (C) (0.37 g, 11%).

### Preparation of Compound (1)

Compound (C) (0.37 g, 0.21 mmol), 2,4-pentanedione (0.06 mL, 0.62 mmol) and sodium carbonate (0.13 g, 1.25 mmol) were dissolved in 2-ethoxyethanol (10 mL), and the solution was heated for 4 hours. When the reaction was completed, the reaction mixture was cooled to room temperature. Solid precipitate was filtered and purified via silica gel column chromatography (CH₂Cl₂:hexane = 1:1). Recrystallization from CH₂Cl₂/hexane gave the target compound (1) (0.14 g, 37%) as red crystal.

According to the procedure of Preparation Example 1, Compounds (1) to (1017) listed in Table 1 were prepared, and the ¹H NMR and MS/FAB data are shown in Table 2.

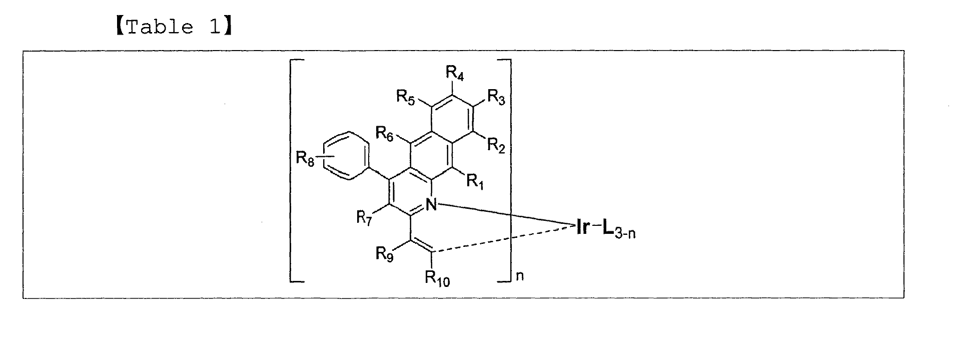

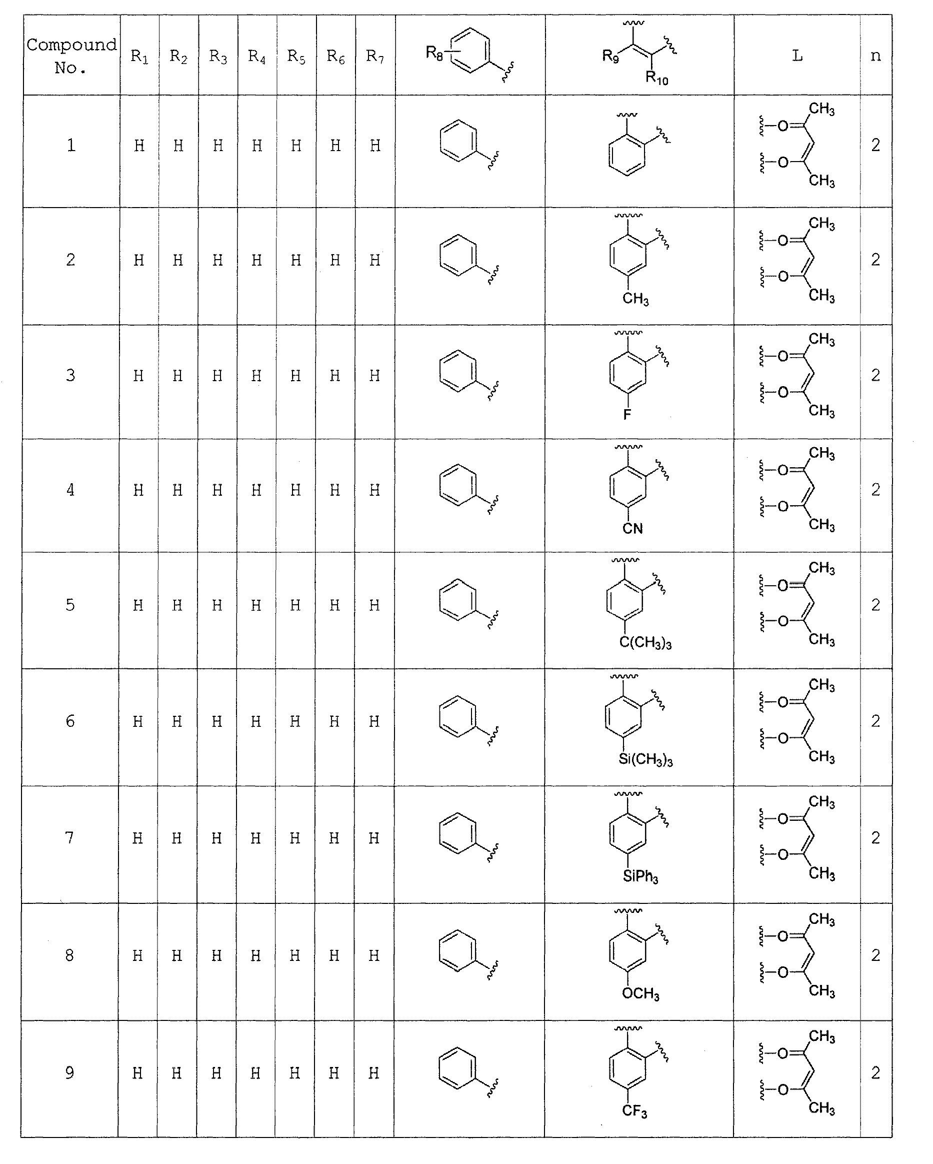

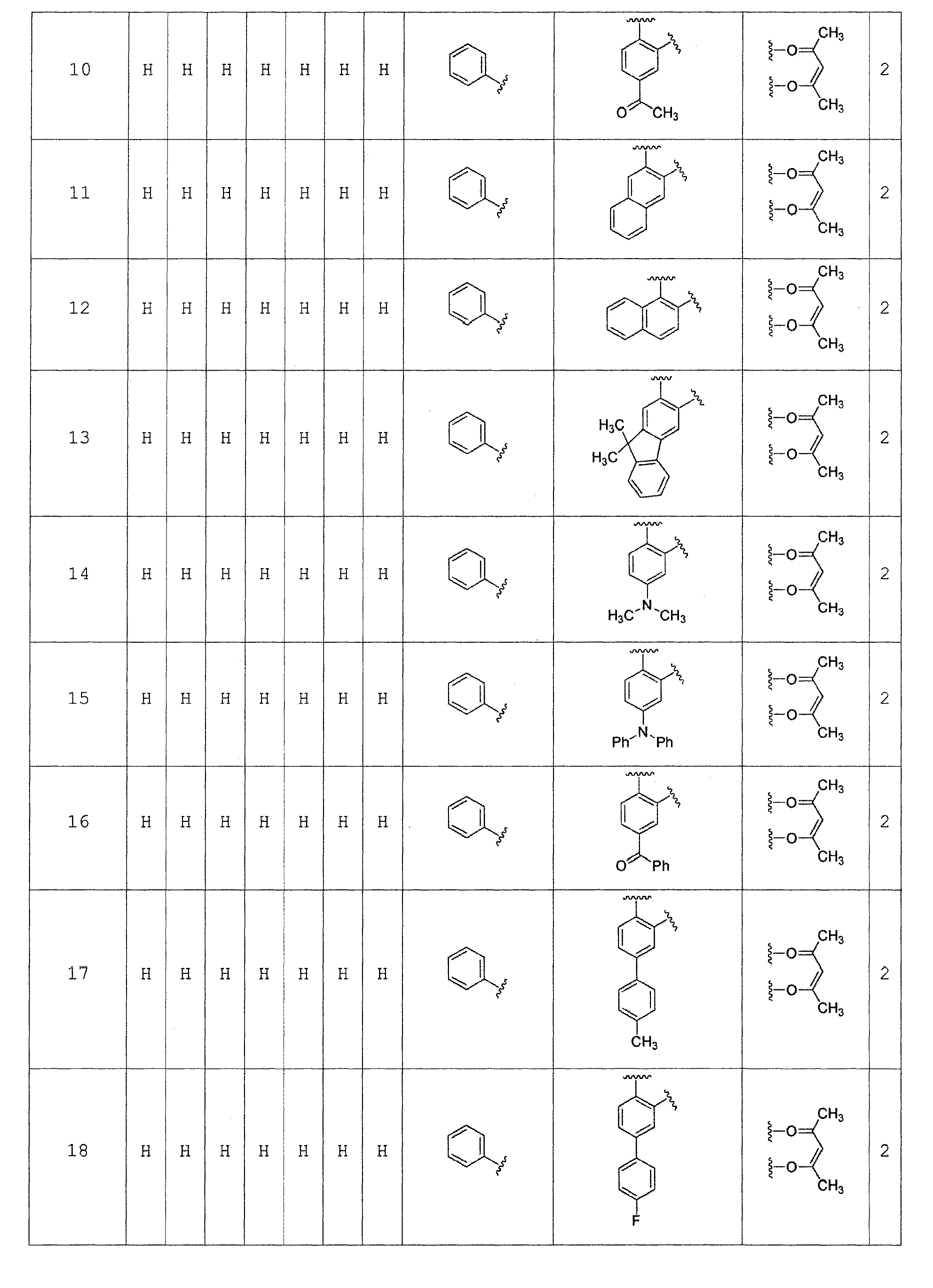

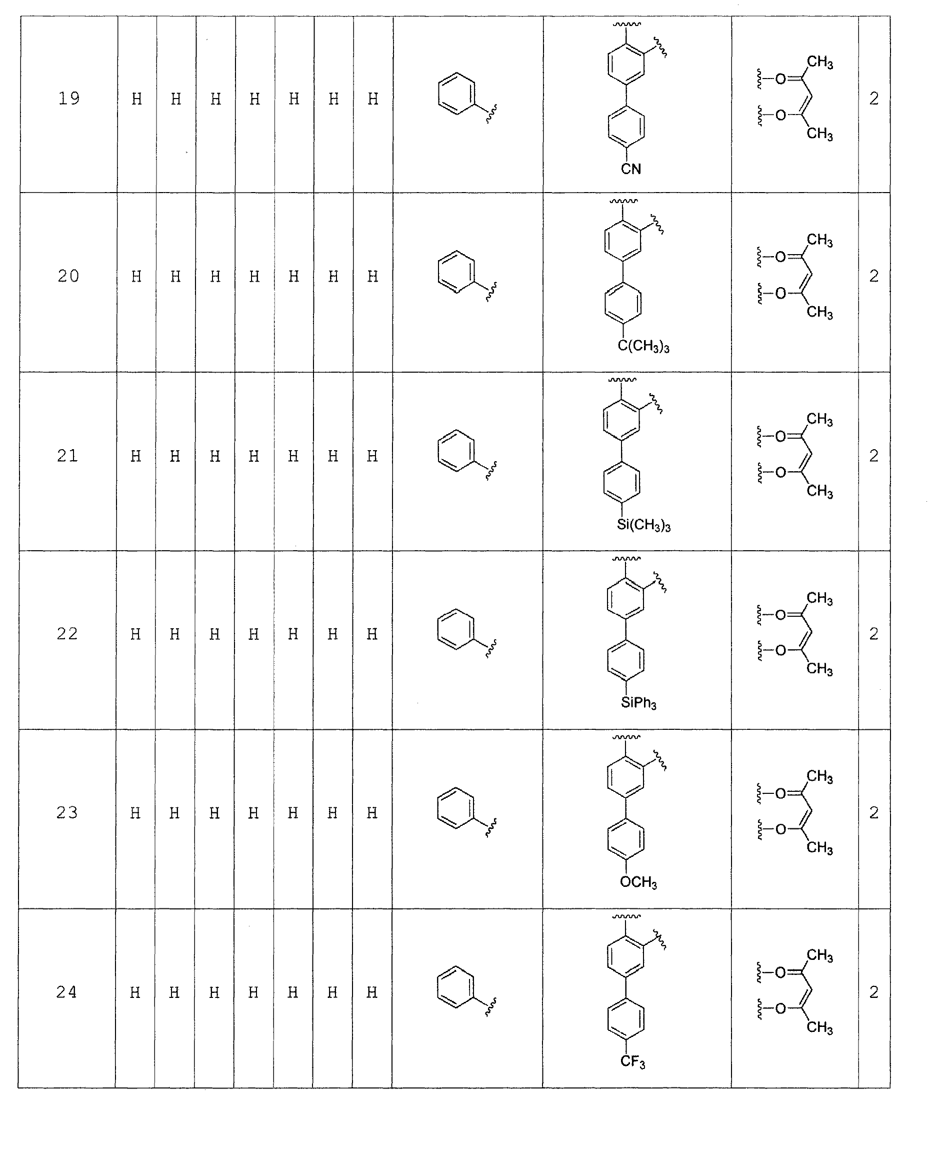

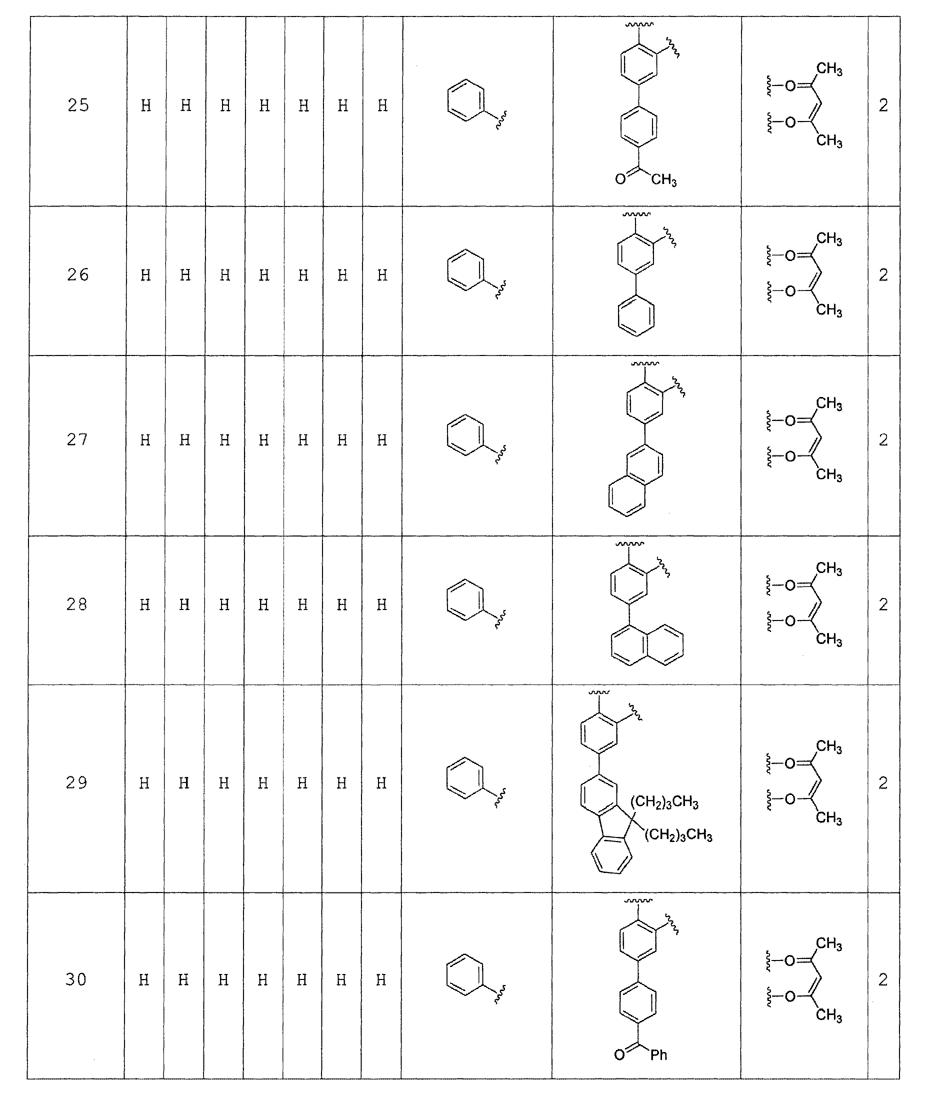

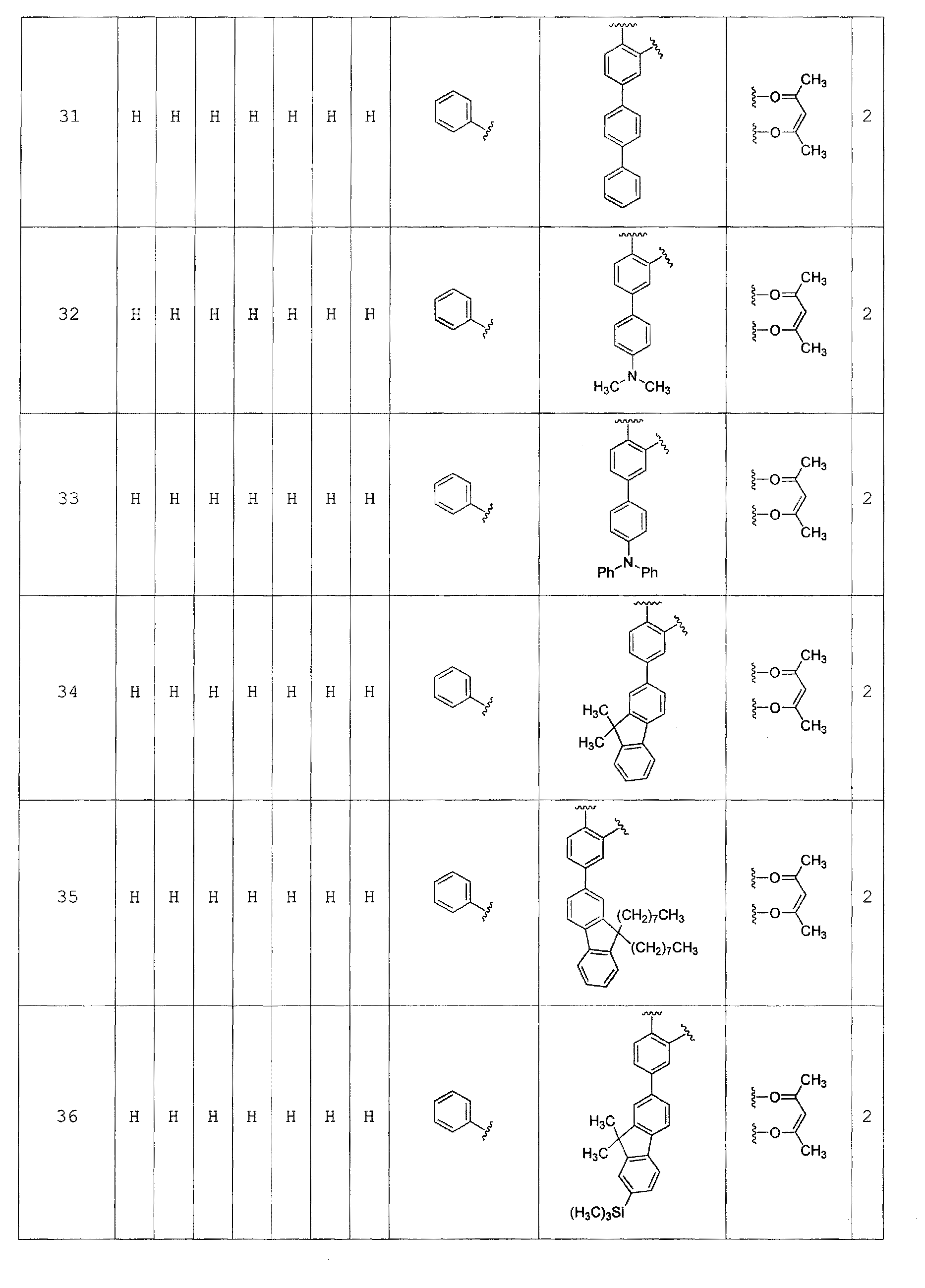

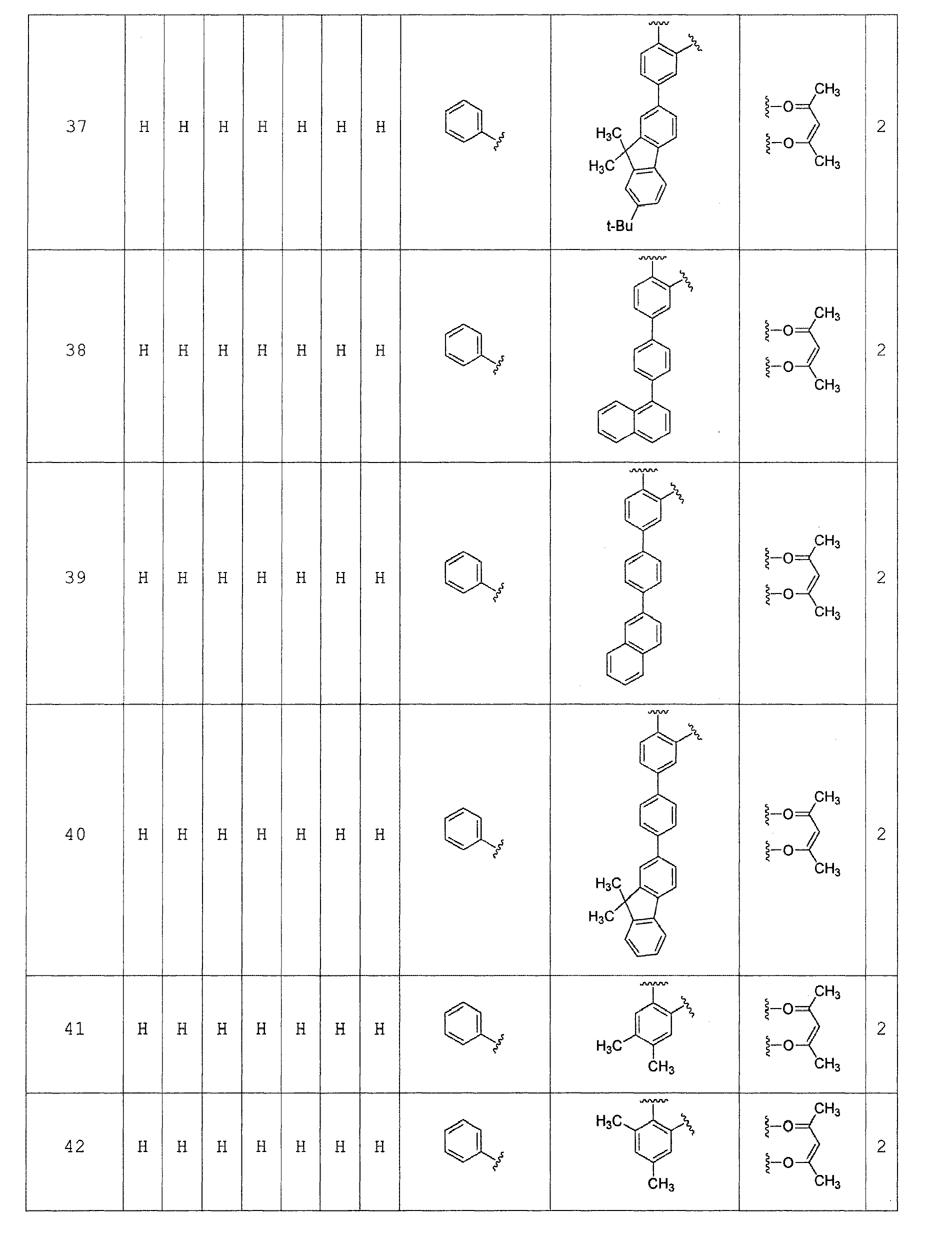

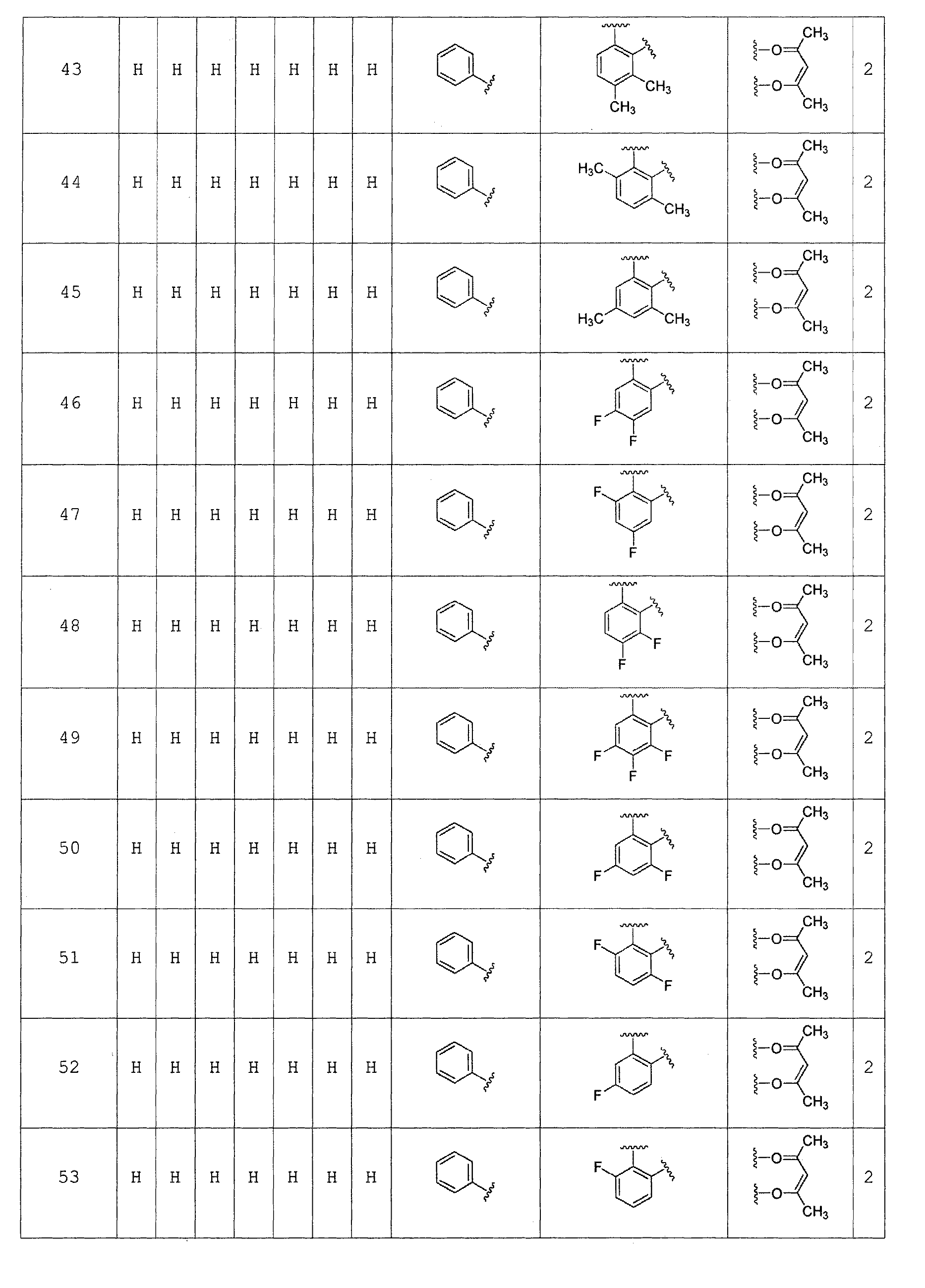

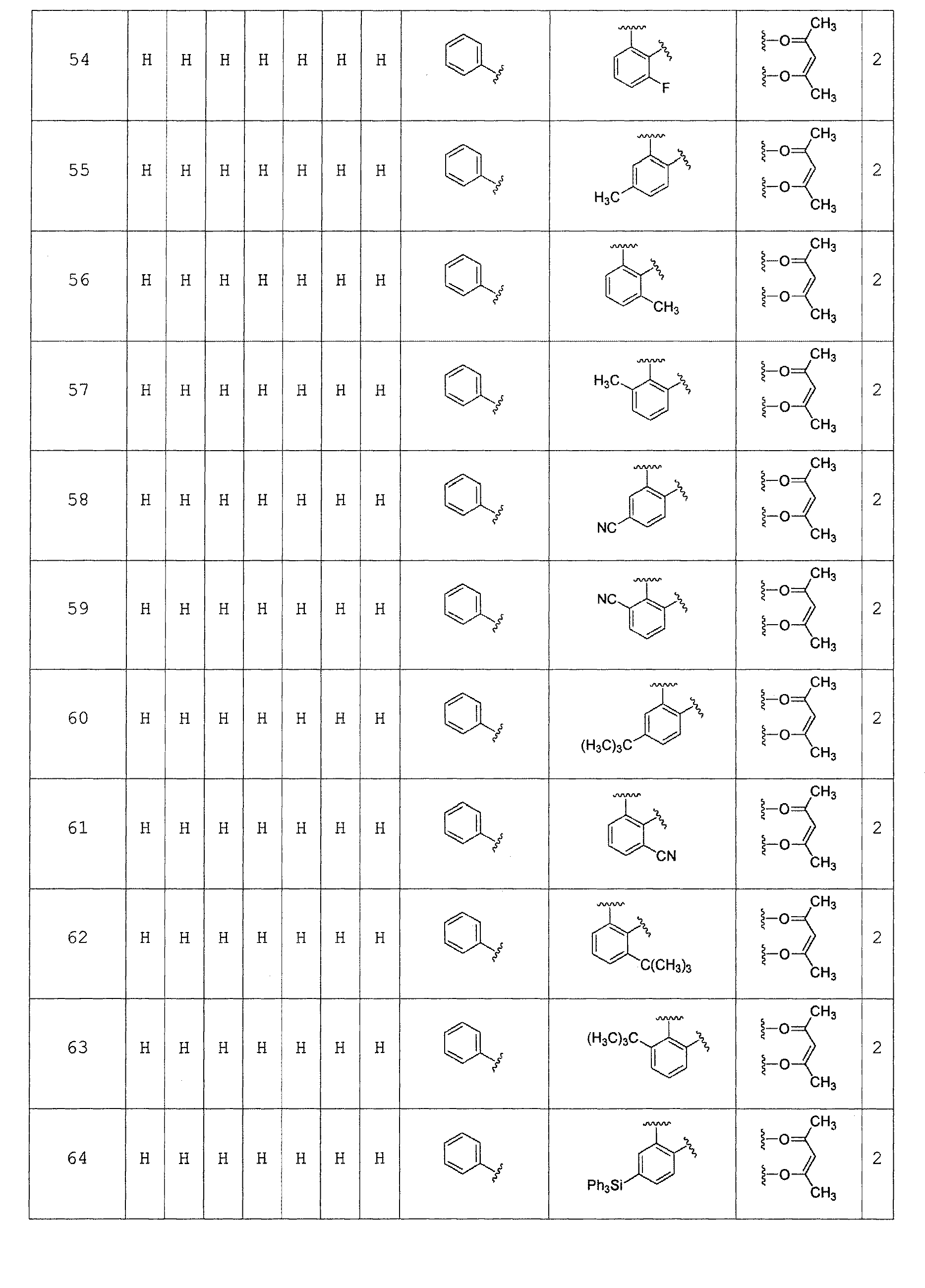

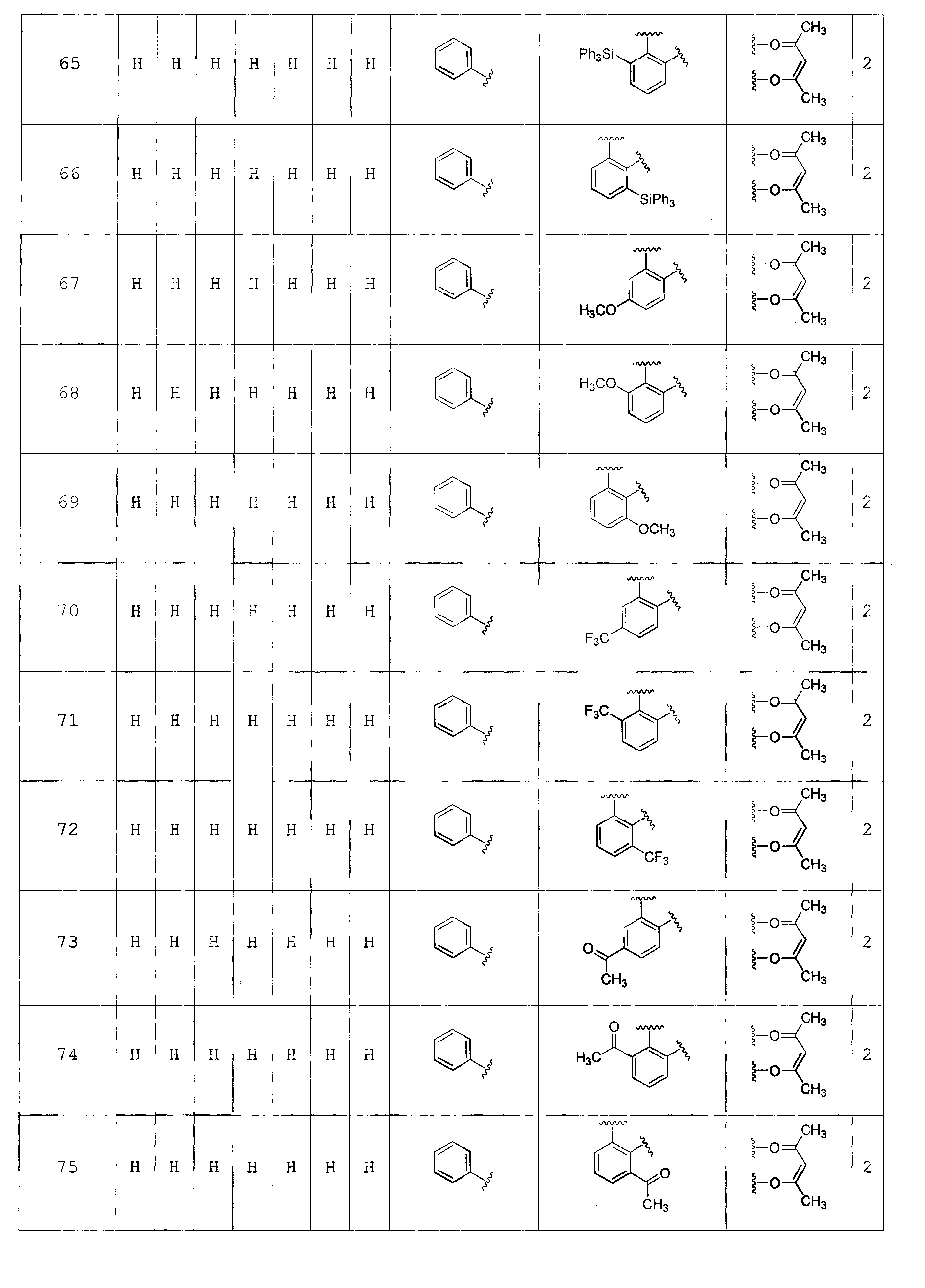

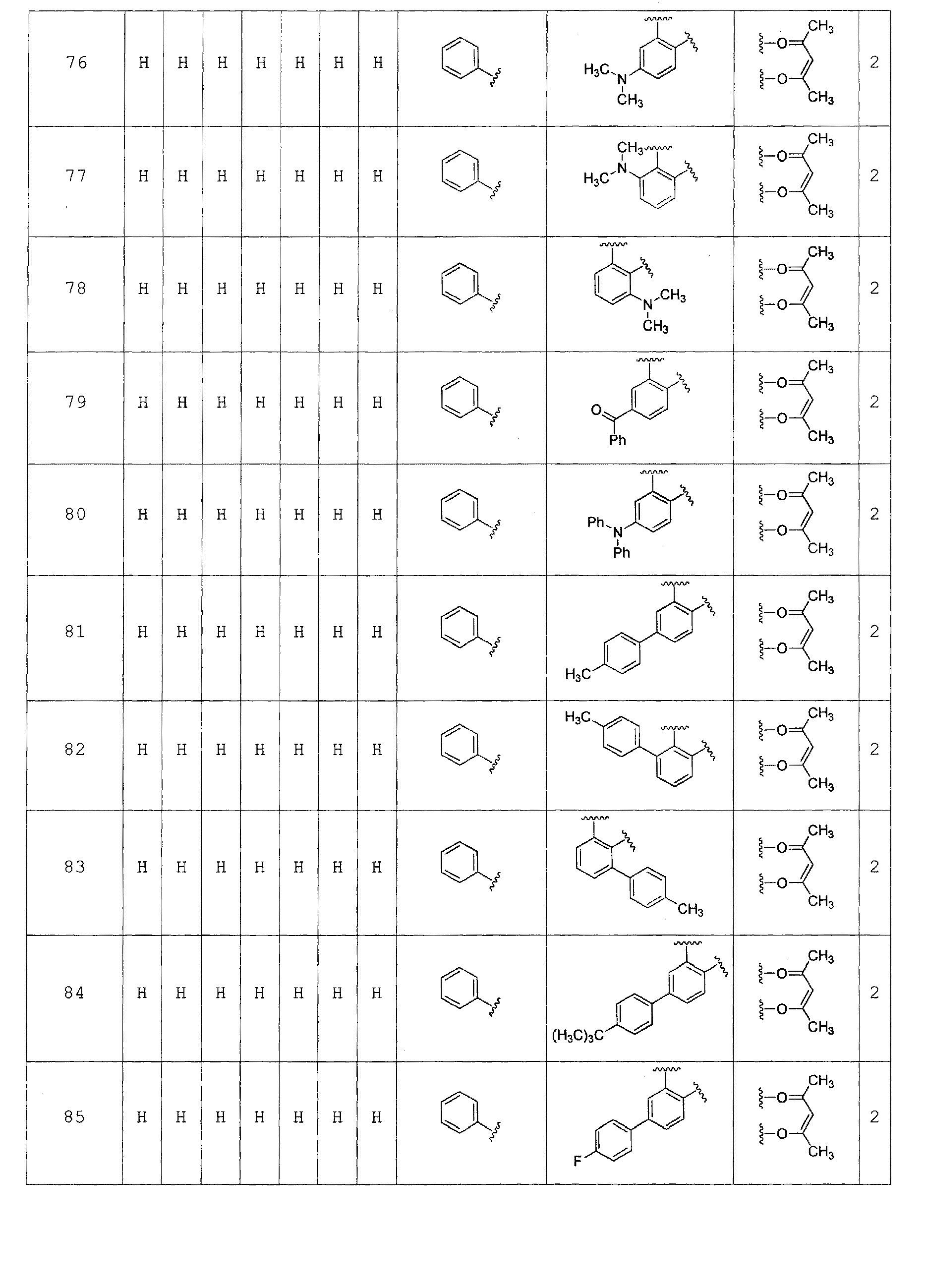

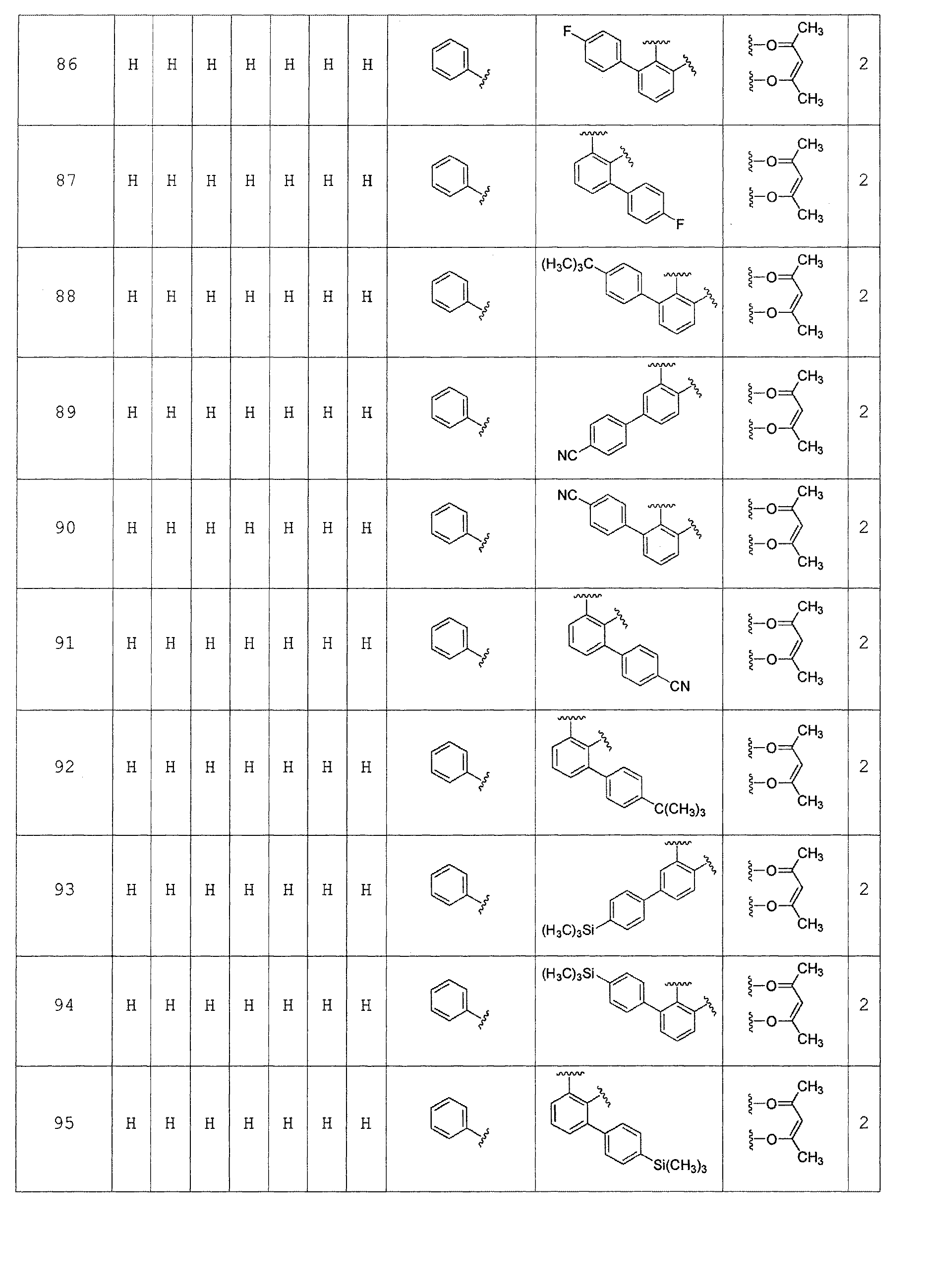

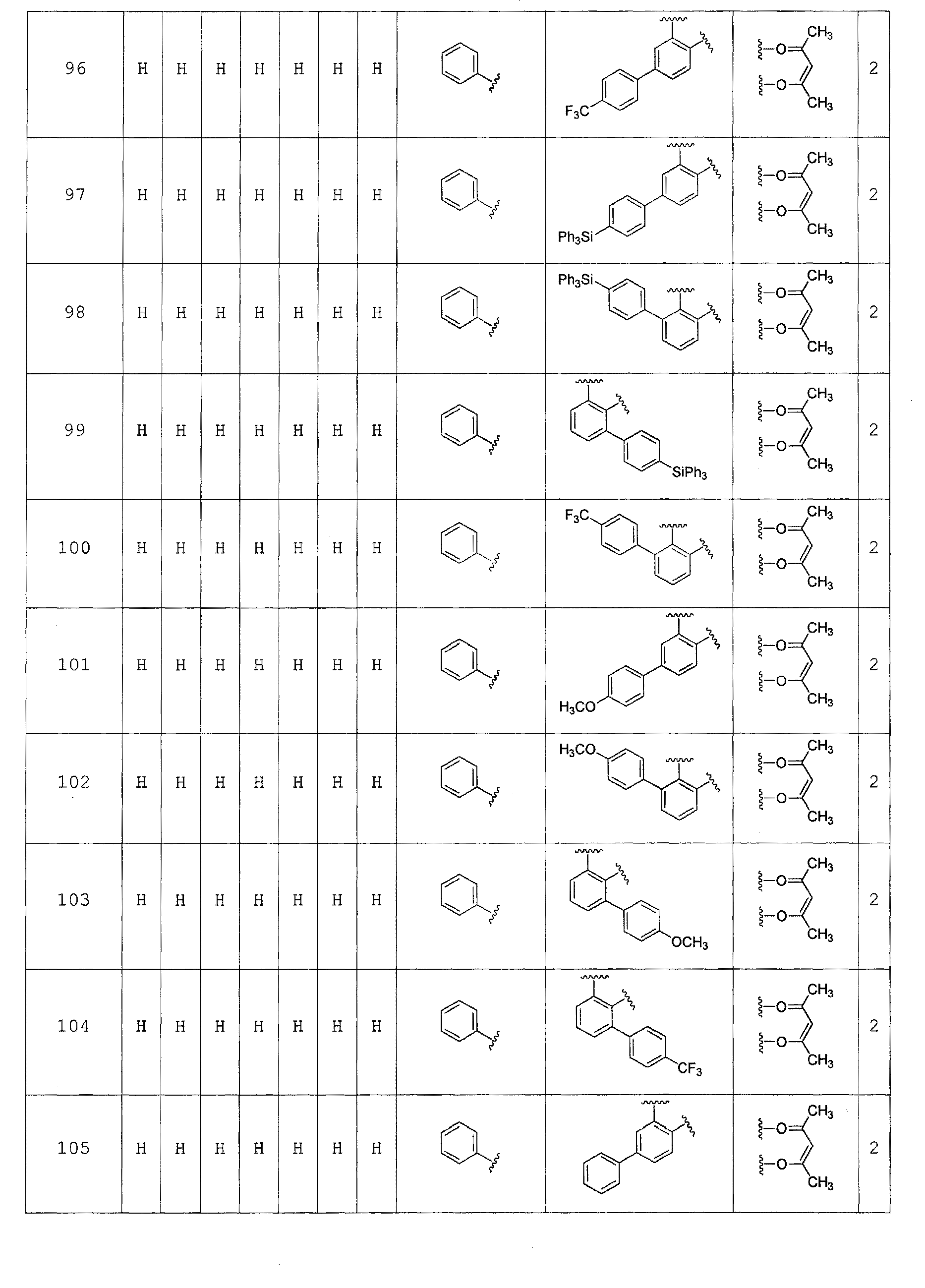

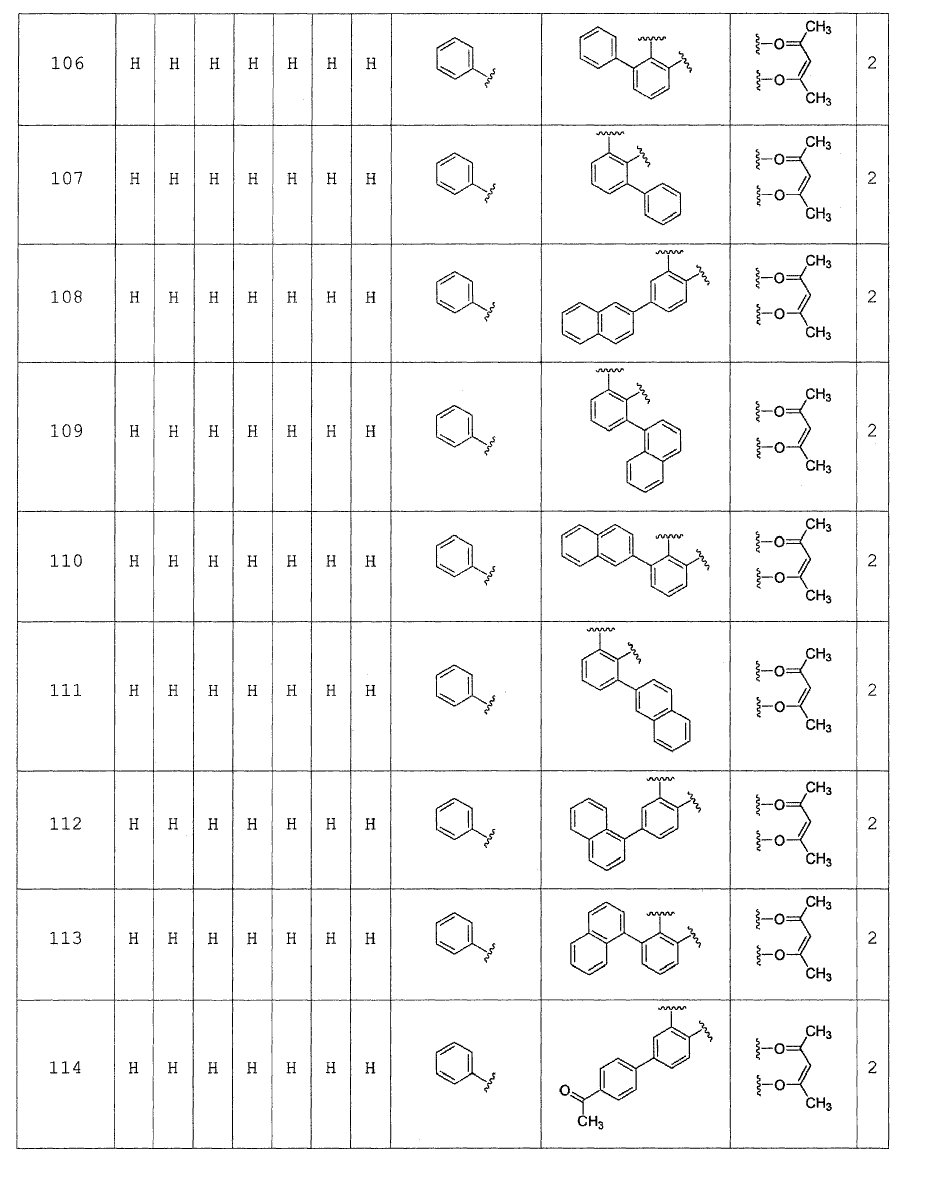

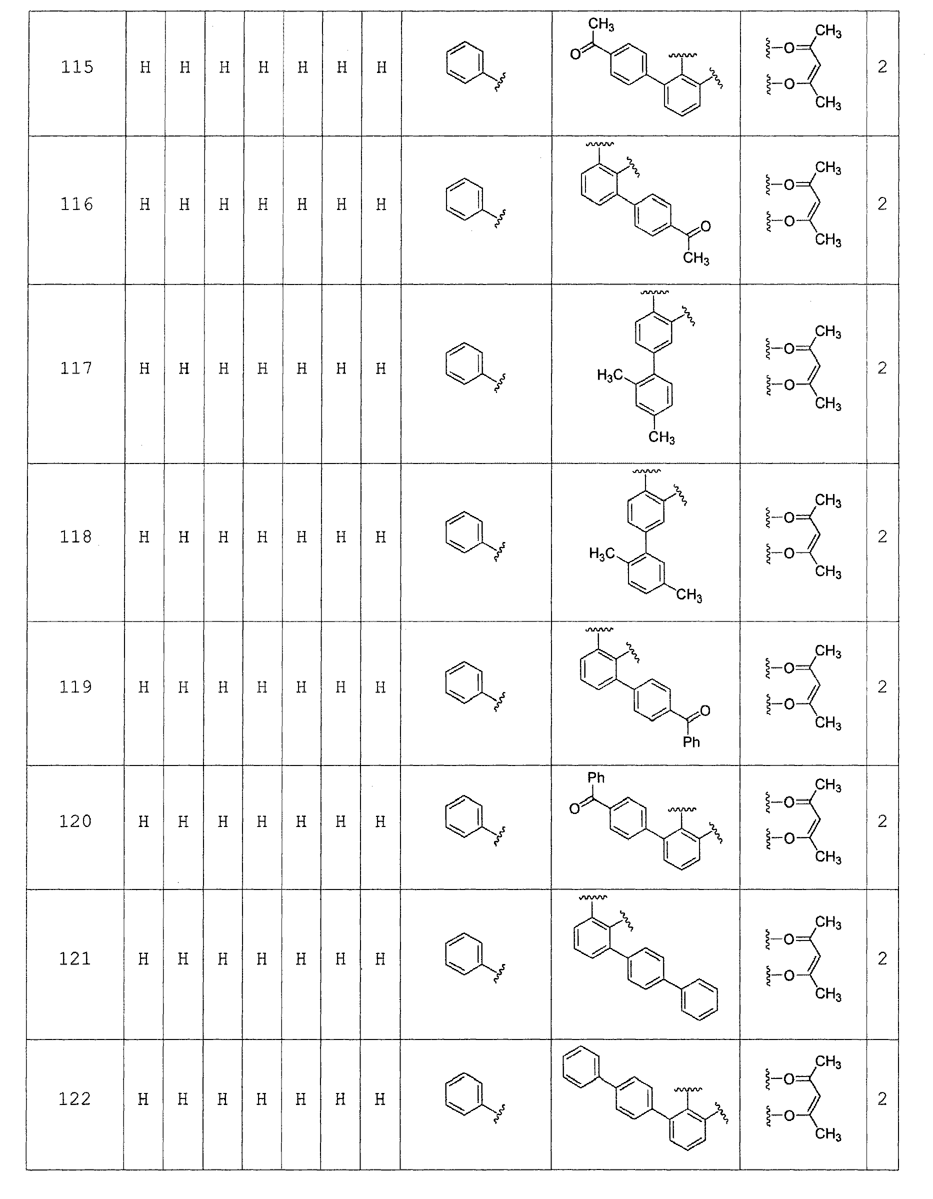

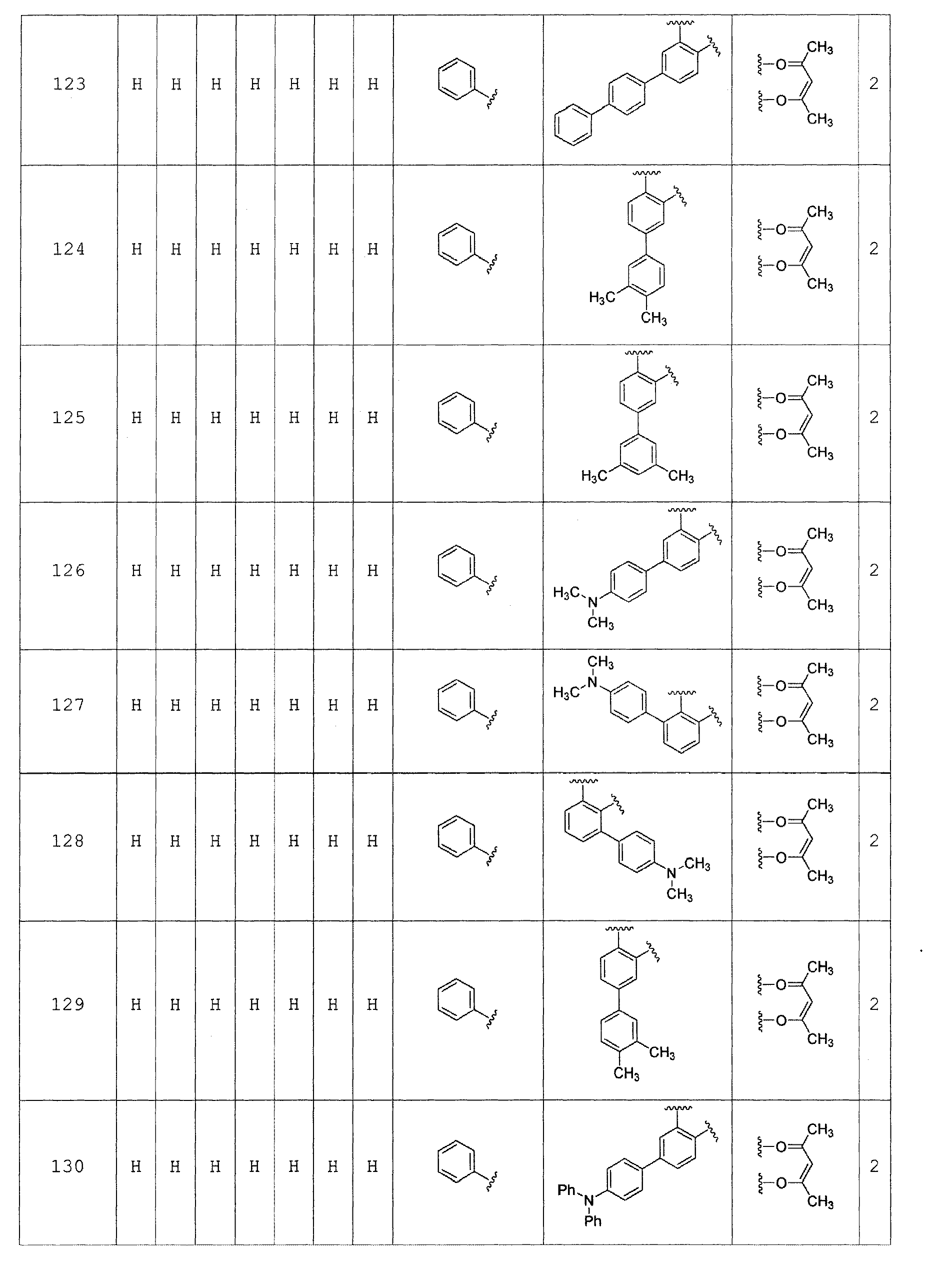

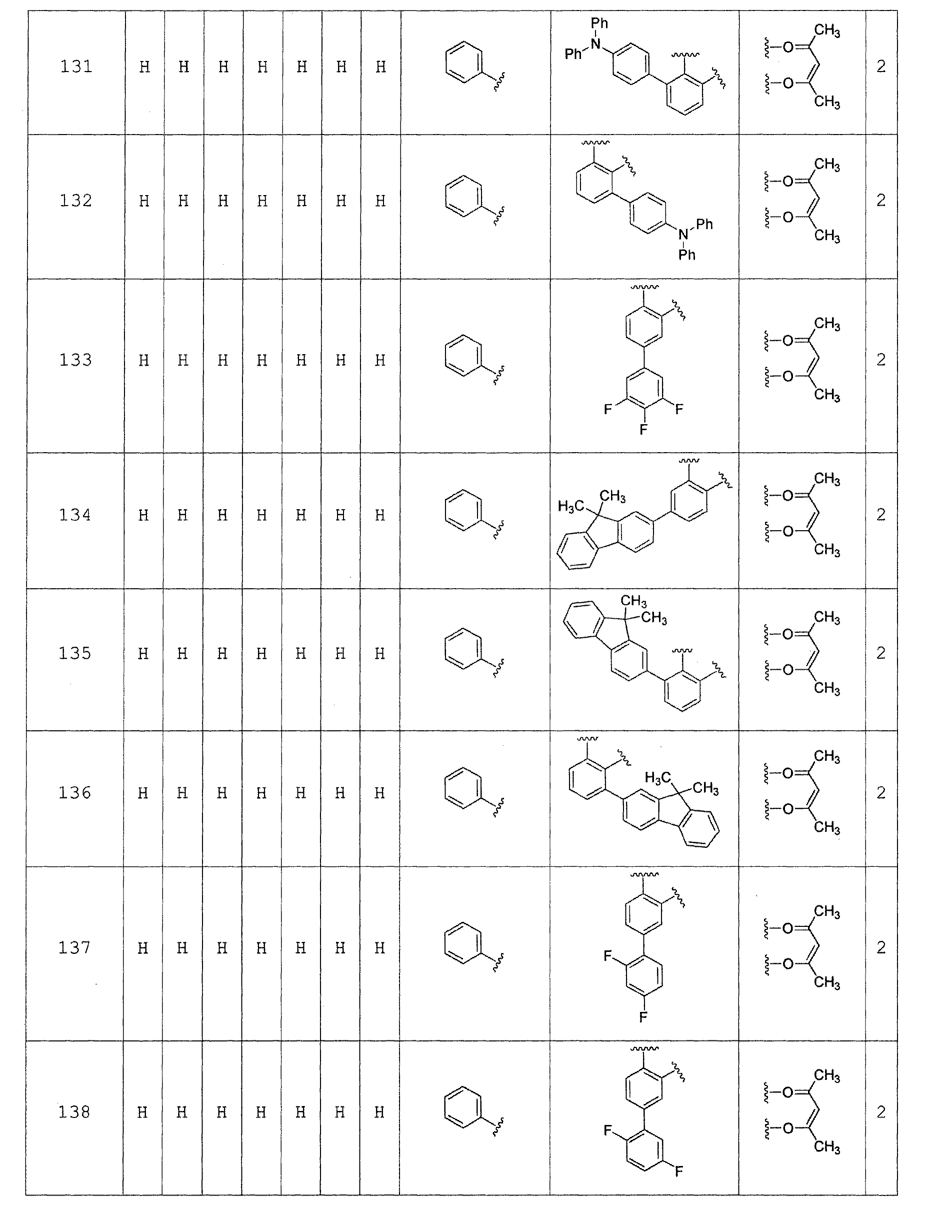

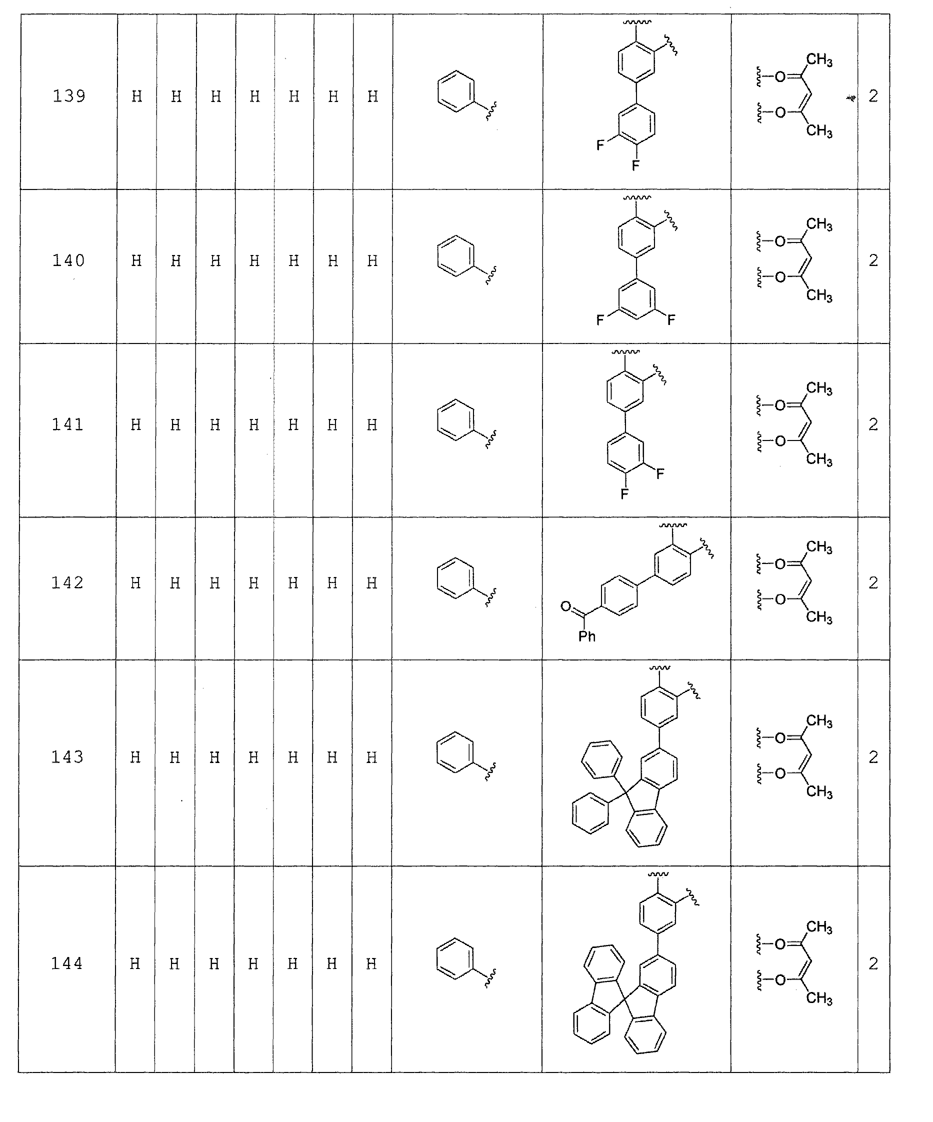

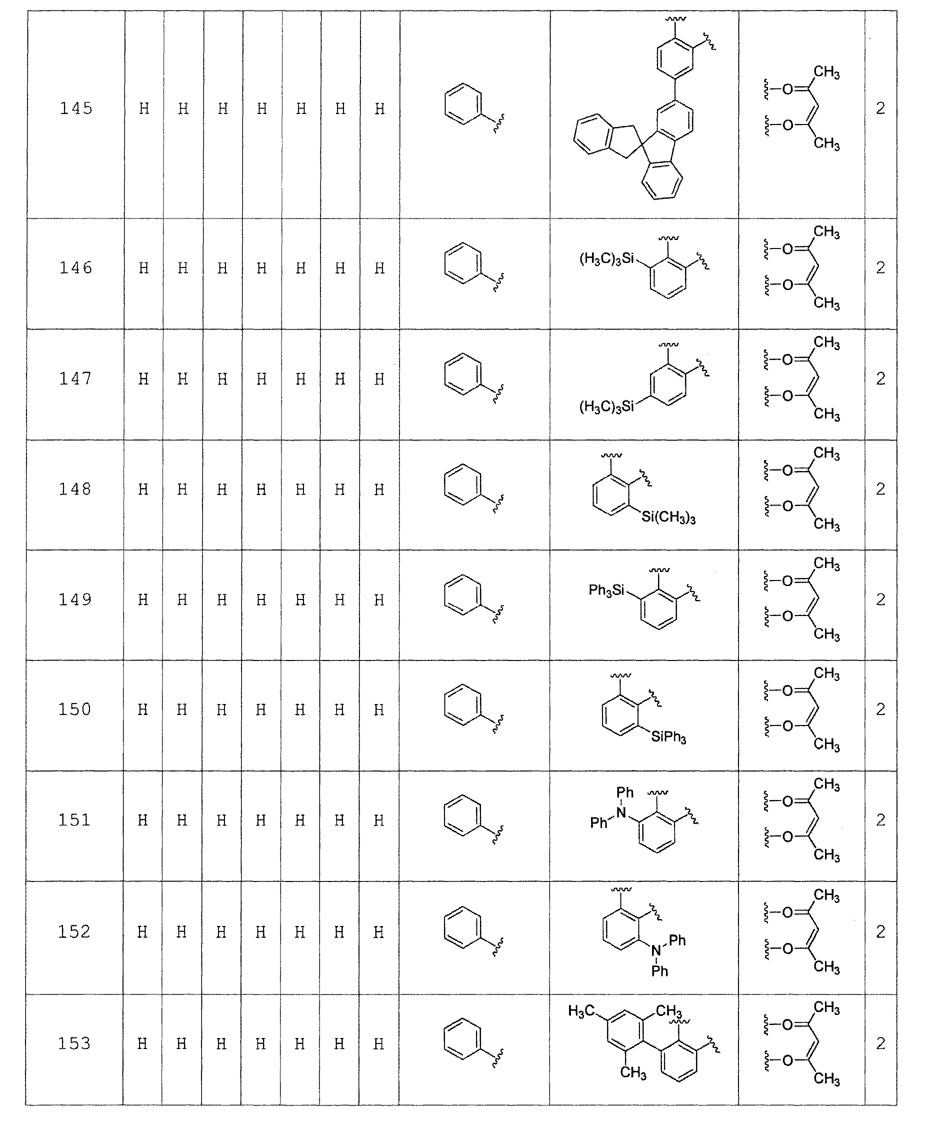

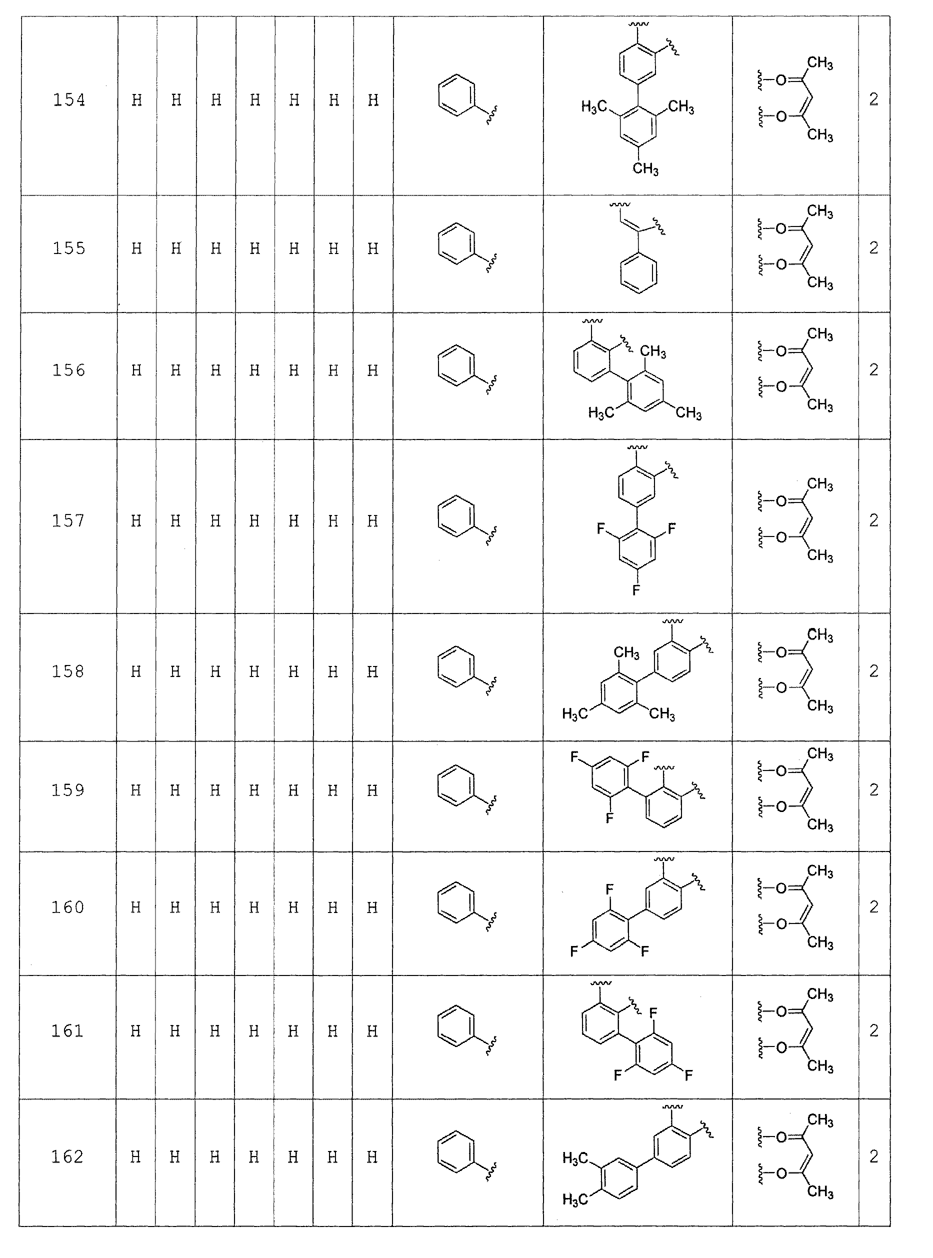

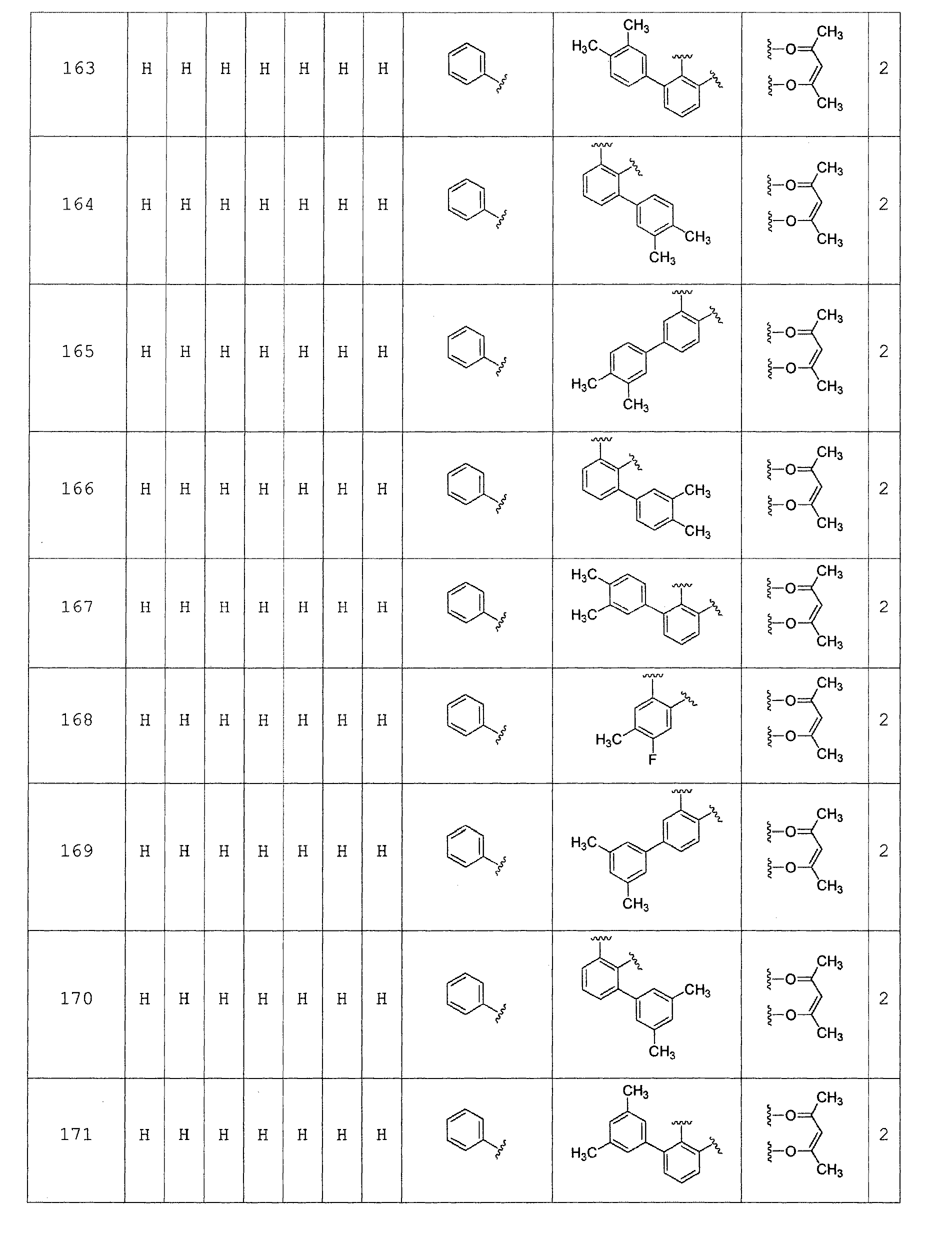

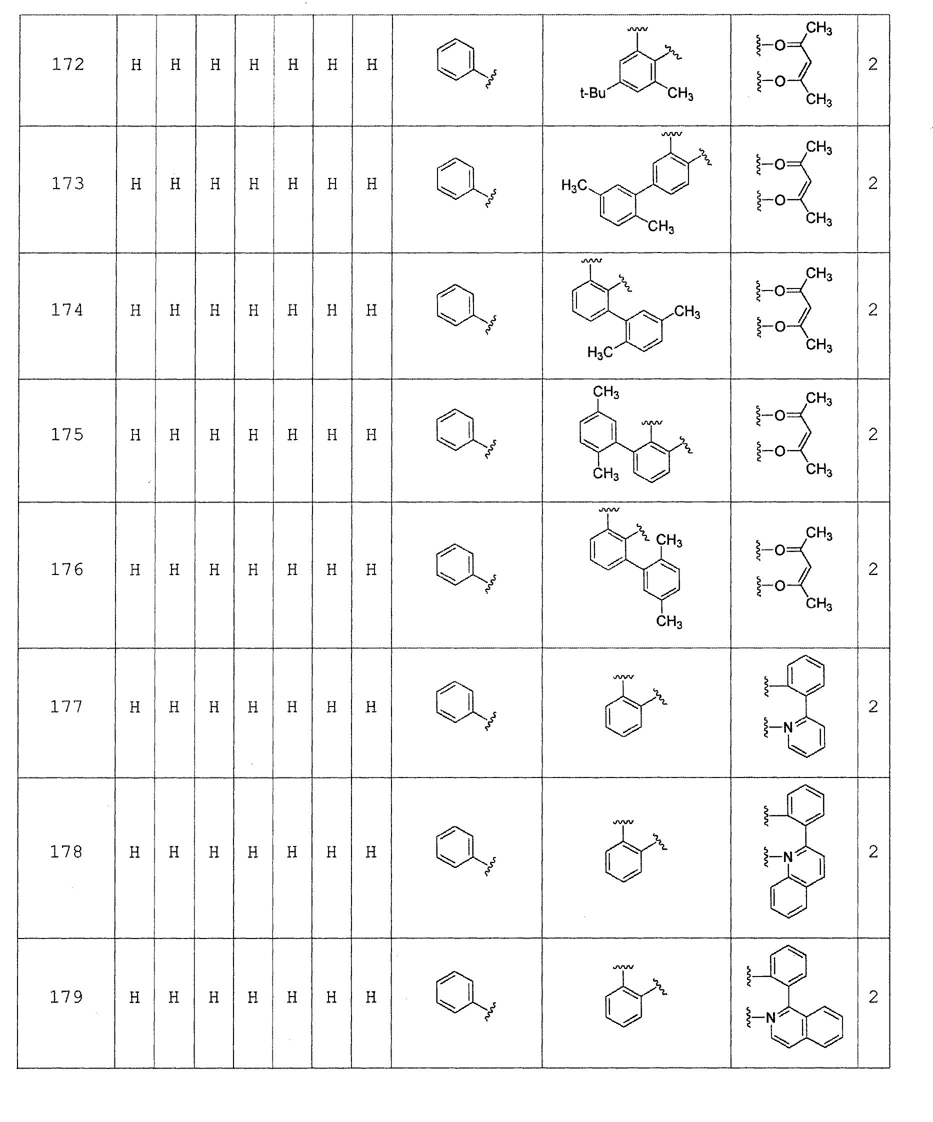

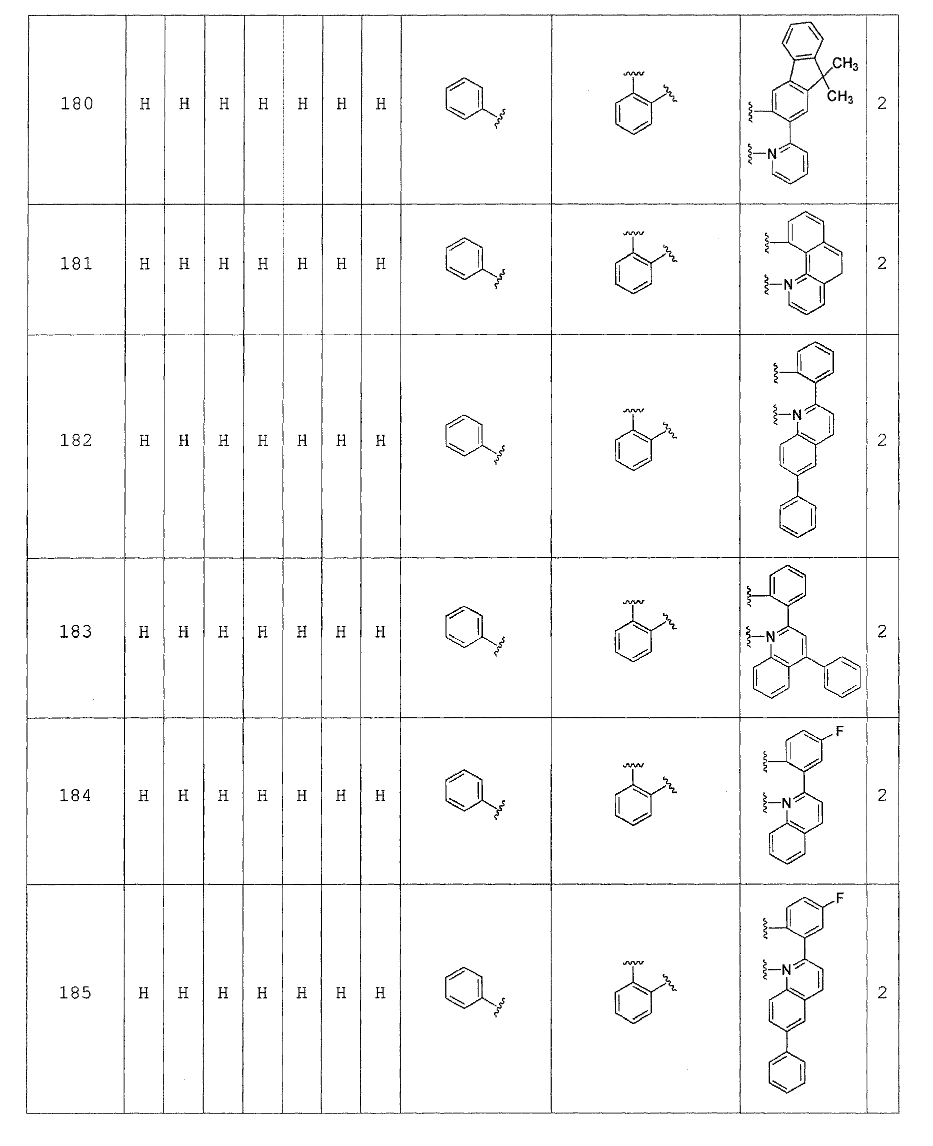

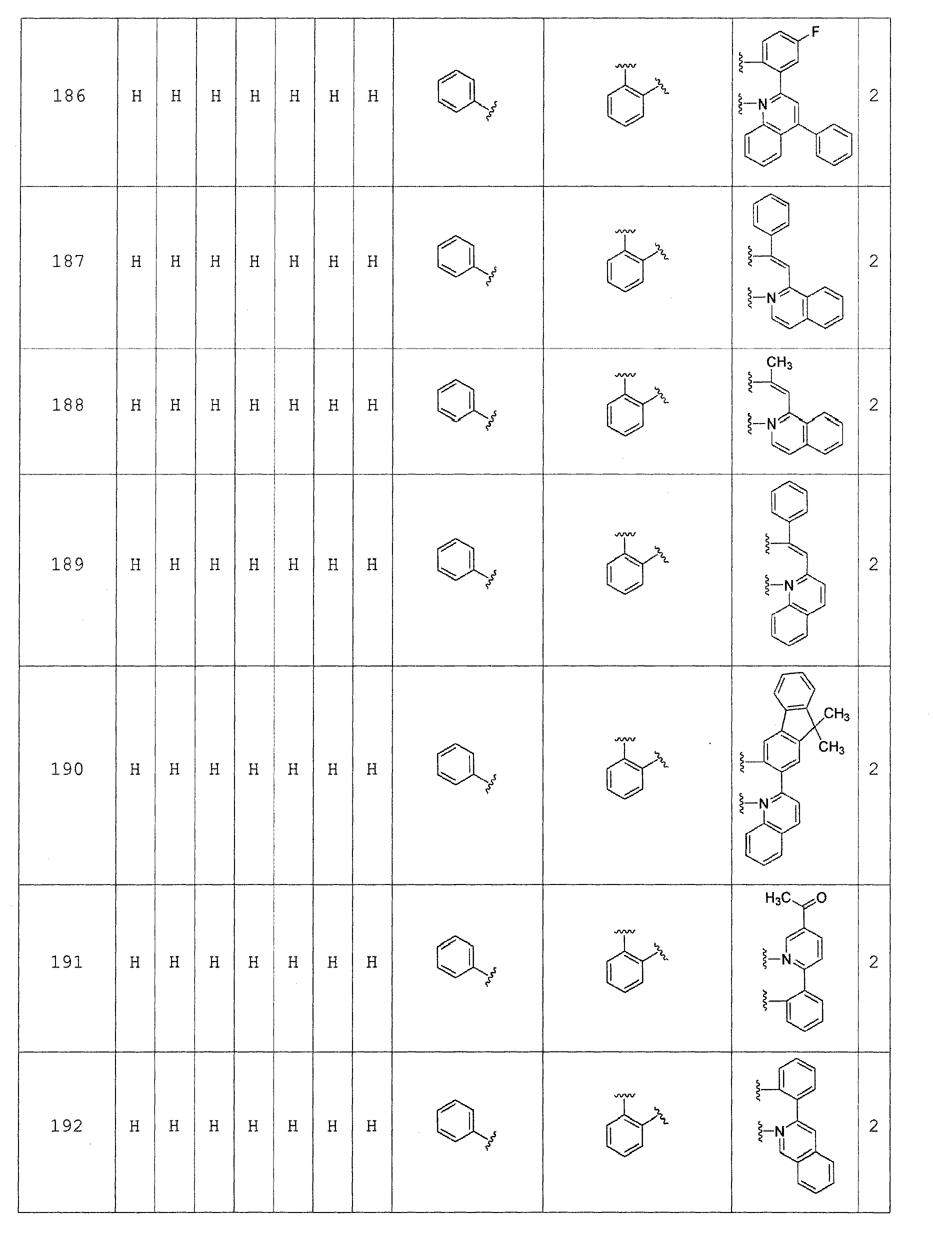

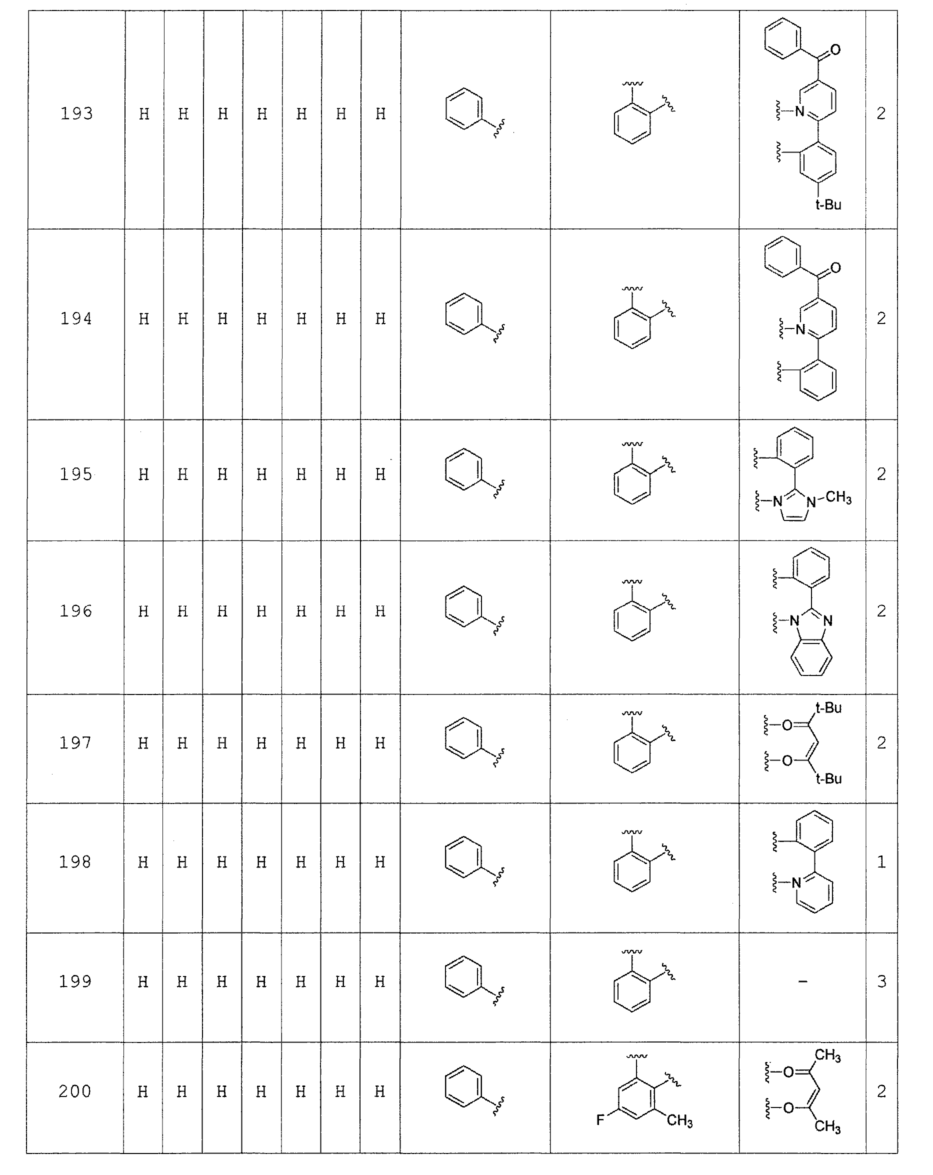

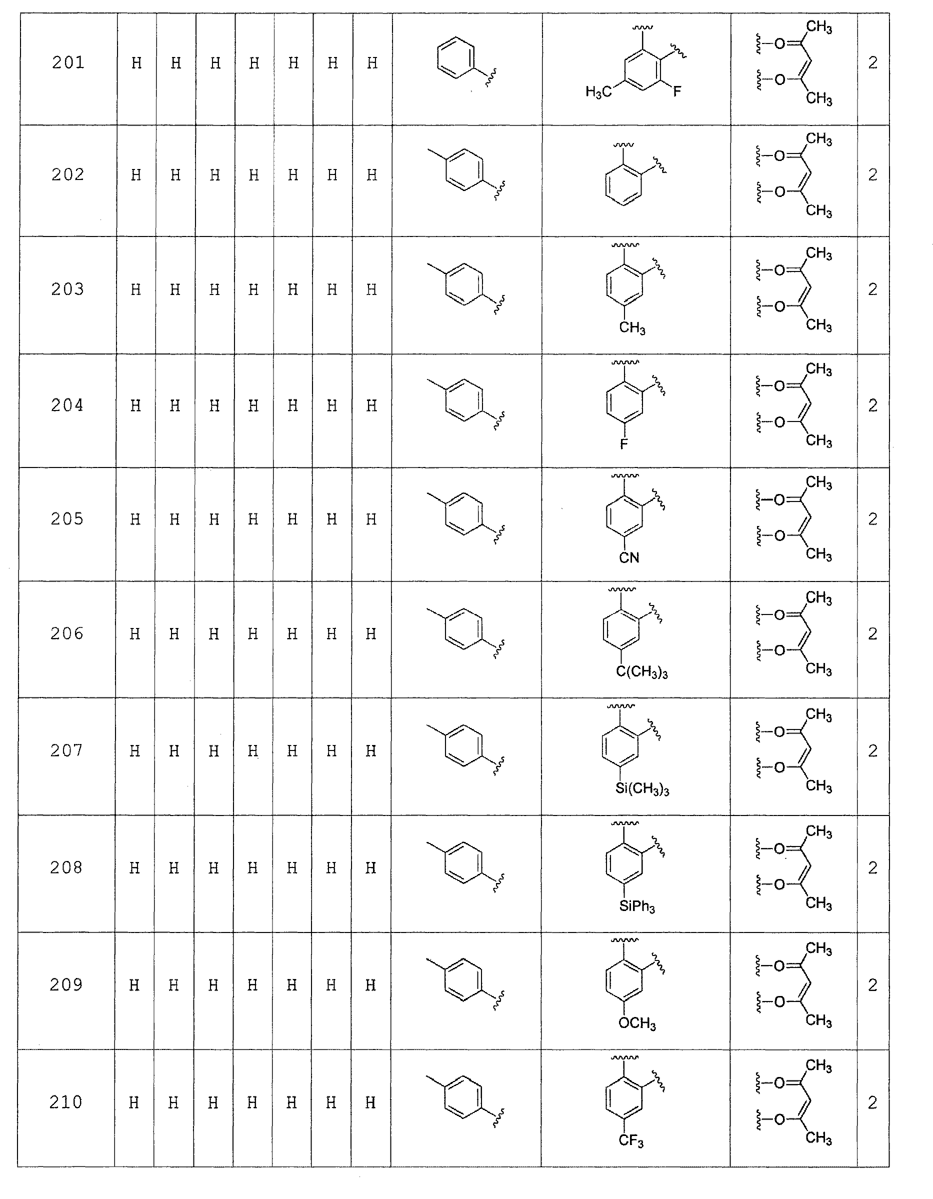

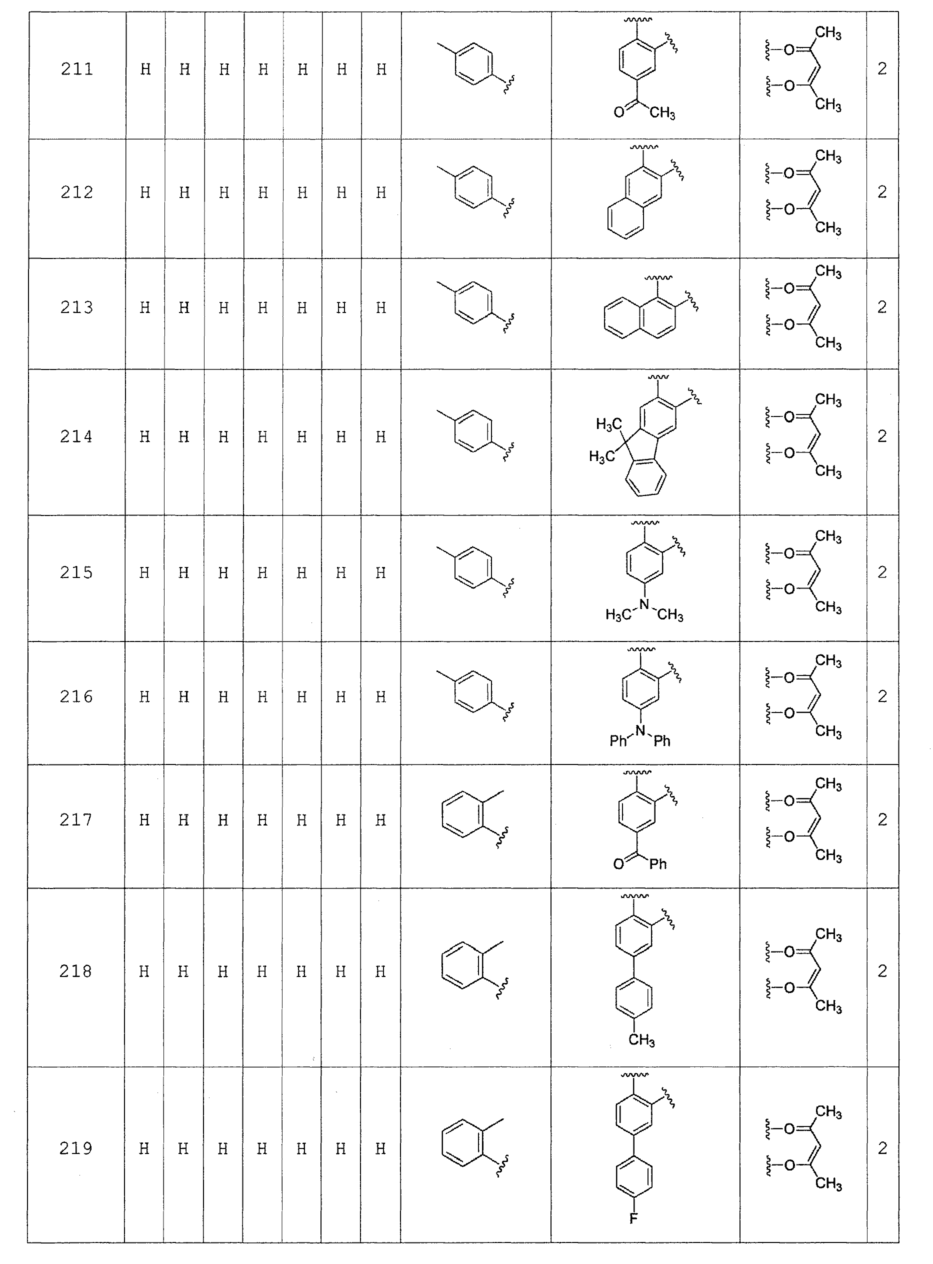

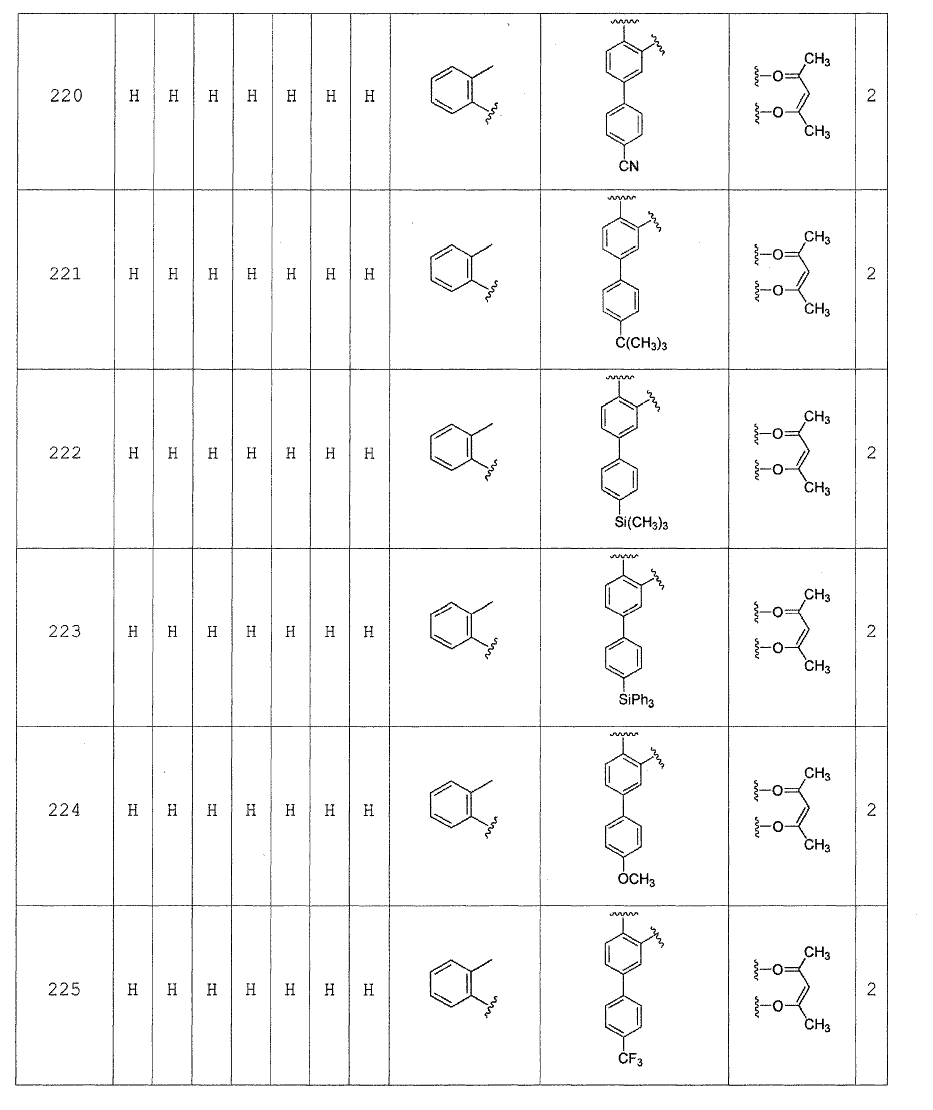

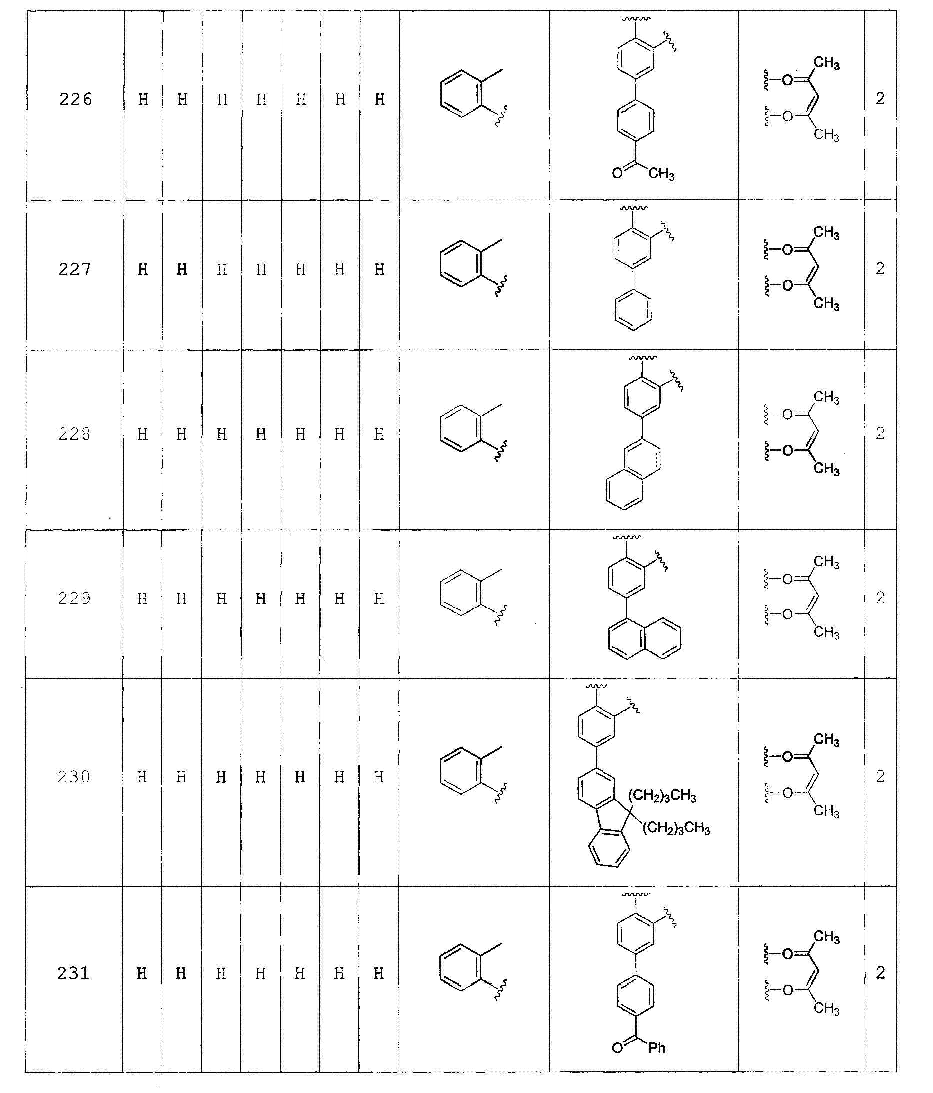

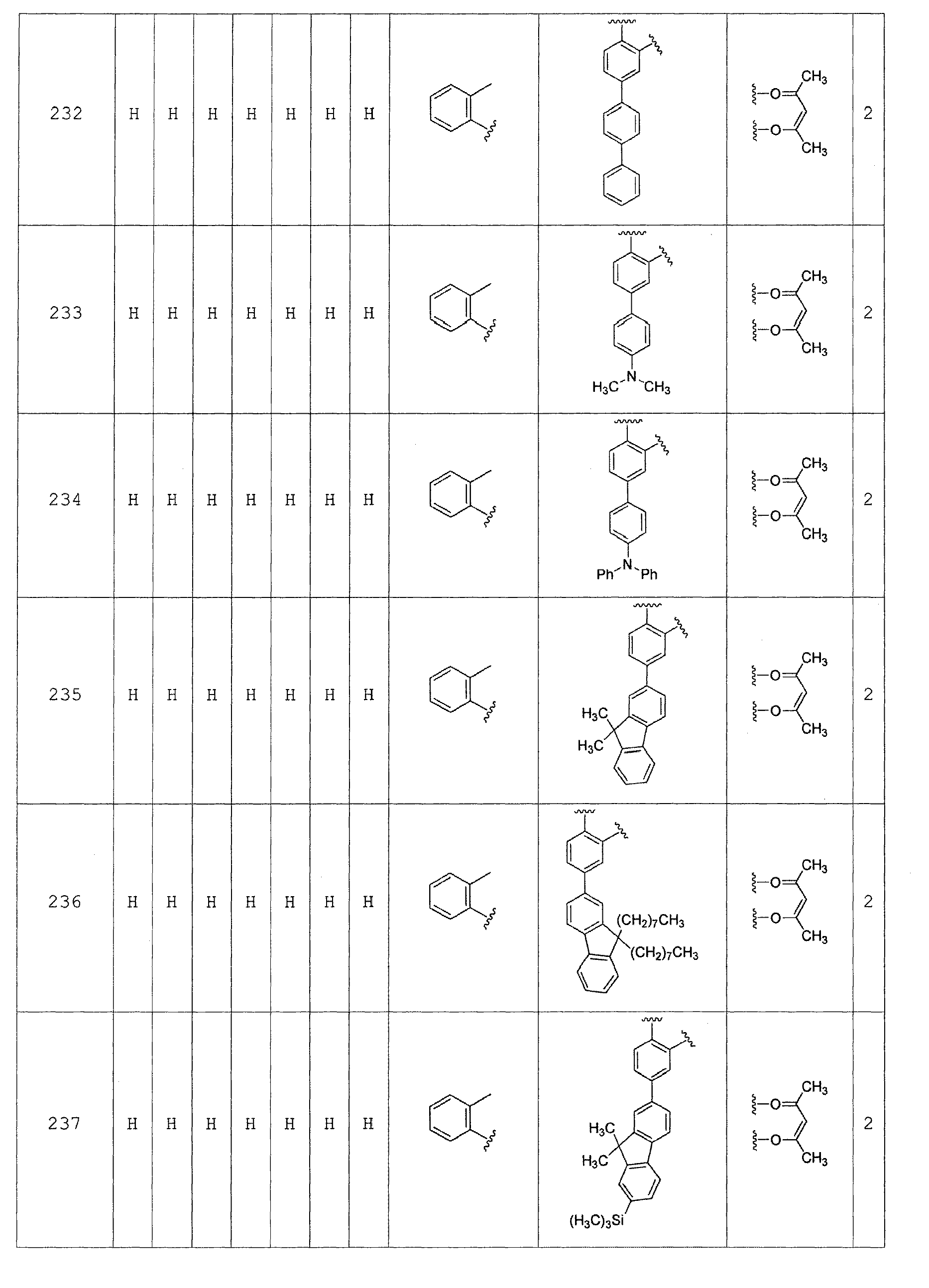

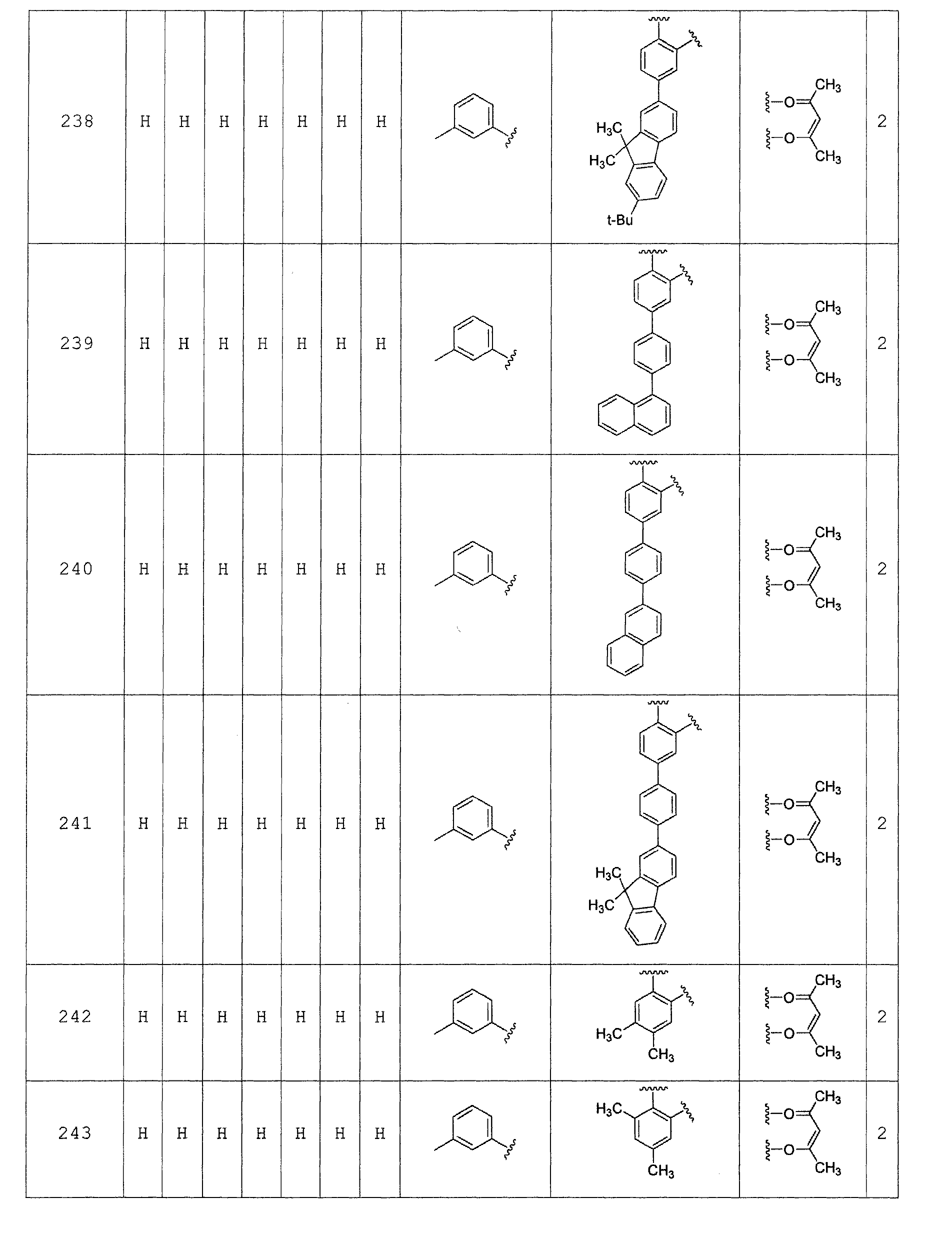

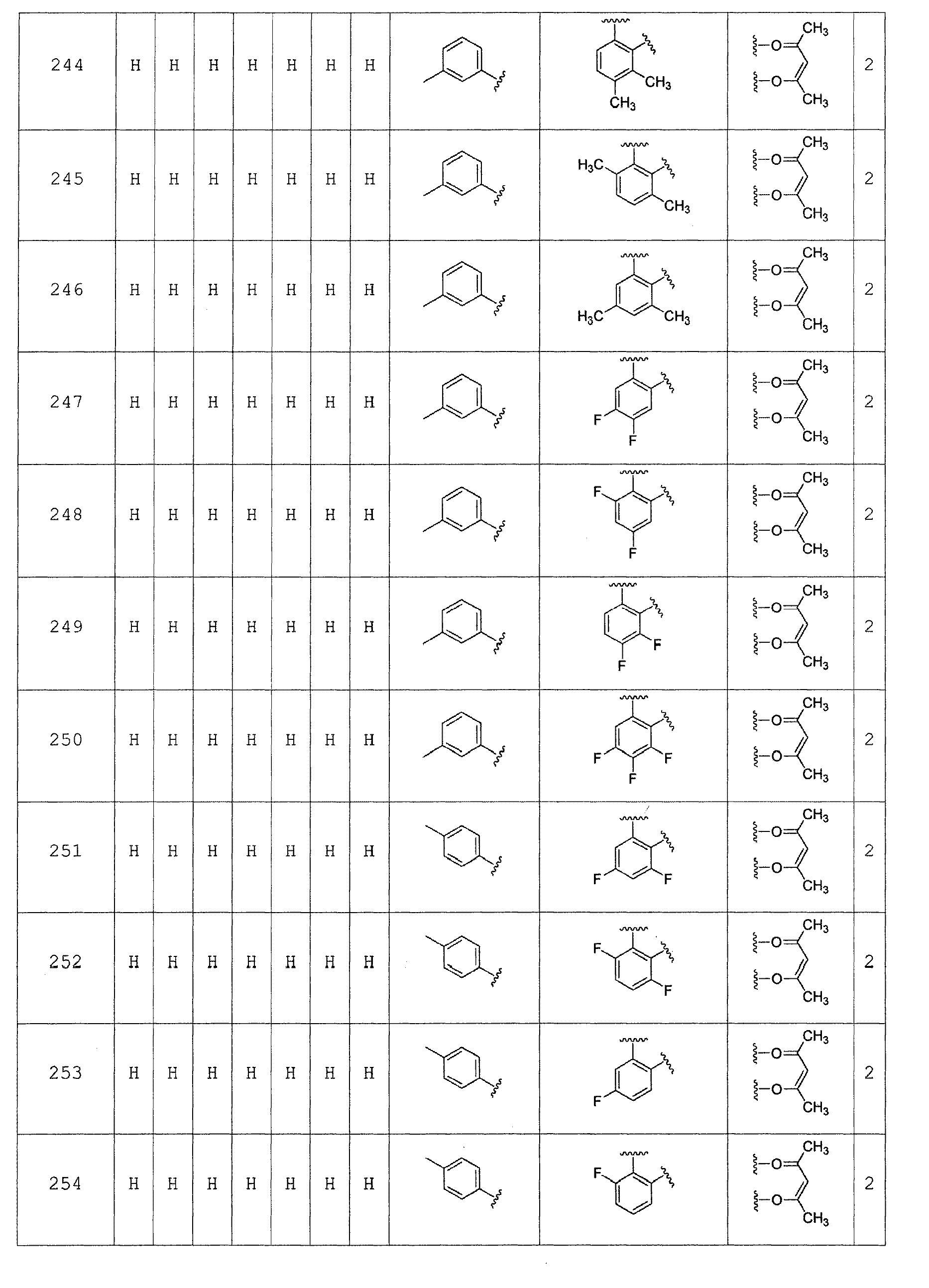

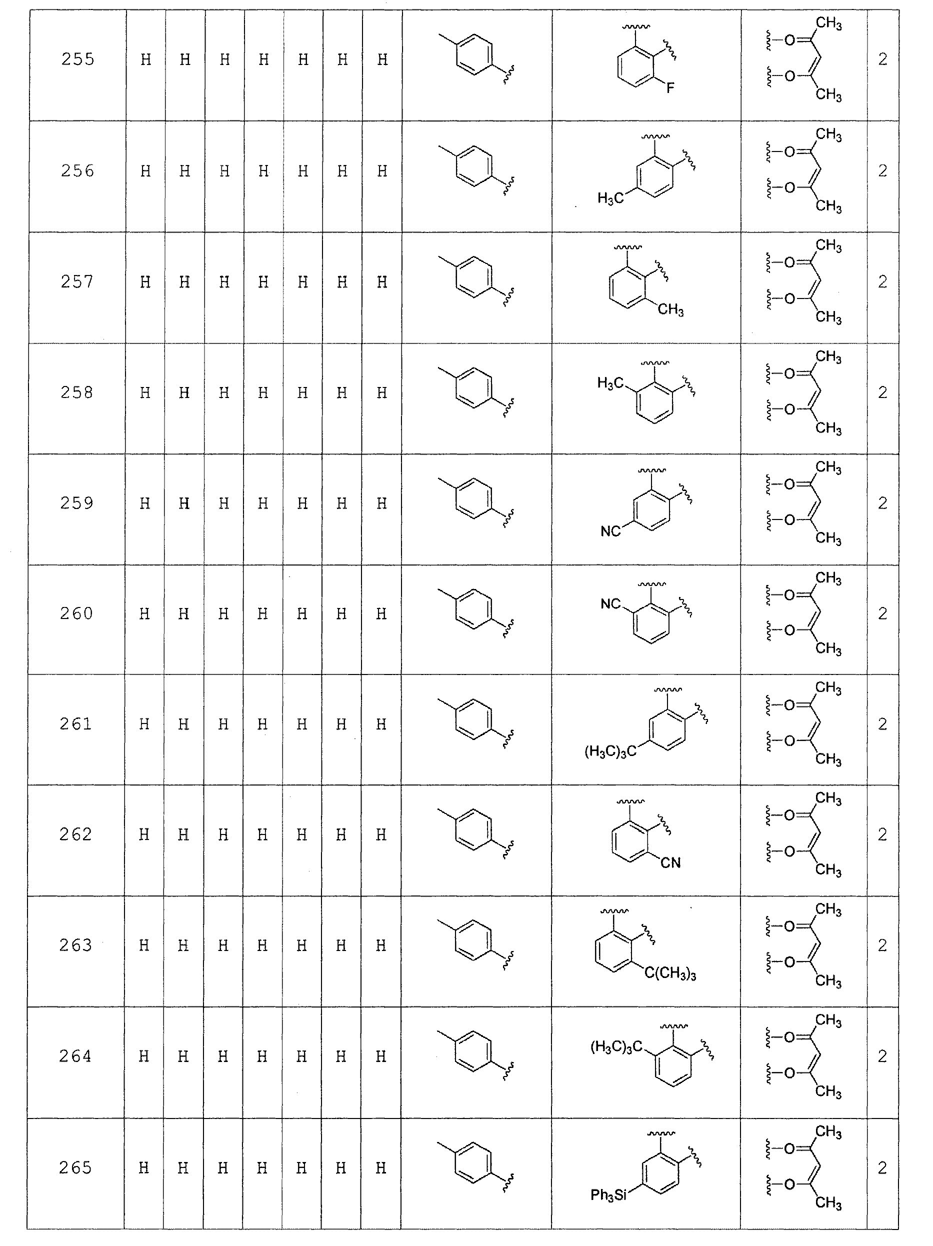

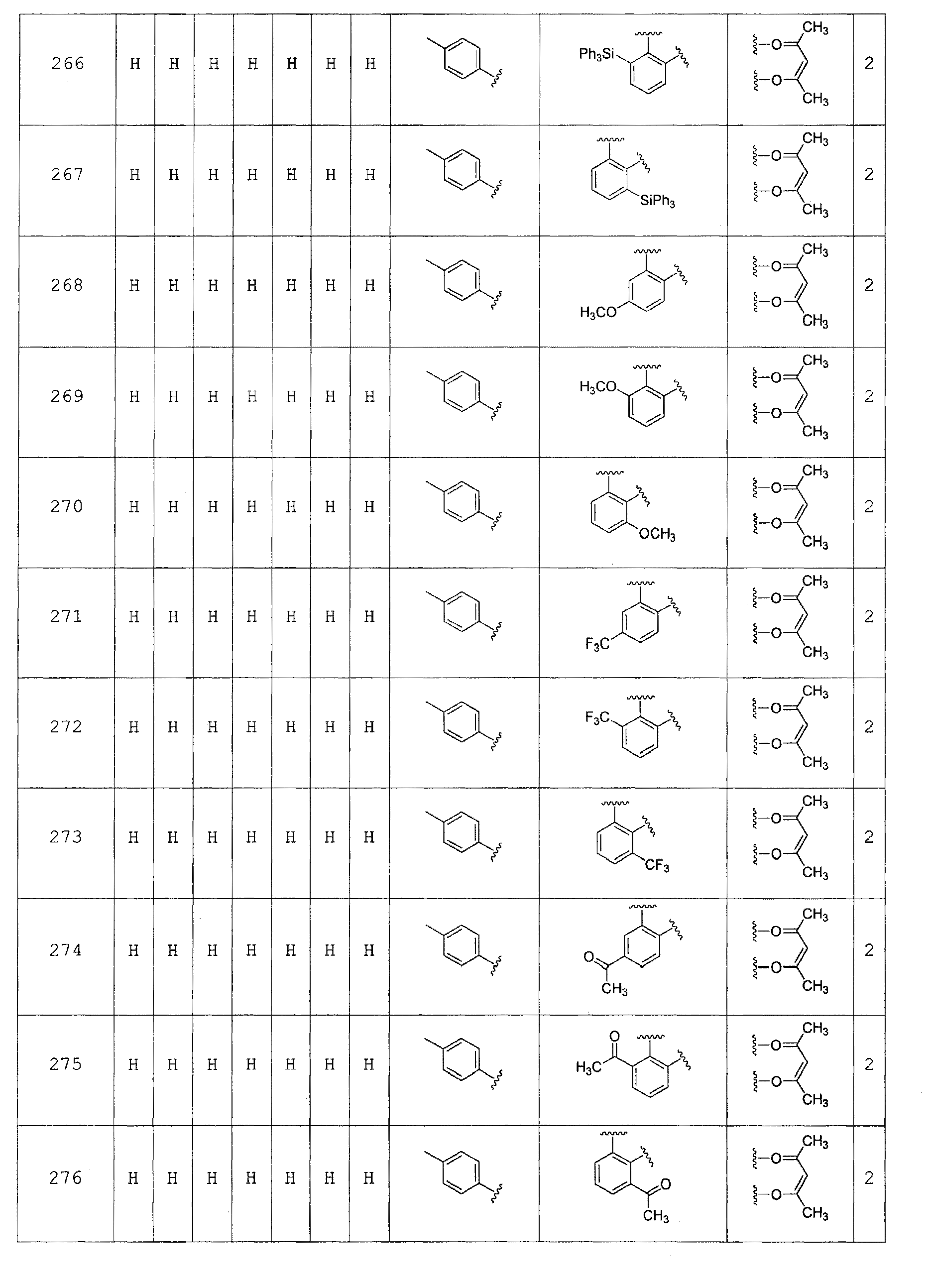

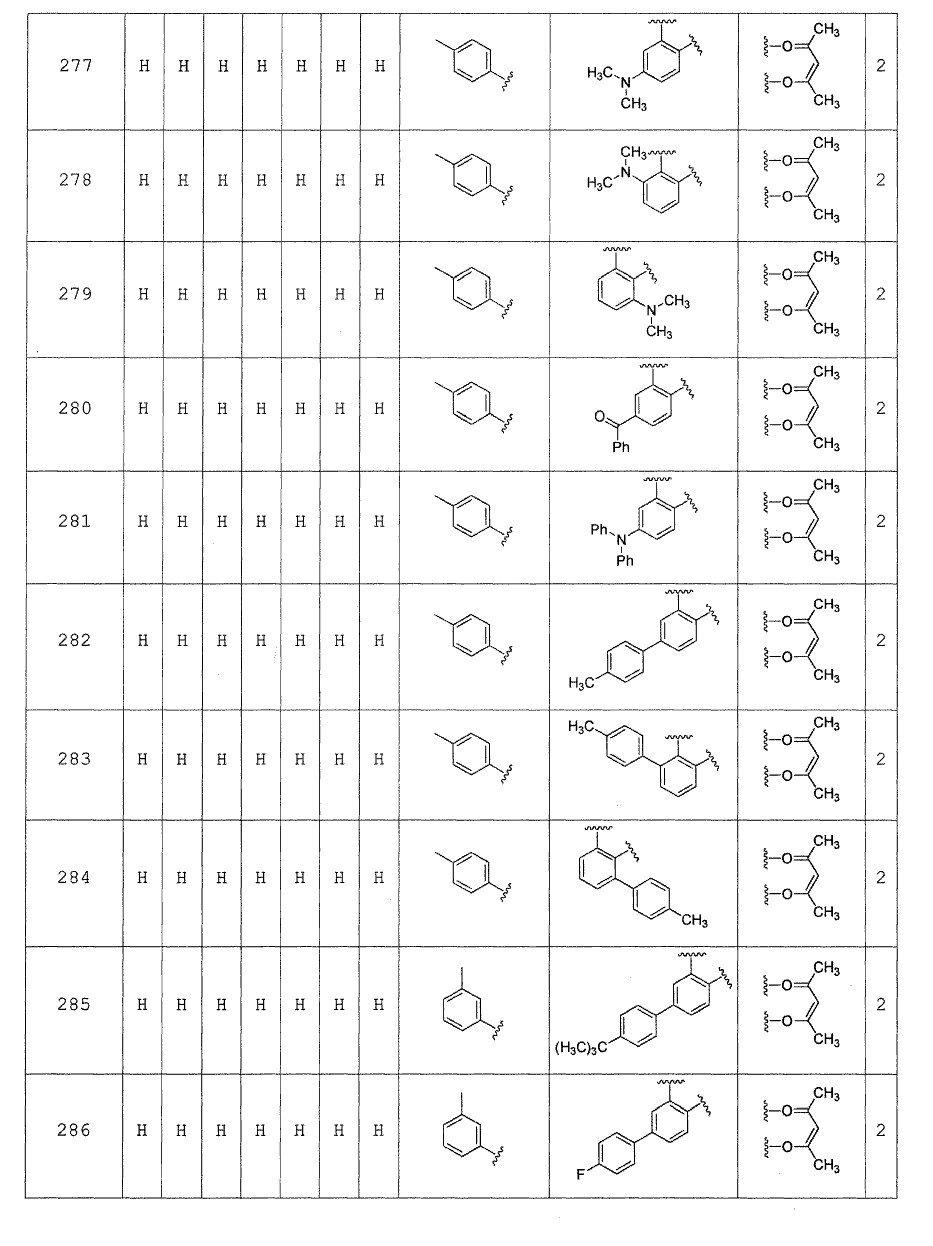

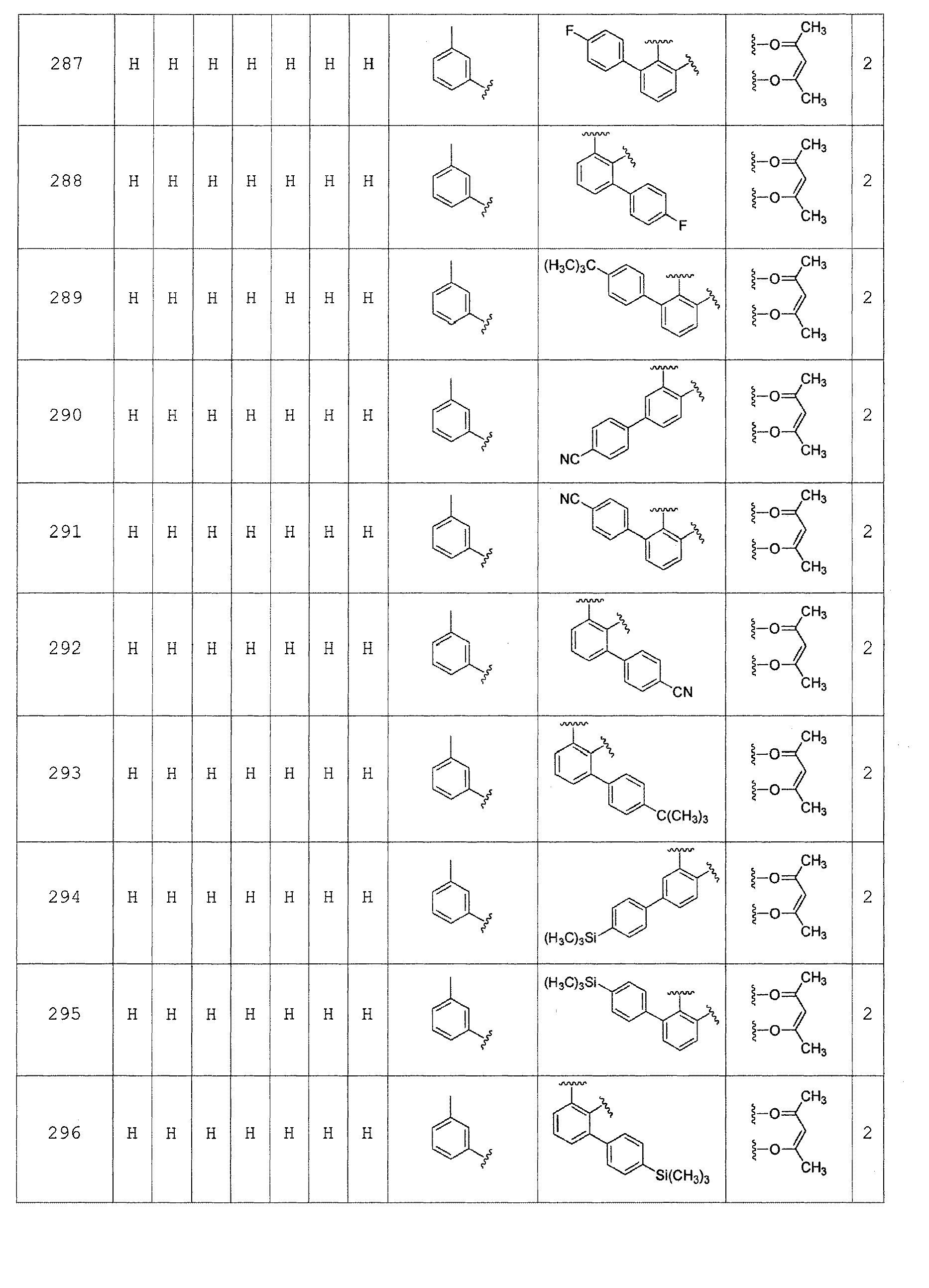

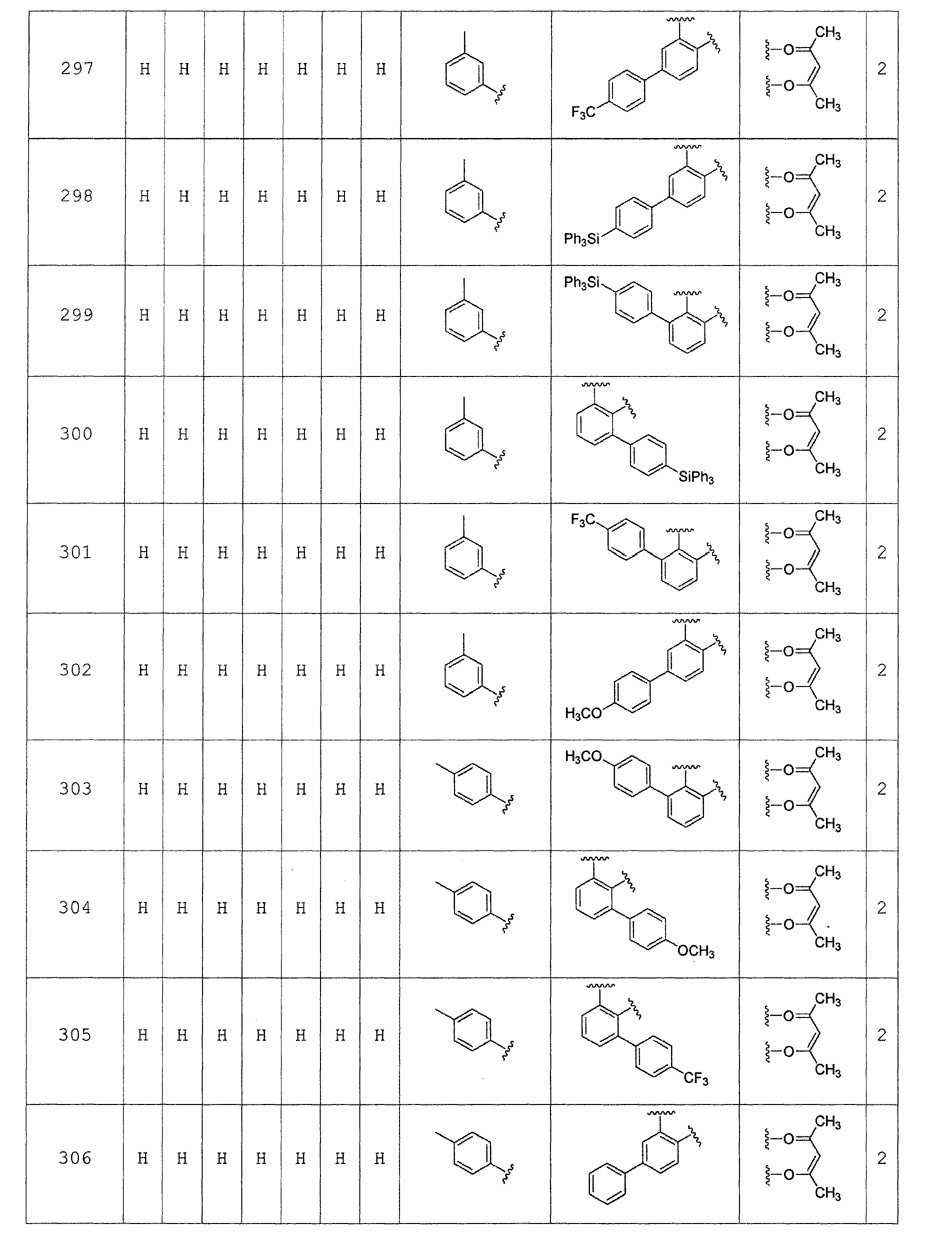

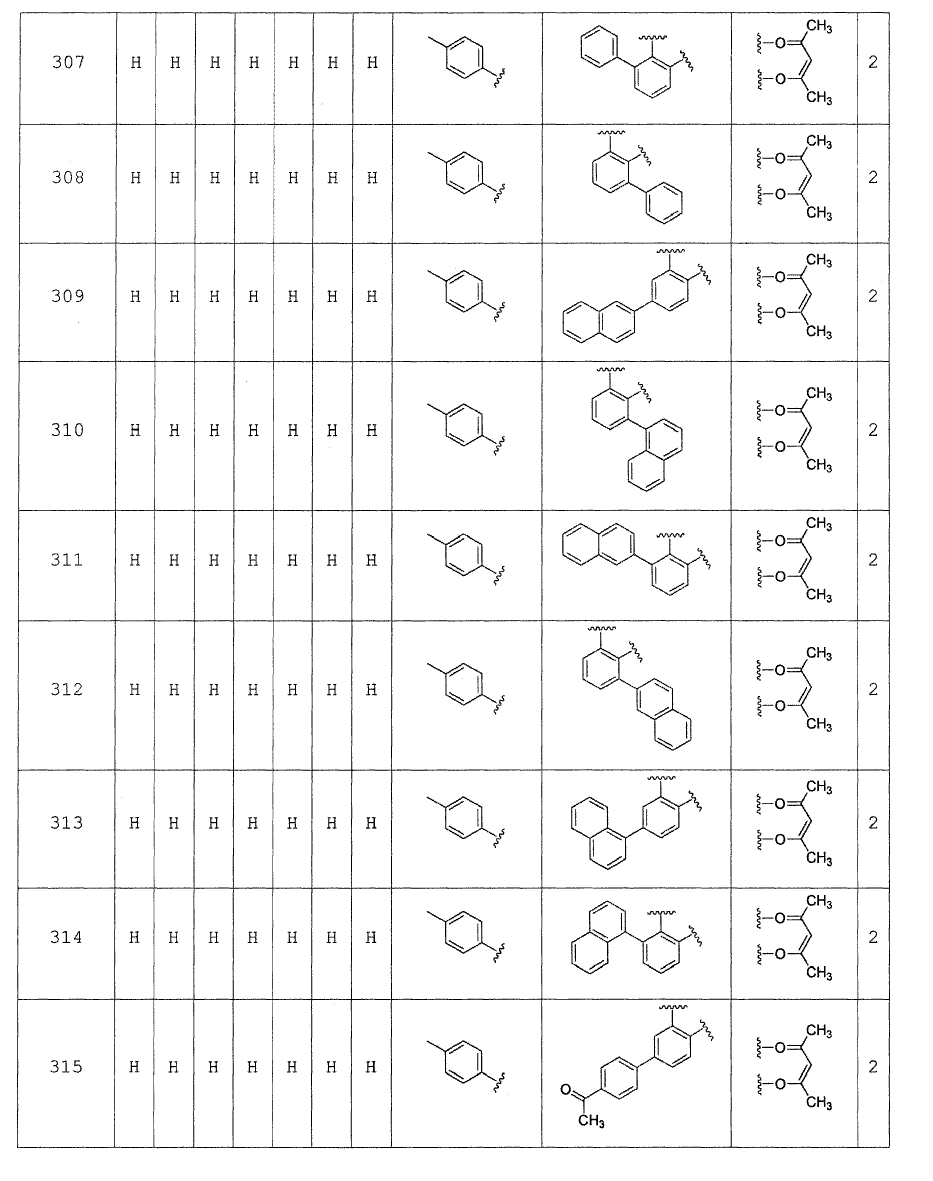

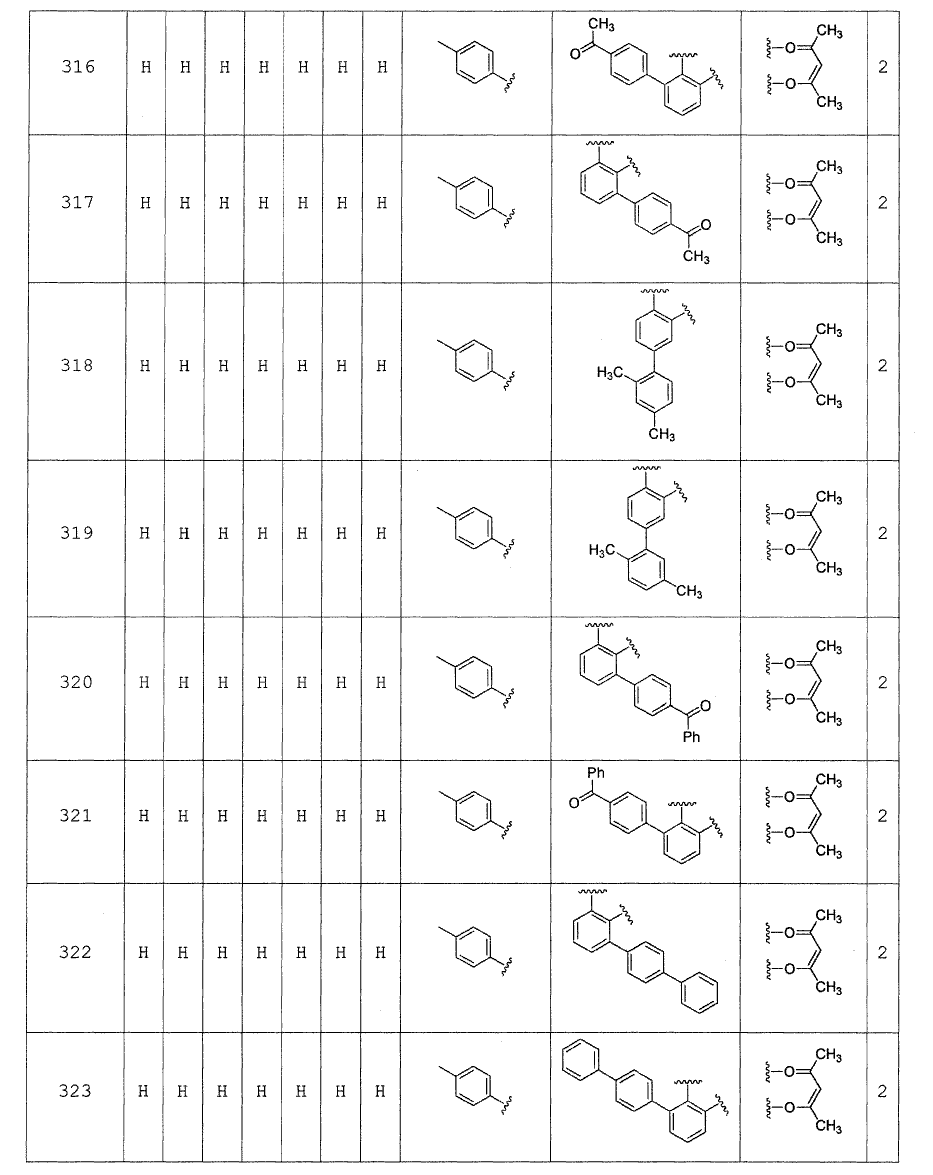

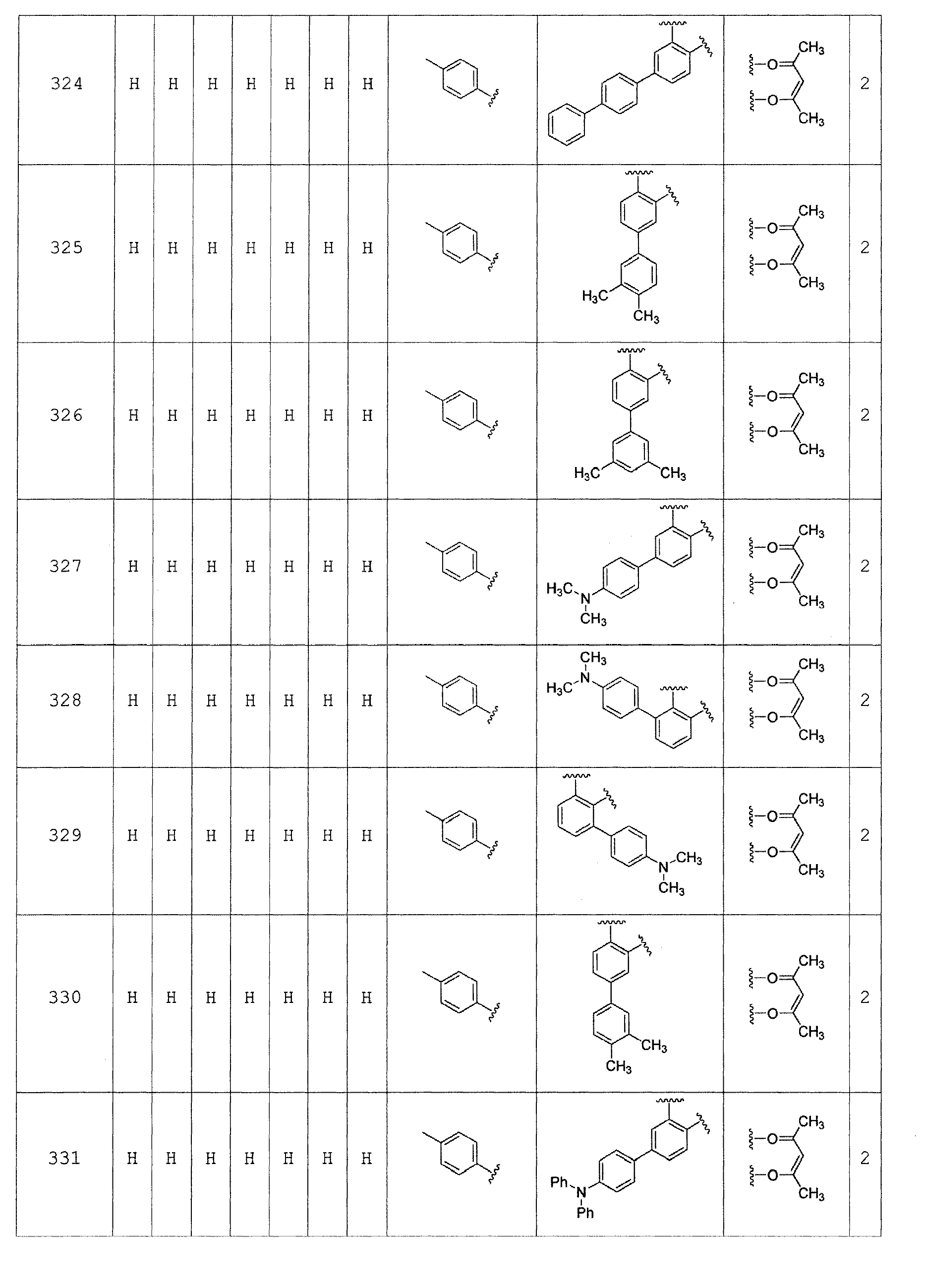

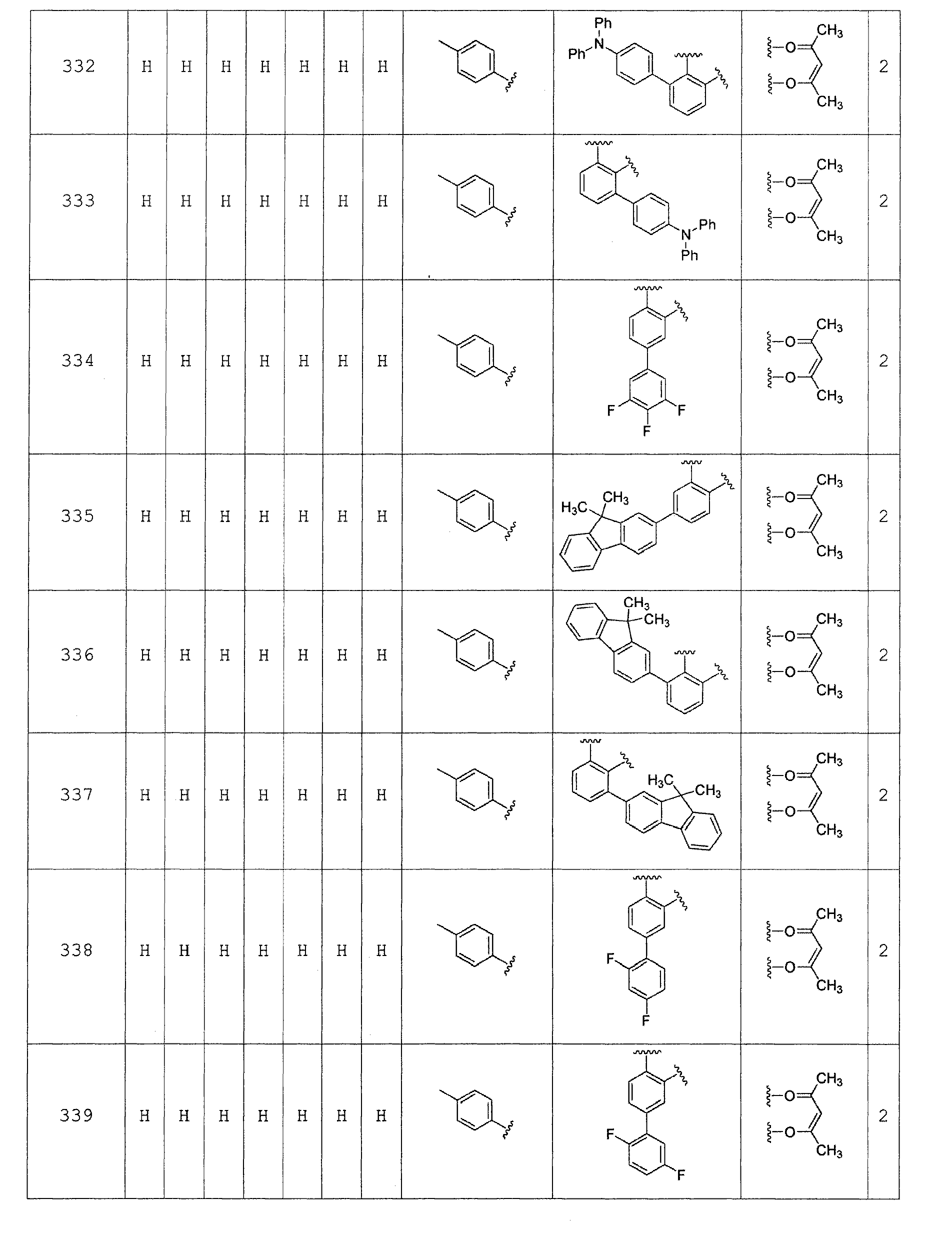

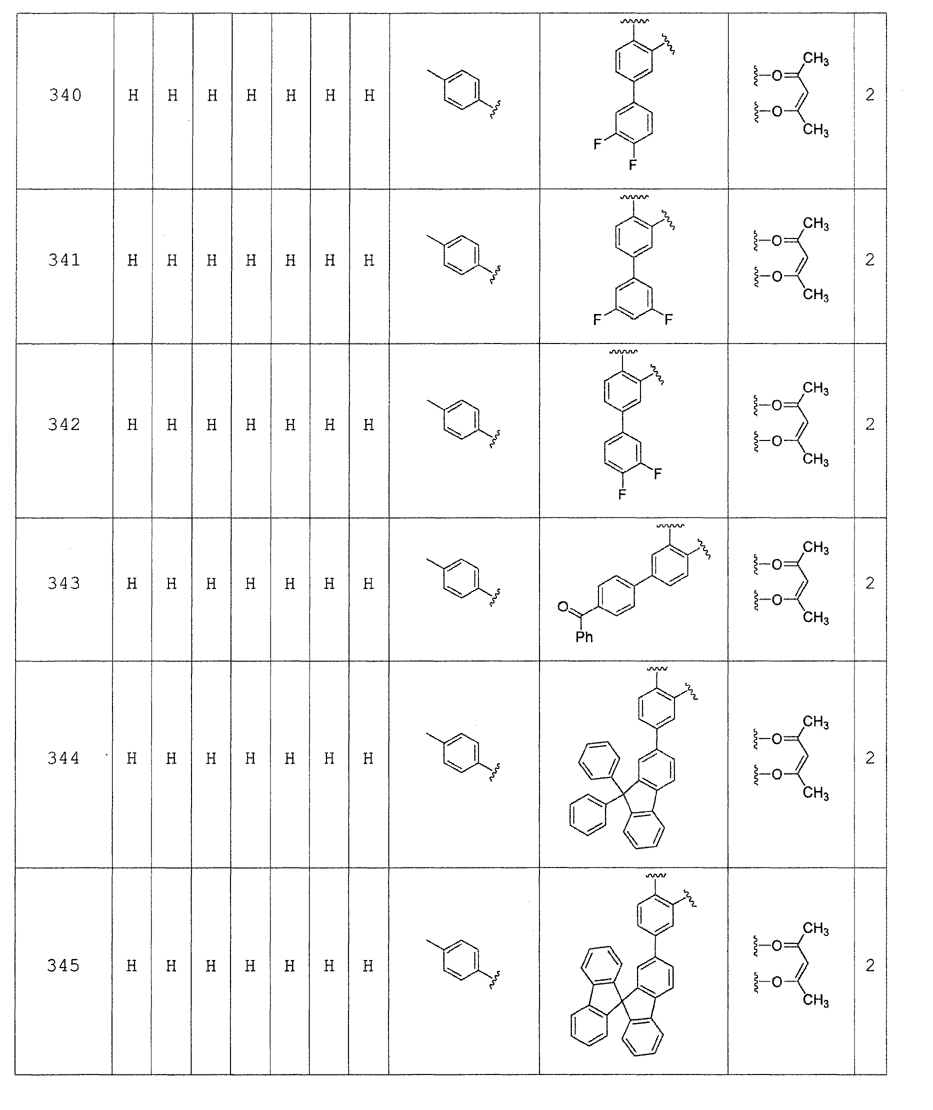

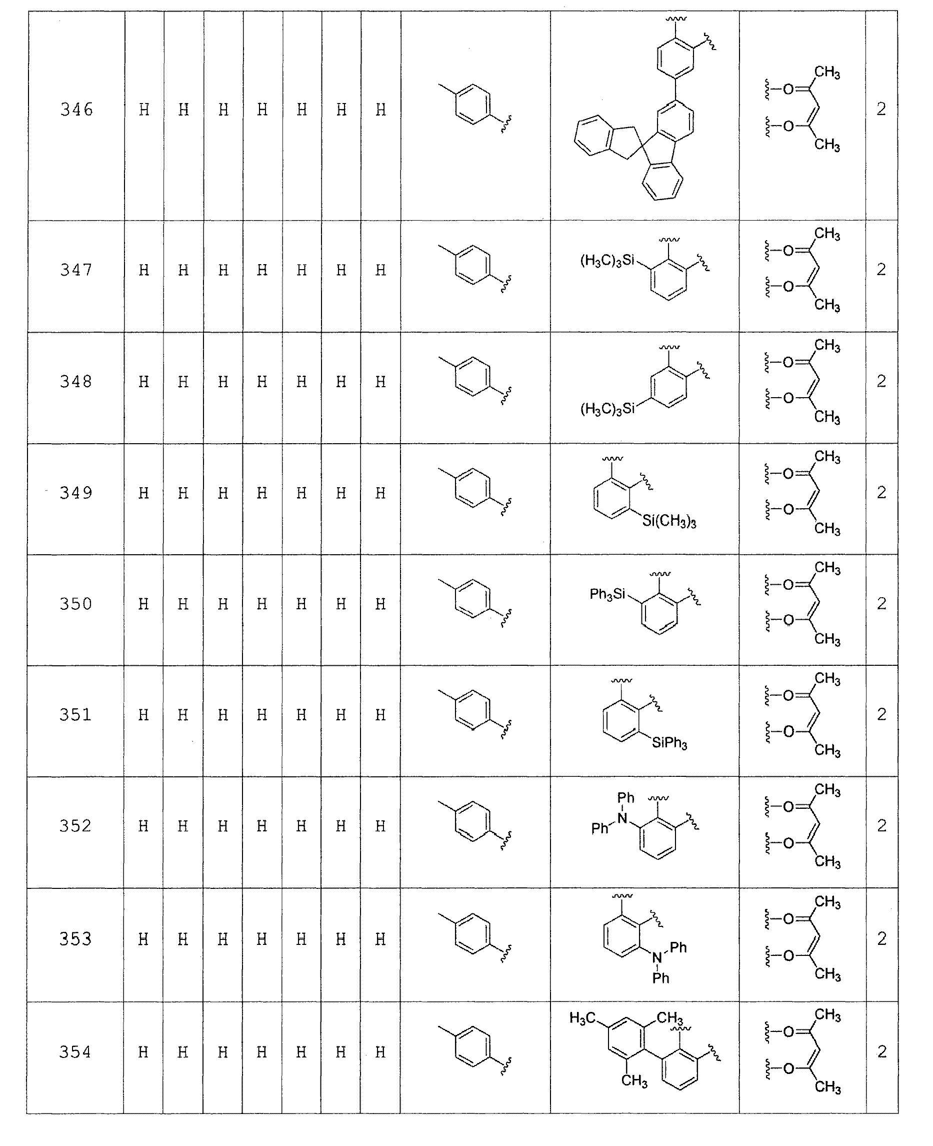

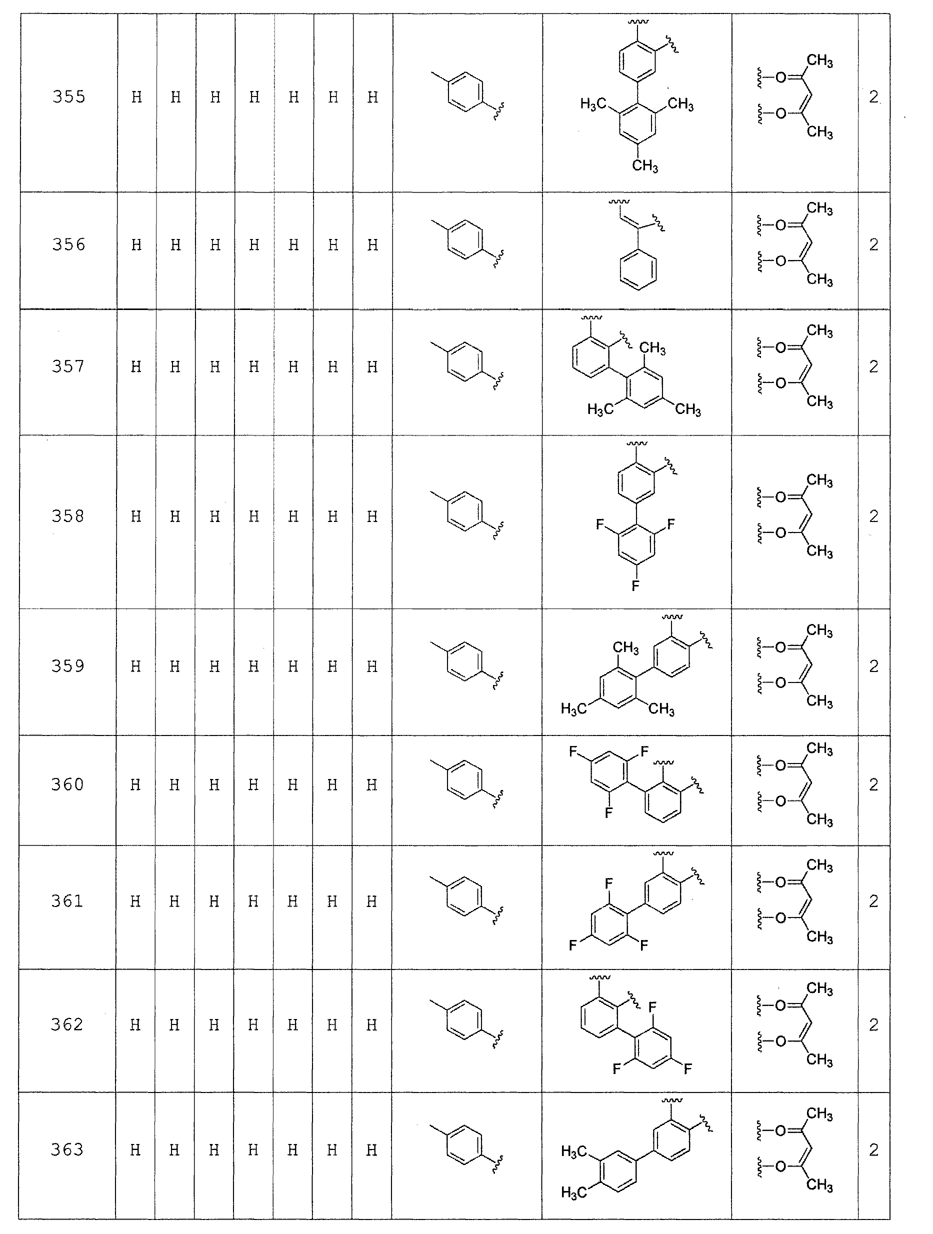

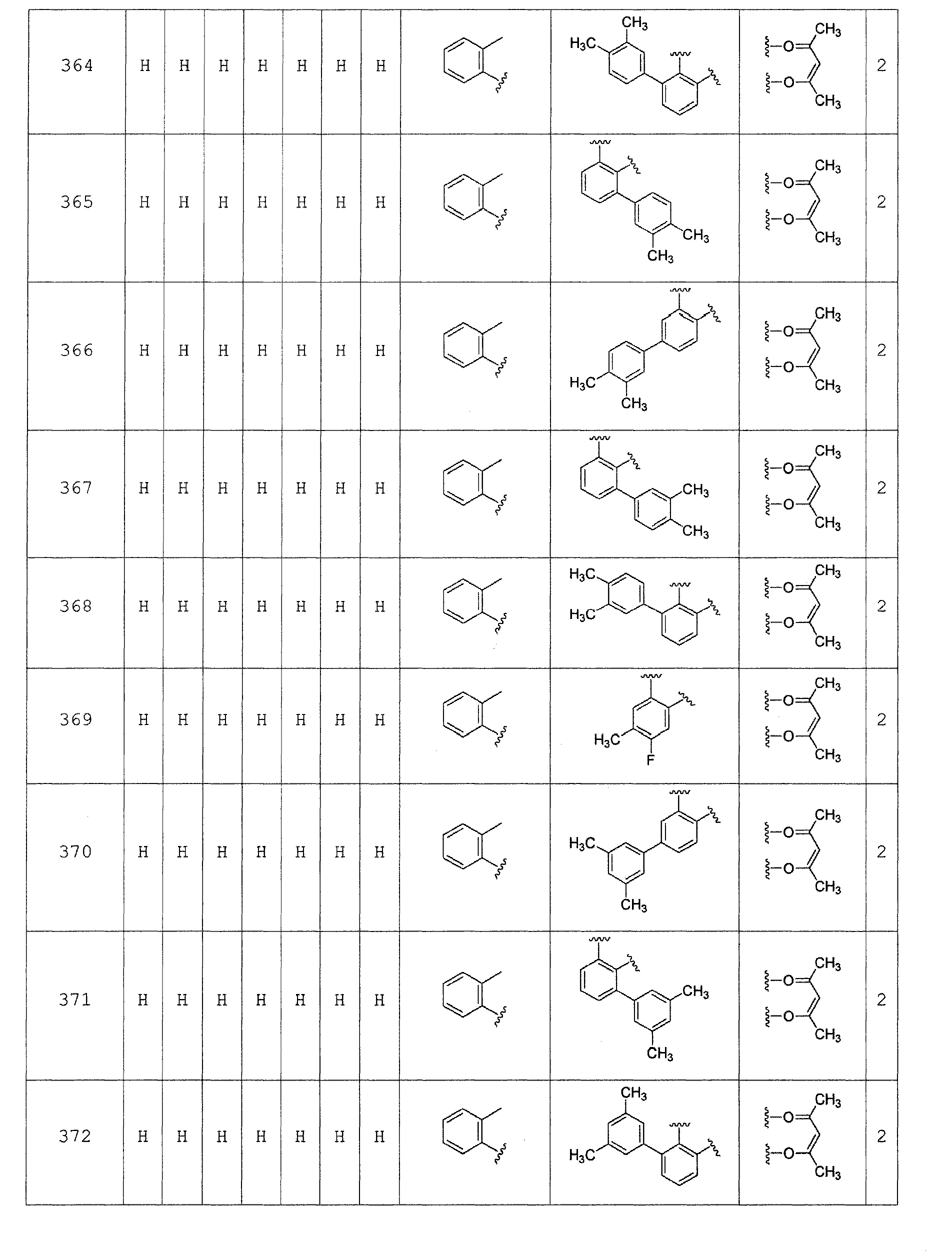

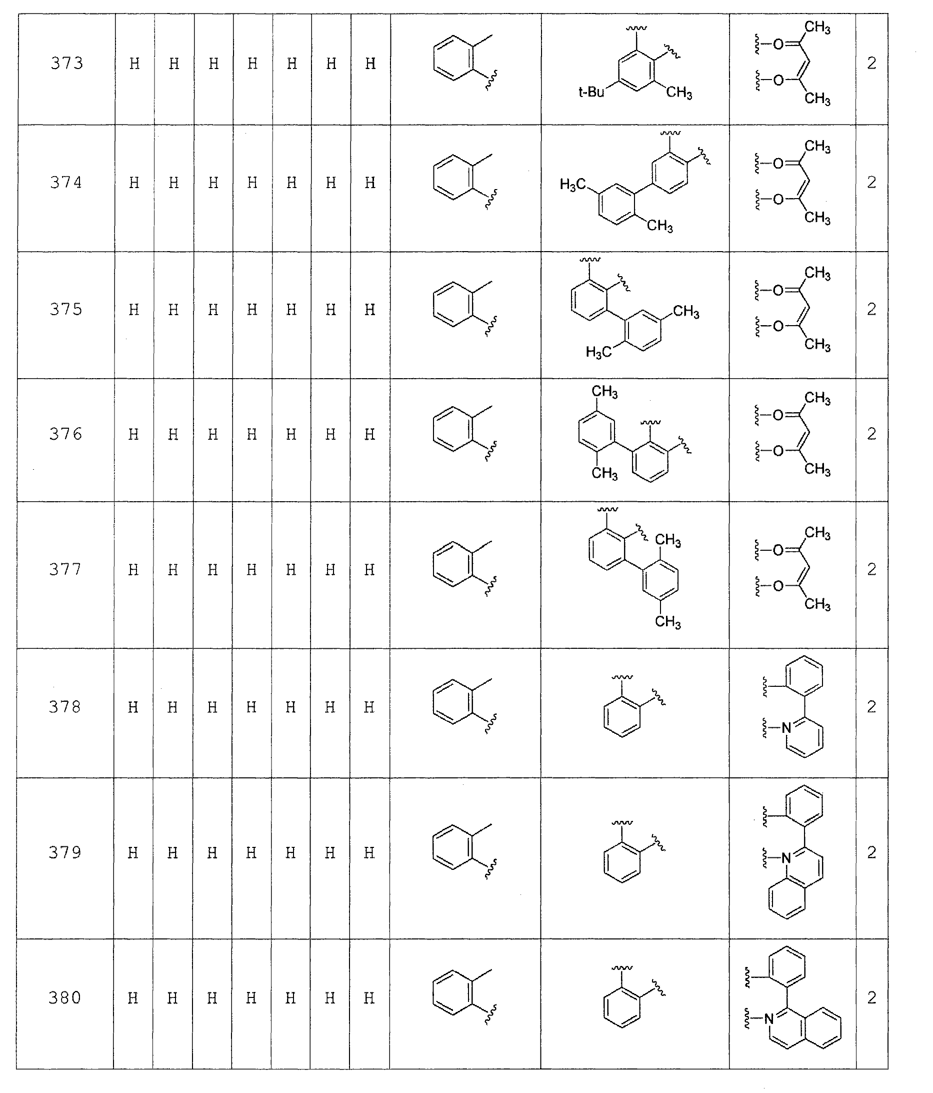

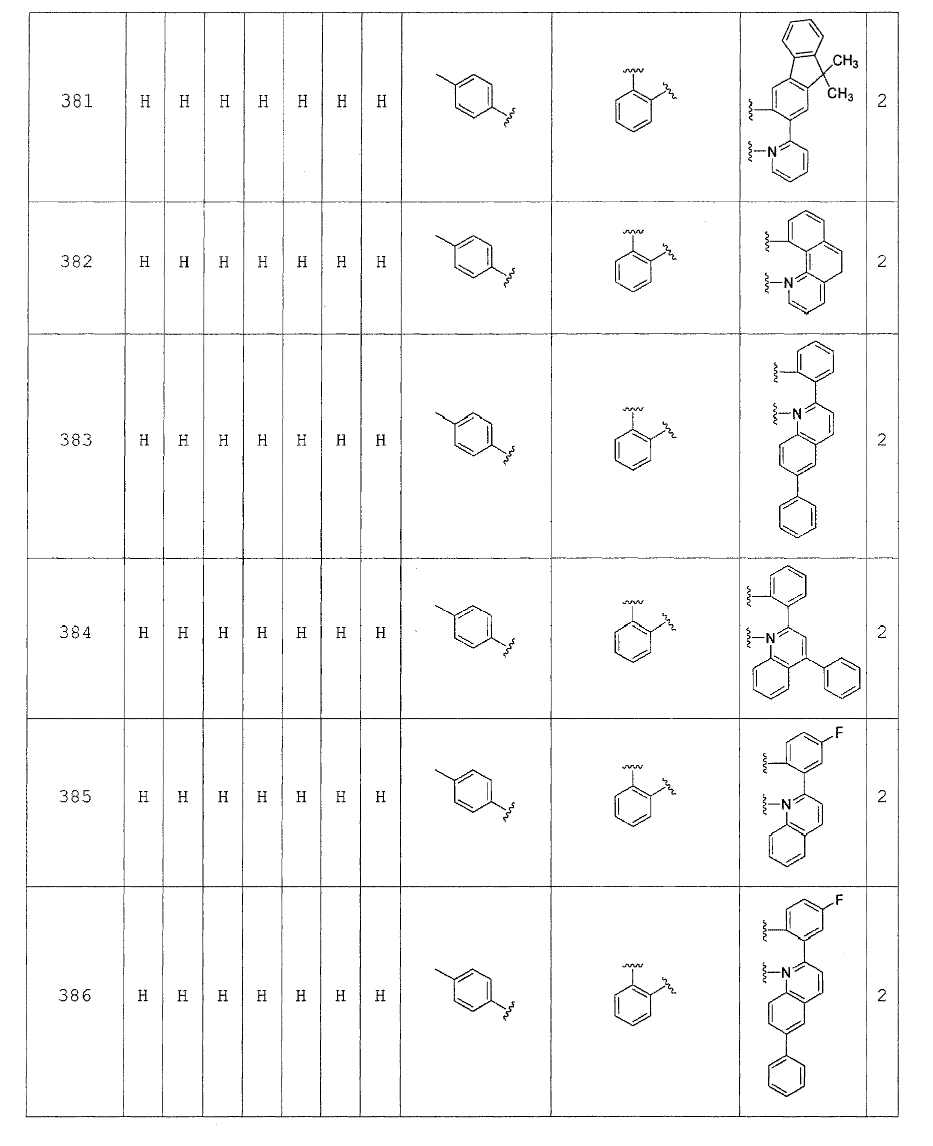

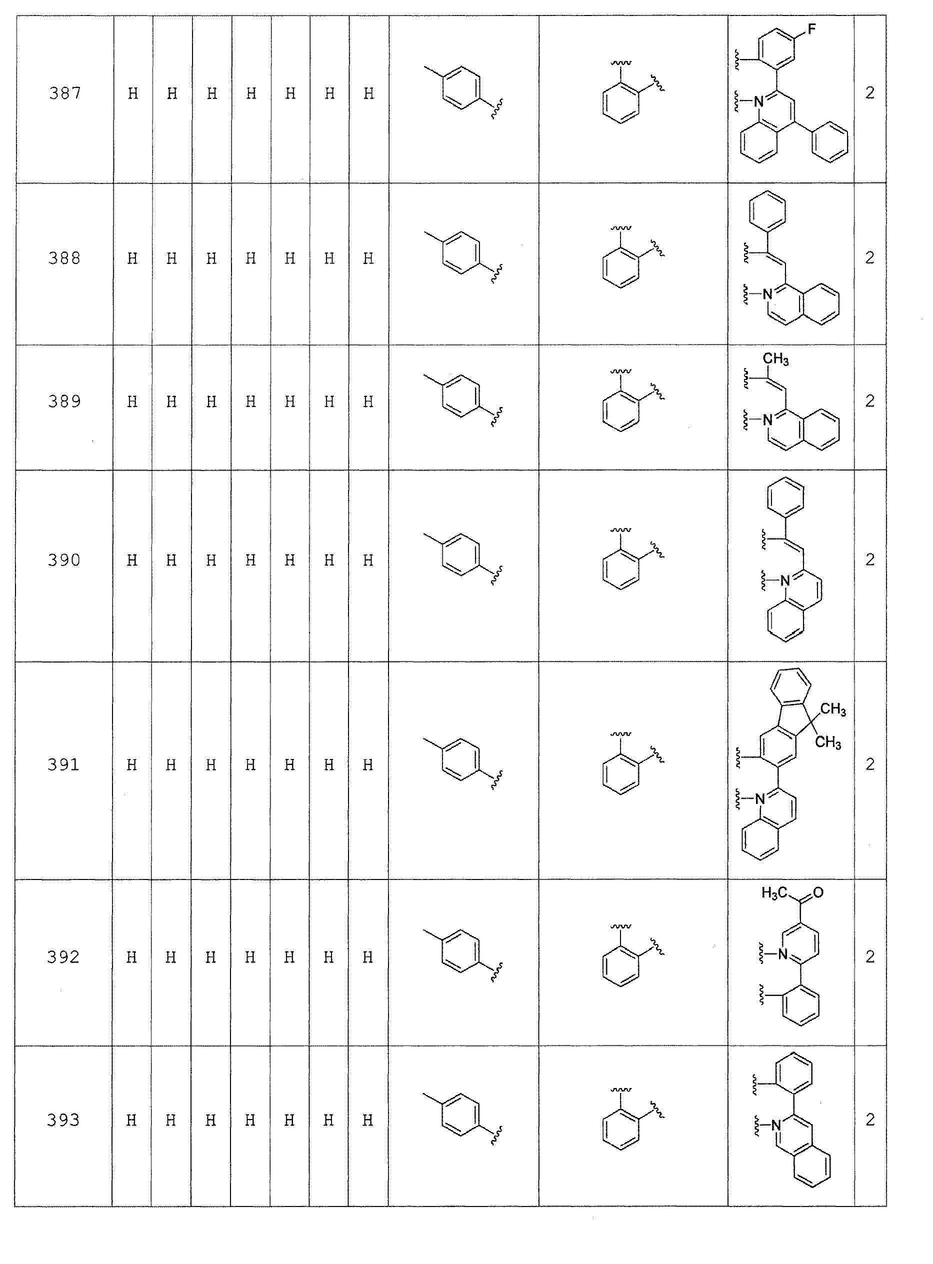

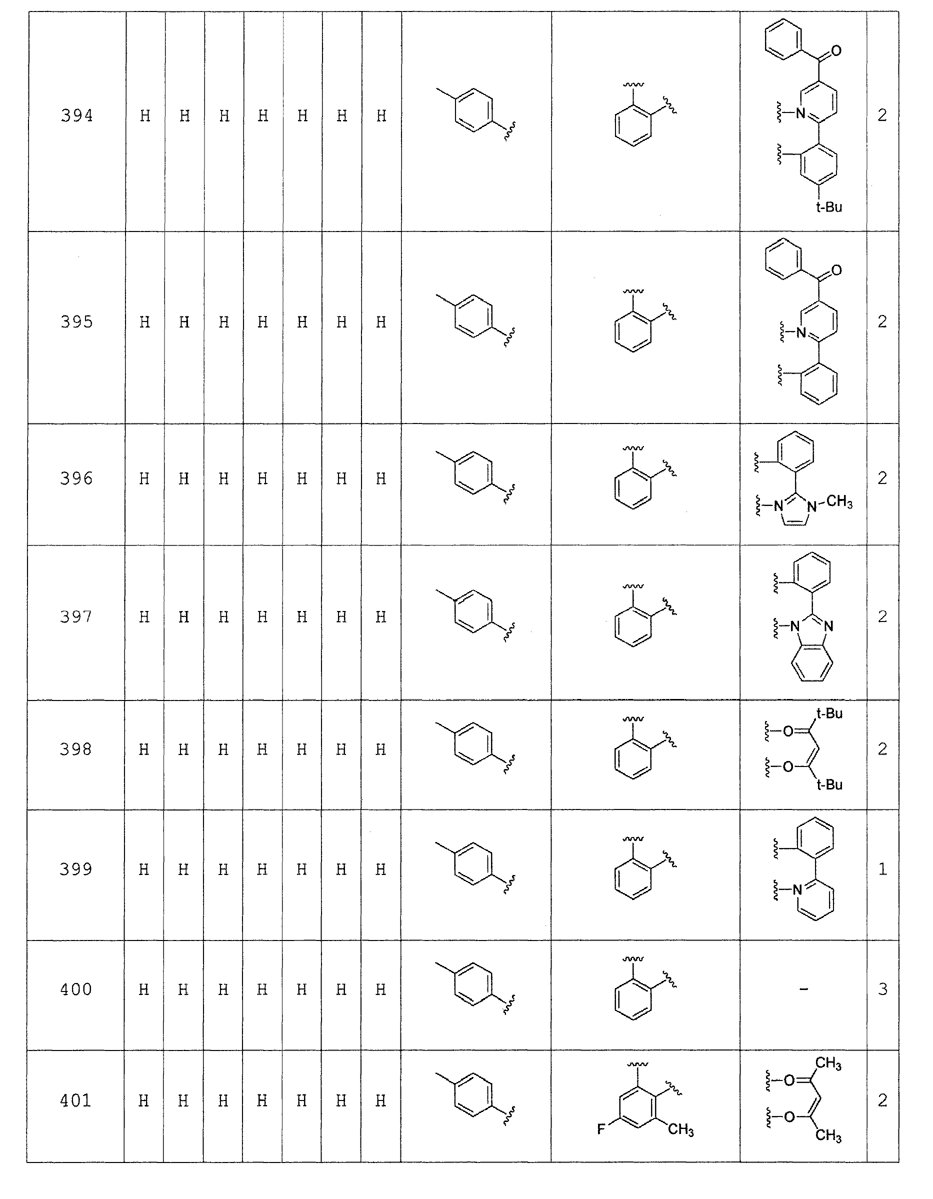

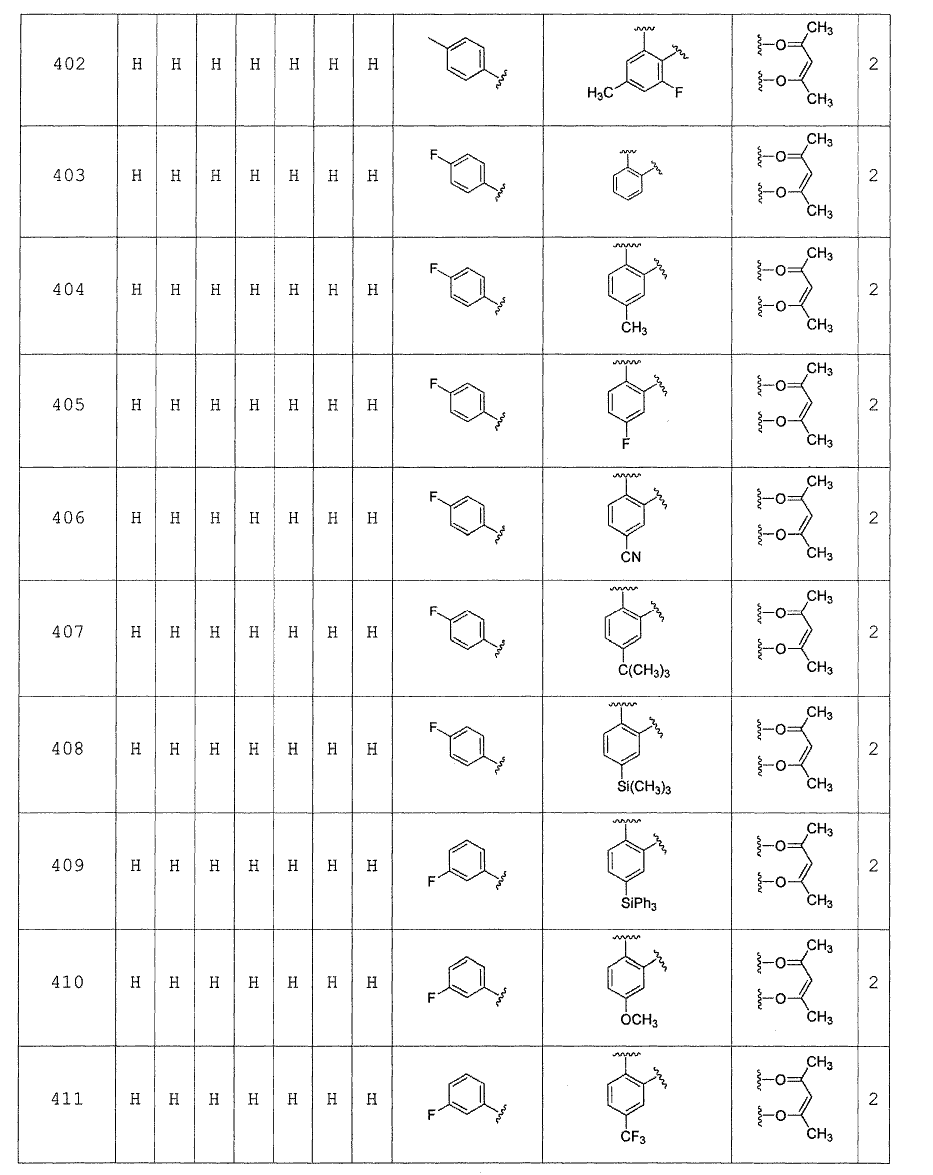

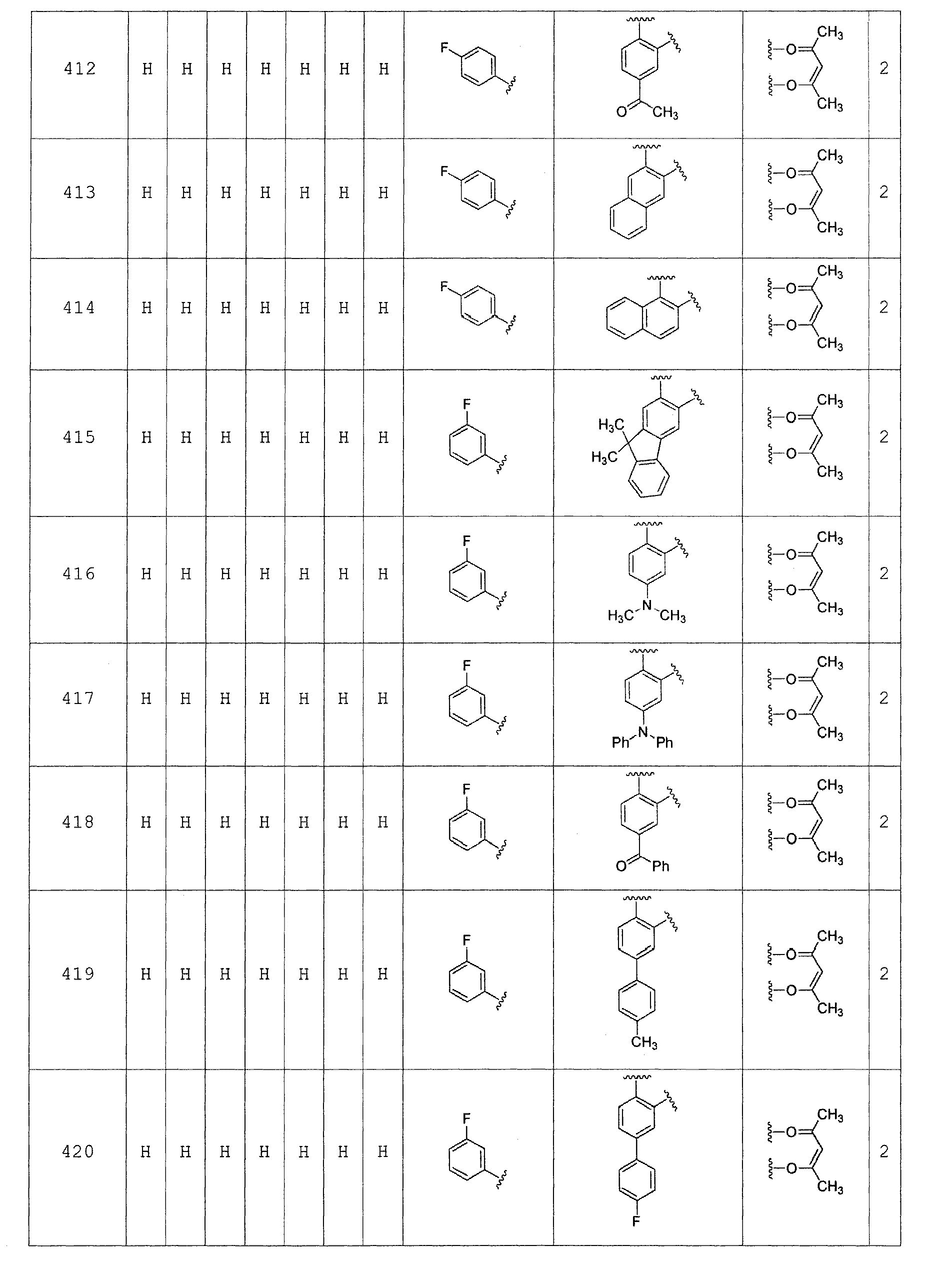

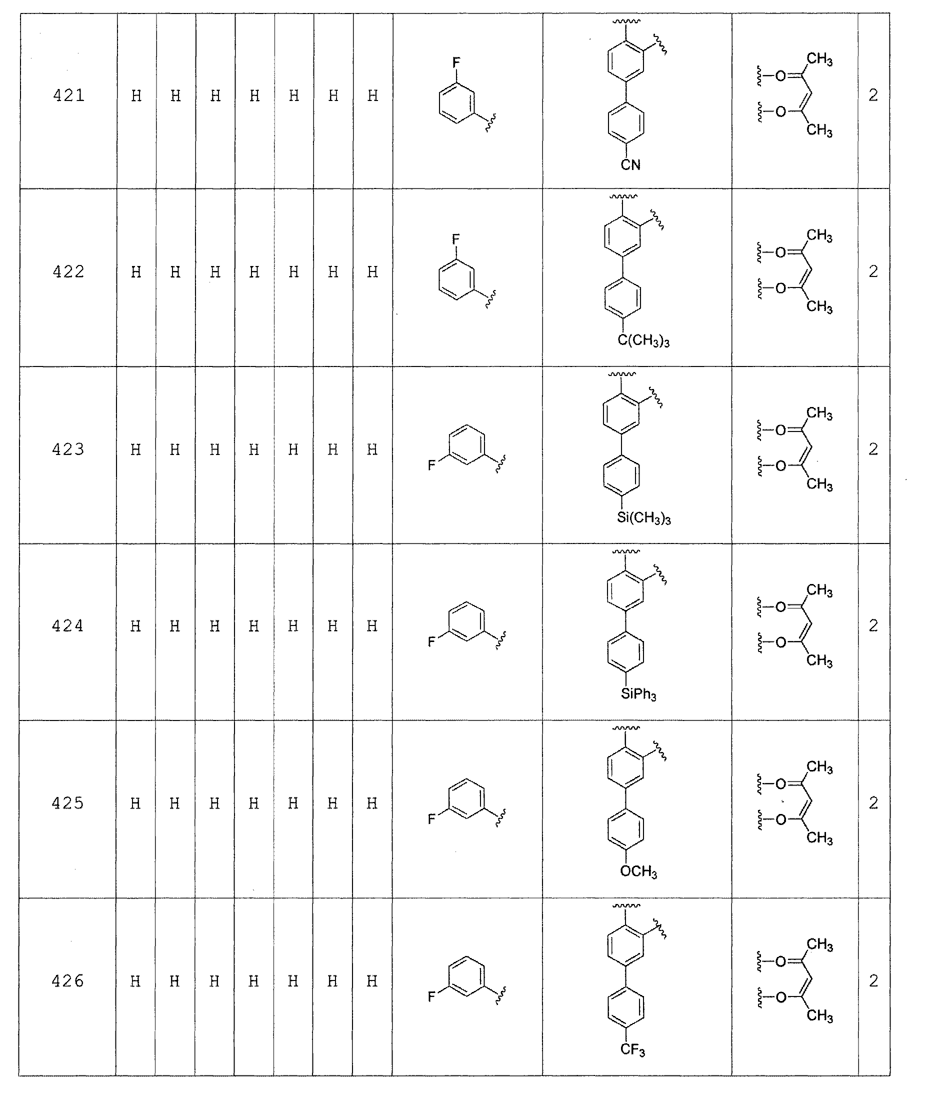

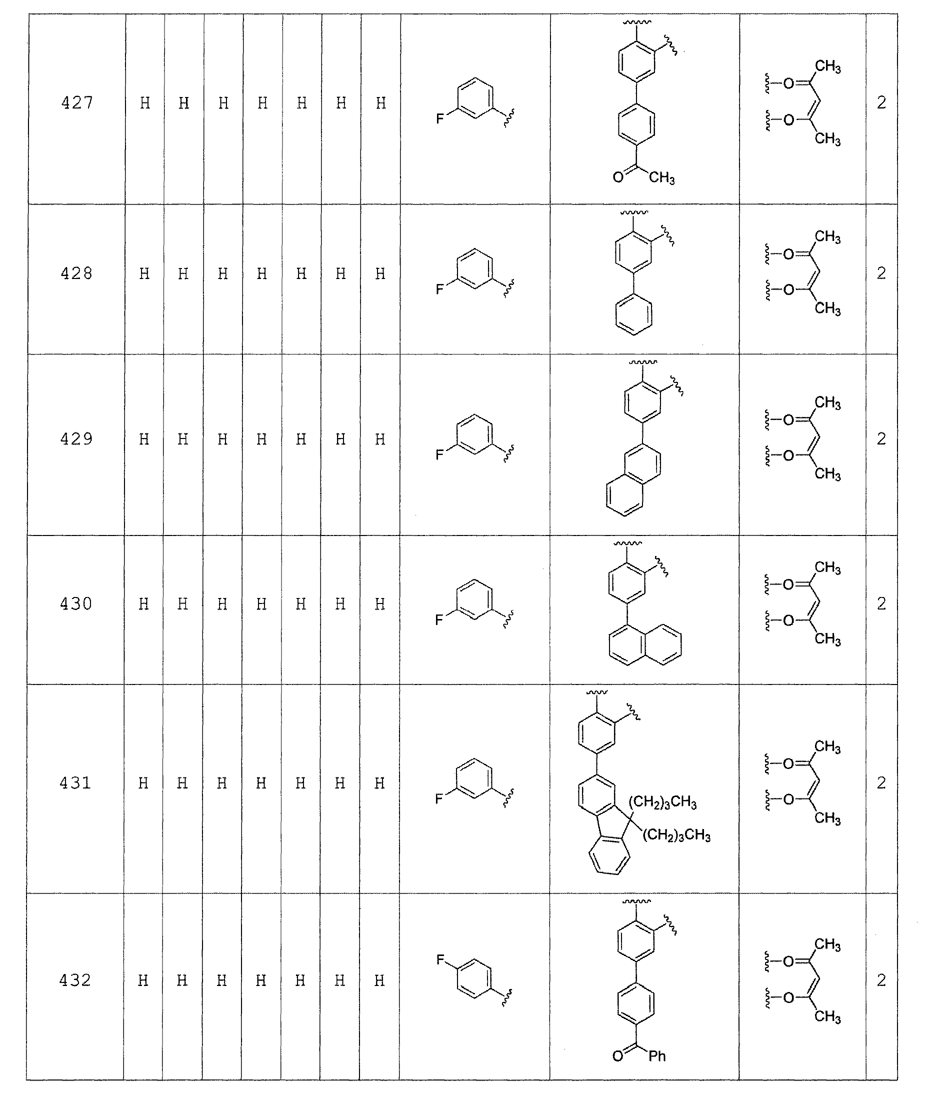

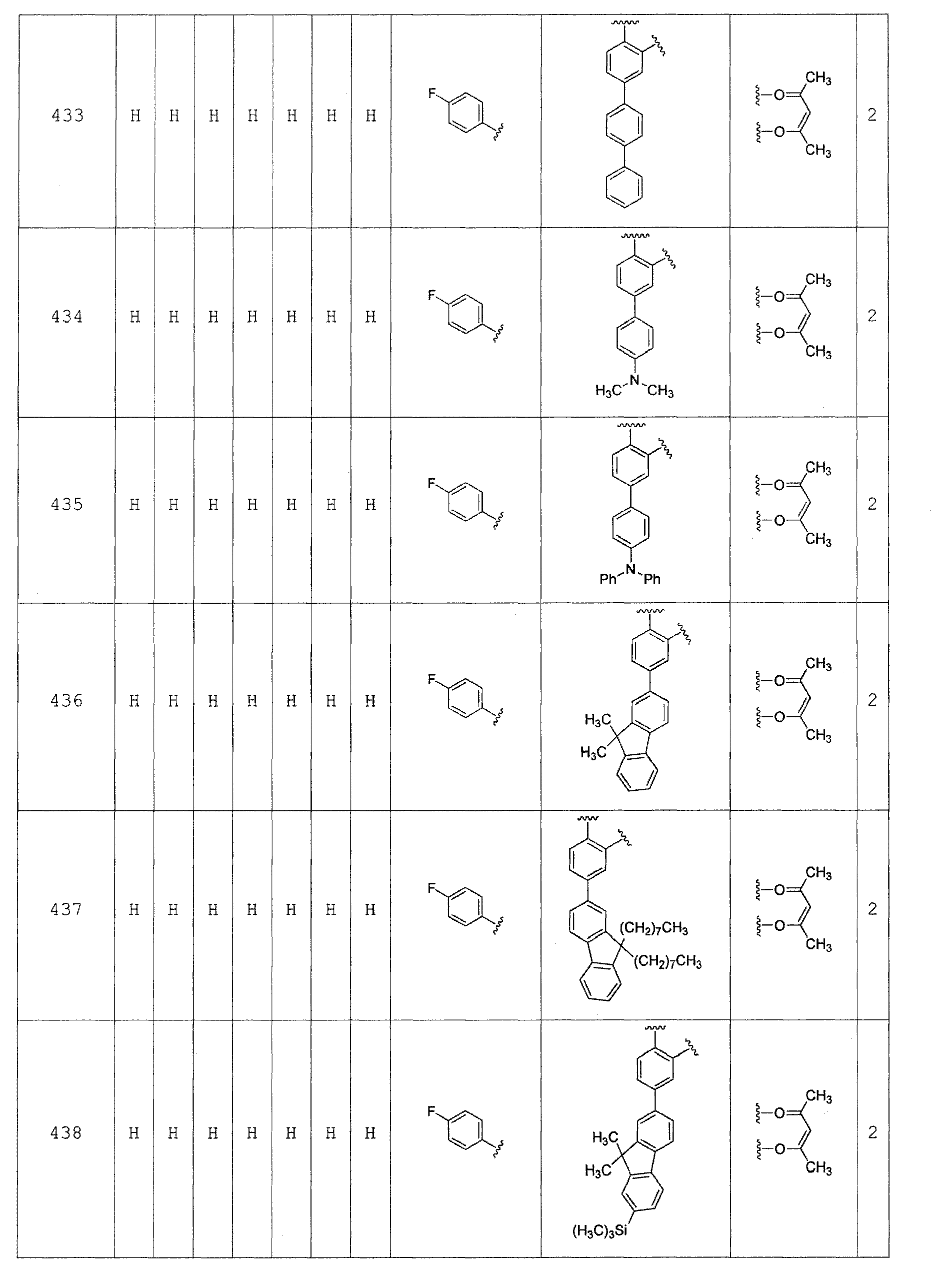

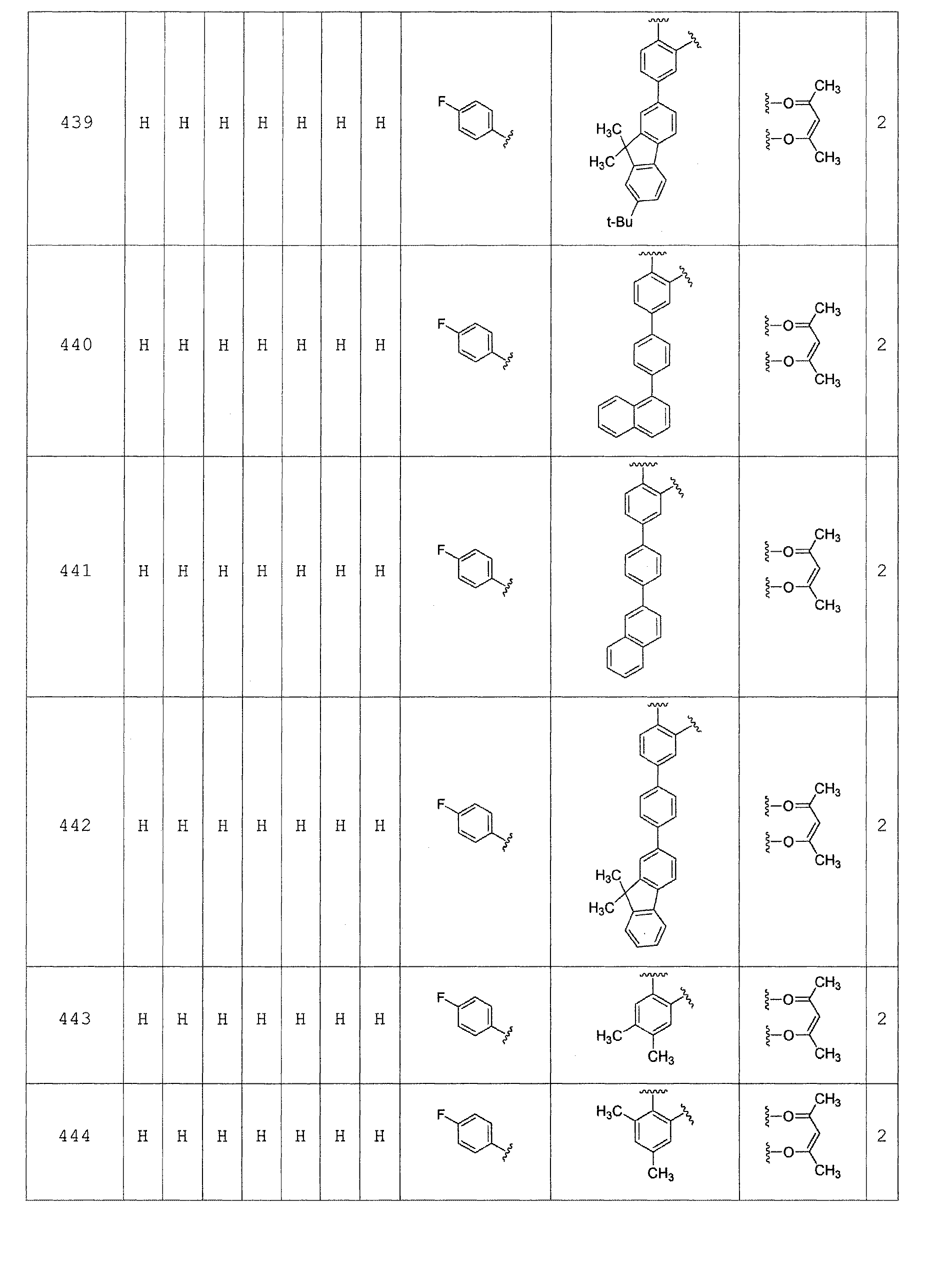

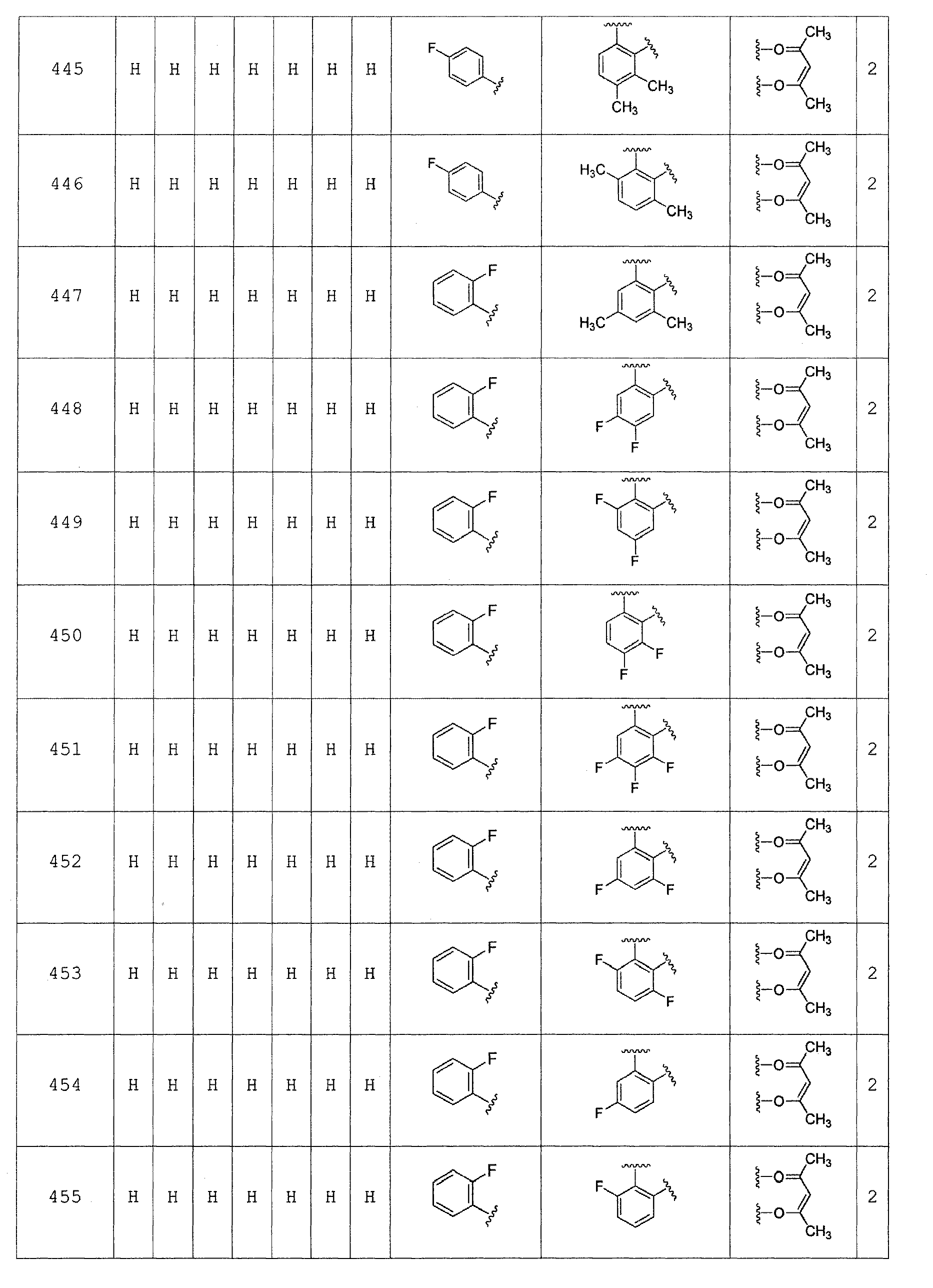

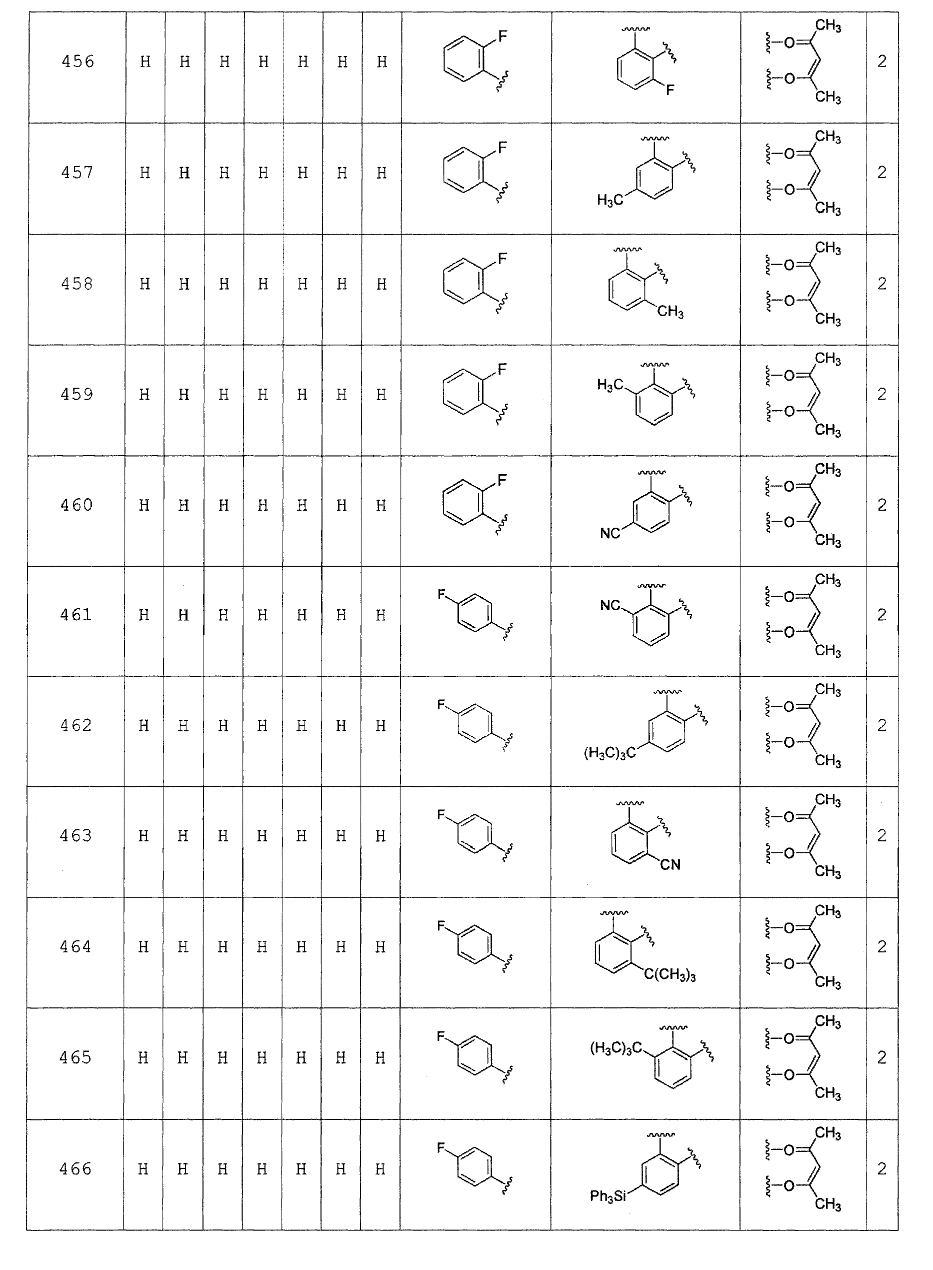

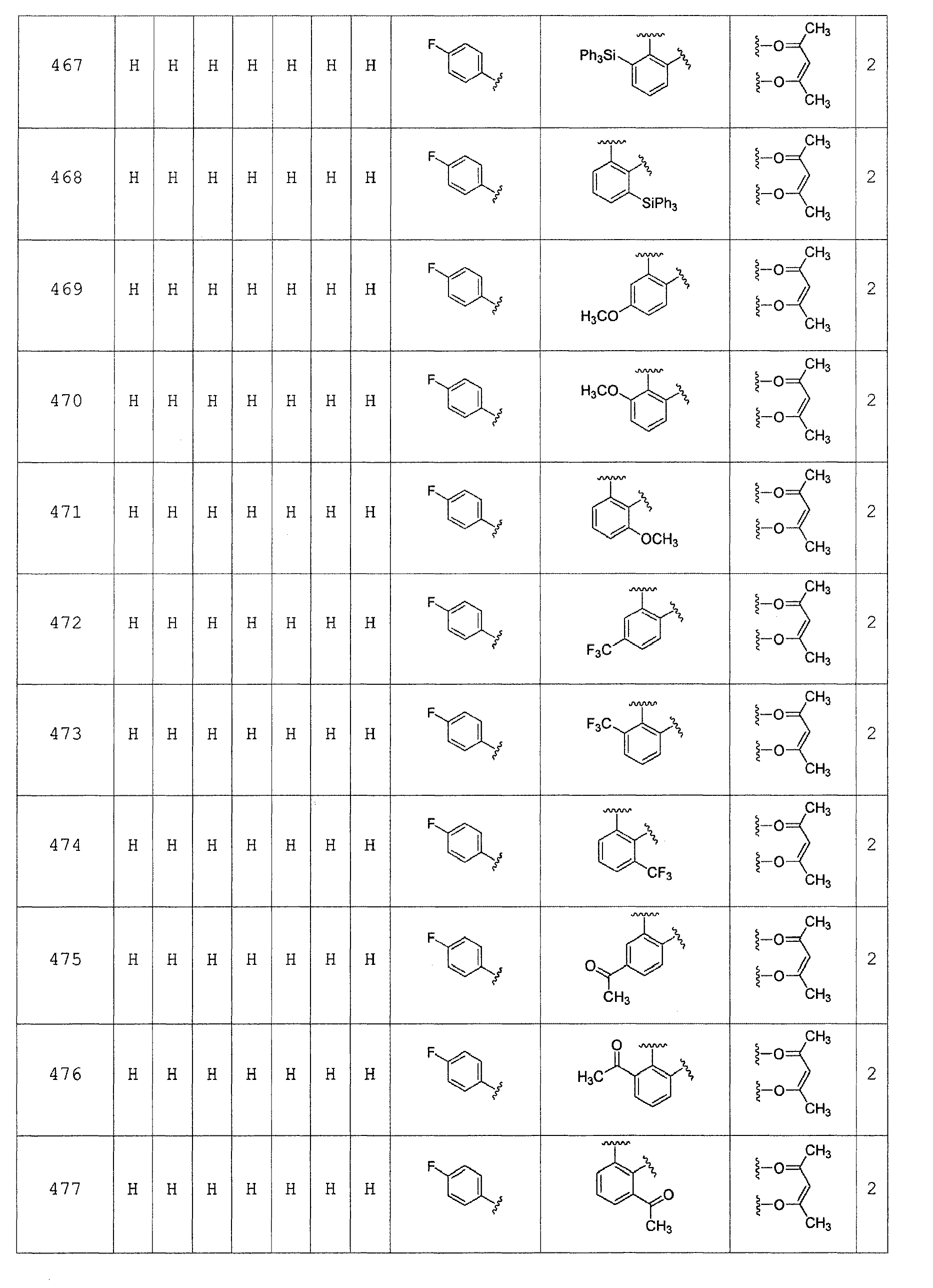

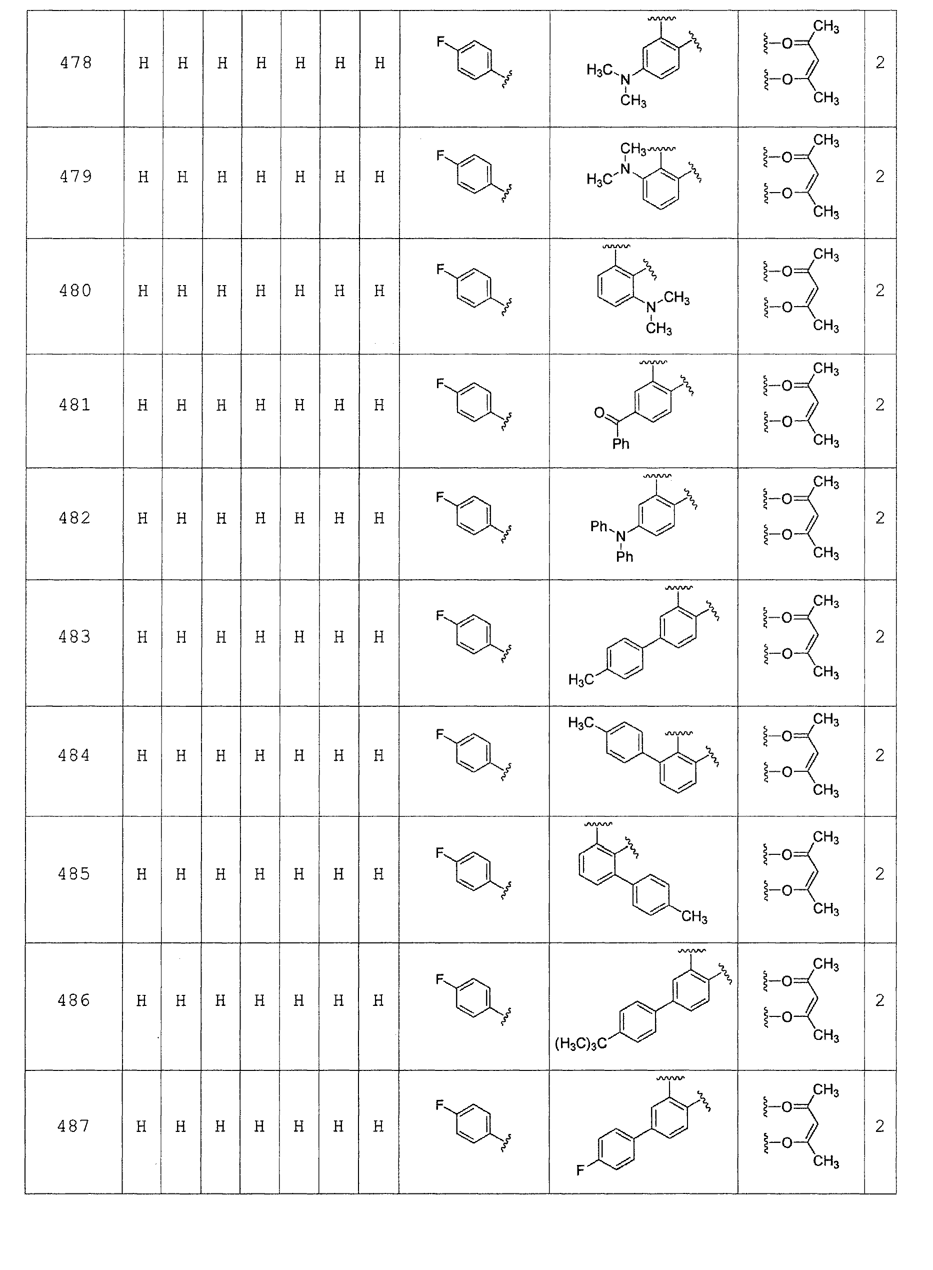

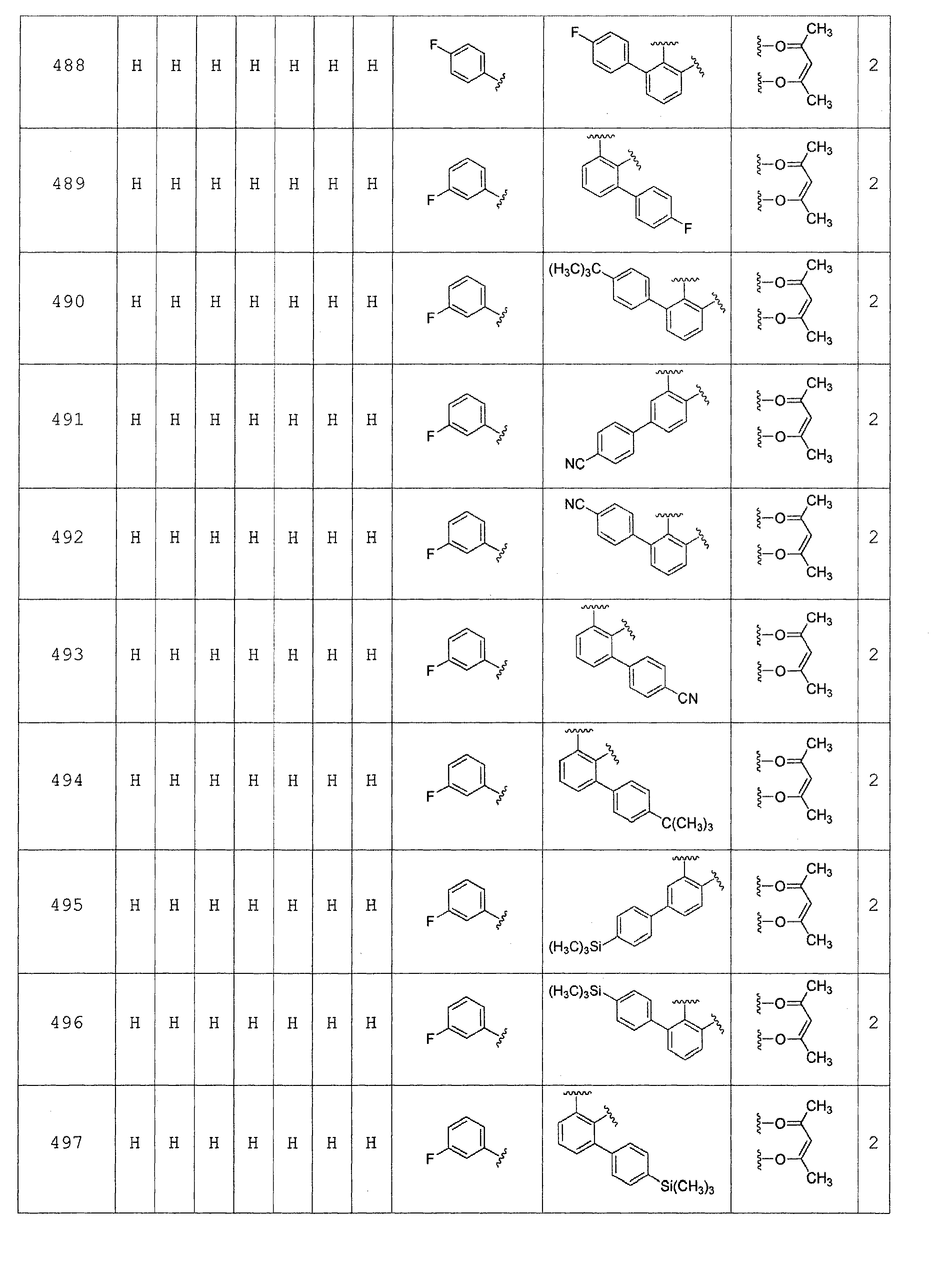

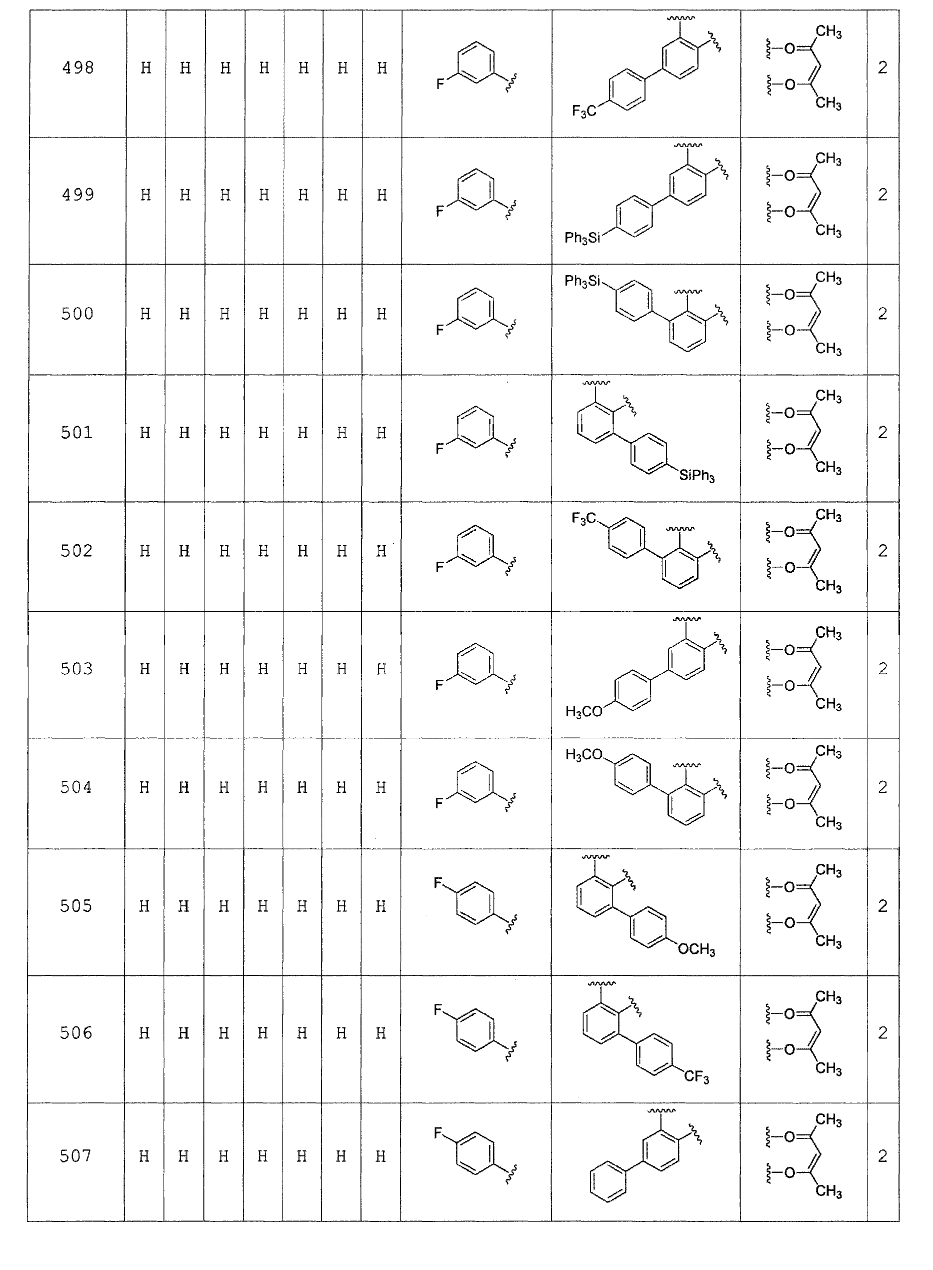

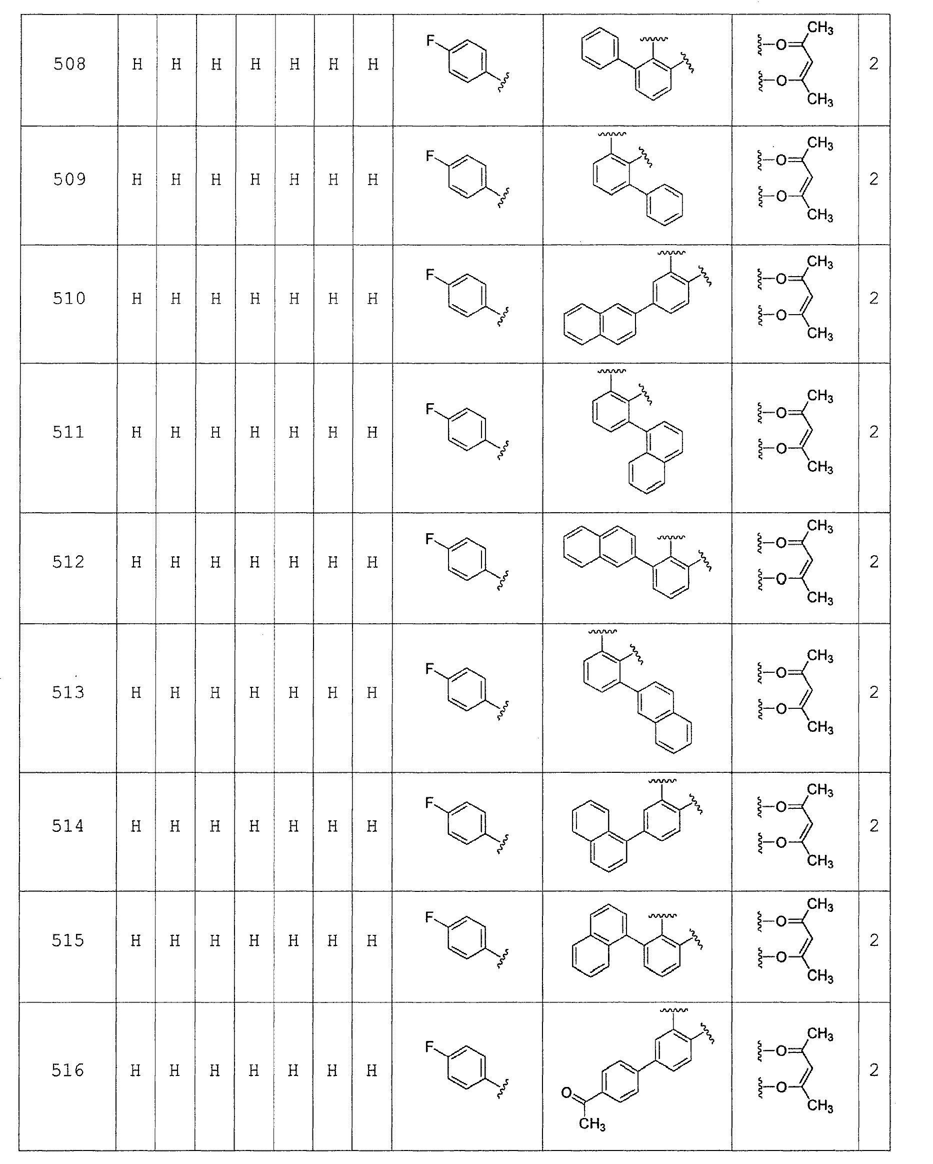

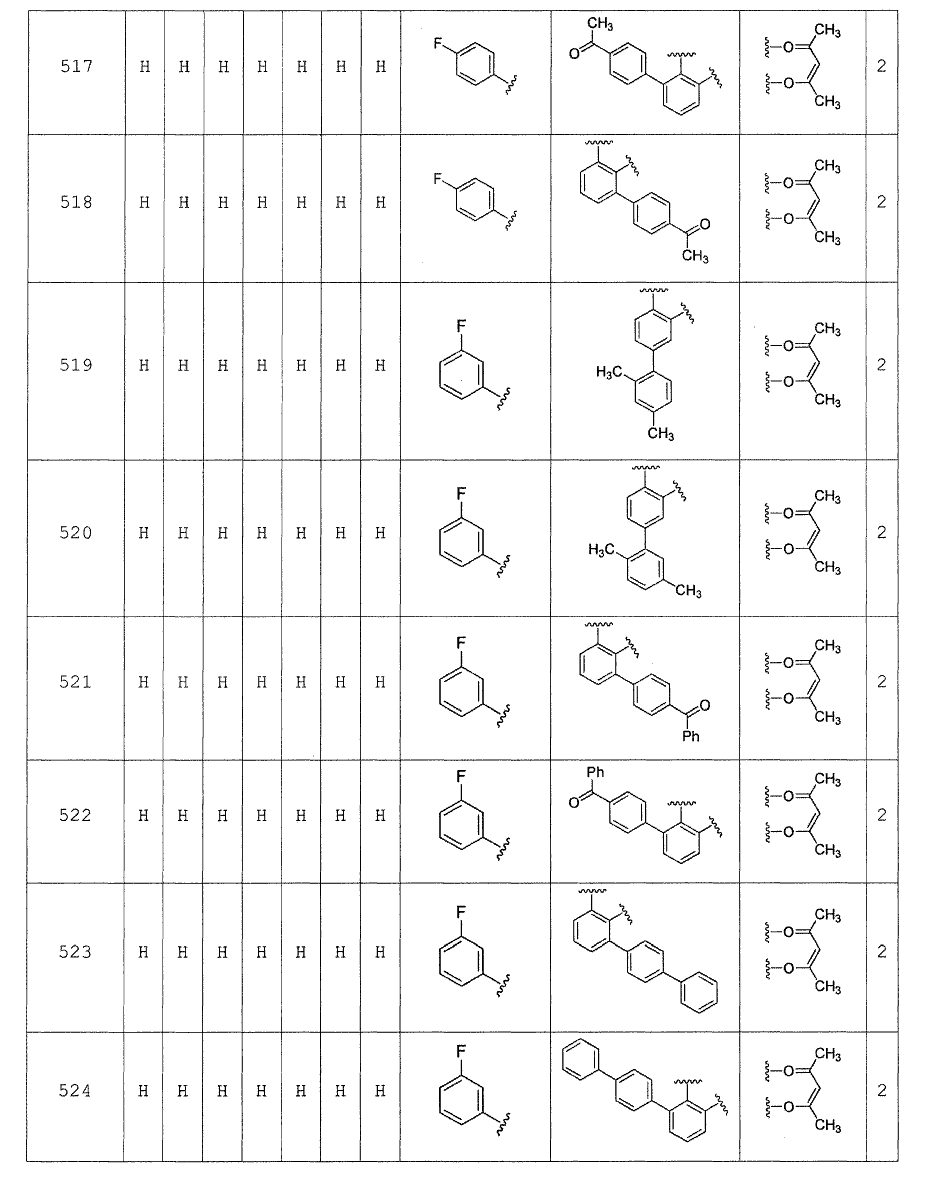

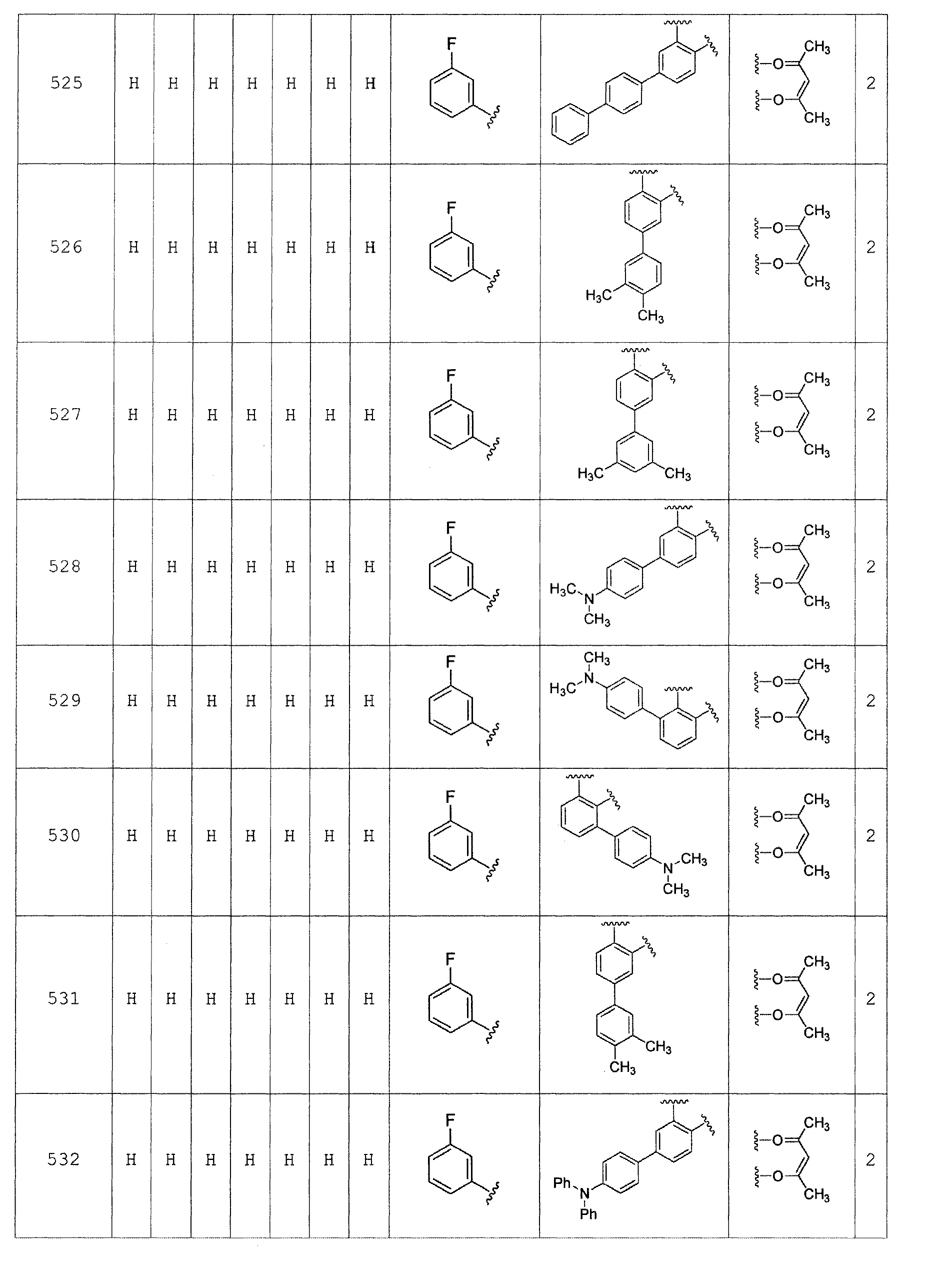

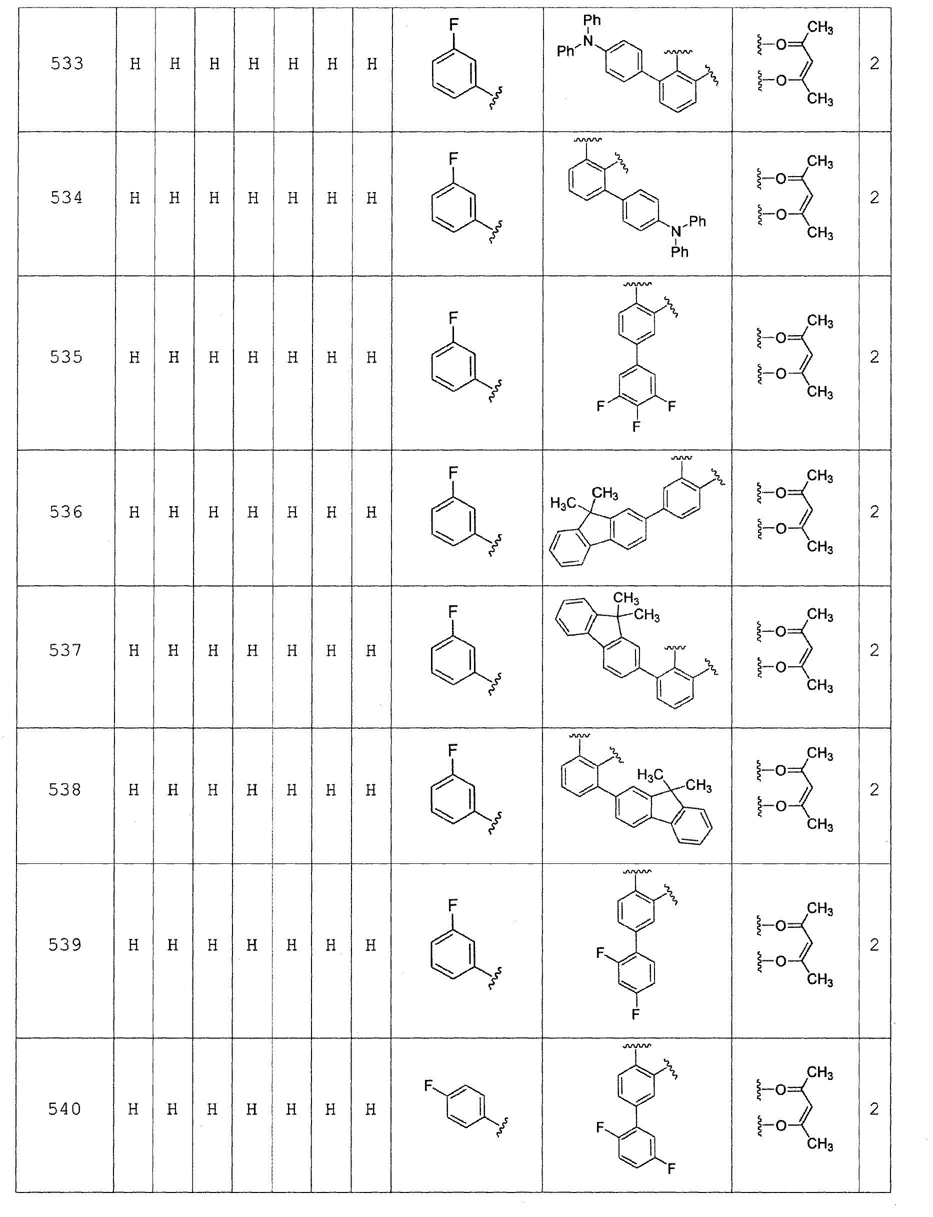

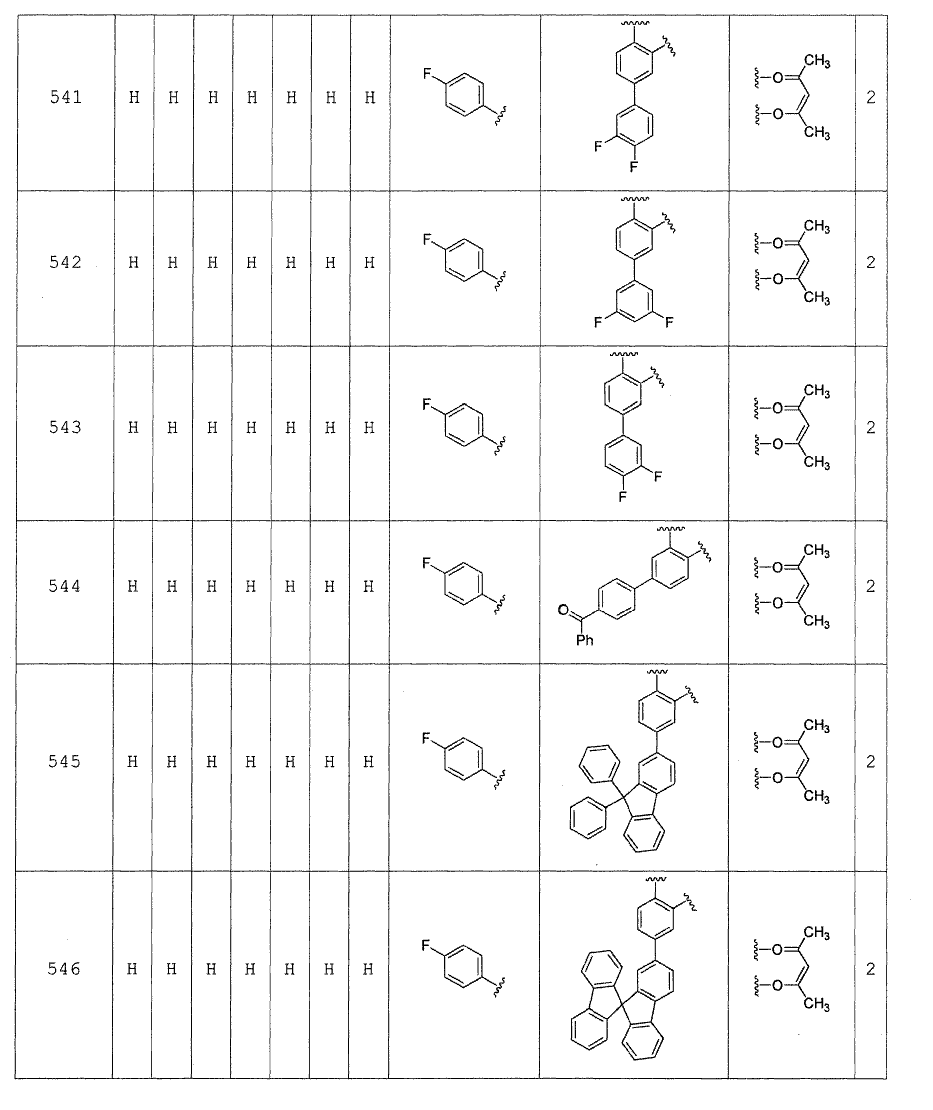

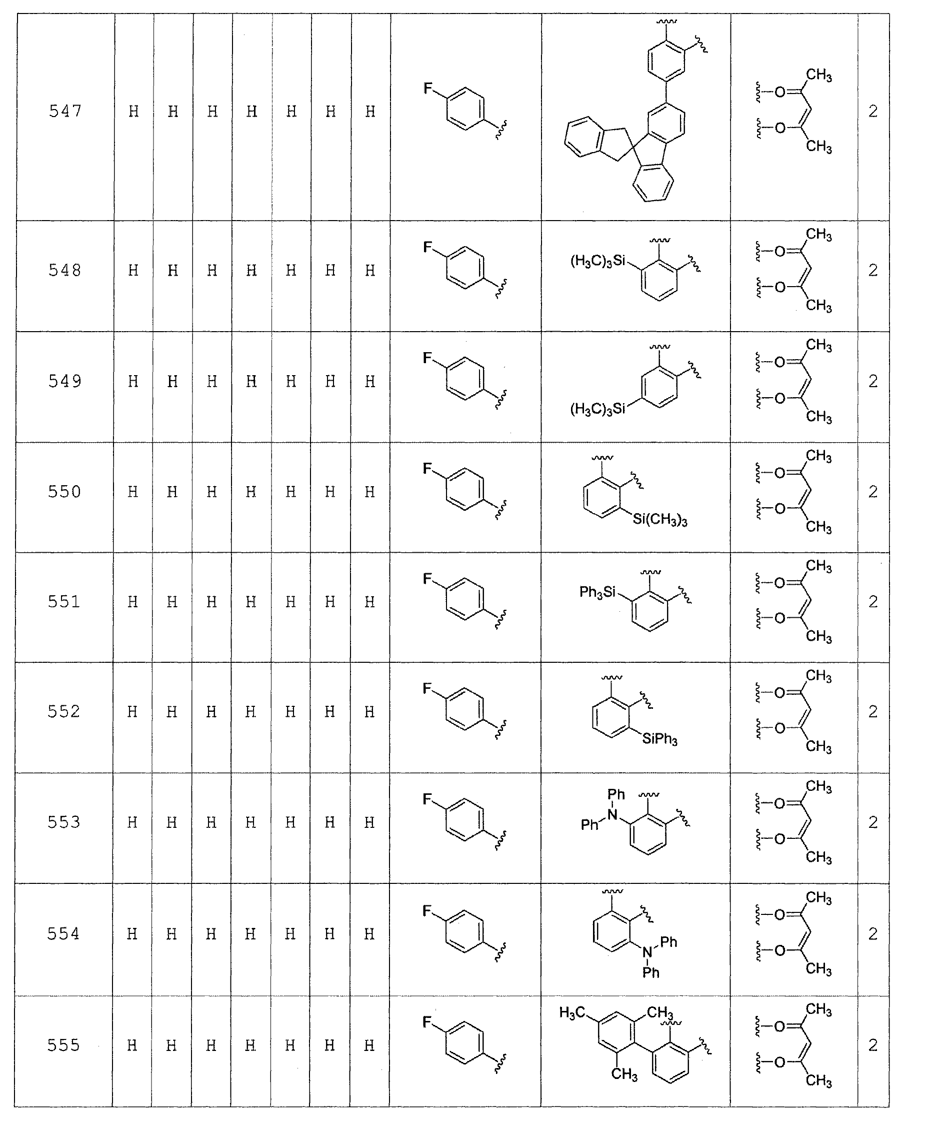

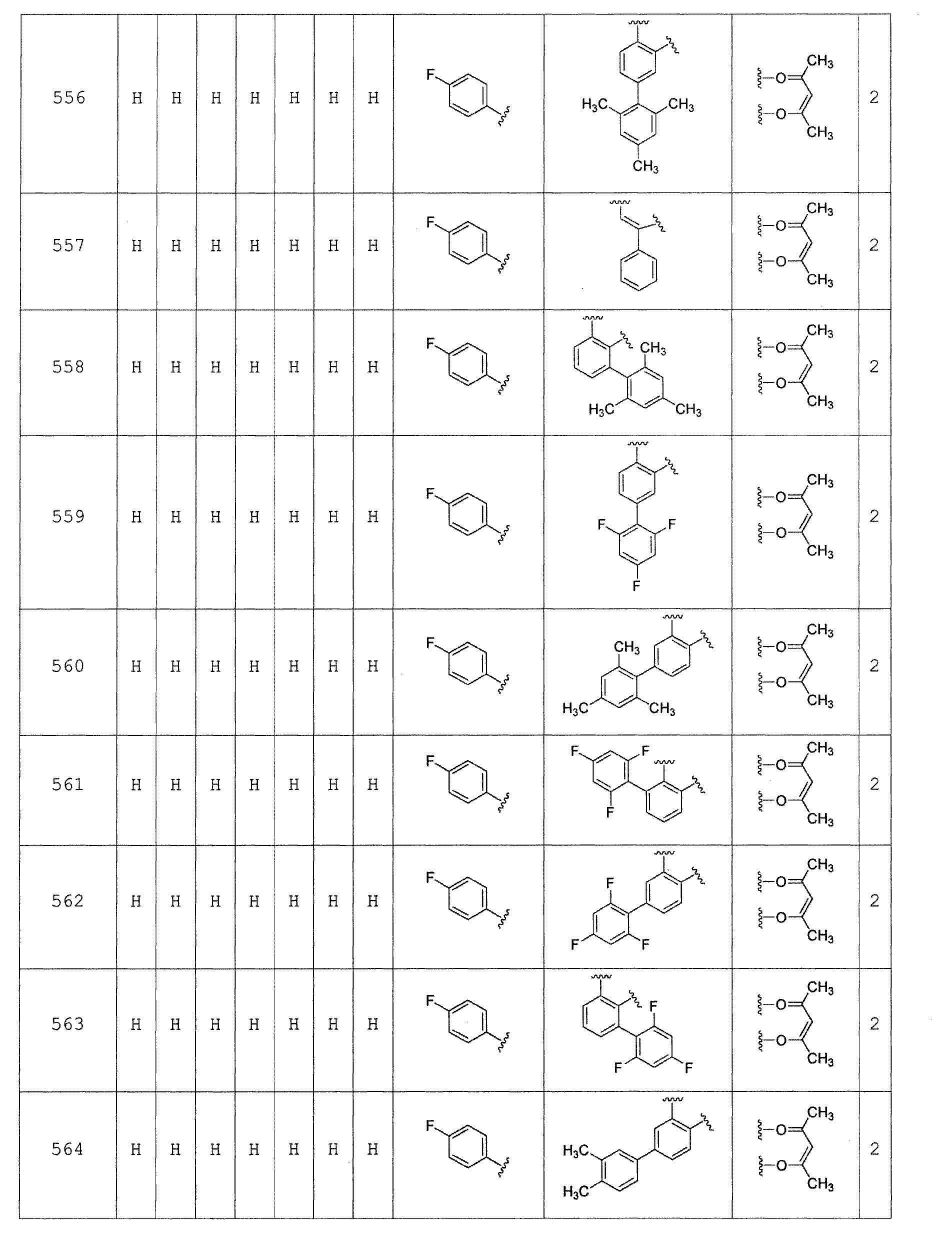

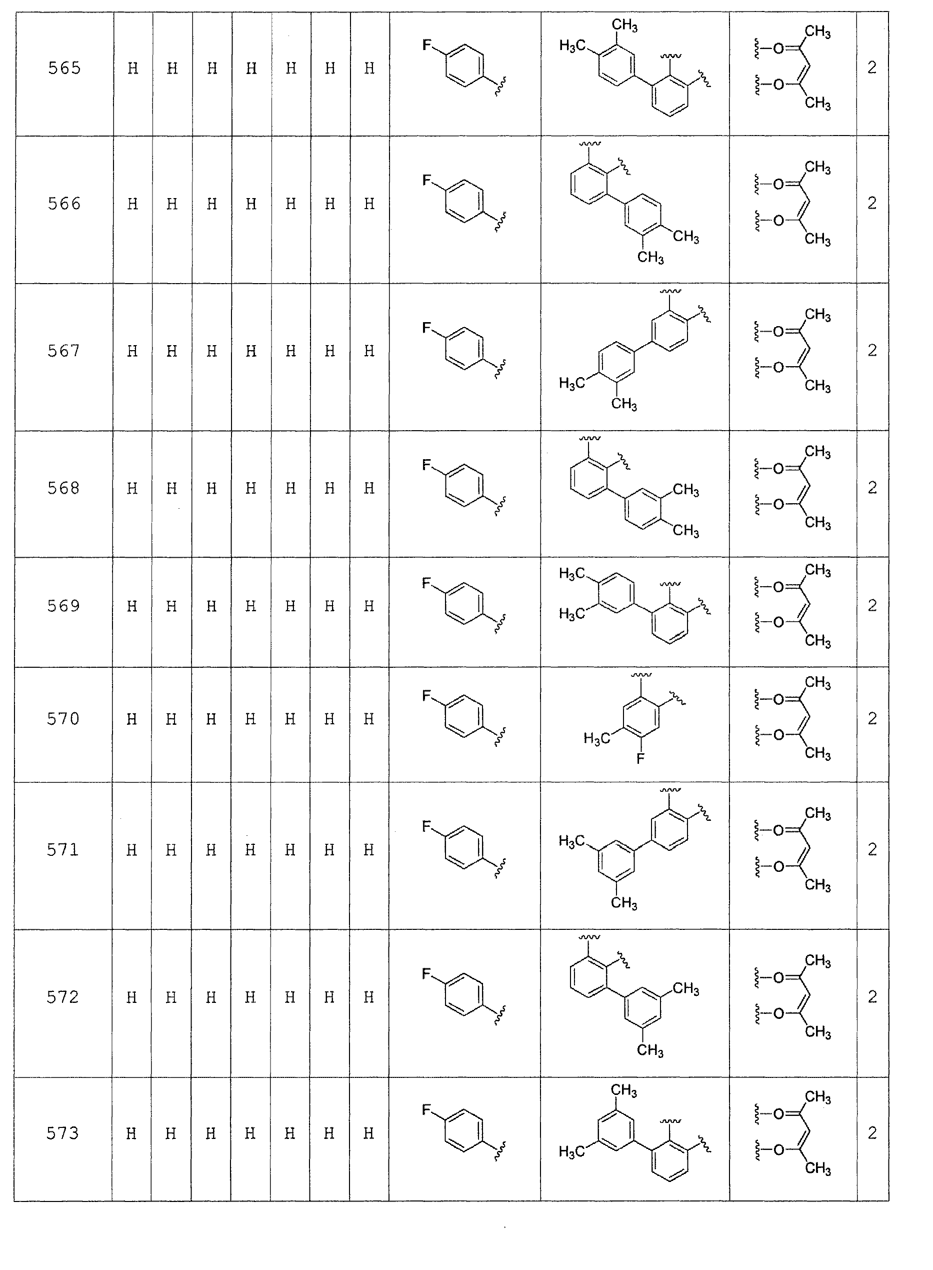

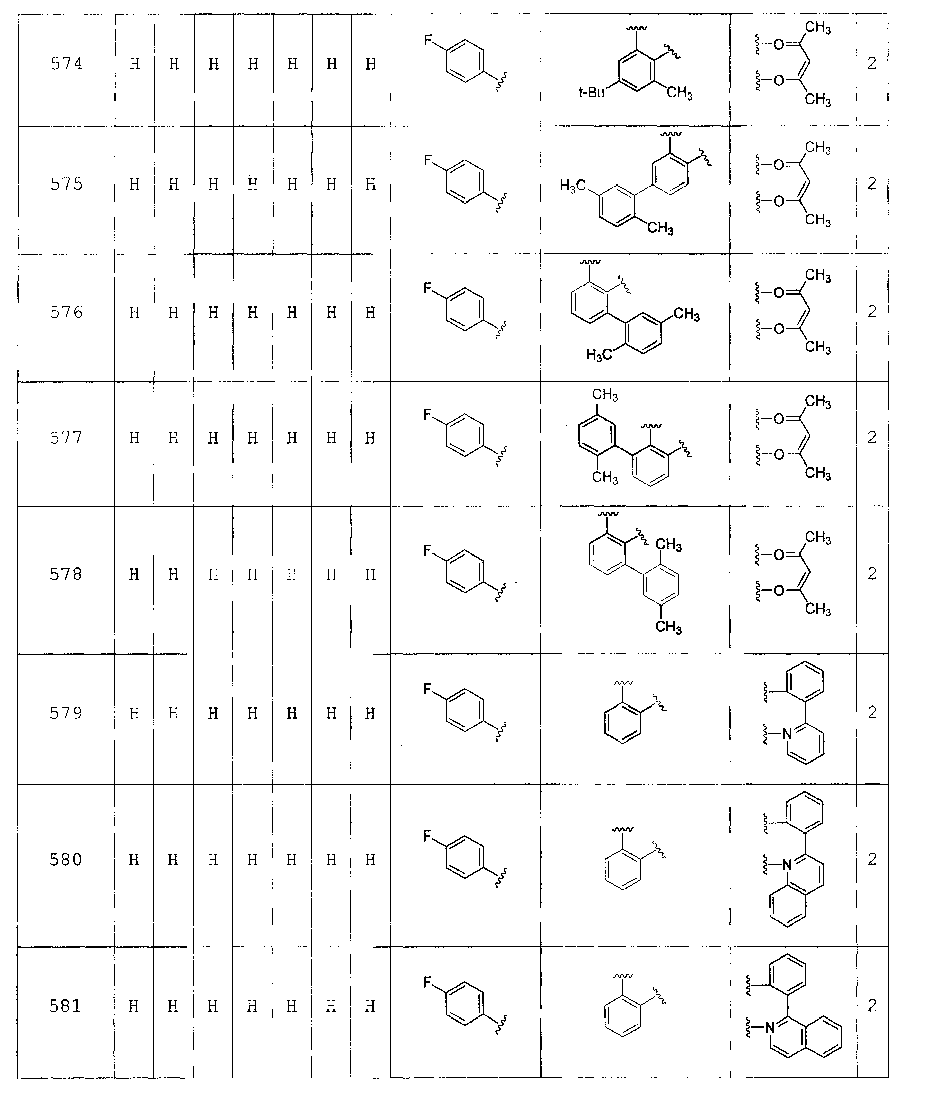

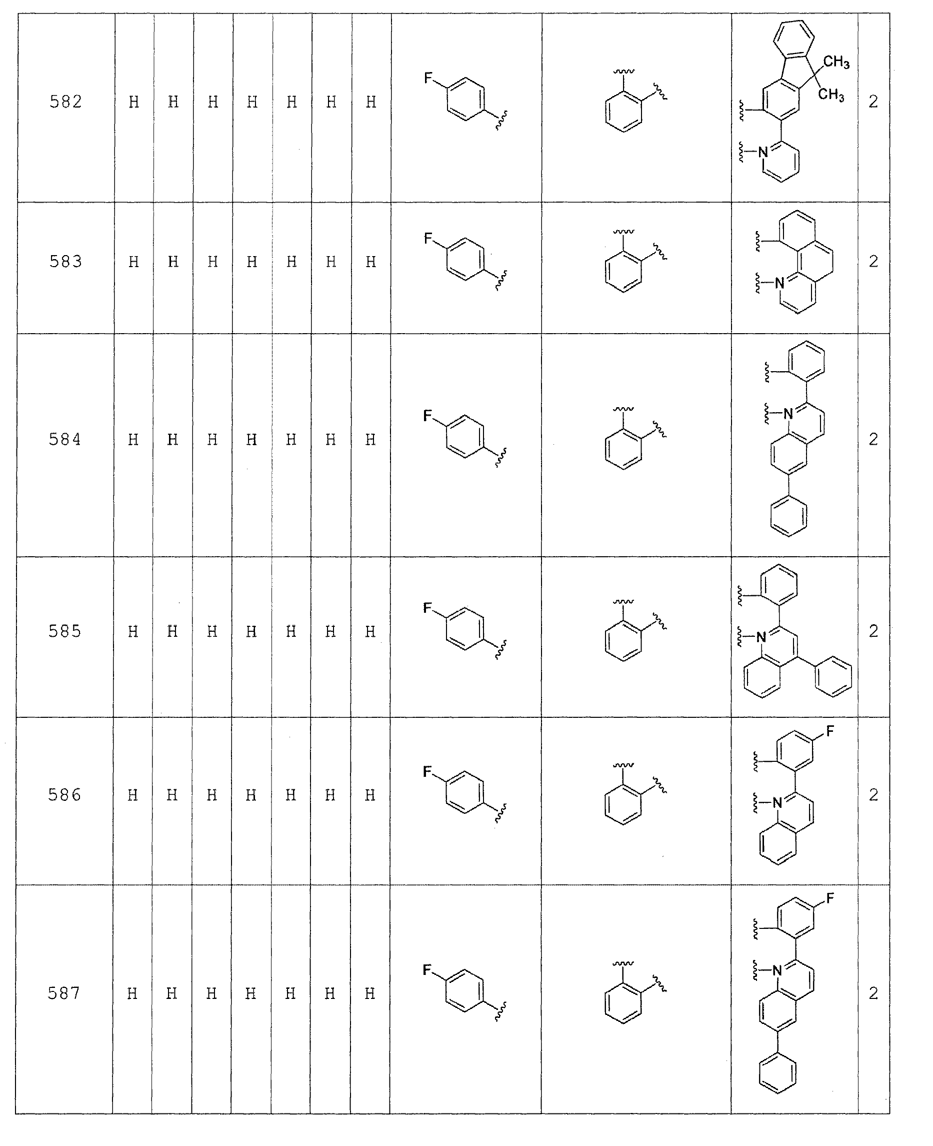

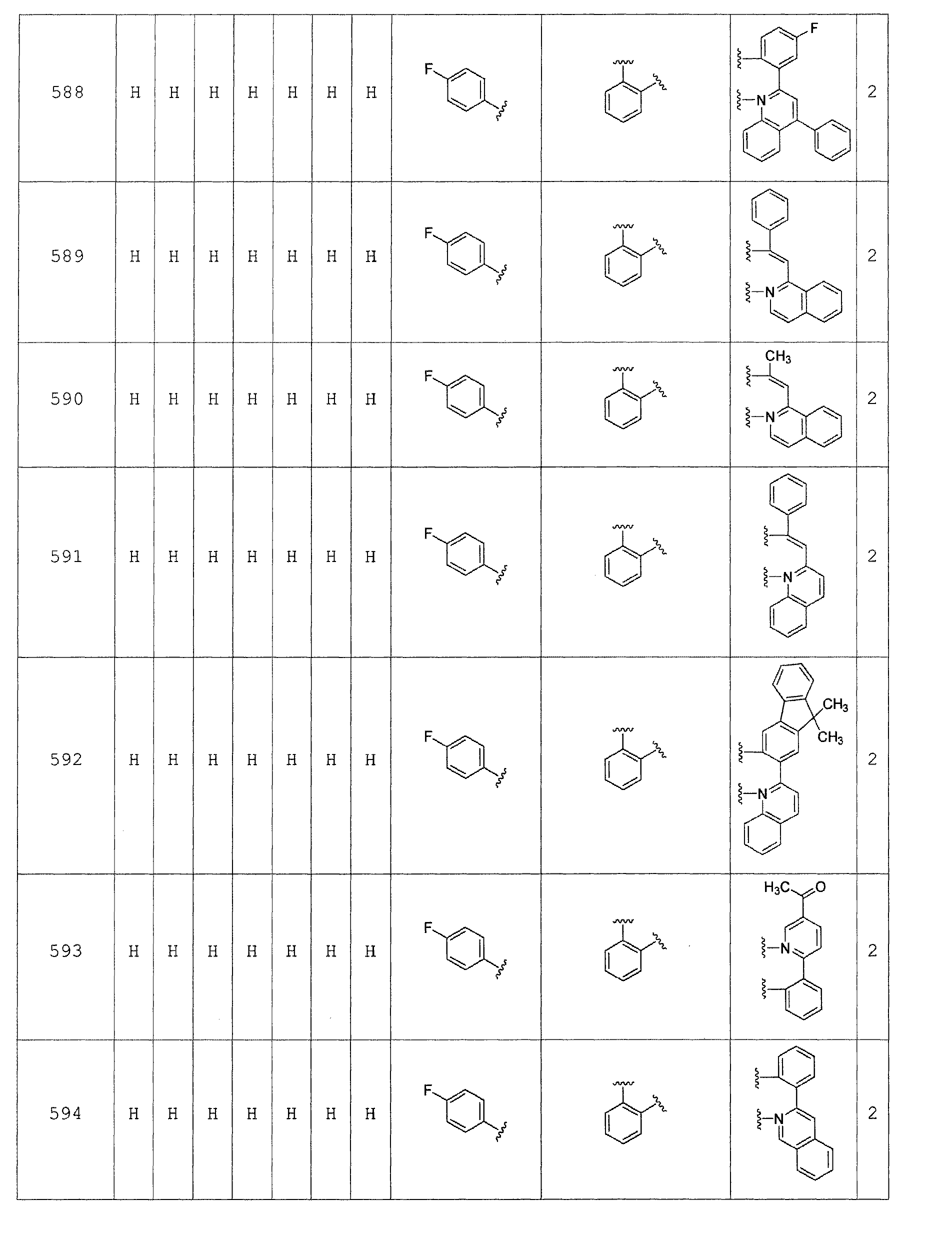

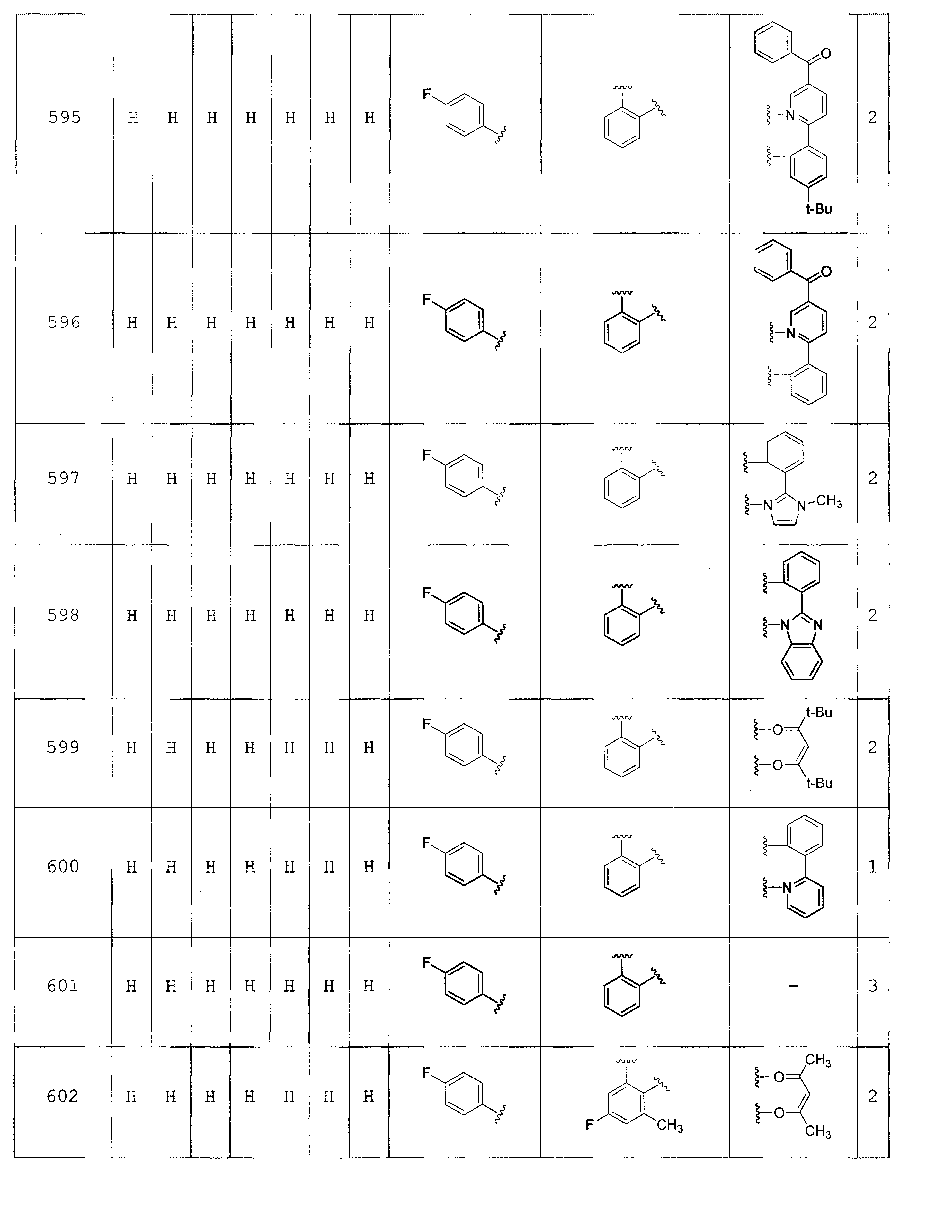

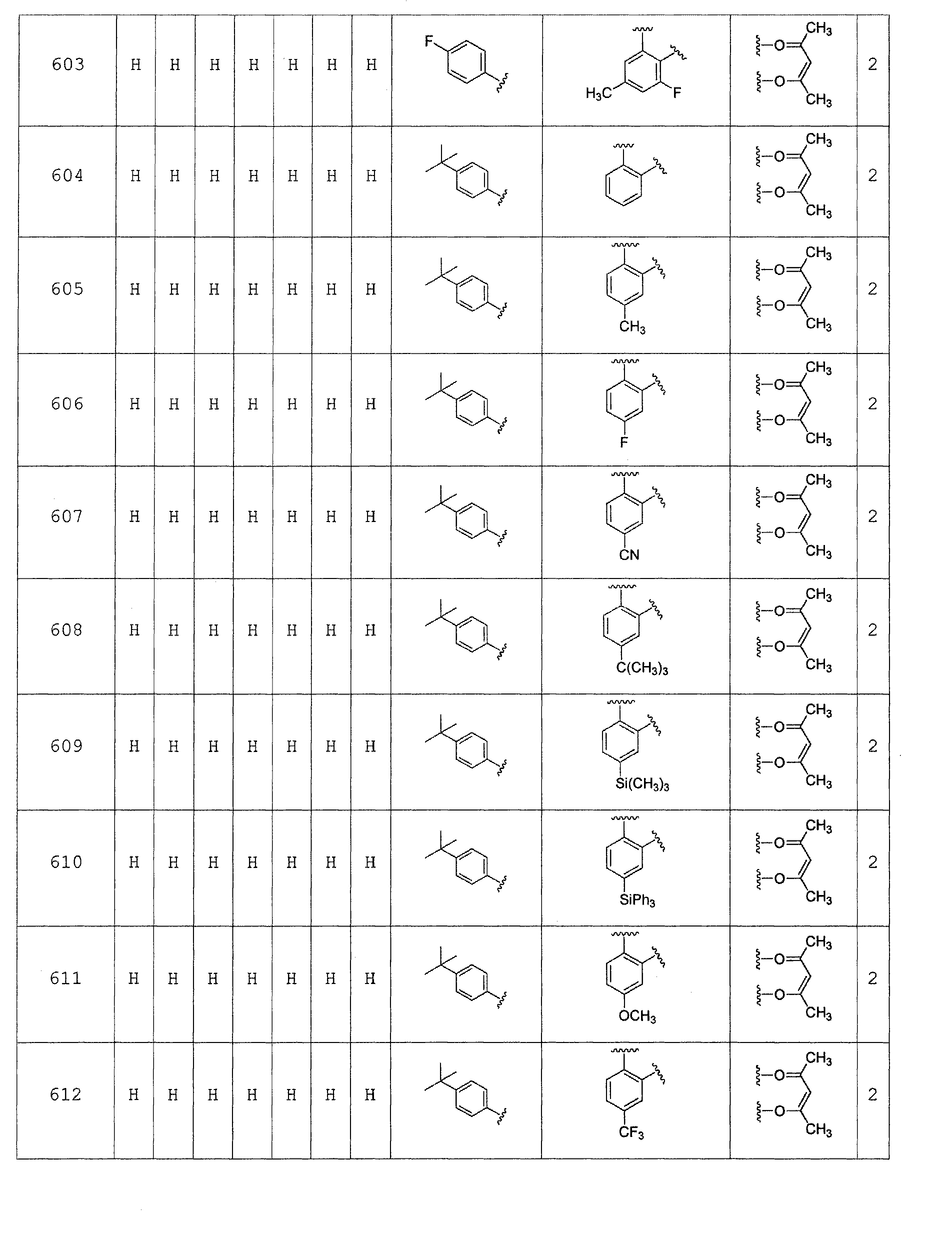

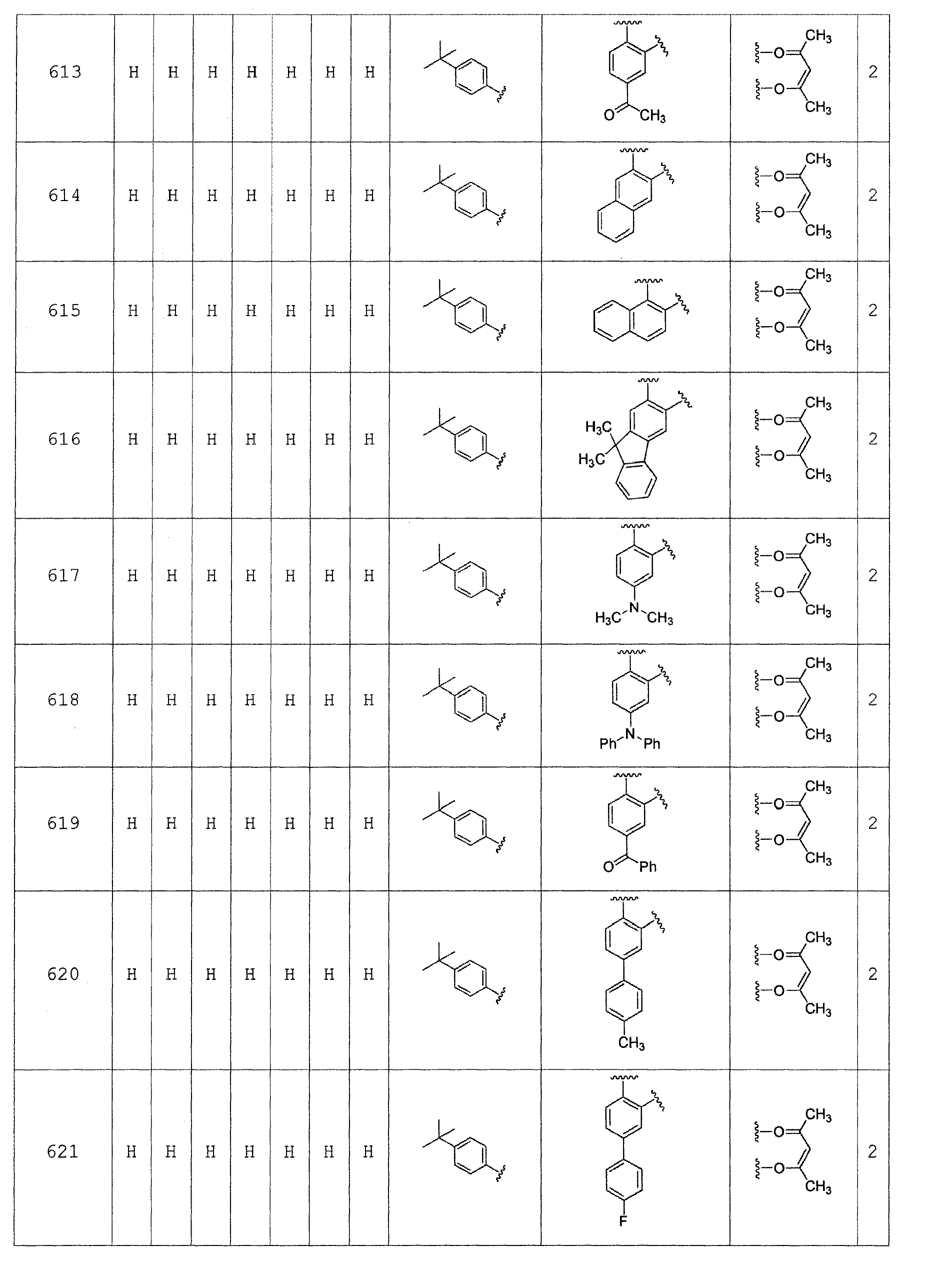

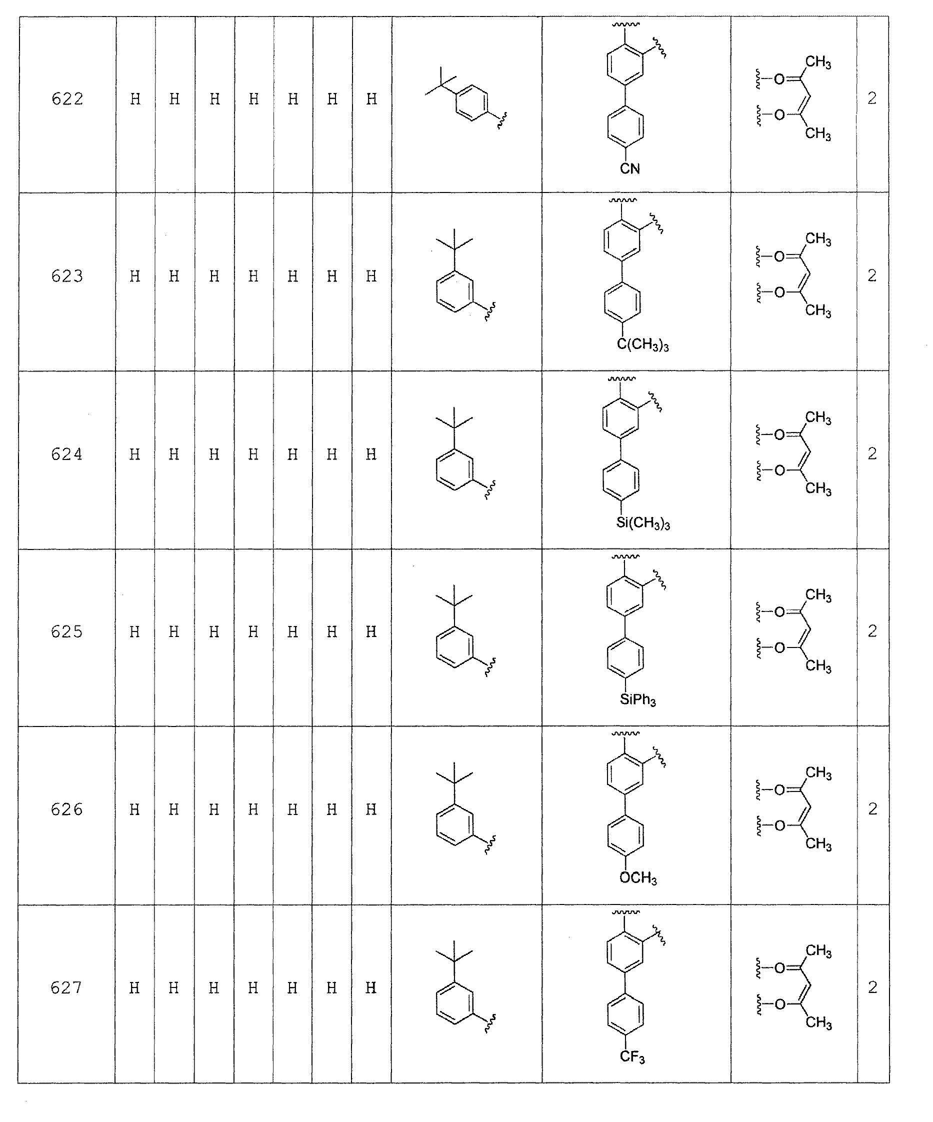

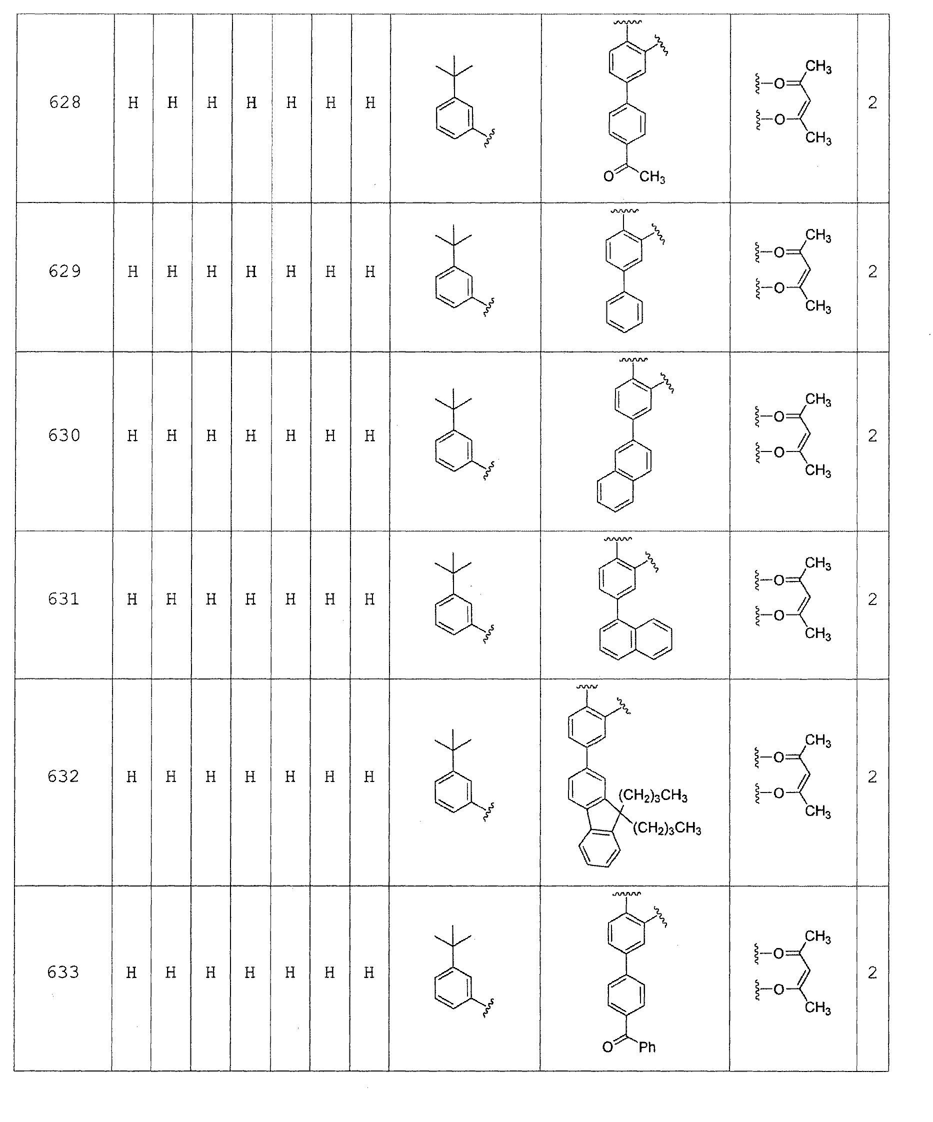

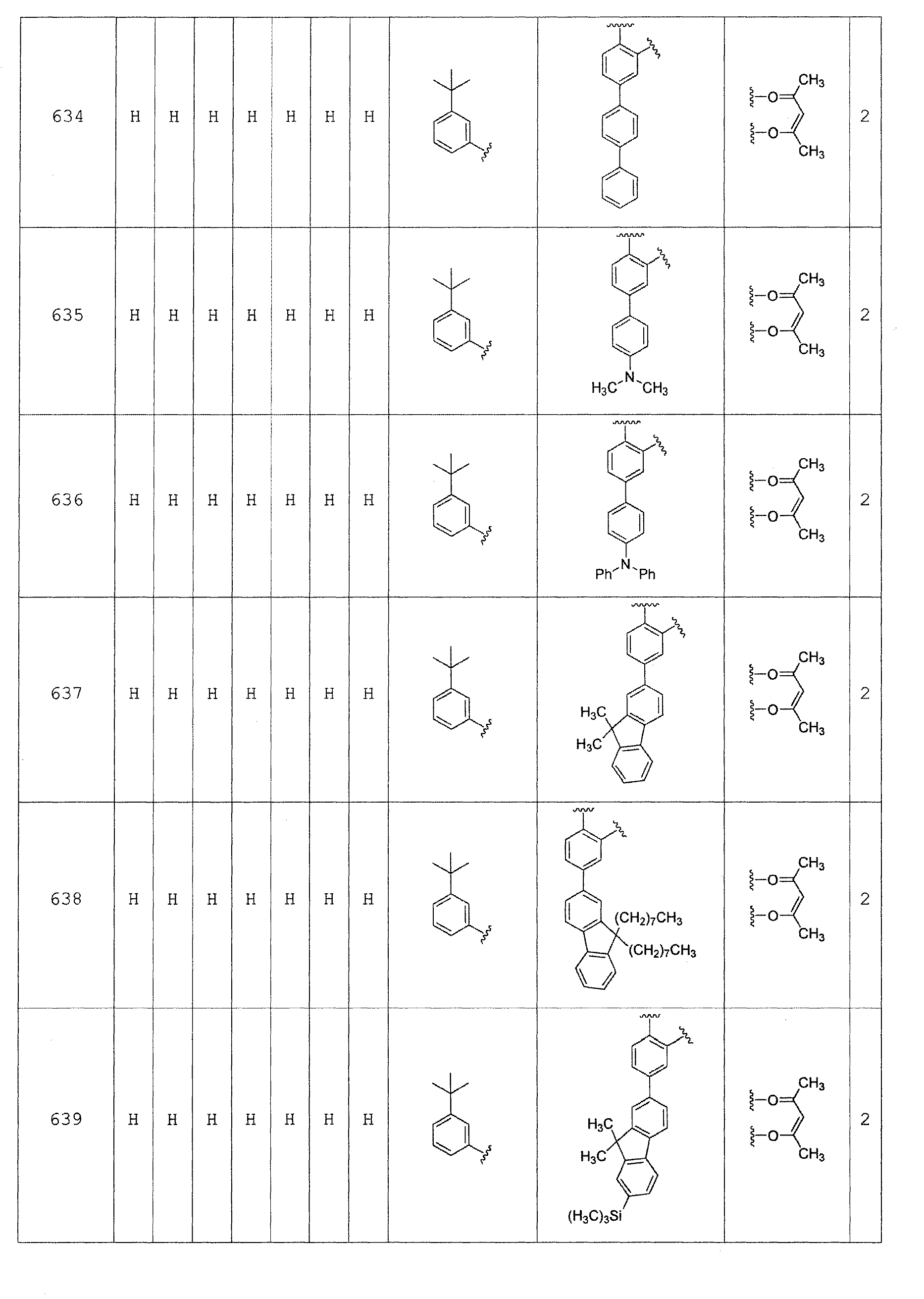

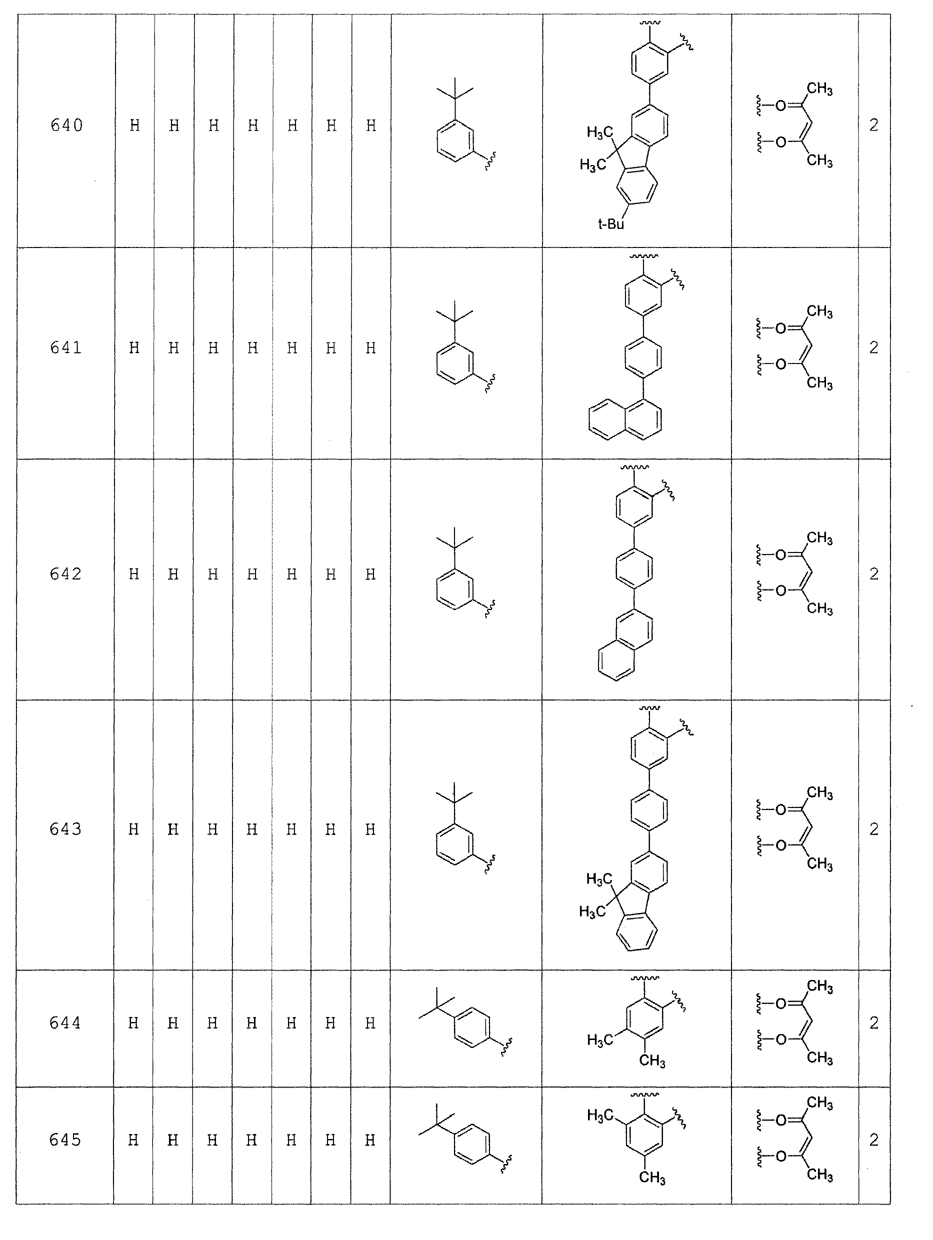

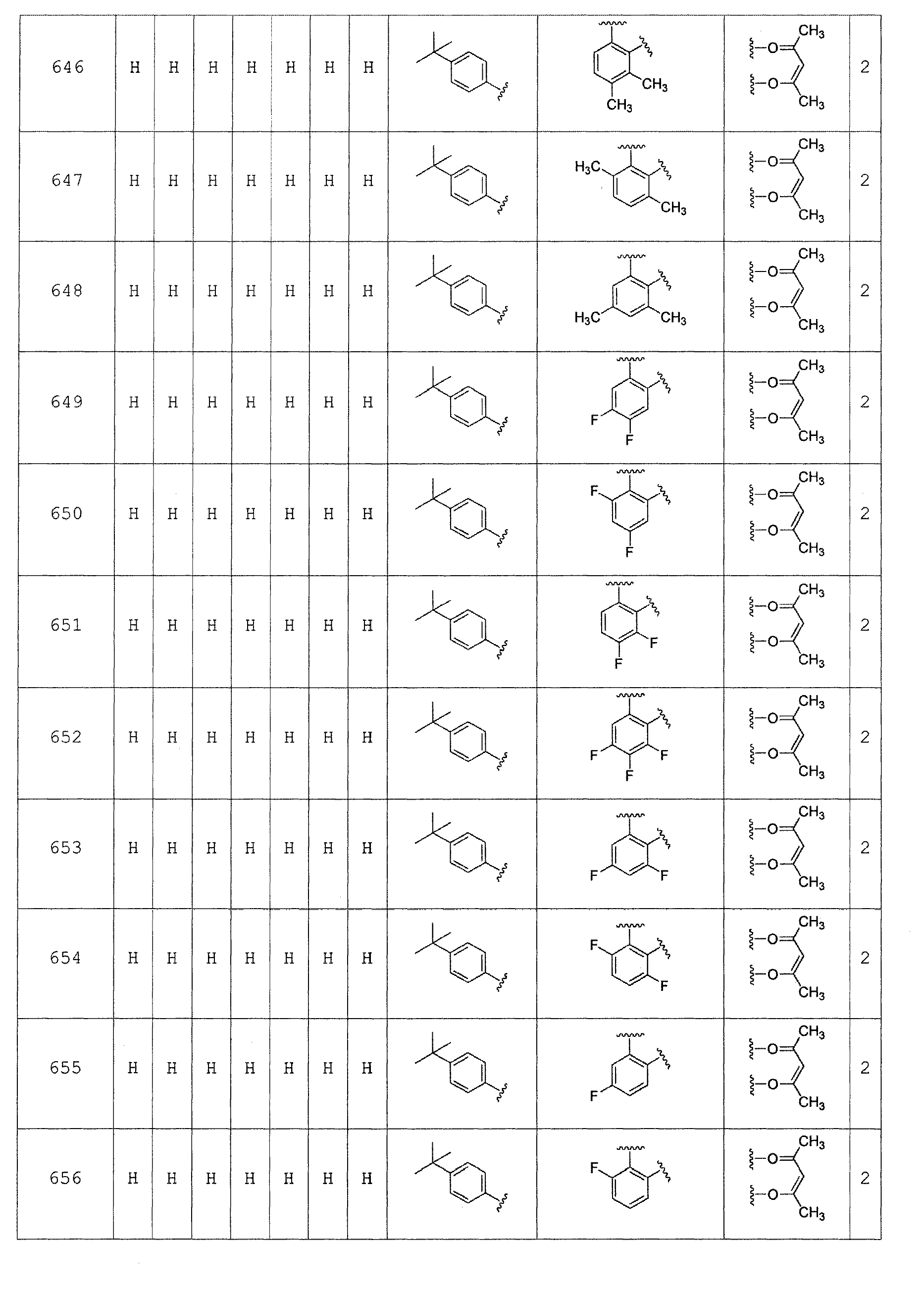

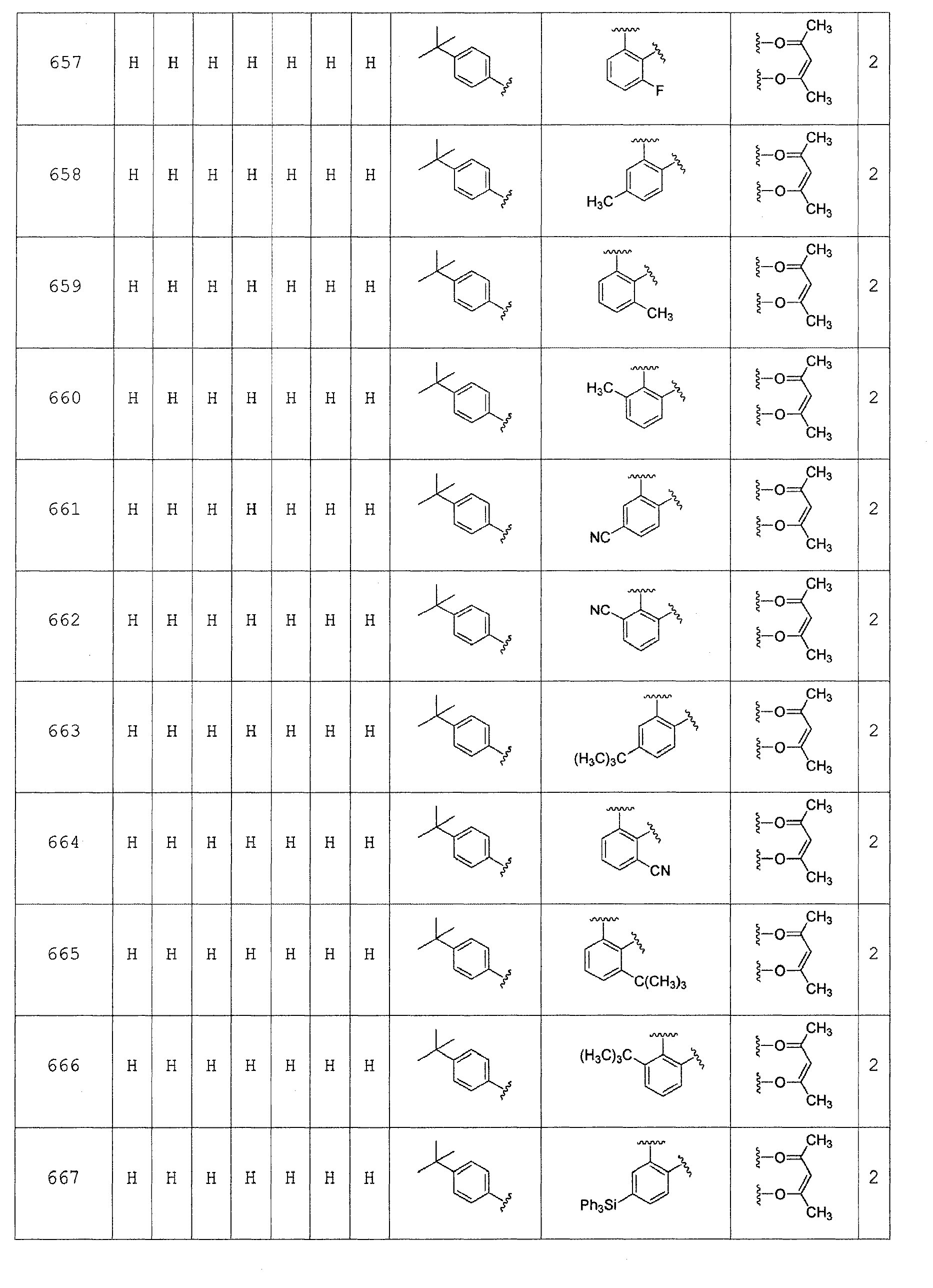

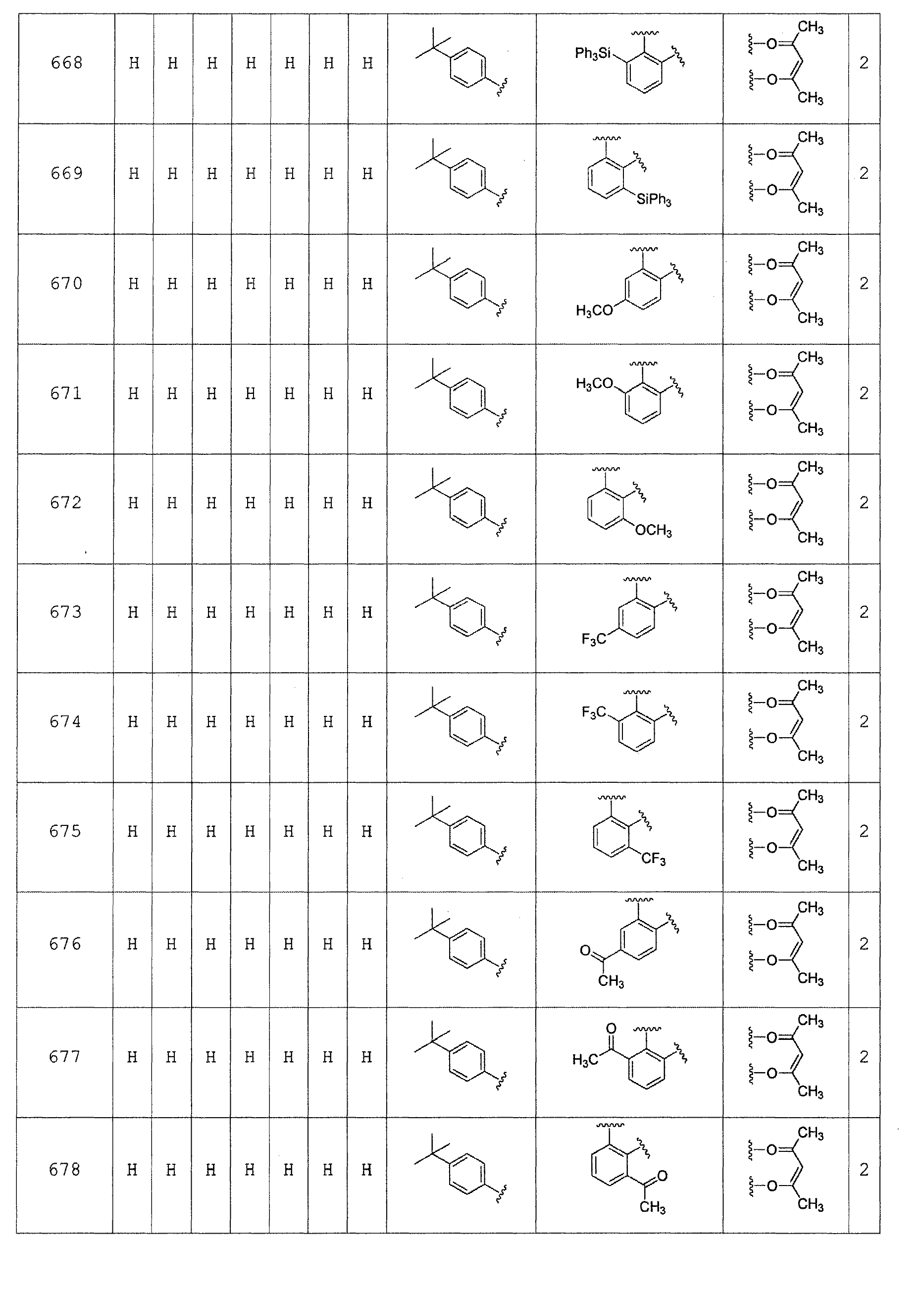

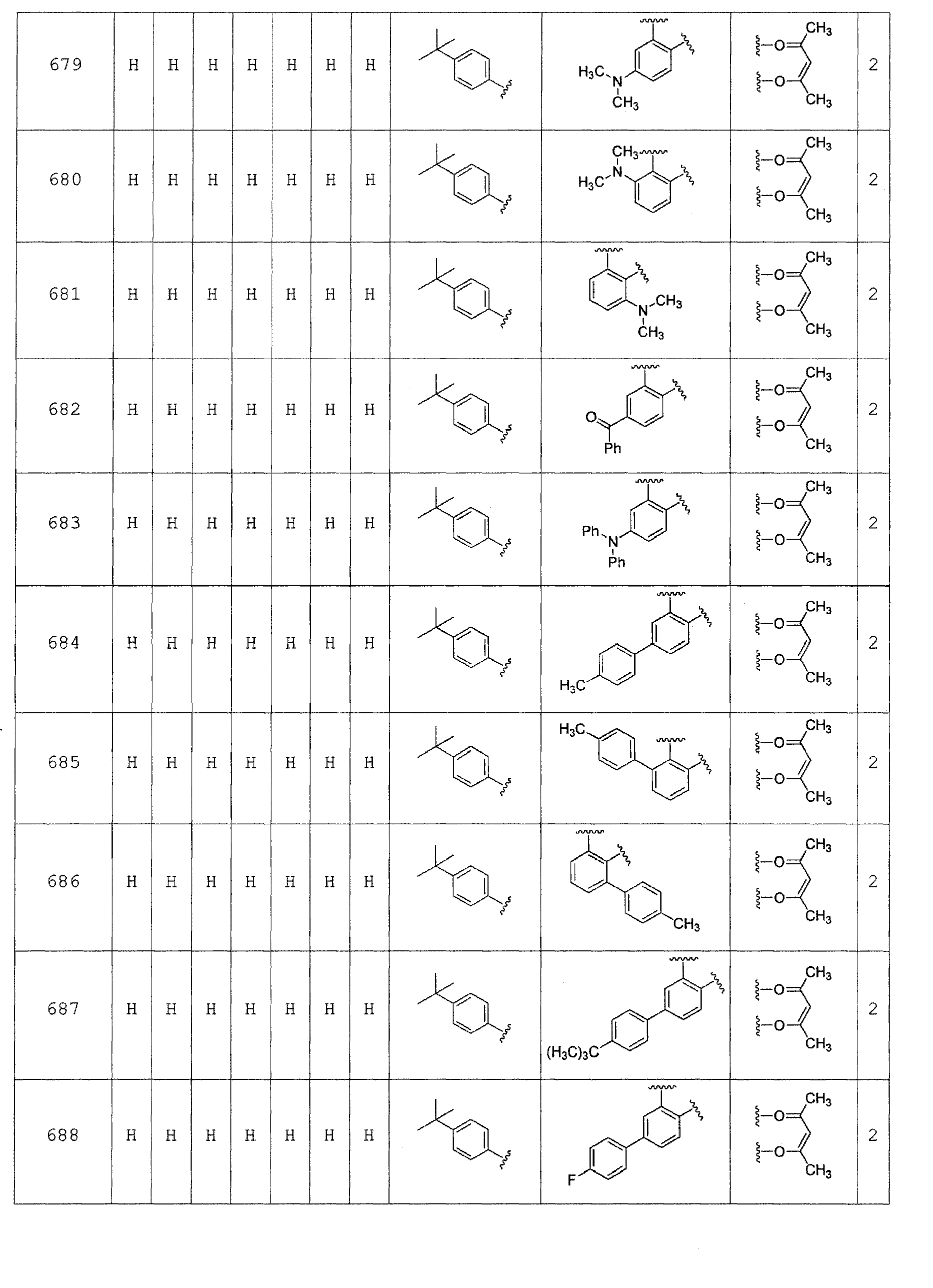

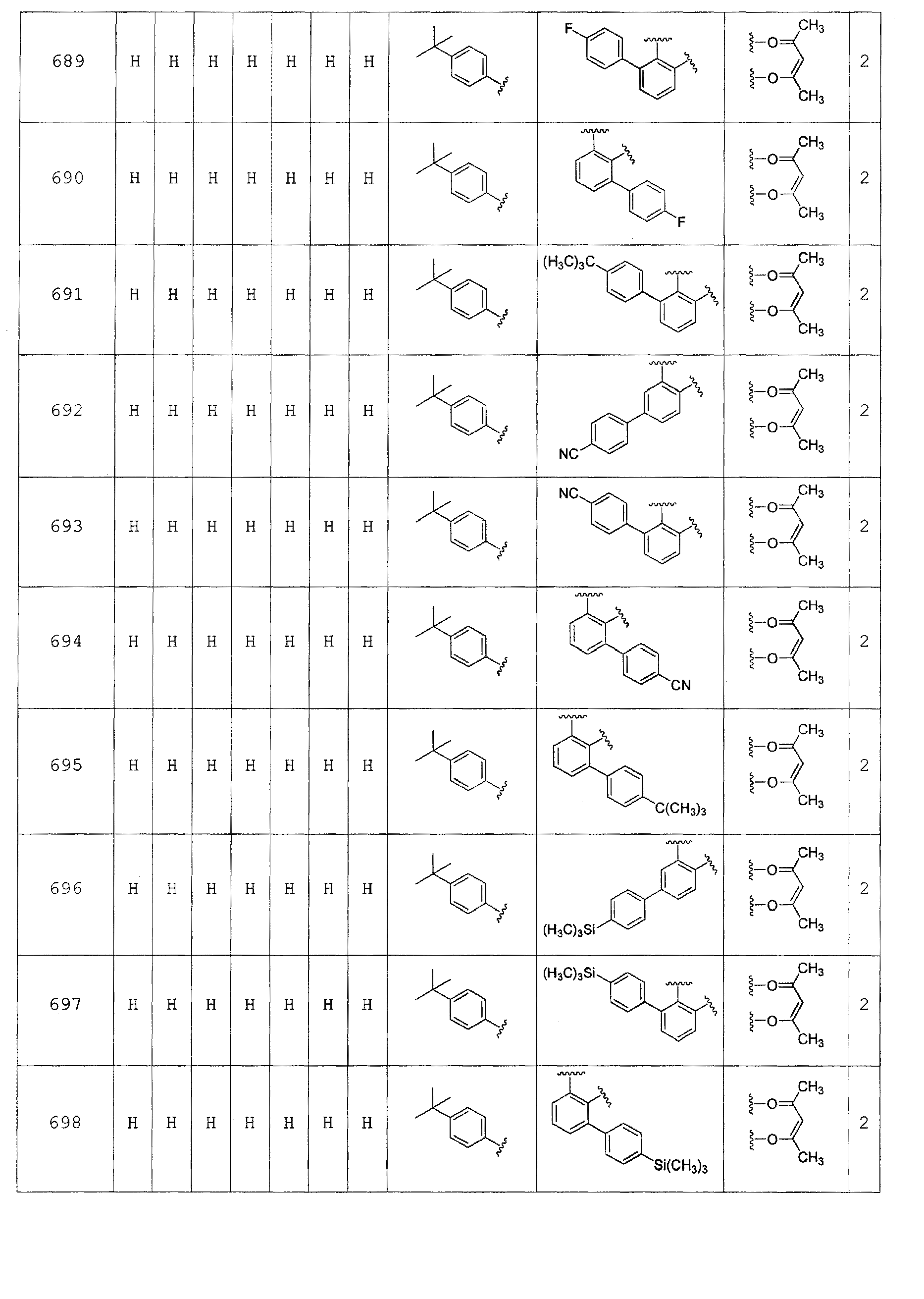

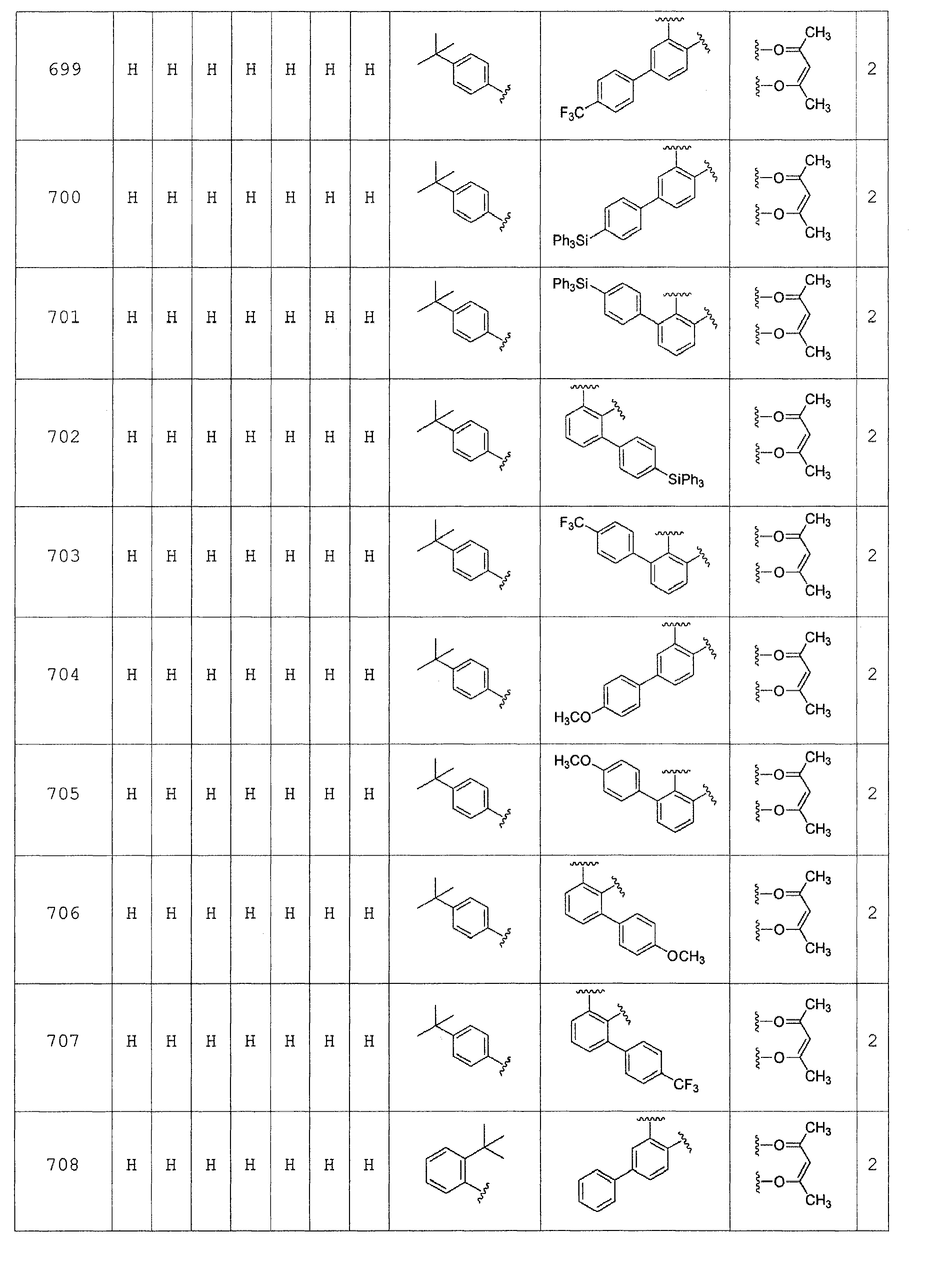

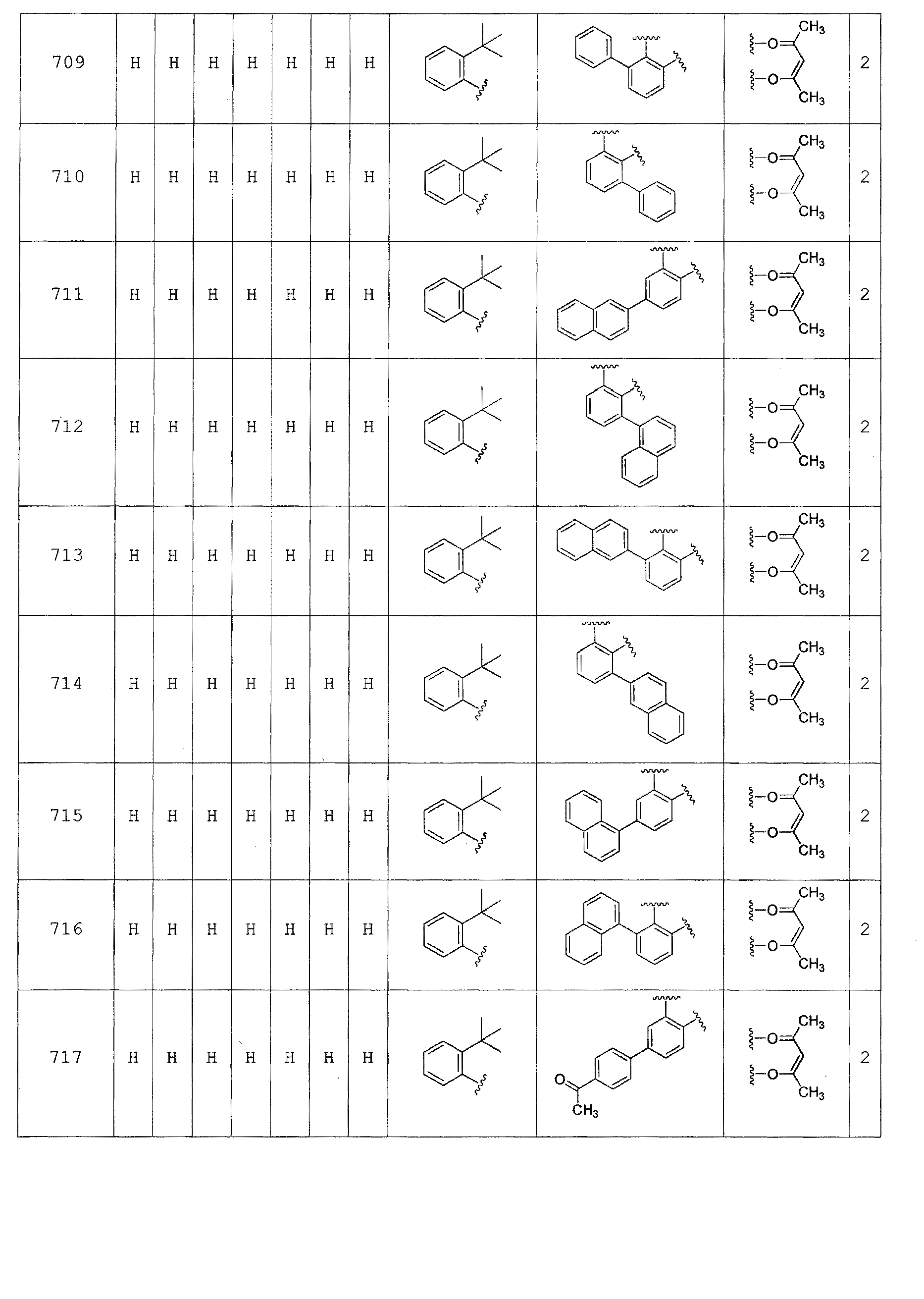

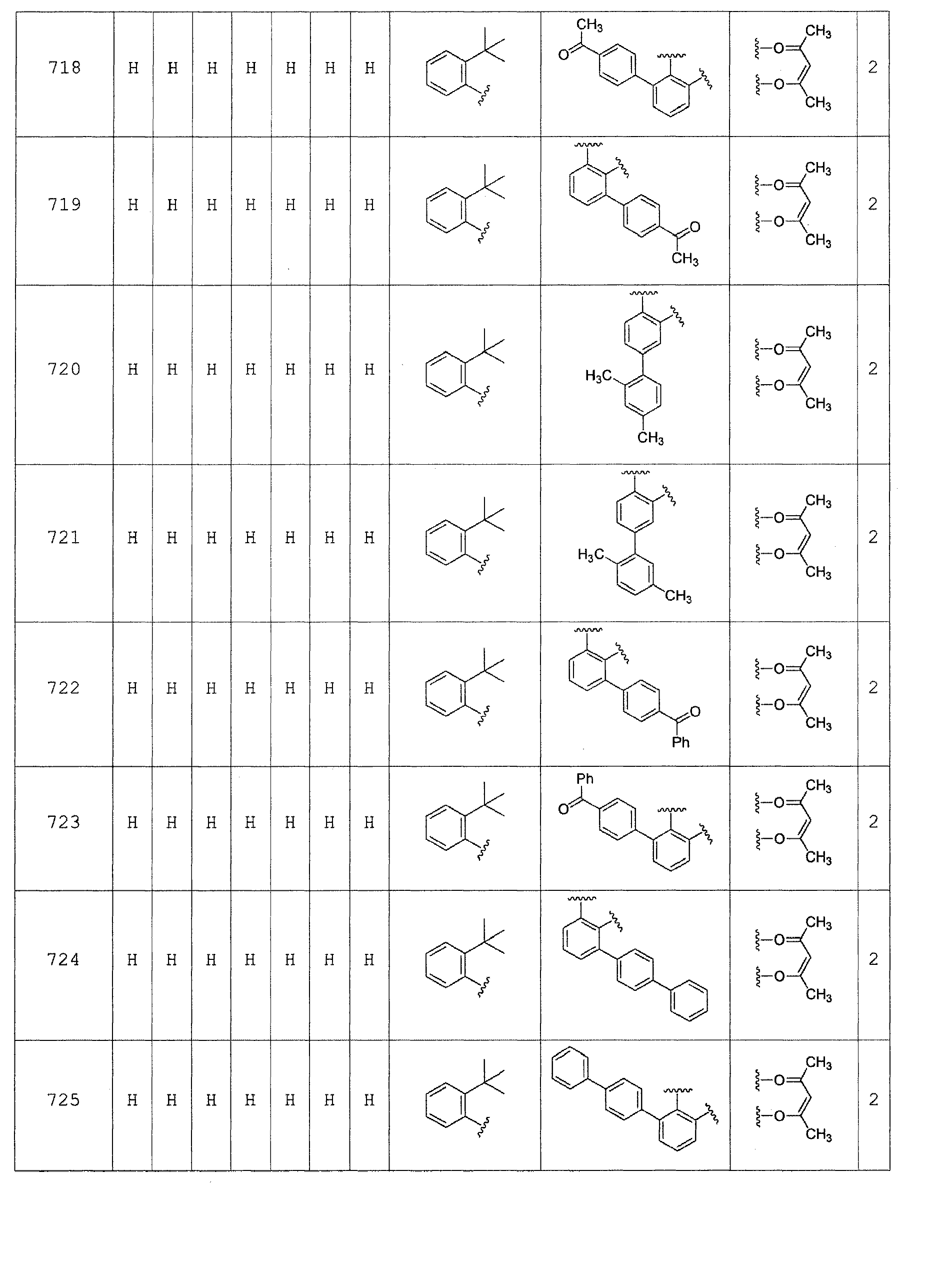

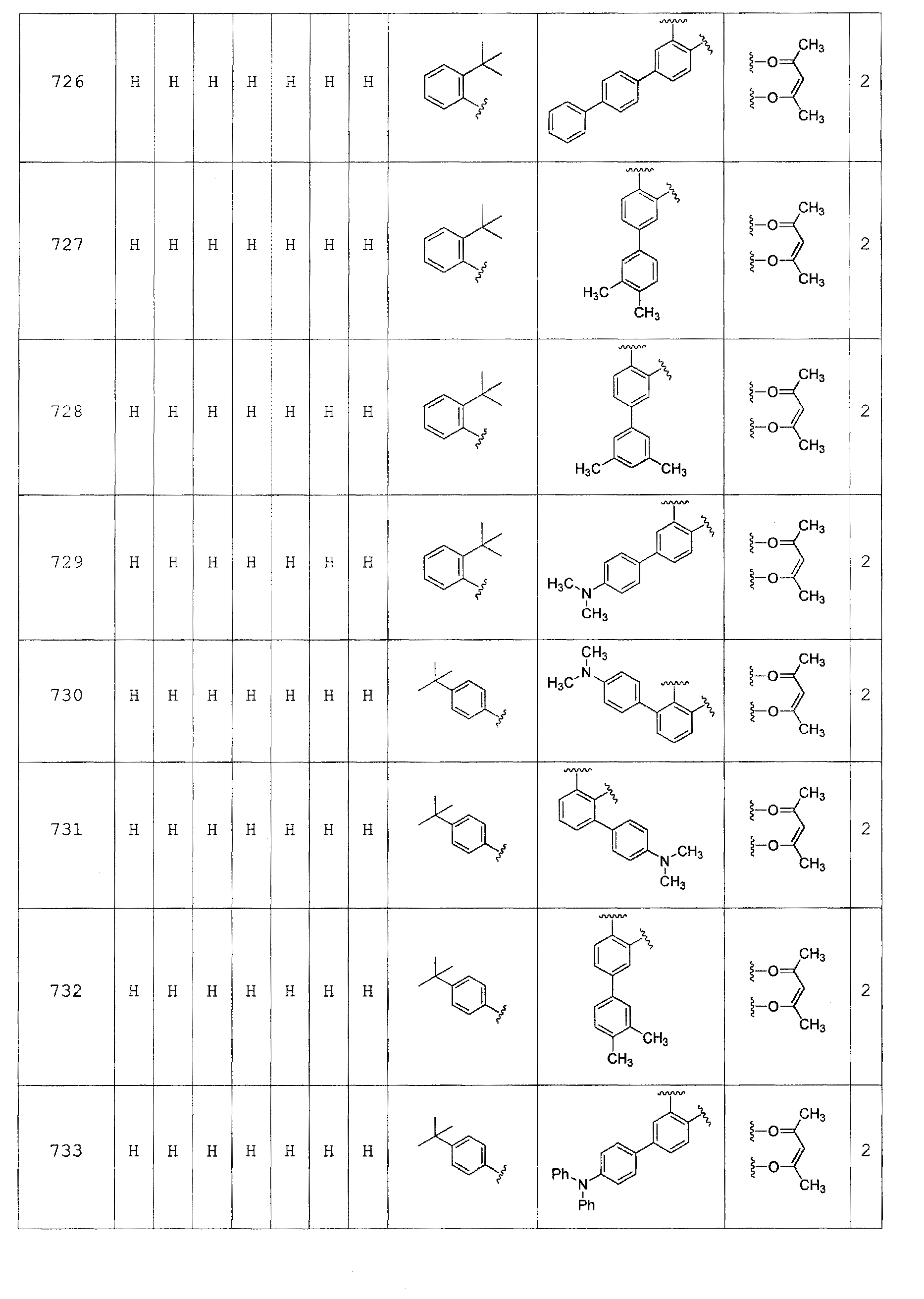

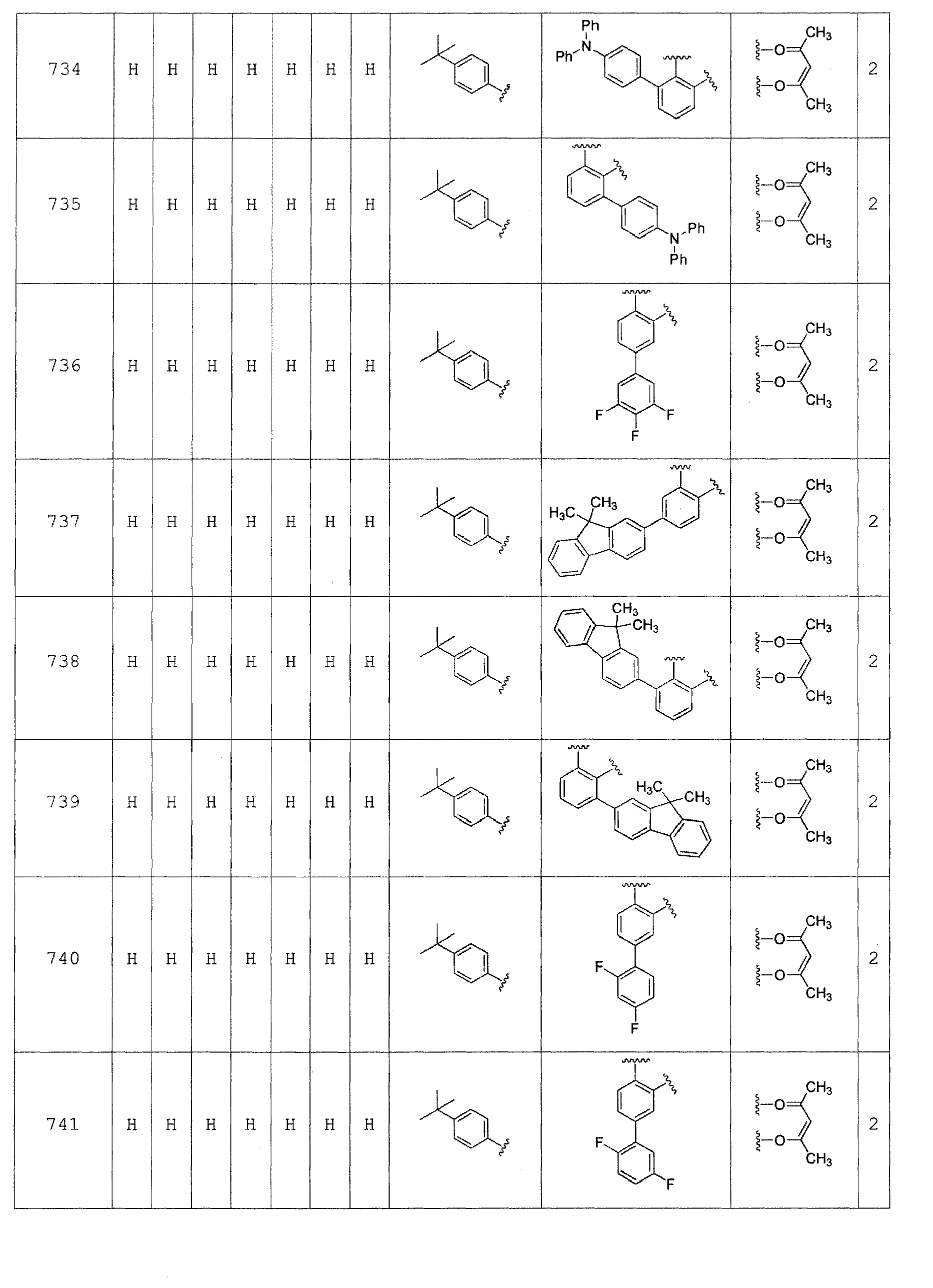

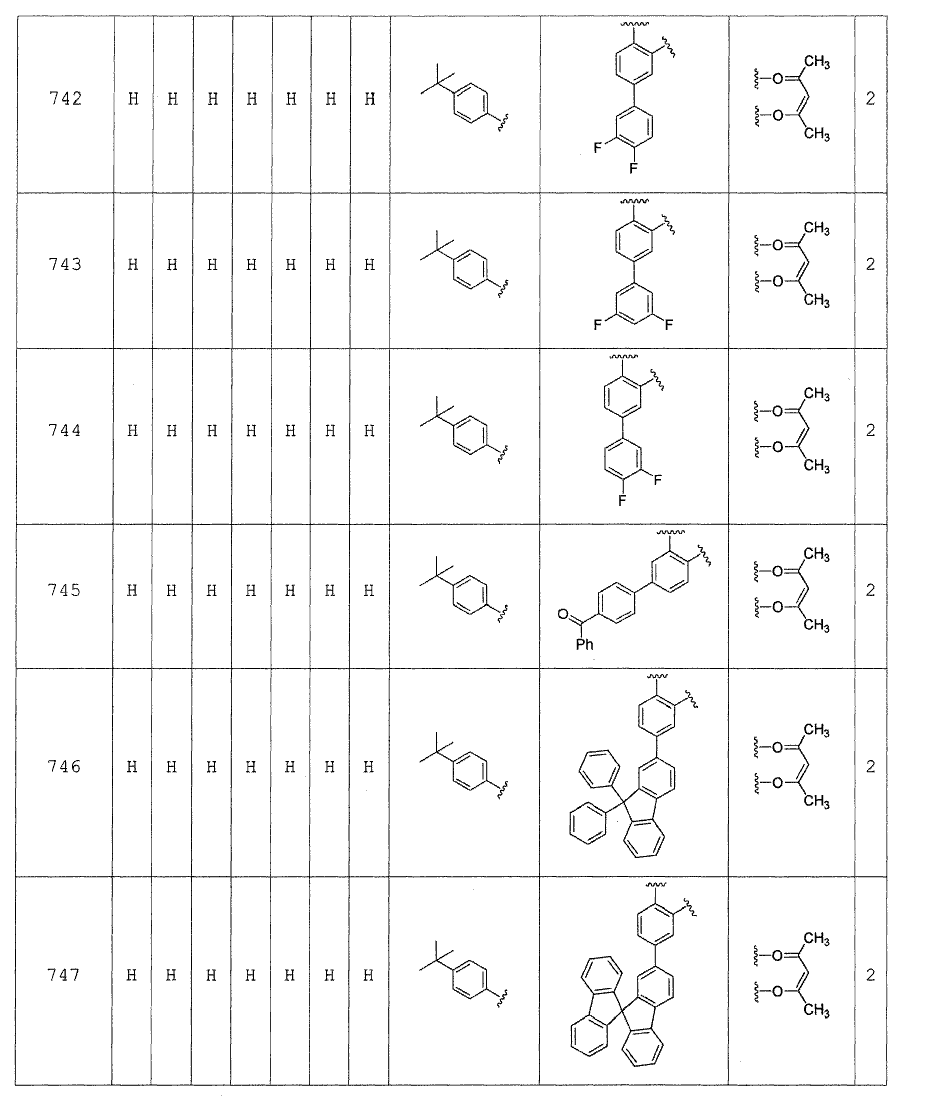

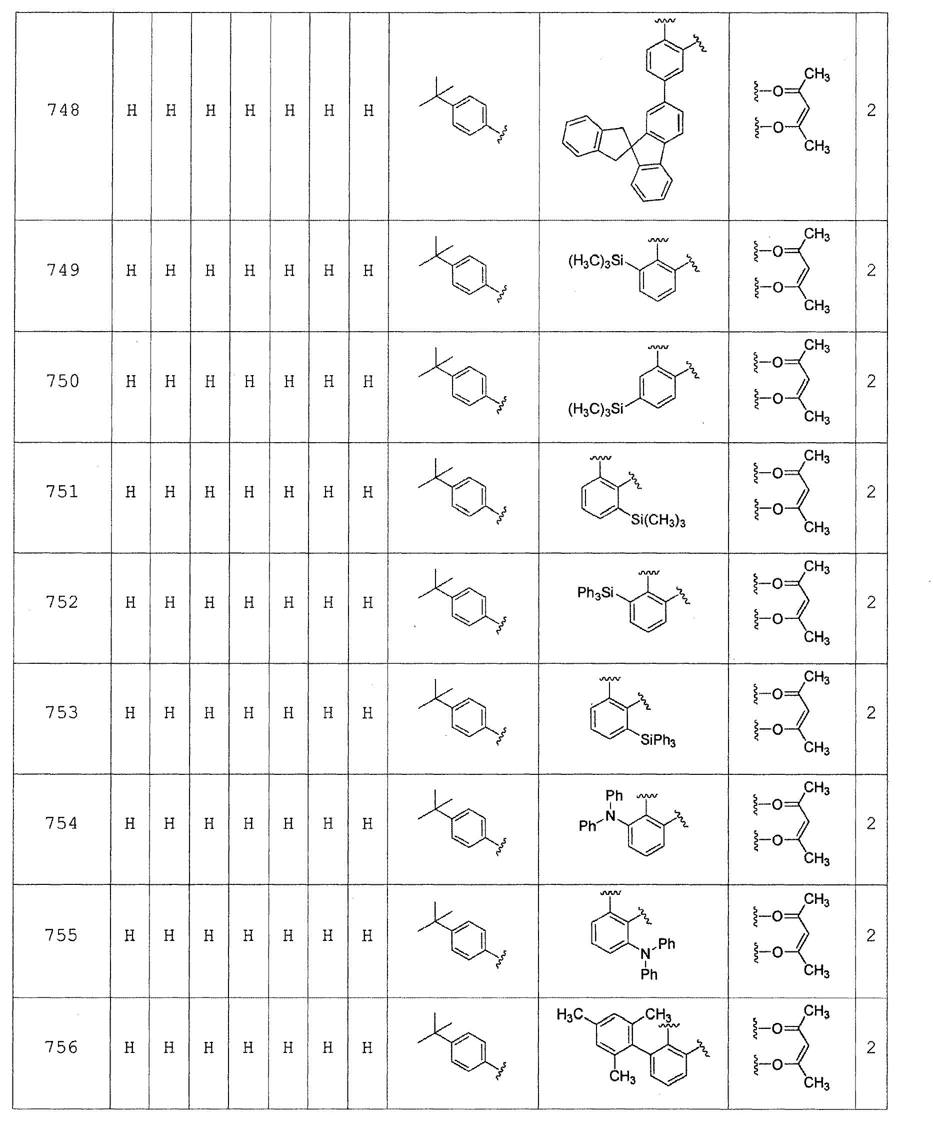

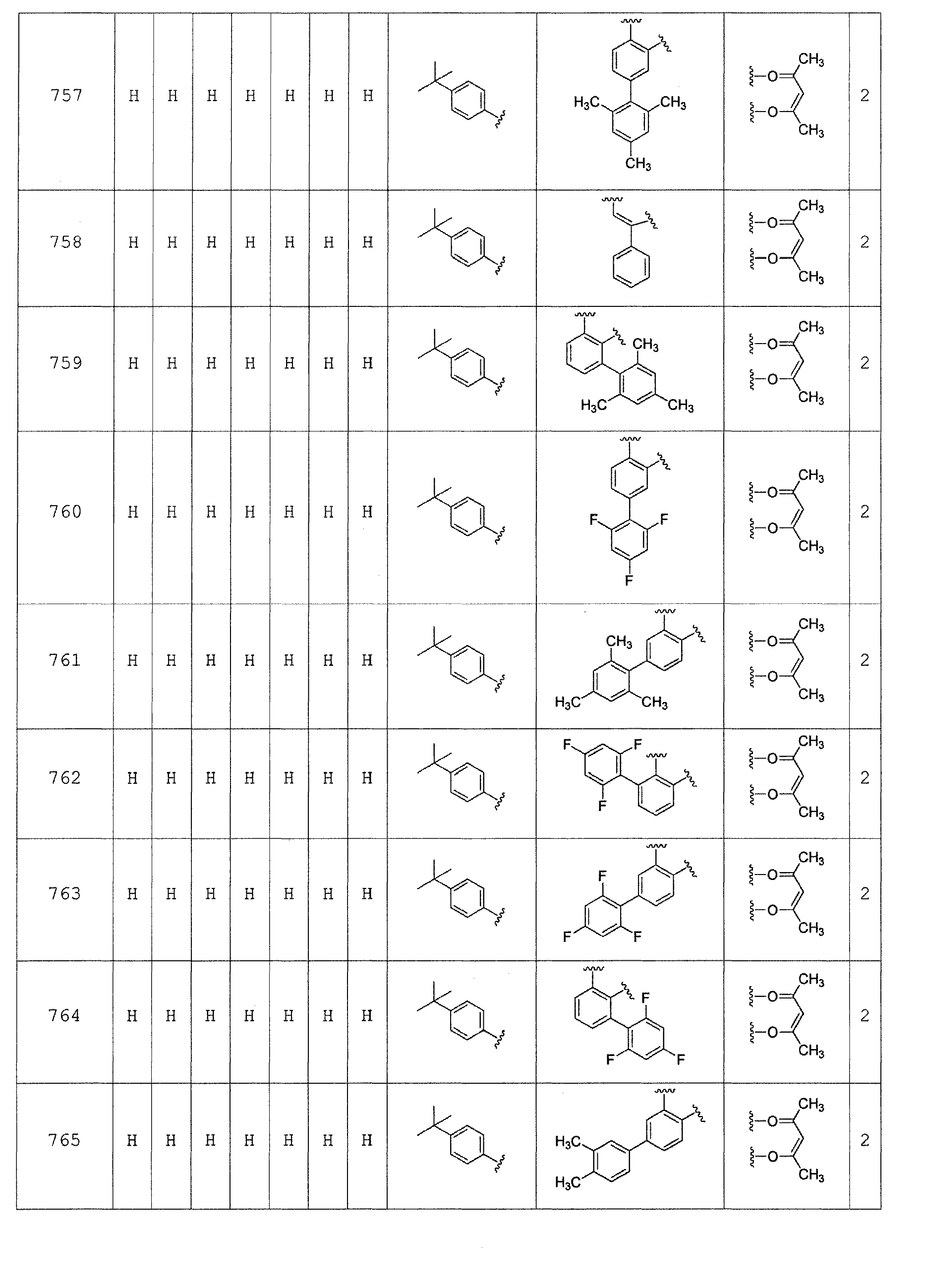

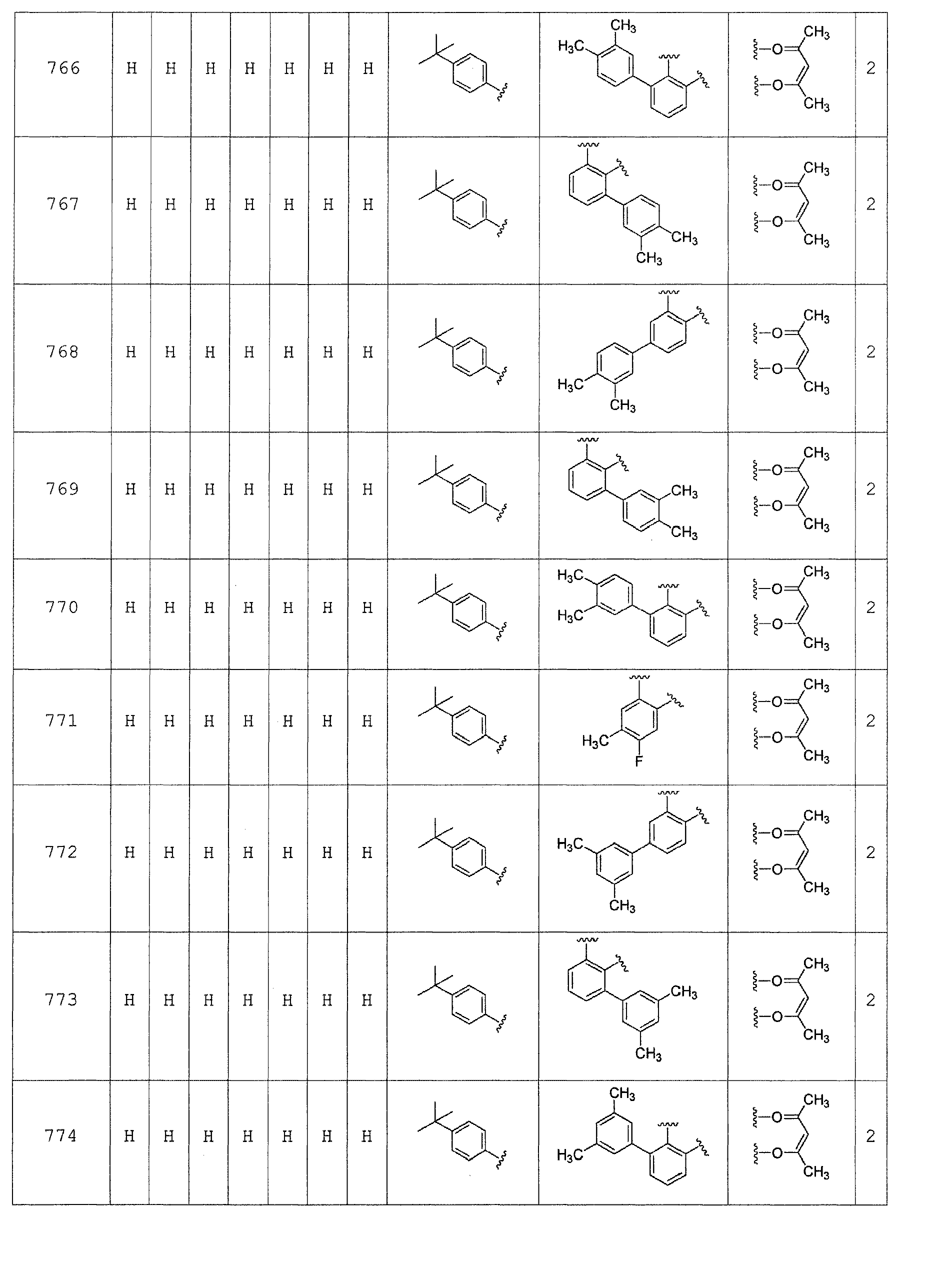

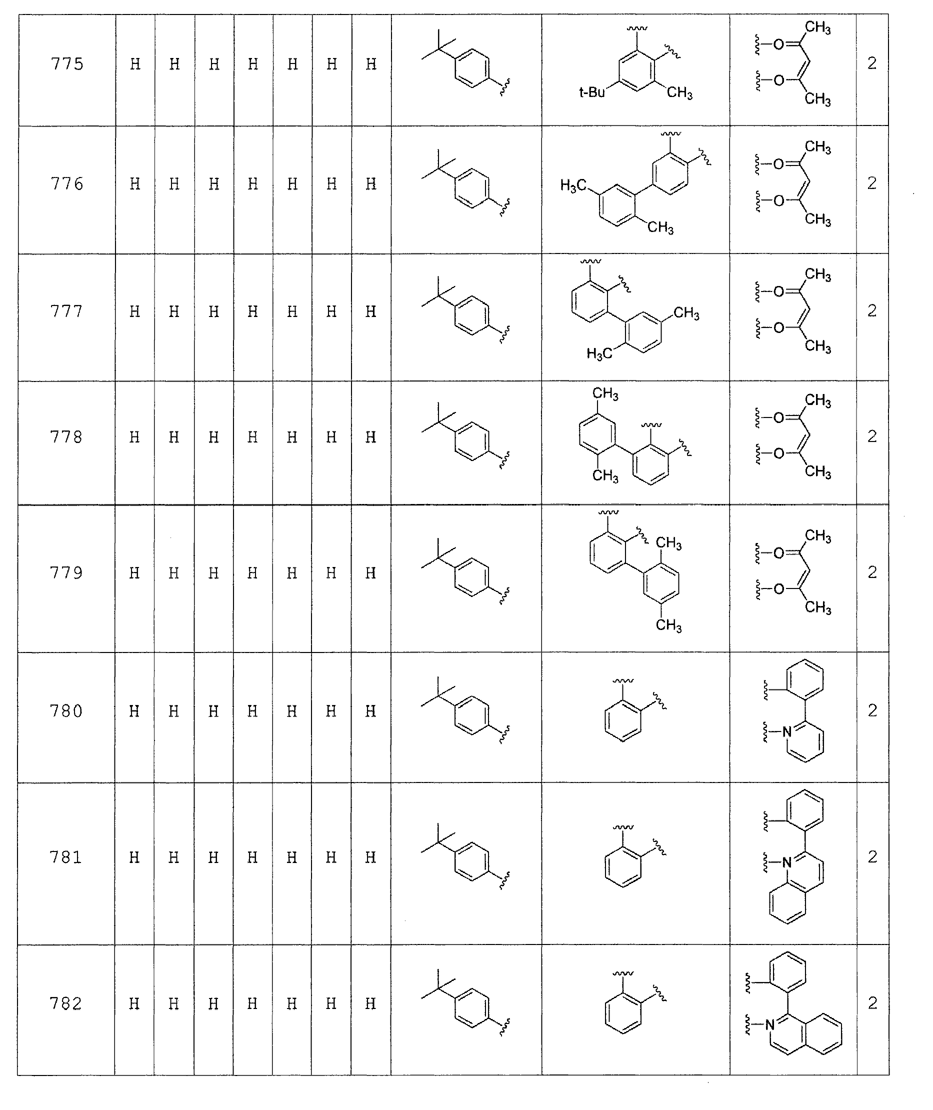

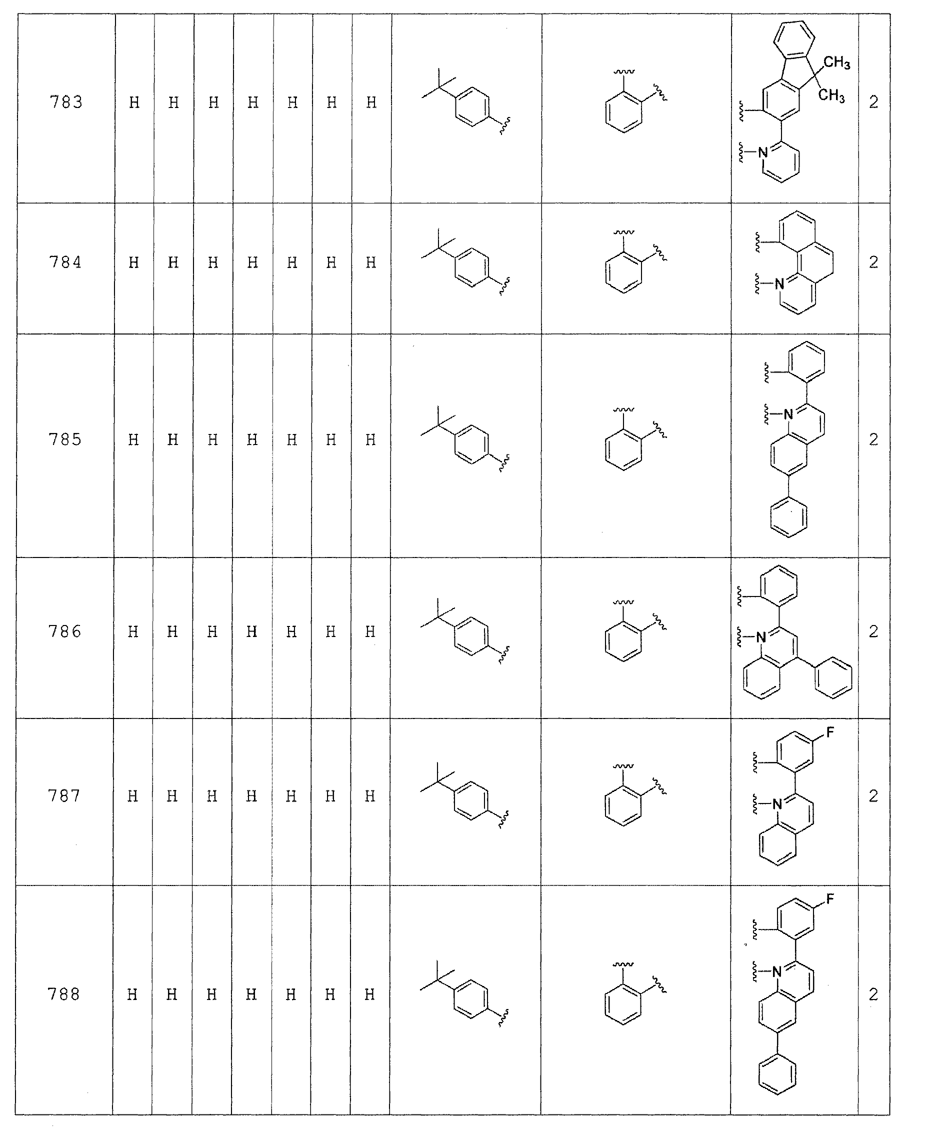

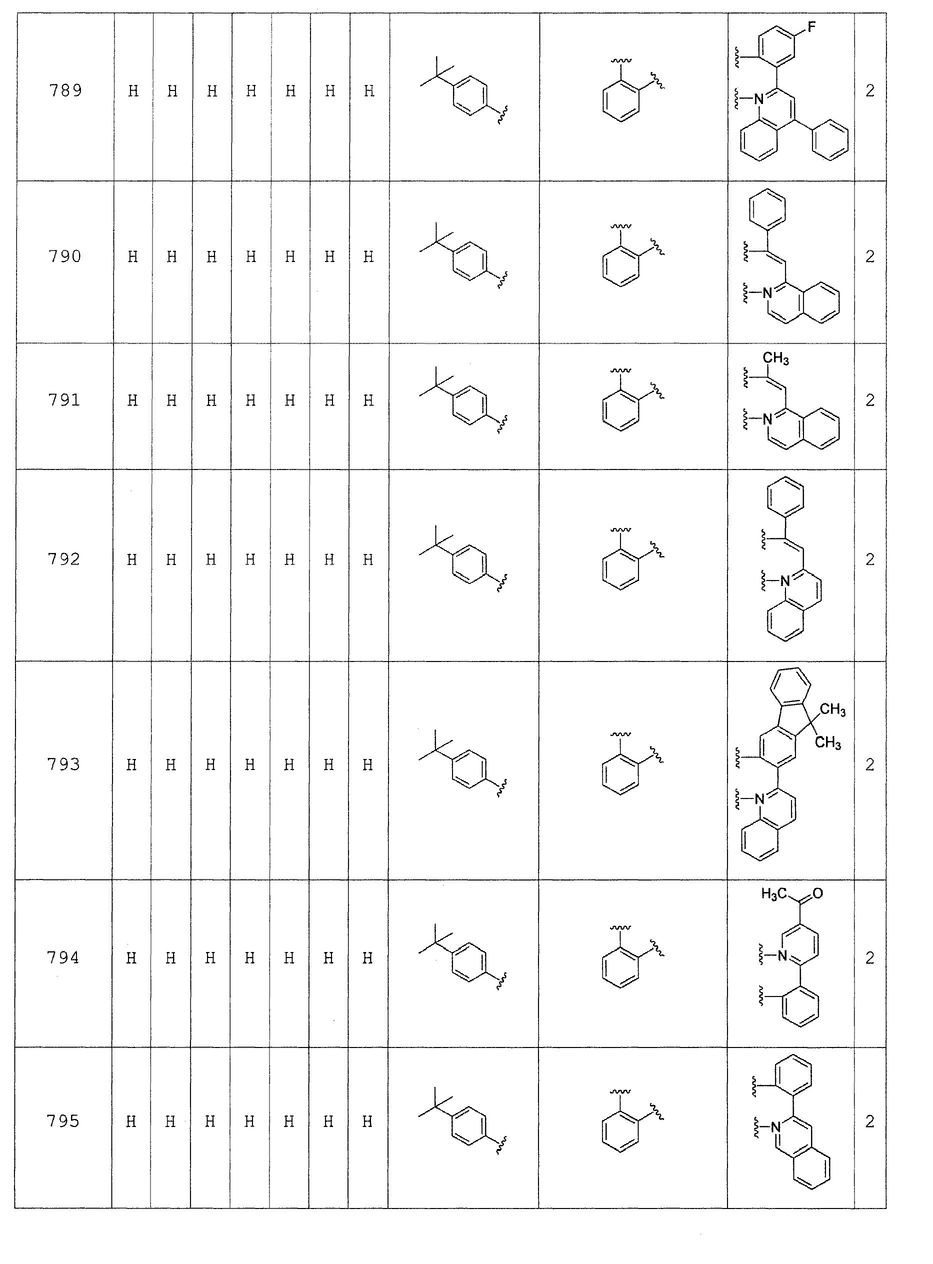

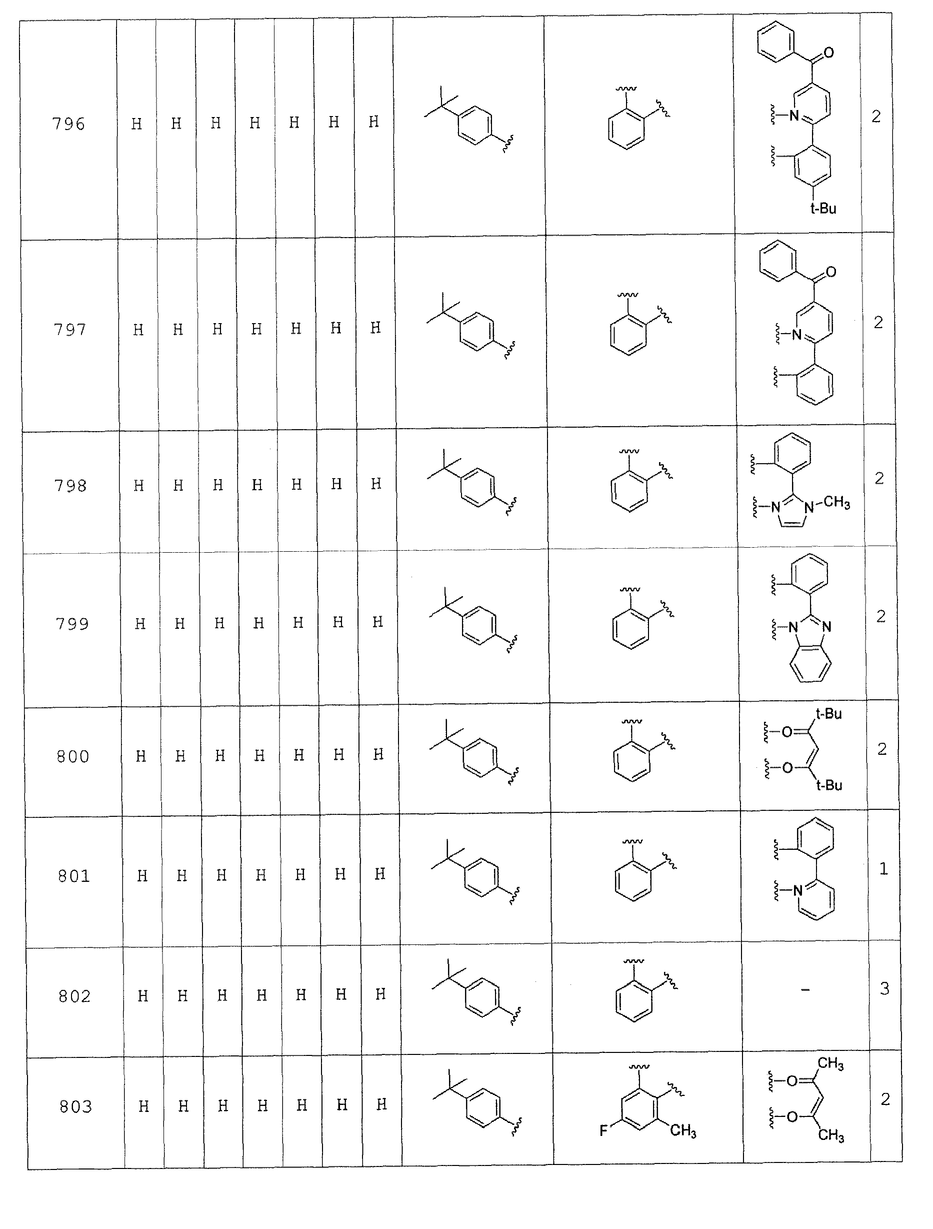

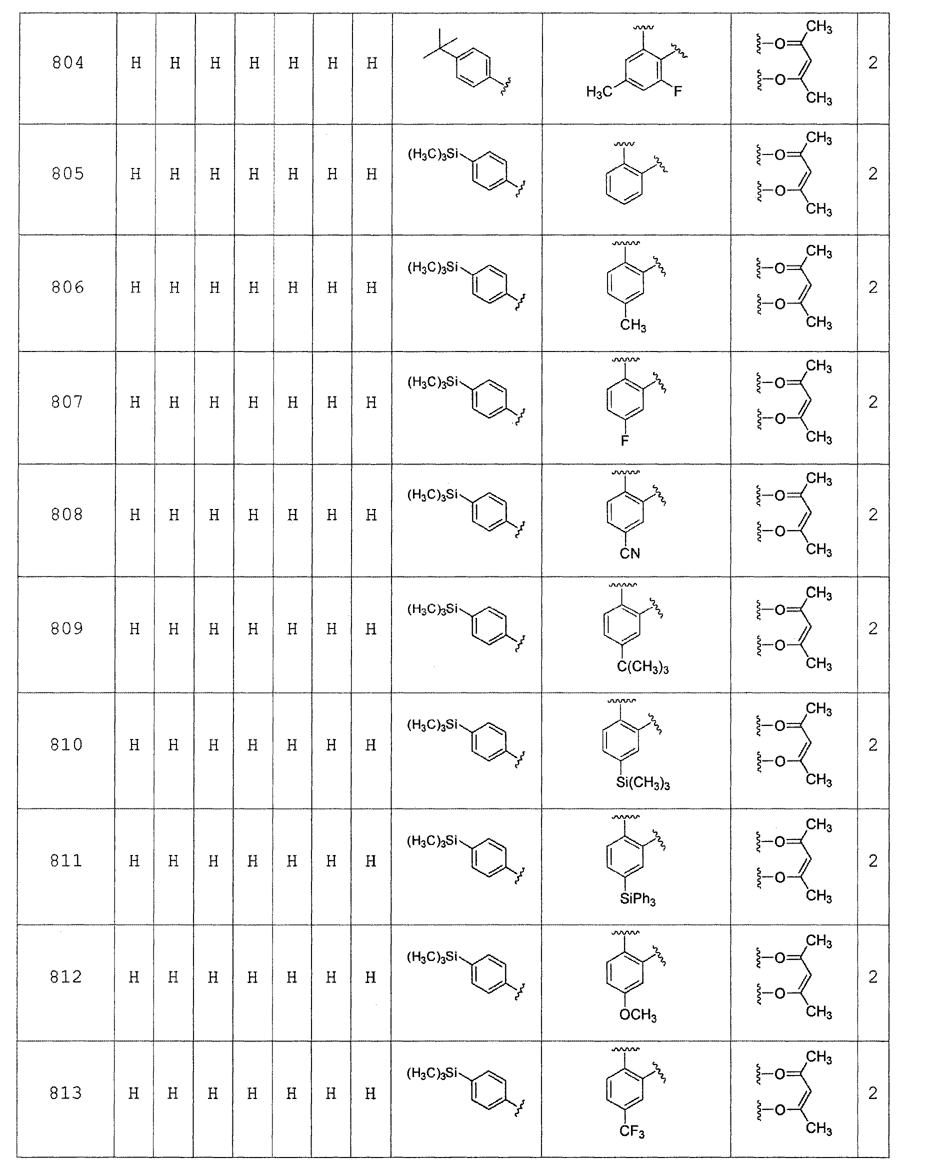

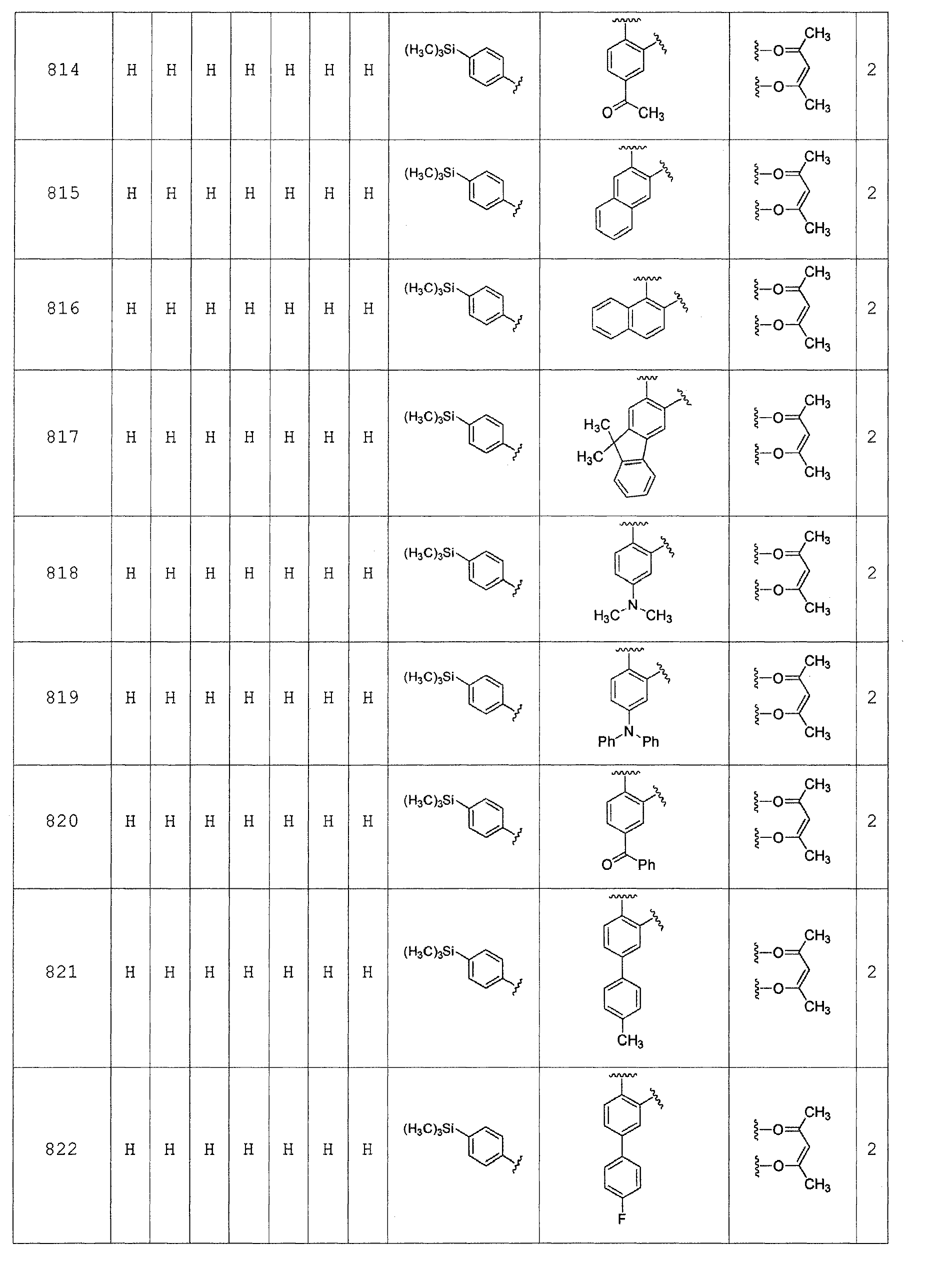

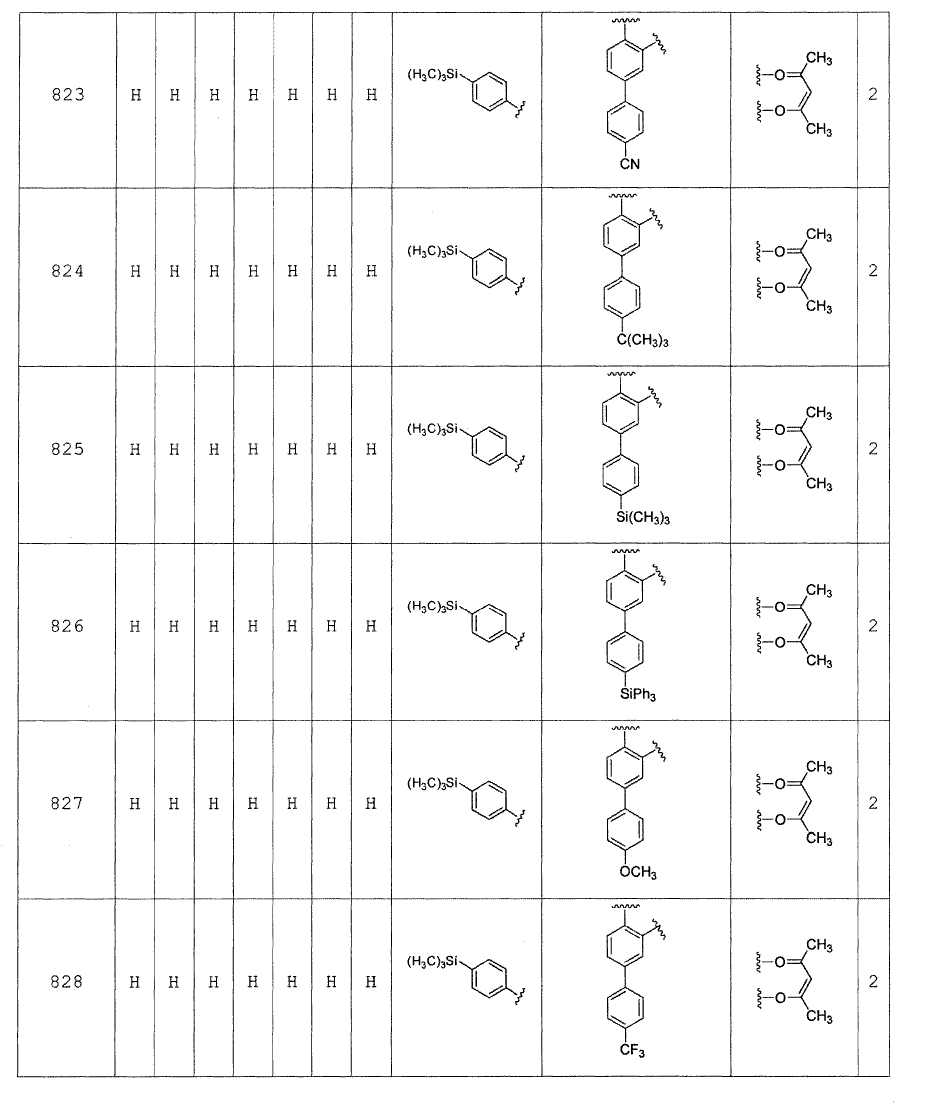

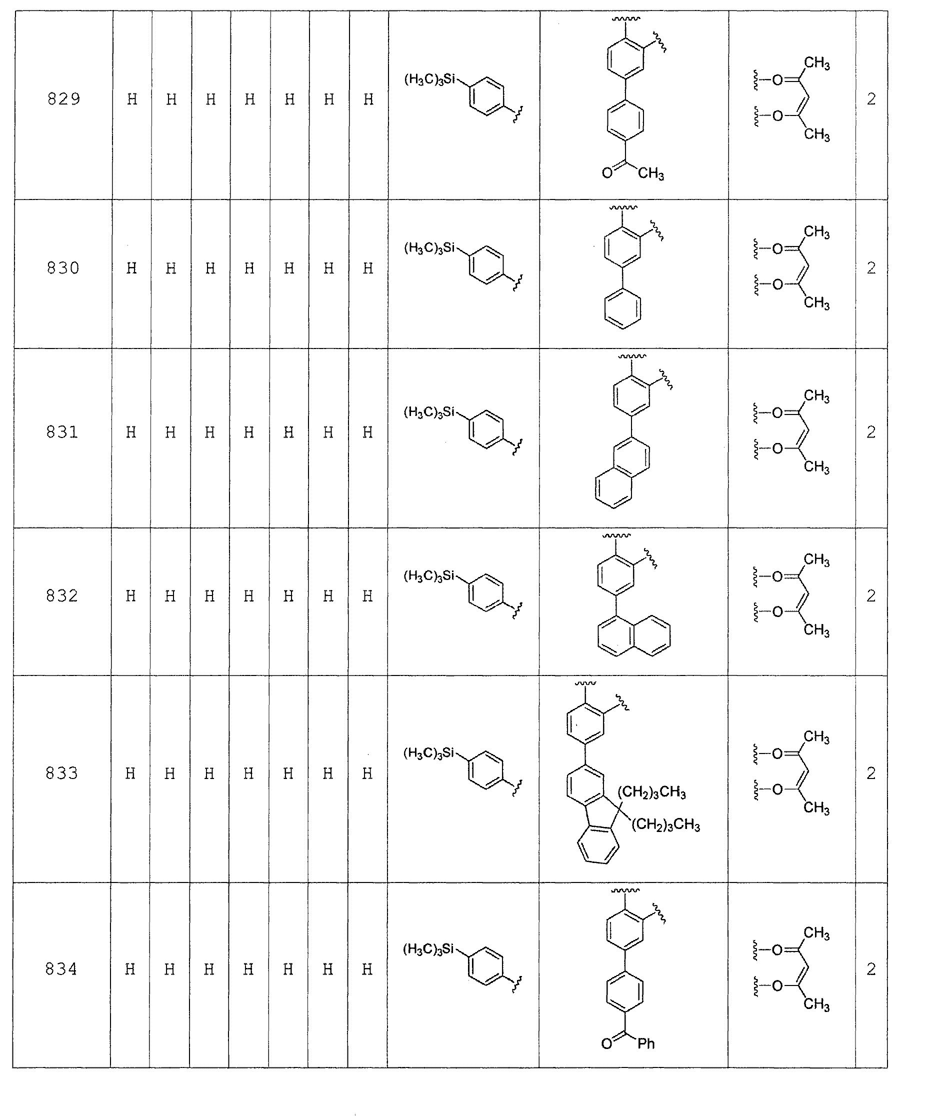

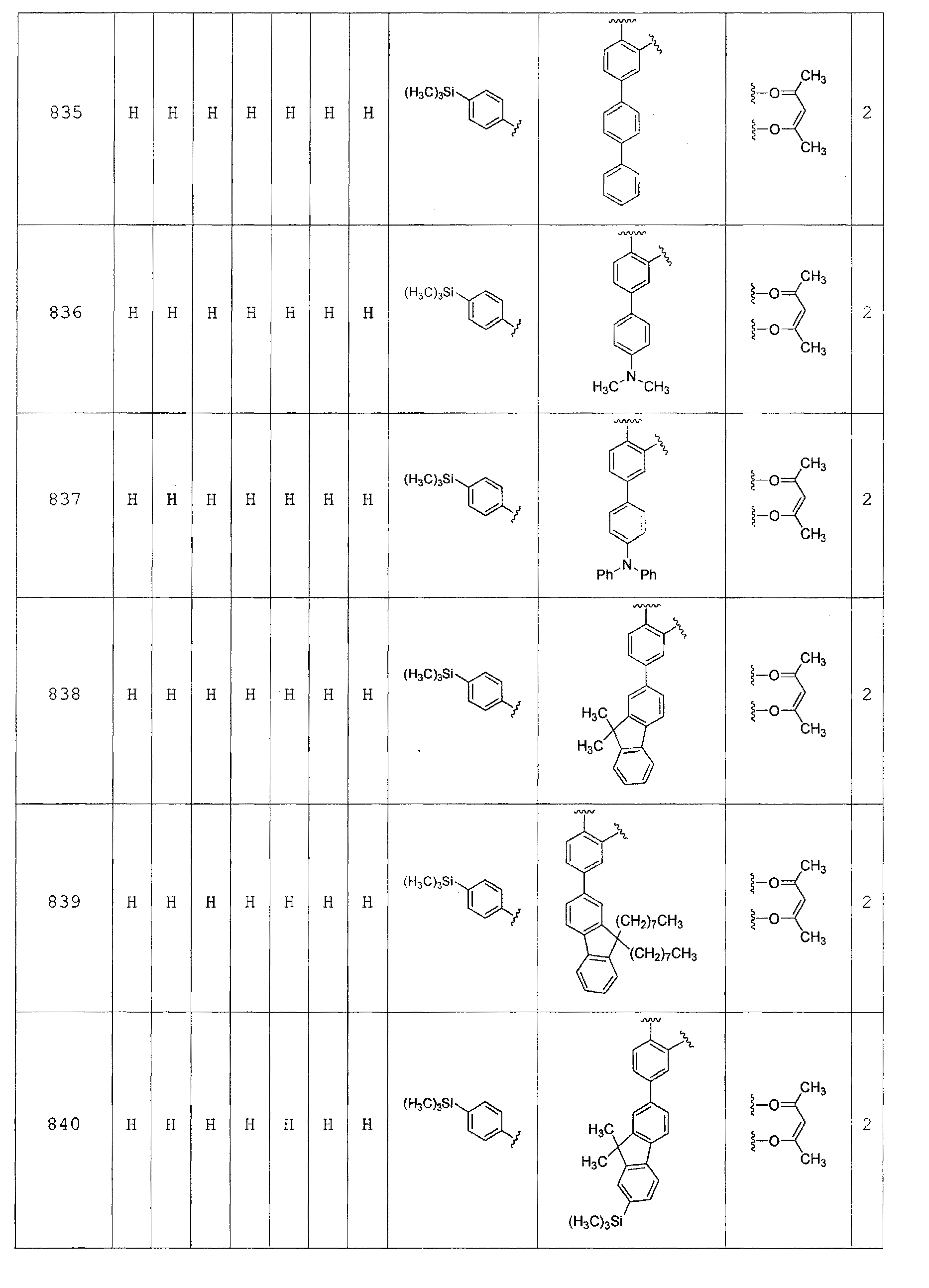

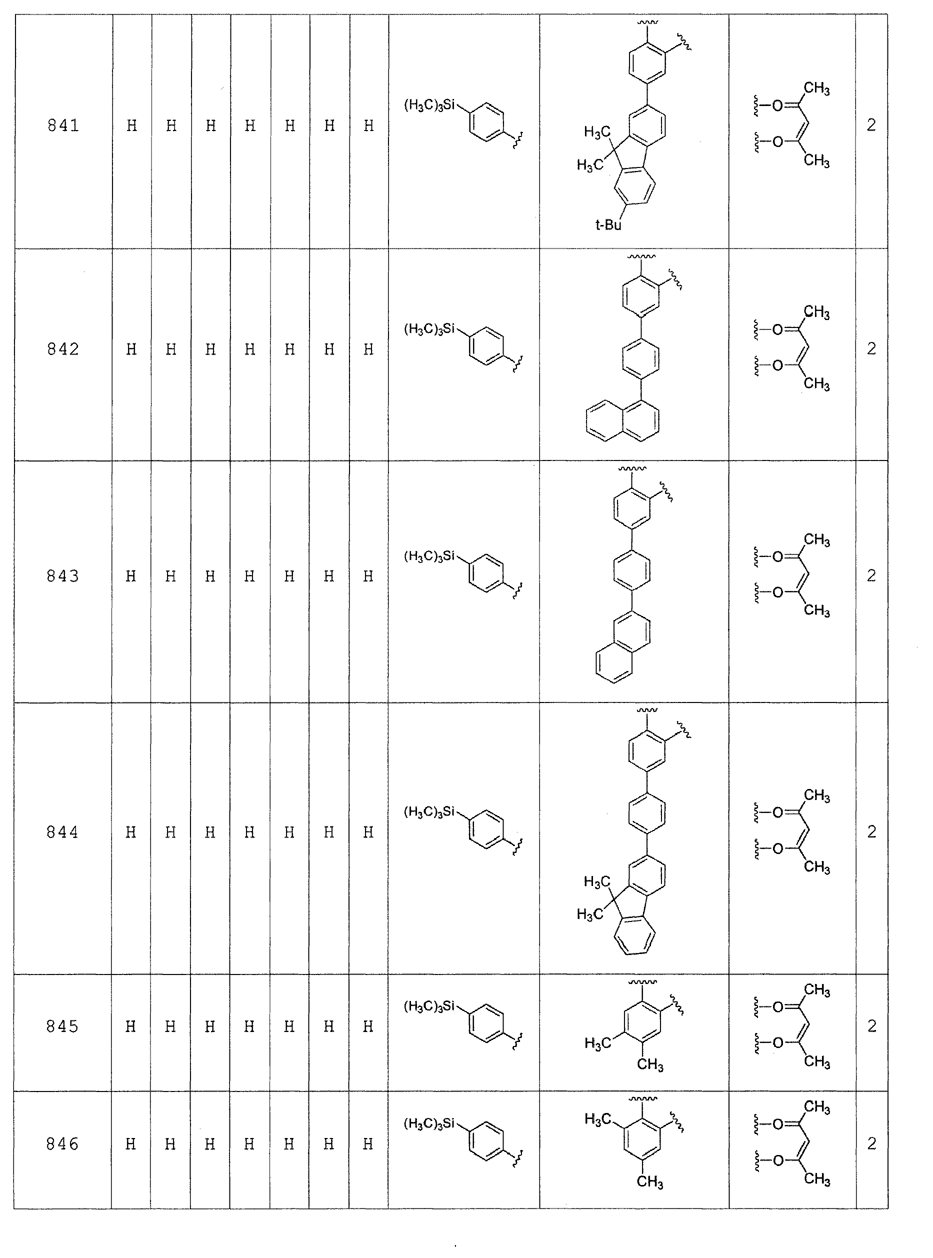

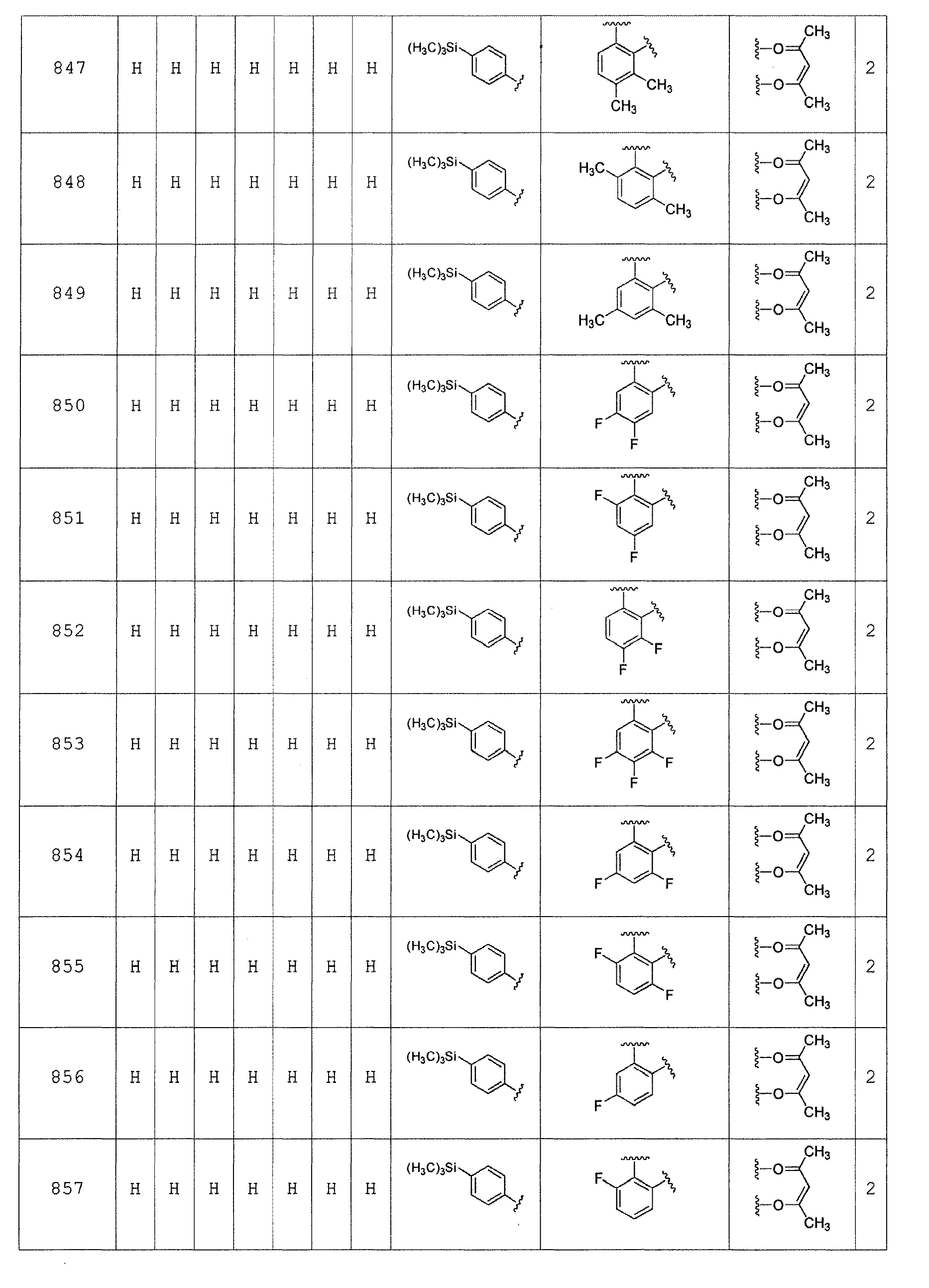

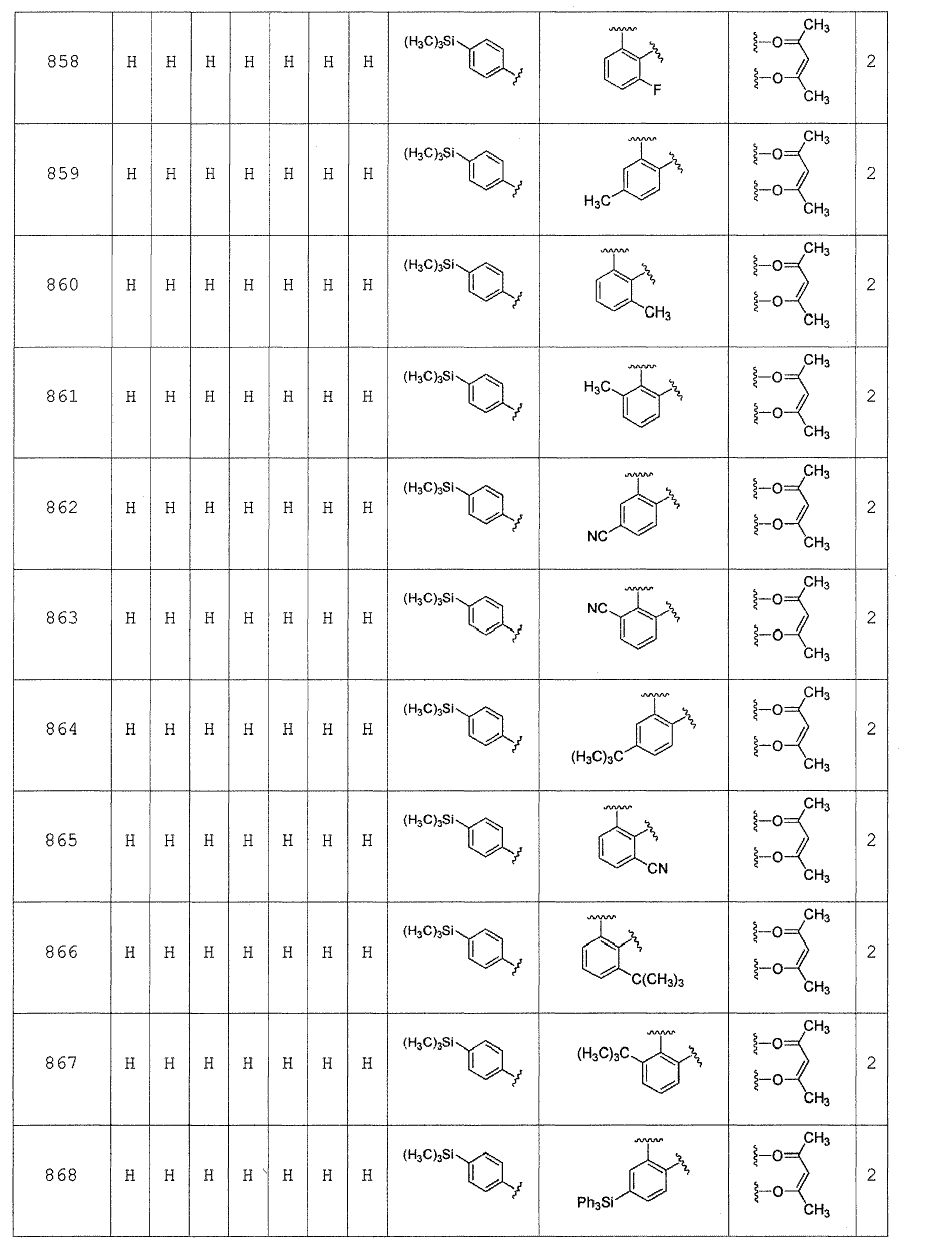

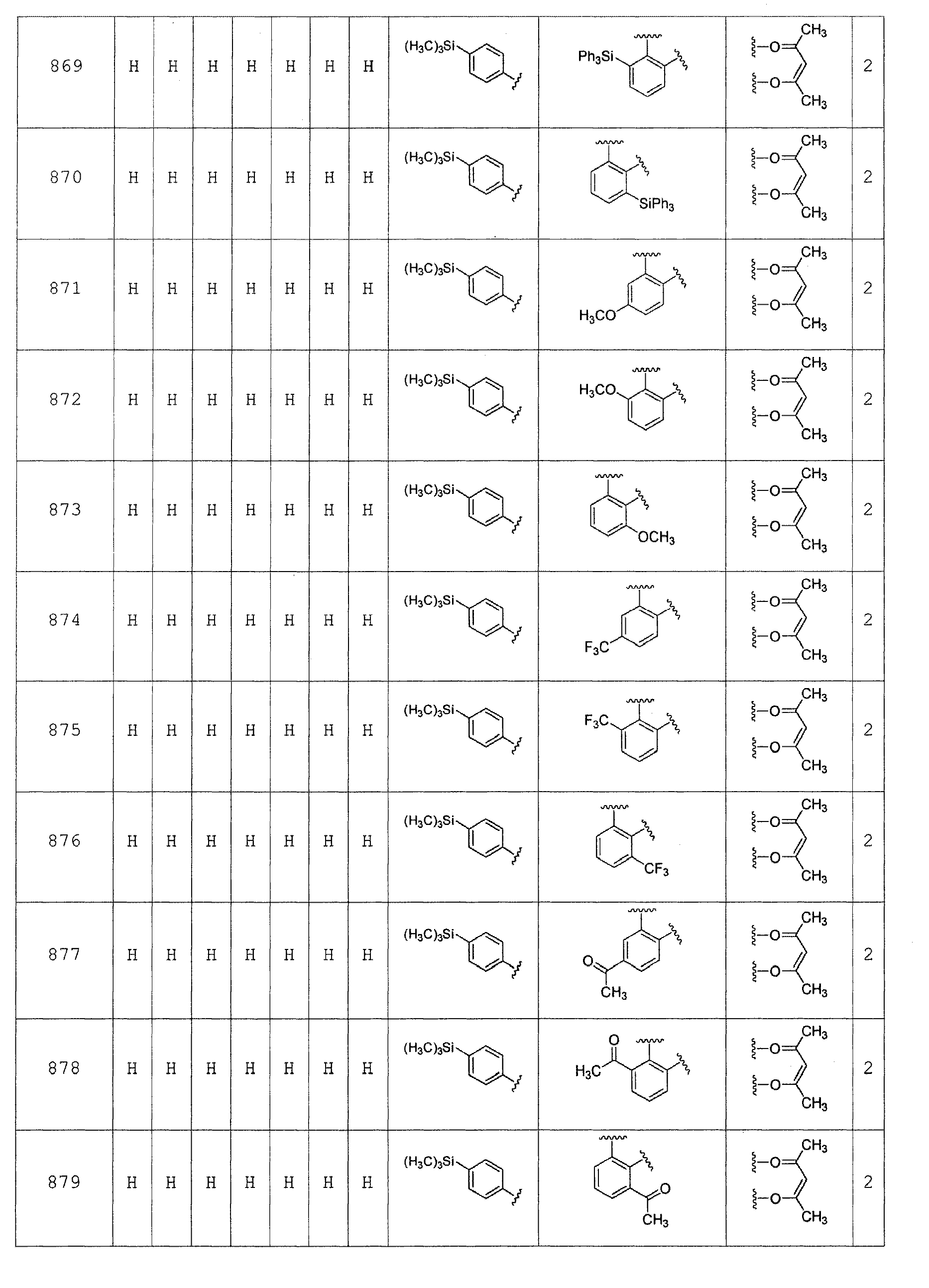

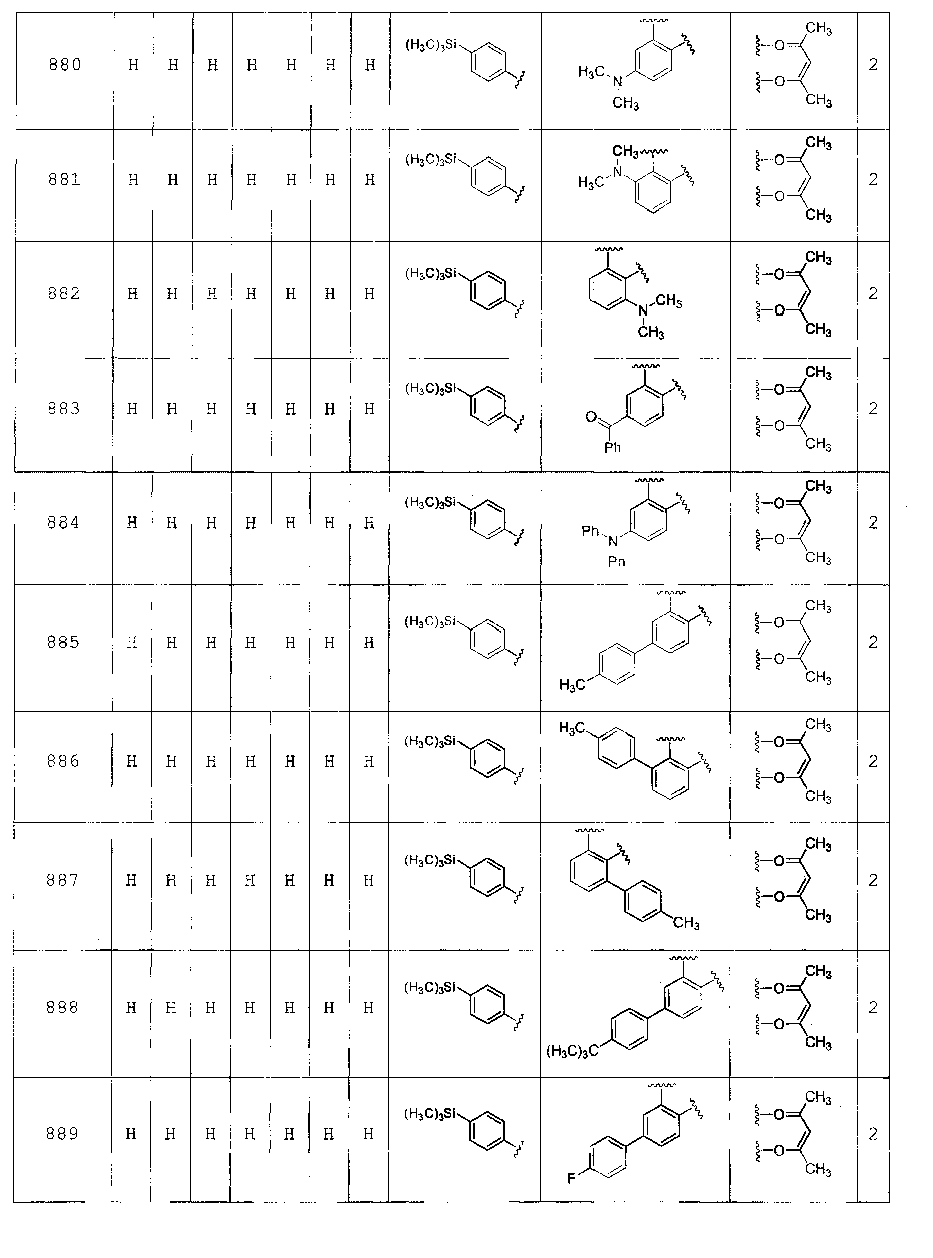

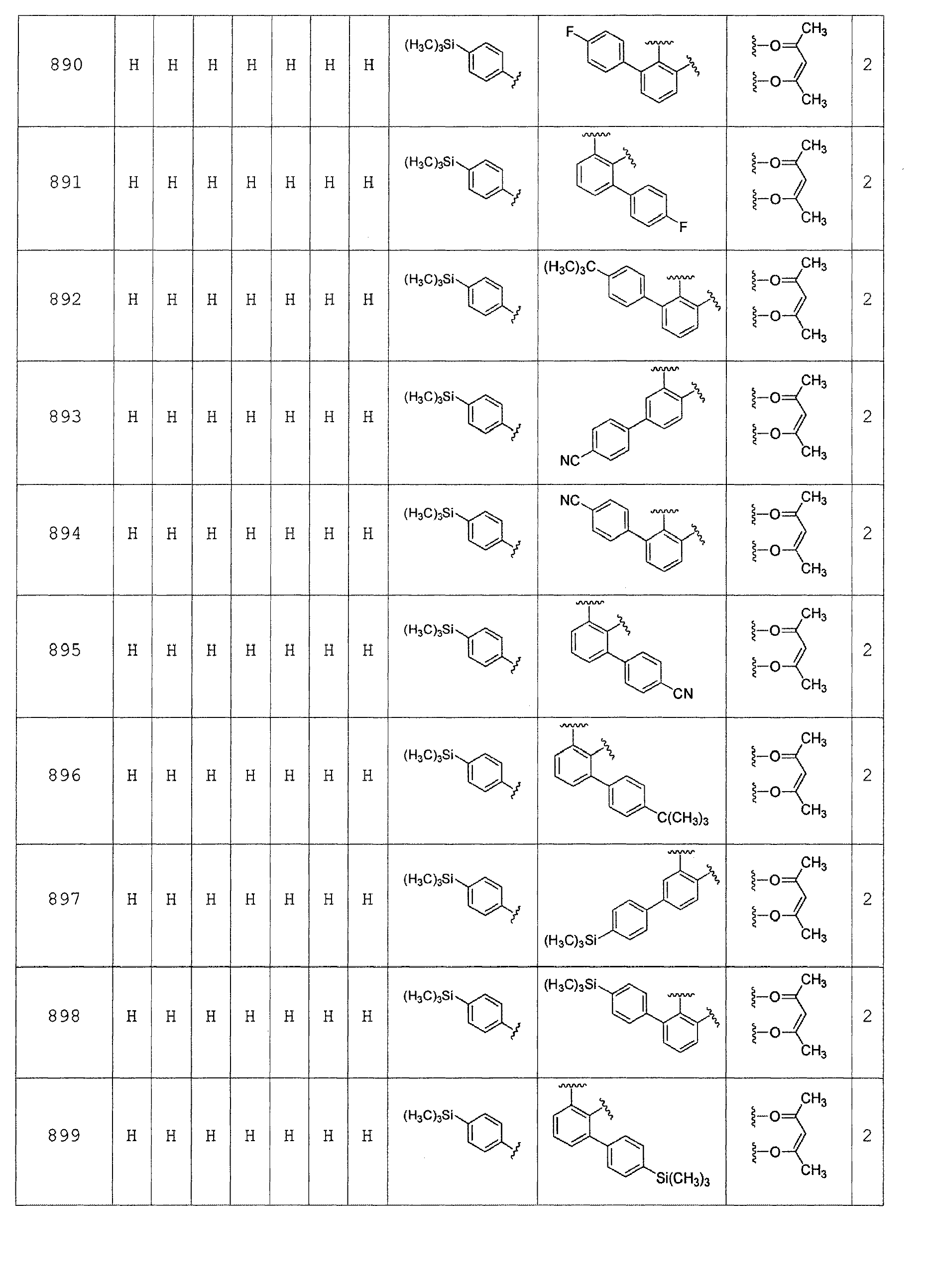

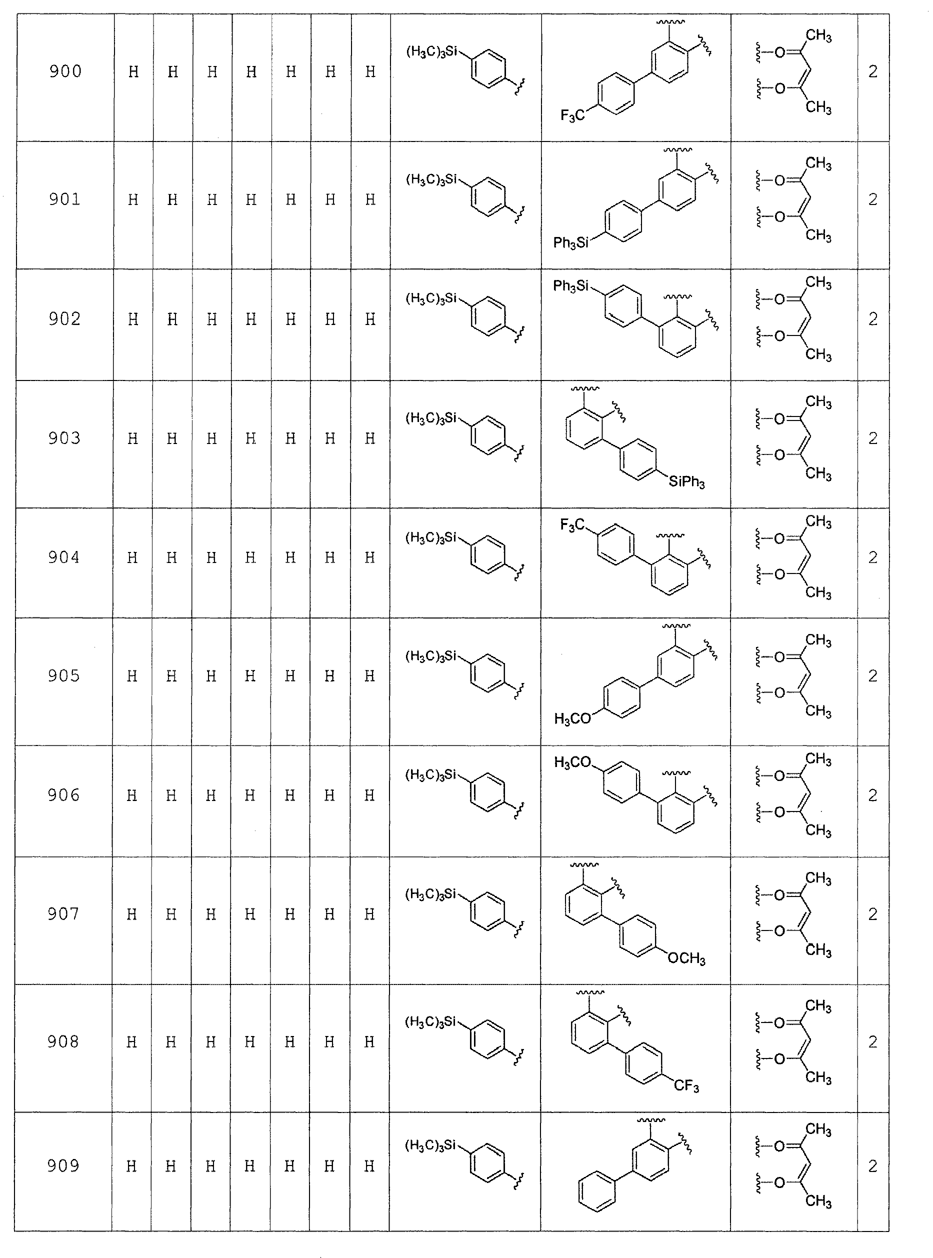

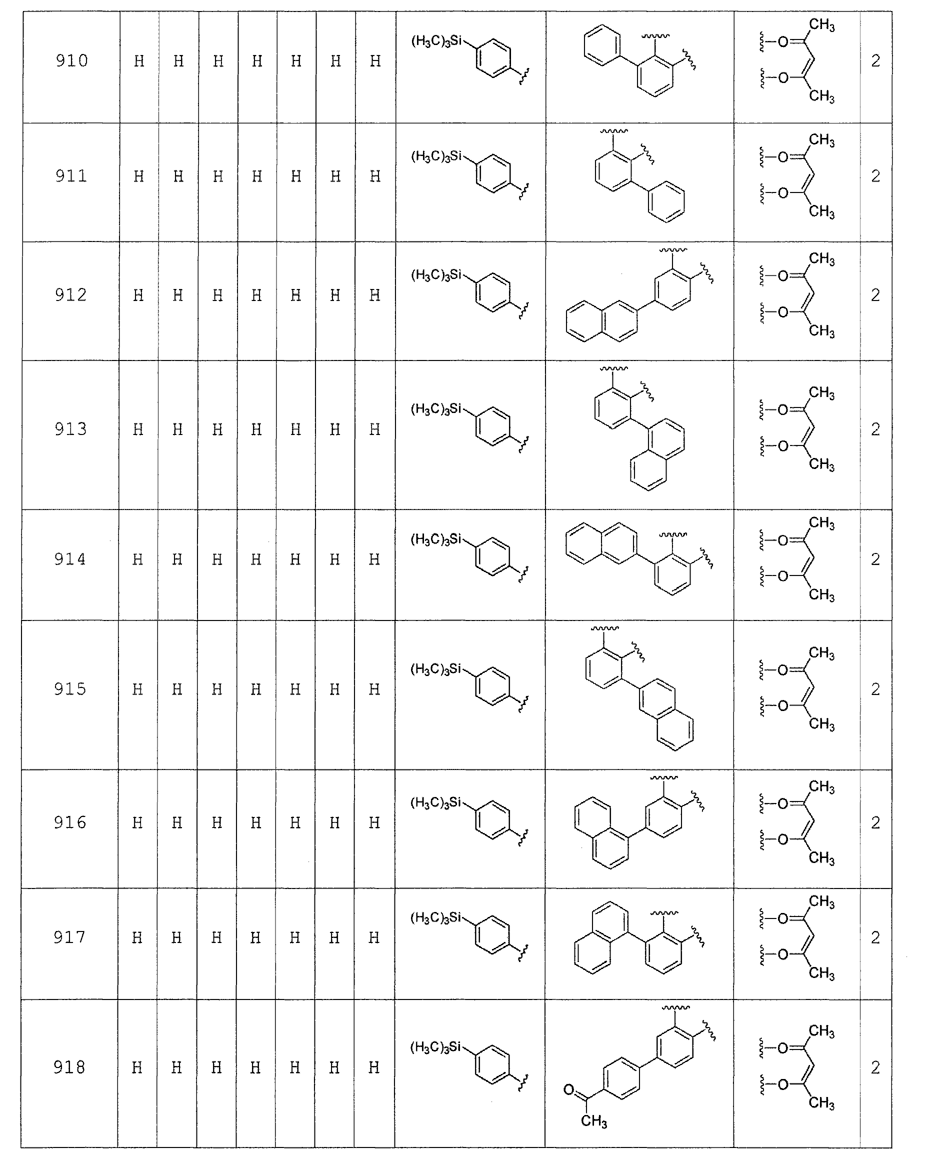

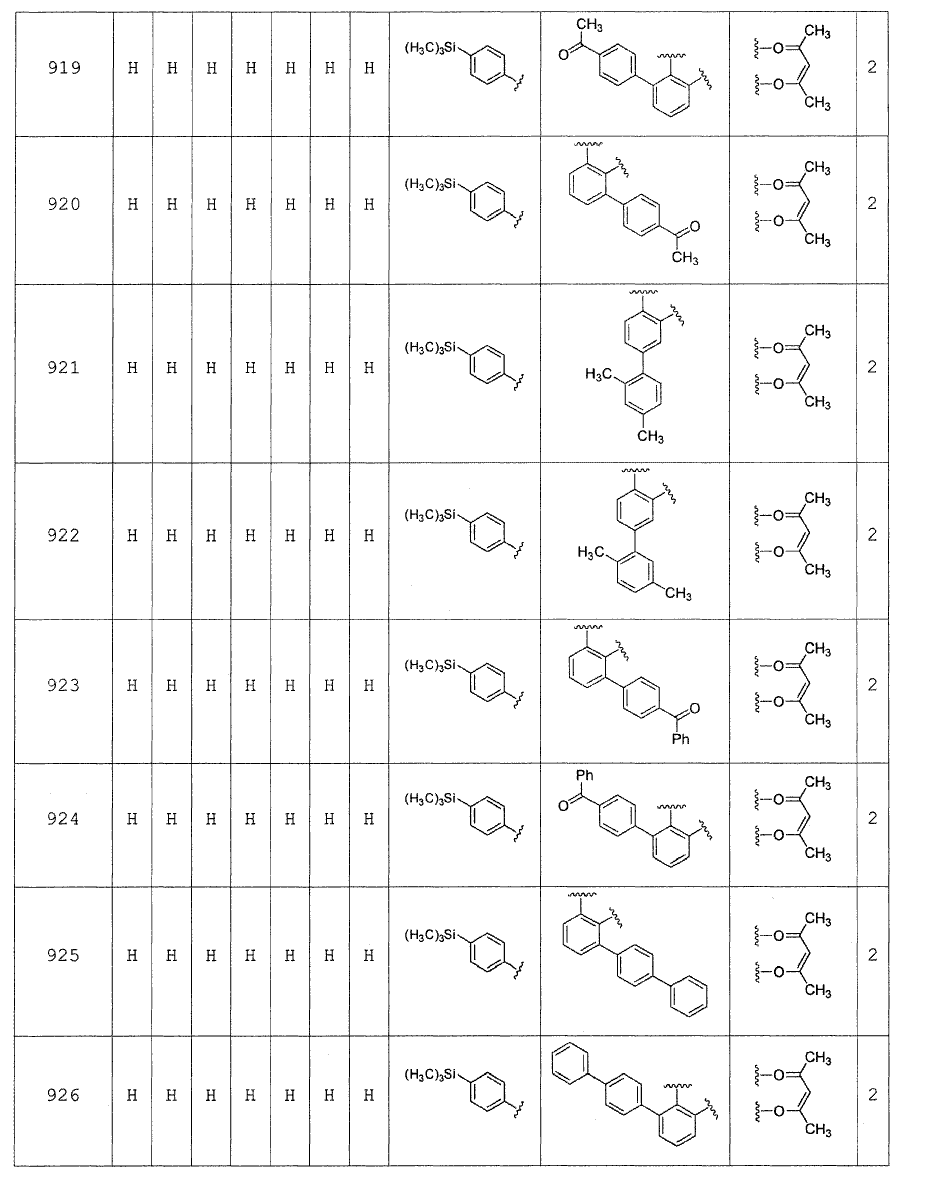

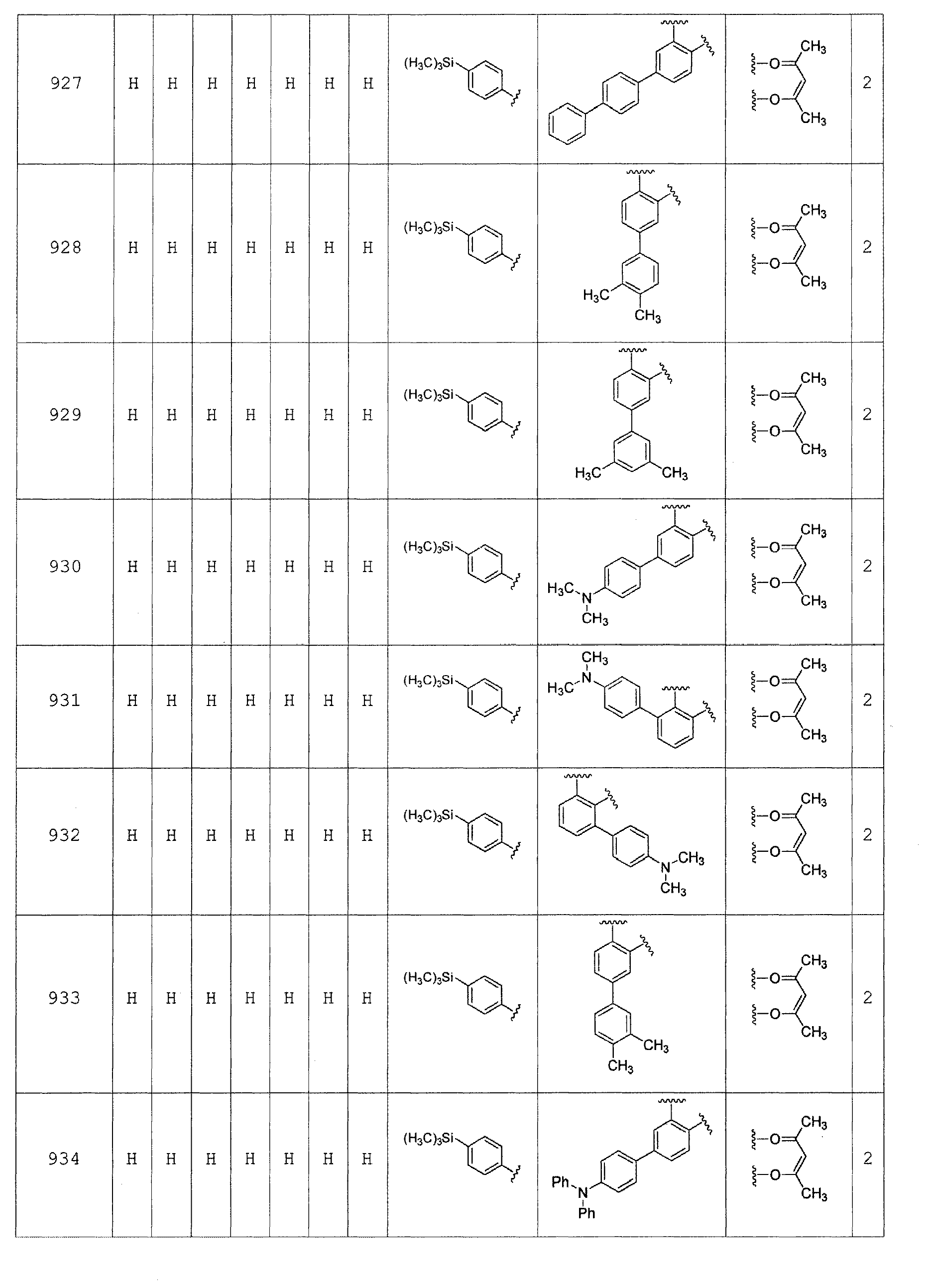

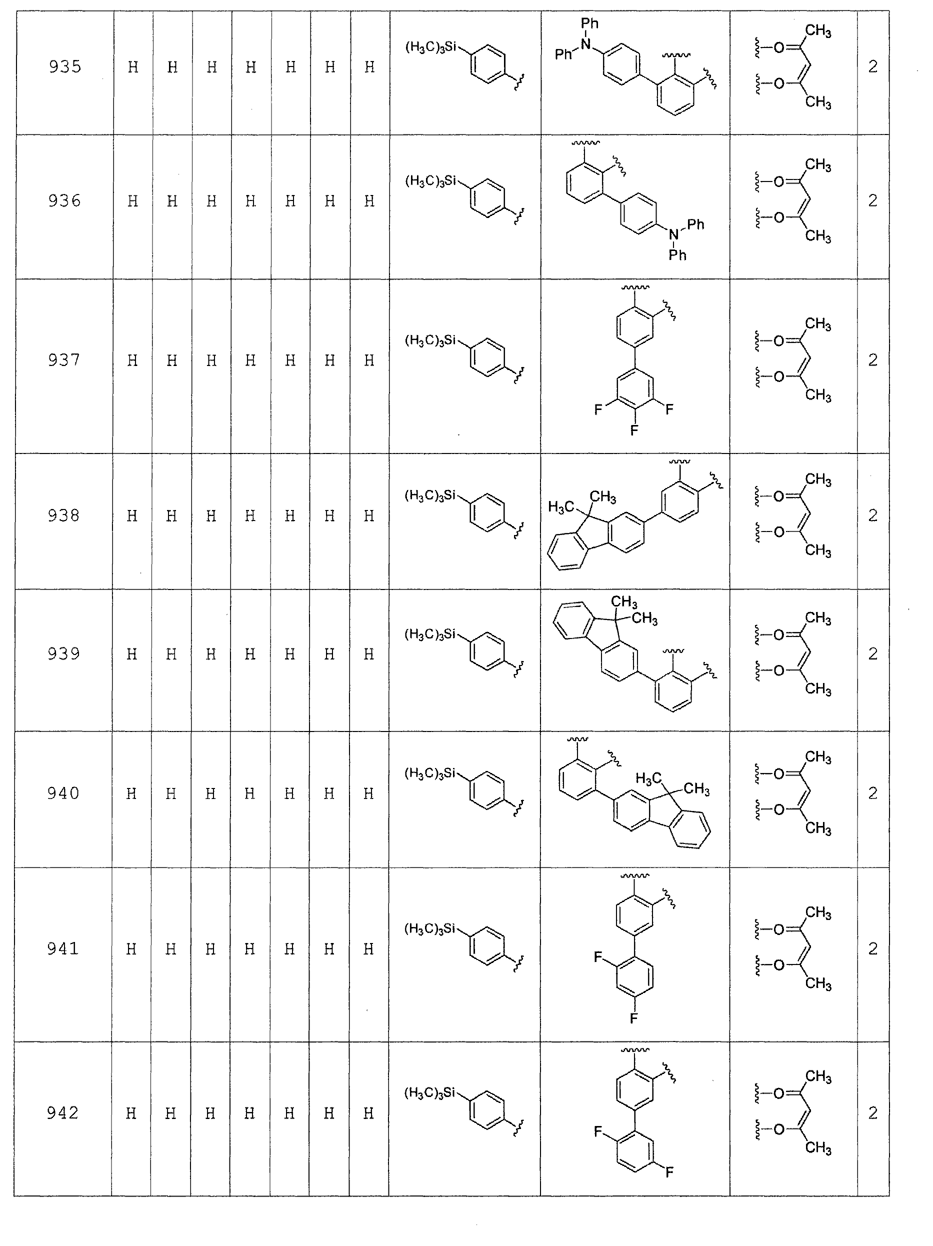

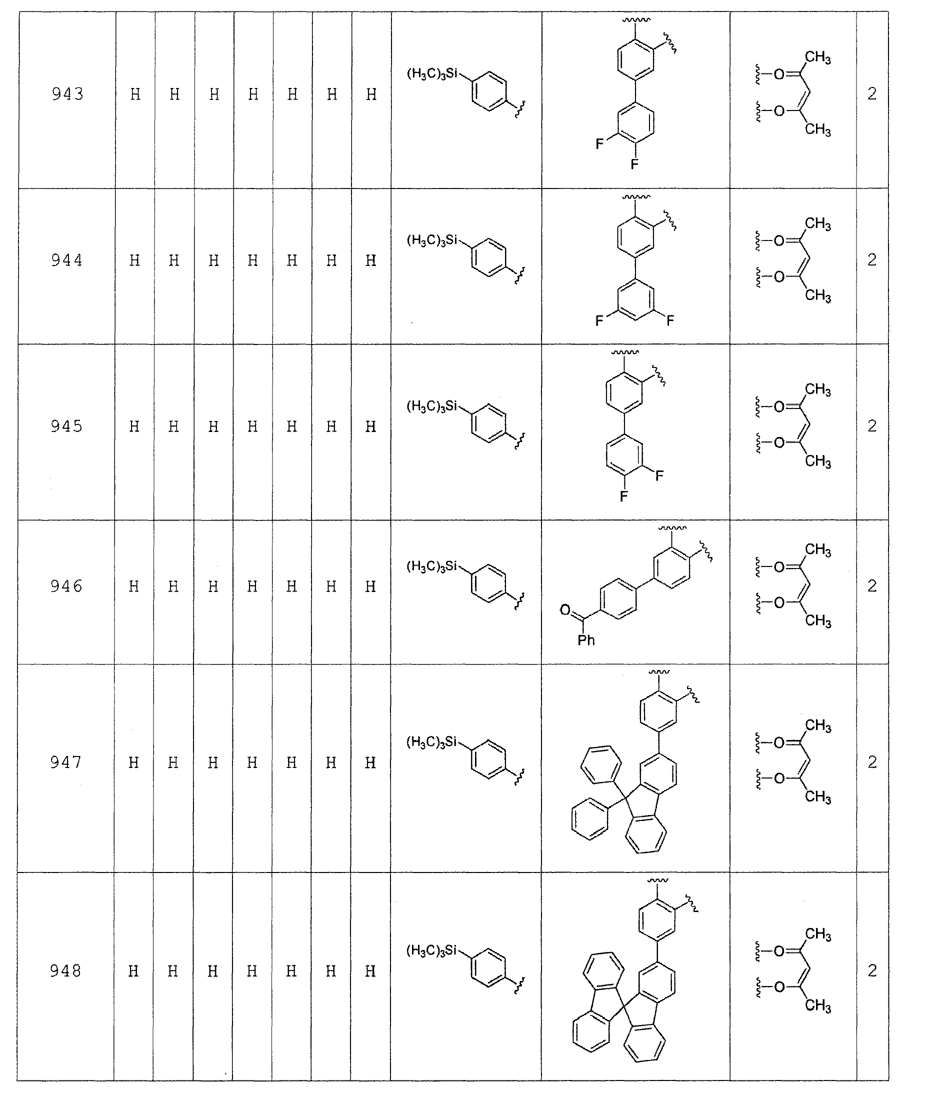

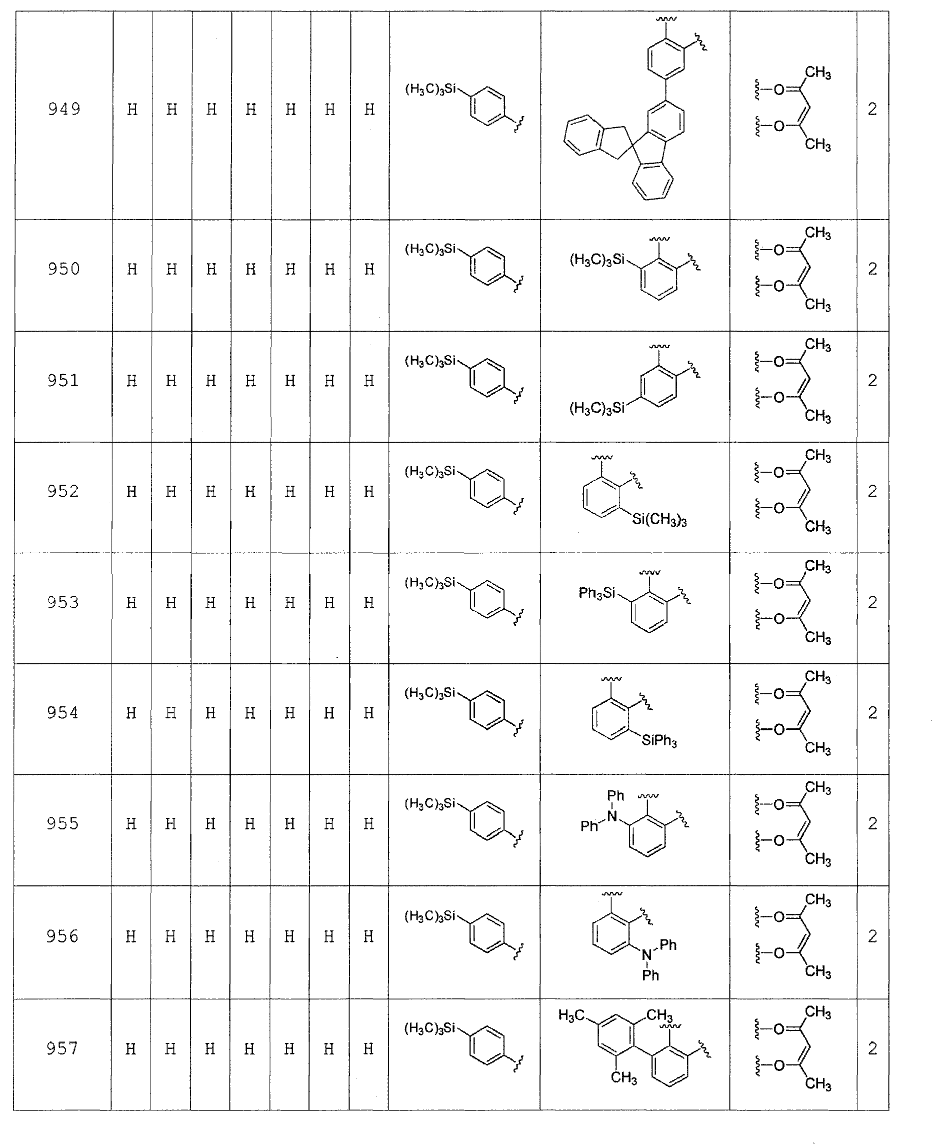

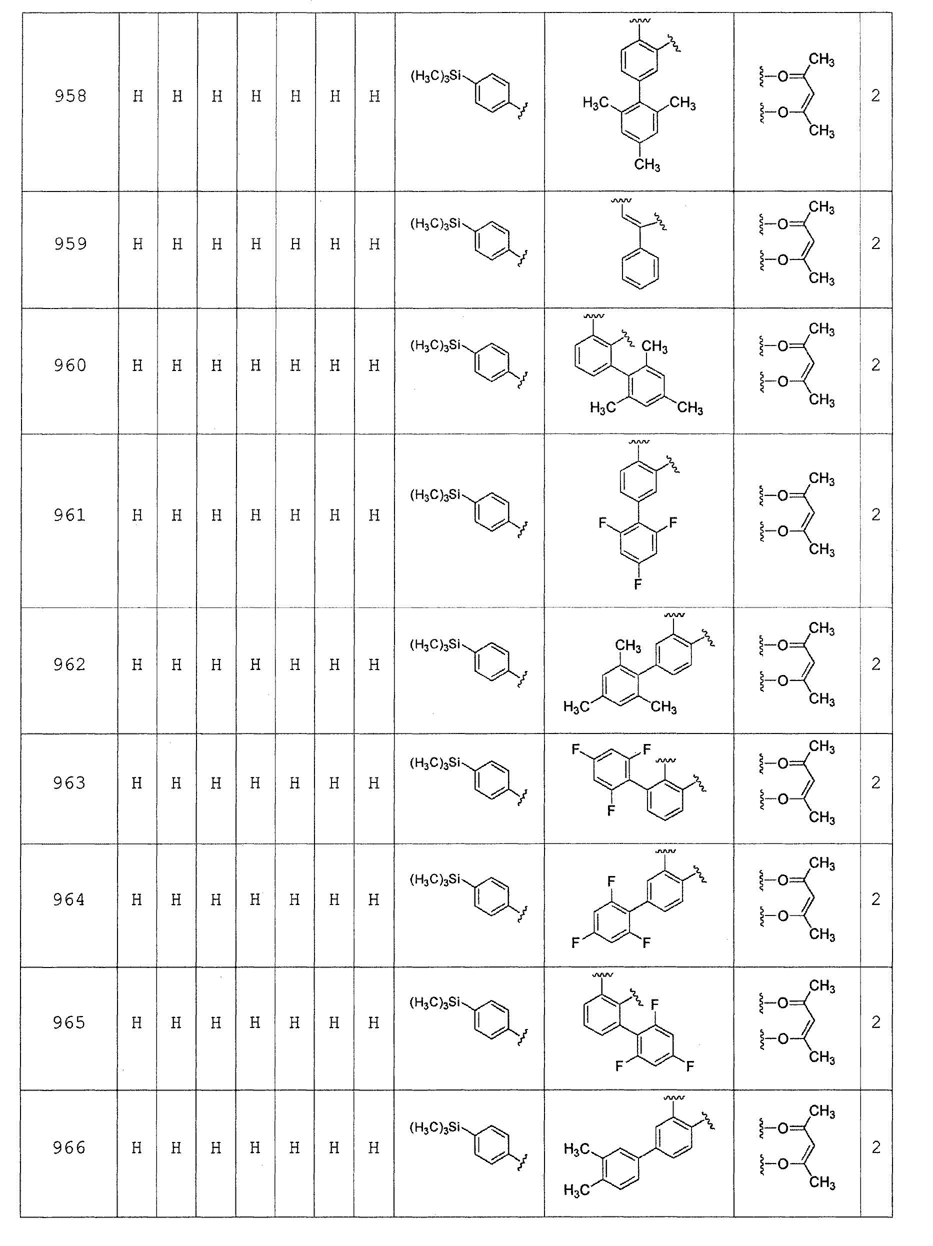

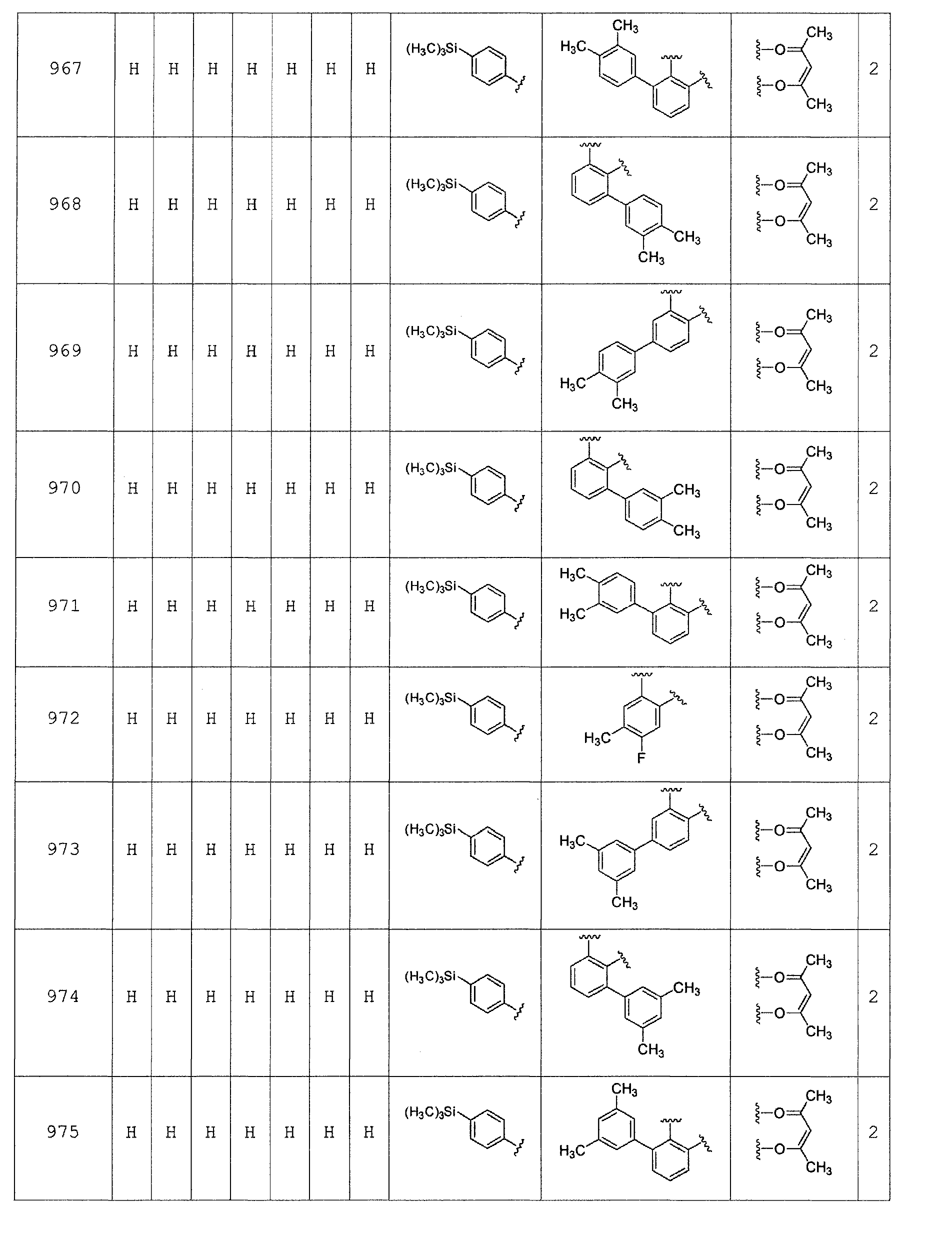

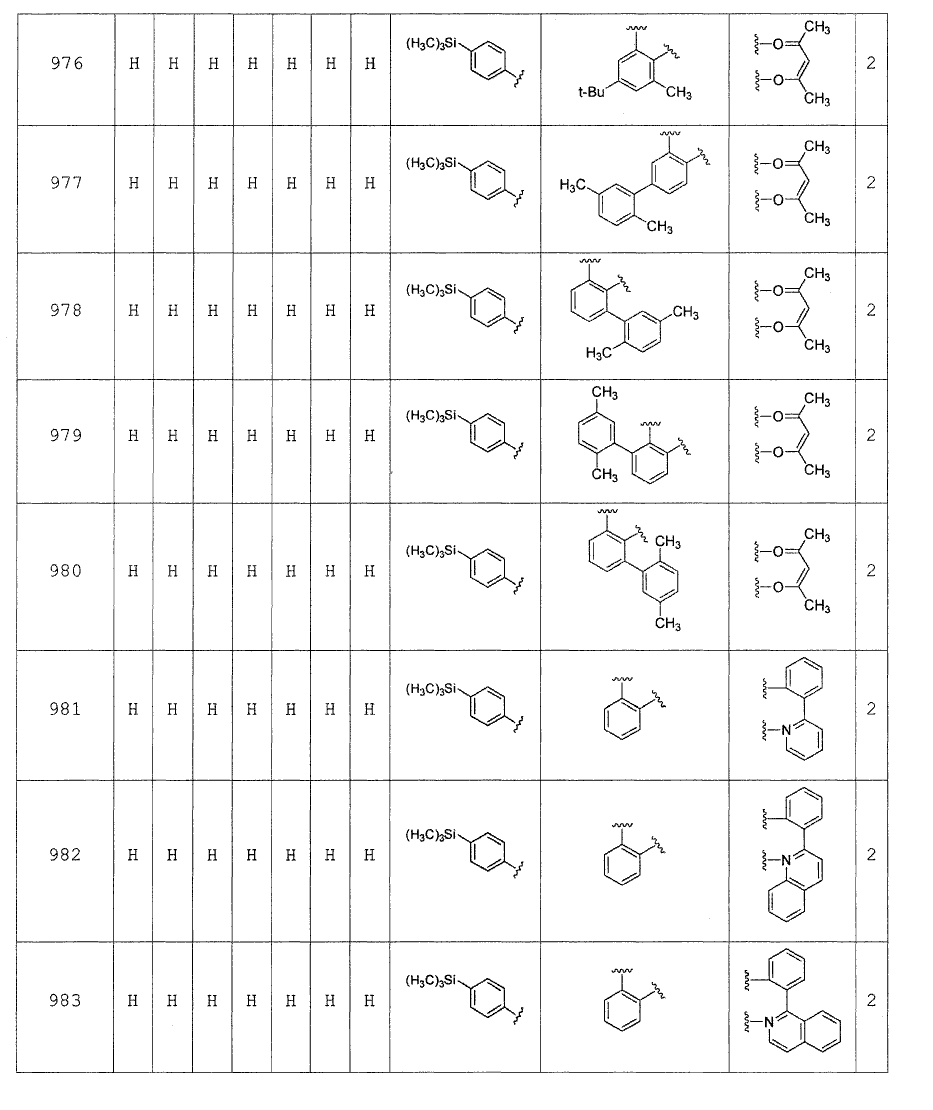

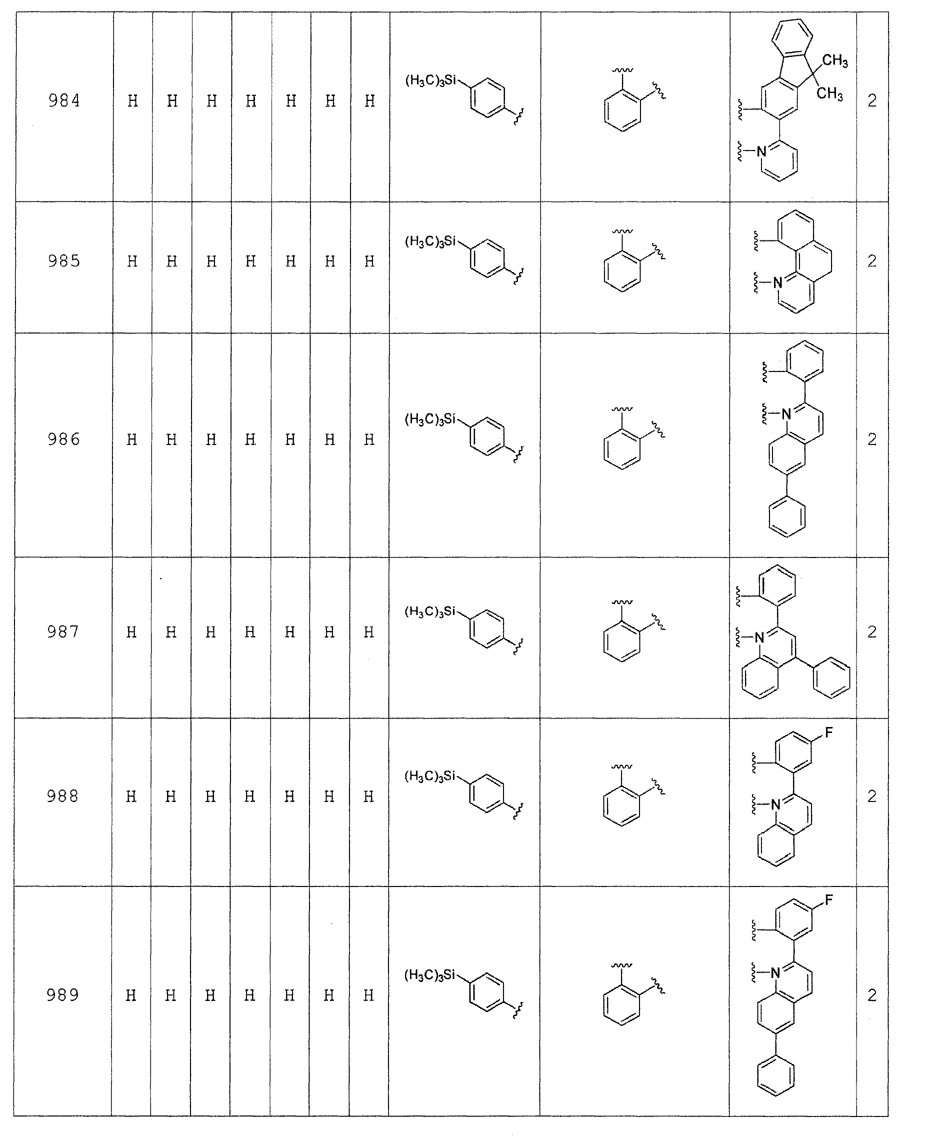

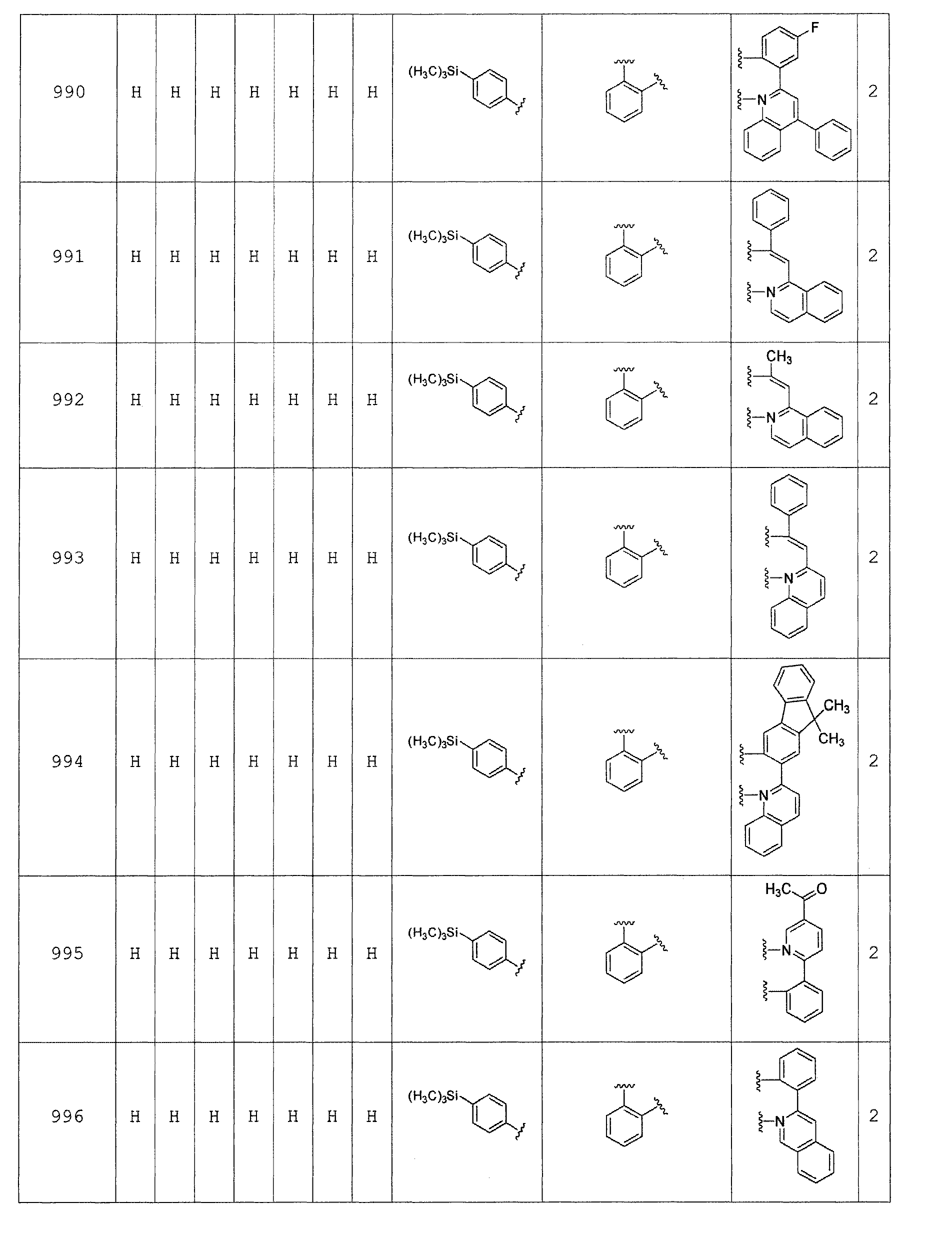

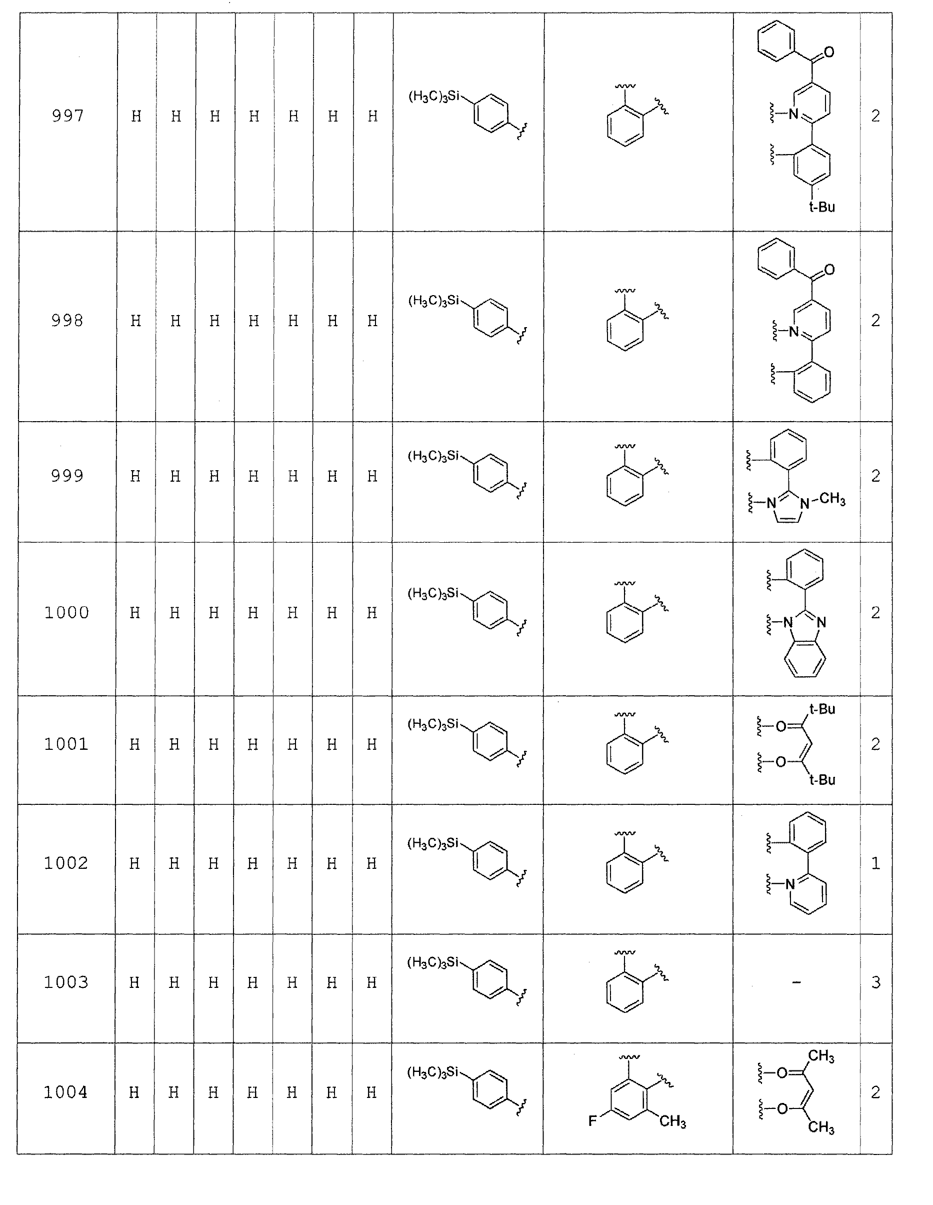

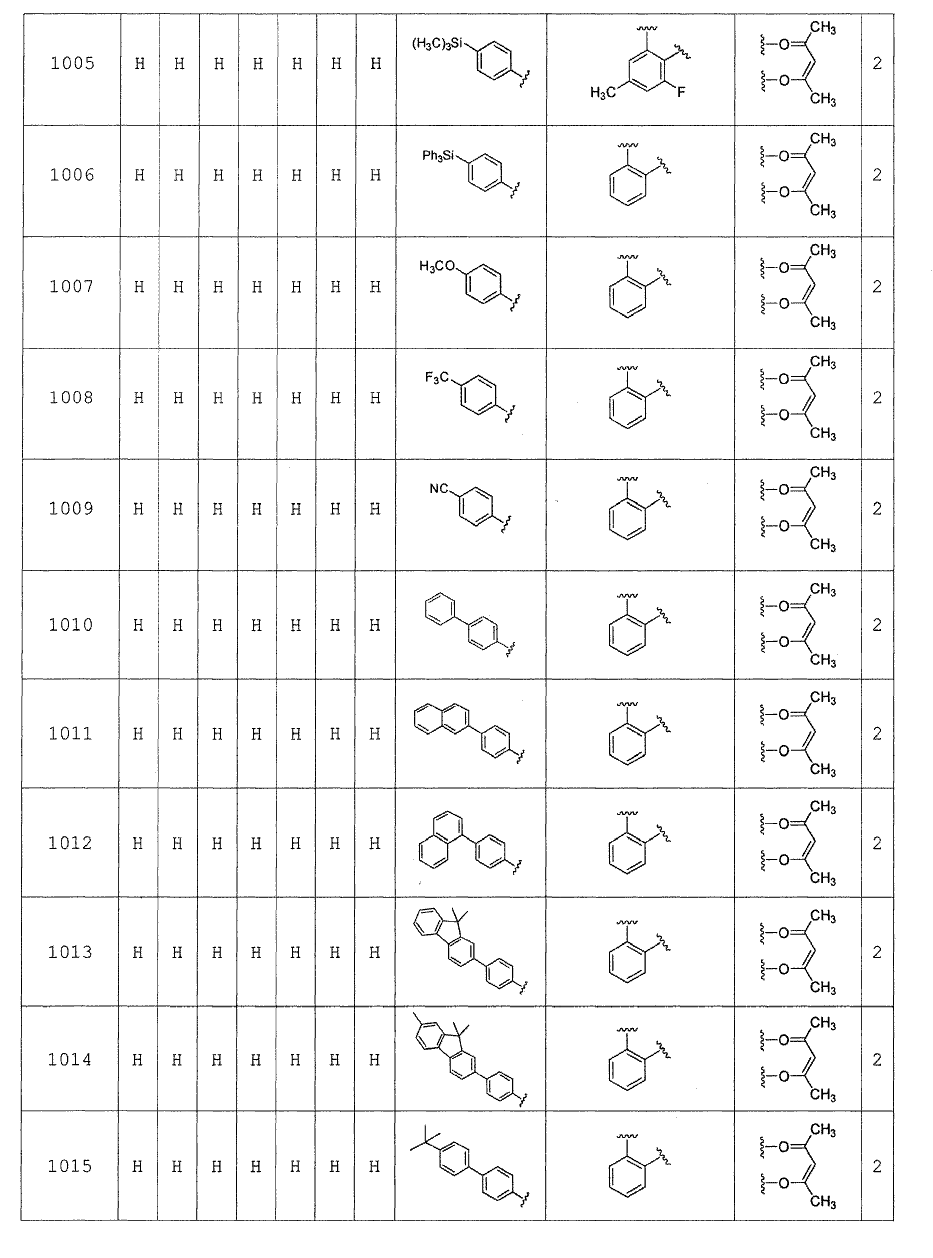

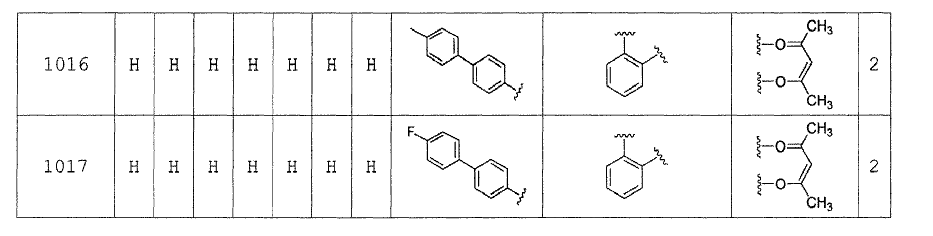

**[Table 2]**

| Compound No. | ¹H NMR(CDCl₃, 200 MHz) | MS/FAB | |
|---|---|---|---|
| | | found | calculated |
| **1** | δ = 8.30(m, 2H), 8.16(m, 4H), 8.05(m, 2H), 7.79(m, 4H), 7.67-7.64(m, 8H), 7.54-7.41(m, 12H), 4.60(s, 1H), 2.05(S, 6H) | 952 | 952.26 |
| **3** | δ = 8.1-8.0(m, 4H), 7.7-7.6(m, 8H), 7.48(m, 4H), 7.32-7.22(m, 10H), 7.1(m, 4H), 6.10(s, 1H), 2.30(s, 3H), 1.71(s, 3H) | 988 | 988.11 |
| **11** | δ = 8.40(s, 2H), 8.05(m, 2H), 7.76(m, 2H), 7.68-7.64(m, 12H), 7.48(m, 4H), 7.32(m, 12H), 7.22(m, 2H), 4.62(s, 1H), 2.05(s, 6H) | 1052 | 1052.27 |
| **42** | δ = 8.16(m, 4H), 8.05(m, 2H), 7.79(m, 4H), 7.67-7.64(m, 8H), 7.51(m, 4H), 7.41(m, 2H), 7.19-7.13(m, 4H), 4.60(s, 1H), 2.59(s, 6H), 2.34(s, 6H), 2.05(s, 6H) | 1008 | 1008.25 |
| **65** | δ = 8.1(m, 2H), 7.7-7.6(m, 10H), 7.54-7.48(m, 16H), 7.4-7.36(m, 22H), 7.32-7.22(m, 10H), 6.10(s, 1H), 2.30(s, 3H), 1.71(s, 3H) | 1468 | 1468.91 |
| **103** | δ = 8.1(m, 2H), 7.9(m, 2H), 7.7-7.6(m, 8H), 7.5-7.4(m, 8H), 7.37-7.32(m, 12H), 7.22-6.83(m, 6H), 6.10(s, 1H), 3.73(s, 6H), 2.30(s, 3H), 1.71(s, 3H) | 1164 | 1164.37 |
| **105** | δ = 8.21-8.16(m, 6H), 8.05(m, 2H), 7.79(m, 4H), 7.64-7.60(m, 10H), 7.54-7.41(m, 18H), 4.59(s, 1H), 2.06(s, 6H) | 1104 | 1104.35 |
| **143** | δ = 8.1(m, 4H), 7.90-7.84(m, 4H), 7.77-7.70(m, 4H), 7.67-7.60(m, 12H), 7.55-7.48(m, 6H), 7.38-7.32(m, 10H), 7.28-7.22(m, 4H), 7.14-7.06(m, 20H), 6.10(s, 1H), 2.30(s, 3H), 1.71(s, 3H) | 1584 | 1584.92 |
| **148** | δ = 8.1-8.0(m, 4H), 7.7-7.6(m, 8H), 7.5-7.48(m, 6H), 7.32-7.22(m, 12H), 6.10(s, 1H), 2.30(s, 3H), 1.71(s, 3H), 0.66(s, 18H) | 1096 | 1096.49 |
| **172** | δ = 8.05(m, 2H), 7.83(s, 2H), 7.68-7.64(m, 8H), 7.48(m, 4h), 7.32(m, 8H), 7.22(m, 2H), 7.11(m, 2H), 4.62(s, 1H), 2.35(s, 6H), 2.05(s, 6H), 1.34(s, 18H) | 1092 | 1092.41 |
| **179** | δ = 8.42(m, 1H), 8.30(m, 3H), 8.16(m, 4H), 8.05(m, 2H), 7.92(m, 1H), 7.79(m, 4H), 7.67-7.41(m, 26H), 7.10(m, 1H) | 1257 | 1257.52 |
| **192** | δ = 8.1-8.0(m, 6H), 7.7-7.6(m, 11H), 7.48-7.40(m, 6H), 7.32-7.22(m, 19H) | 1057 | 1057.27 |
| **217** | δ = 8.1(m, 4H), 7.8-7.7(m, 10H), 7.67-7.45(m, 8H), 7.36-7.32(m, 12H), 7.12(m, 4H), 6.10(s, 1H), 2.35(s, 6H), 2.30(s, 3H), 1.71(s, 3H) | 1188 | 1188.39 |
| **248** | δ 8.05(m, 2H), 7.68(m, 8H), 7.36-7.32(m, 8H), 7.12(m, 4H), 6.83-6.77(m, 4H), 4.62(s, 1H), 2.35(s, 6H), 2.12(s, 6H) | 1052 | 1052.17 |
| **261** | δ = 8.1-8.0(m, 4H), 7.7(m, 2H), 7.67-7.6(m, 6H), 7.36-7.30(m, 12H), 7.12(m, 4H), 6.10(s, 1H), 2.35(s, 6H), 2.30(s, 3H), 1.71(s, 3H), 1.34(s, 18H) | 1092 | 1092.39 |
| **271** | 5 = 8.2-8.1(m, 4H), 7.7-7.5(m, 10H), 7.36-7.30(m, 10H), 7.12(m, 4H), 6.10(s, 1H), 2.35(s, 6H), 2.30(s, 3H), 1.71(s, 3H) | 1116 | 1116.18 |
| **306** | δ = 8.2-8.1(m, 4H), 7.7-7.6(m, 8H), 7.5-7.4(m, 8H), 7.36-7.32(m, 12H), 7.22-7.12(m, 6H), 6.10(s, 1H), 2.35(s, 6H), 2.30(s, 3H), 1.71(s, 3H) | 1132 | 1132.37 |
| **314** | δ = 8.05(m, 2H), 7.68-7.57(m, 18H), 7.38-7.31(m, 18H), 7.12(m, 4H), 4.58(s, 1H), 2.35(s, 6H), 2.08(s, 6H) | 1232 | 1232.51 |
| **378** | δ = 8.56(m, 1H), 8.1-8.0(m, 5H), 7.7-7.6(m, 8H), 7.54-7.47(m, 2H), 7.36-7.30(m, 17H), 7.12-6.98(m, 5H), 2.35(s, 6H) | 1035 | 1035.26 |
| **410** | δ = 8.16(m, 4H), 8.06-8.05(m, 4H), 7.67-7.64(m, 8H), 7.39-7.30(m, 8H), 7.08(m, 4H), 4.60(s, 1H), 3.93(s, 6H), 2.06(s, 6H) | 1048 | 1048.19 |
| **536** | δ = 8.21(s, 2H), 8.05(m, 2H), 7.90-7.84(m, 4H), 7.77(m, 2H), 7.68-7.64(m, 8H), 7.60-7.50(m, 6H), 7.46-7.41(m, 6H), 7.38-7.28(m, 4H), 7.03(m, 4h), 4.62(s, 1H), 2.08(s, 6H), 1.67(s, 12H) | 1372 | 1372.65 |
| **557** | δ = 8.1(m, 2H), 7.7-7.6(m, 8H), 7.46(m, 4H), 7.32-7.30(m, 8H), 7.21-7.02(m, 10H), 7.2(s, 2H), 6.10(s, 1H), 2.30(s, 3H), 1.71(s, 3H) | 1040 | 1040.18 |
| **604** | δ = 8.1-8.0(m, 4H), 7.7-7.6(m, 8H), 7.4-7.3(m, 18H), 6.10(s, 1H), 2.30(s, 3H), 1.71(s, 3H), 1.34(s, 18H) | 1064 | 1064.34 |
| **652** | δ = 8.16(m, 4H), 8.05(m, 2H), 7.78(m, 2H), 7.67-7.64(m, 8H), 7.38-7.37(m, 8H), 4.59(s, 1H), 2.05(s, 6H), 1.35(s, 18H) | 1172 | 1172.31 |
| **854** | δ = 8.16(m, 4H), 8.05(m, 2H), 7.80-7.77(m, 6H), 7.67-7.64(m, 8H), 7.46(m, 4H), 6.70(m, 2H), 4.59(s, 1H), 2.05(s, 6H), 0.25(s, 18H) | 1168 | 1168.47 |
| **972** | δ = 8.1(m, 2H), 7.8-7.7(m, 4H), 7.67-7.6(m, 6H), 7.54-7.46(m, 8H), 7.32-6.9(m, 6H), 6.10(s, 1H), 2.35(s, 6H), 2.30(s, 3H), 1.71(s, 3H), 0.66(s, 18H) | 1160 | 1160.52 |
| **1006** | δ = 8.1-8.0(m, 4H), 7.70-7.60(m, 12H), 7.54-7.58(m, 16H), 7.36-7.30(m, 28H), 6.10(s, 1H), 2.30(s, 3H), 1.71(s, 3H) | 1468 | 1468.44 |
| **1007** | δ = 8.15-8.01(m, 4H), 7.71-7.65(m, 8H), 7.35-7.30(m, 14H), 6.85-6.82(m, 4H), 6.10(s, 1H), 3.73(s, 6H), 2.30(s, 3H), 1.71(s, 3H) | 1012 | 1012.29 |
| **1008** | δ = 8.1-8.0(m, 4H), 7.70-7.60(m, 8H), 7.51-7.41(m, 8H), 7.33-7.30(m, 10H), 6.11(s, 1H), 2.31(s, 3H), 1.71(s, 3H) | 1088 | 1088.24 |
| **1009** | δ = 8.1-8.0(m, 4H), 7.71-7.66(m, 10H), 7.60-7.57(m, 6H), 7.32-7.30(m, 10H), 6.10(s, 1H), 2.30(s, 3H), 1.71(s, 3H) | 1002 | 1002.25 |
| **1010** | δ = 8.05-7.99(m, 4H), 7.68-7.64(m, 8H), 7.54-7.48(m, 12H), 7.35-7.22(m, 16H), 4.58(s, 1H), 2.07(s, 6H) | 1104 | 1104.35 |
| **1011** | δ = 8.1-7.89(m, 6H), 7.73-7.6(m, 14H), 7.54-7.32(m, 24H), 6.10(s, 1H), 2.30(s, 3H), 1.71(s, 3H) | 1204 | 1204.36 |
| **1013** | δ = 8.1-8.0(m, 4H), 7.90-7.84(m, 4H), 7.77-7.60(m, 12H), 7.55-7.54(m, 10H), 7.38-7.32(m, 6H), 7.30-7.28(m, 8H), 6.10(s, 1H), 2.30(s, 3H), 1.71(s, 3H), 1.67(s, 12H) | 1336 | 1336.64 |

### [Example 1] Manufacture of an OLED (1)

An OLED device was manufactured by using a red phosphorescent compound according to the invention.

First, a transparent electrode ITO thin film (15 Ω/□) (2) prepared from glass for OLED (produced by Samsung Corning) (1) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injecting layer (3) having 60 nm of thickness on the ITO substrate.

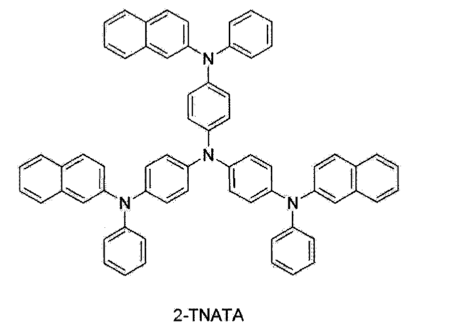

Then, to another cell of the vacuum vapor-deposit device, charged was N,N'-bis(α-naphthyl)-N,N'-diphenyl-4,4'-diamine (NPB), and electric current was applied to the cell to evaporate NPB, thereby providing vapor-deposit of a hole transport layer (4) of 20 nm of thickness on the hole injecting layer.

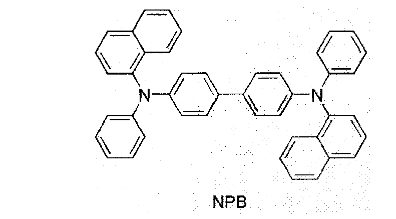

To another cell of said vacuum vapor-deposit device, charged was 4,4`-N,N'-dicarbazole-biphenyl (CBP) as an electroluminescent host material, and an organic electroluminescent compound (Compound 96) according to the present invention was charged to still another cell. The two materials were evaporated at different rates to carry out doping to vapor-deposit an electroluminescent layer (5) having 30 nm of thickness on the hole transport layer. The suitable doping concentration is 4 to 10 mol% on the basis of CBP.

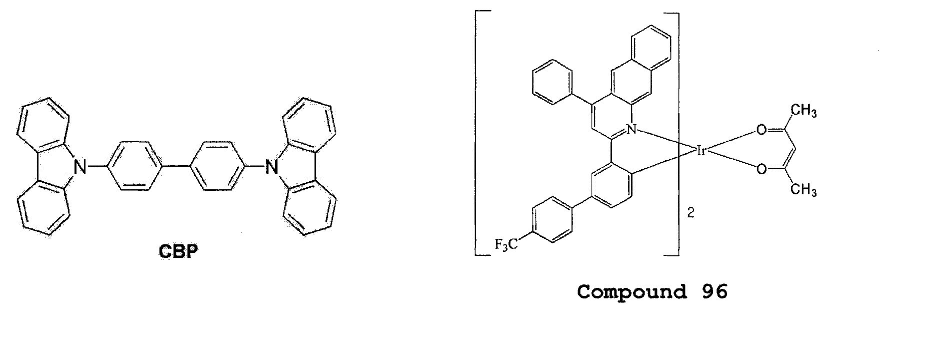

Then, on the electroluminescent layer, bis(2-methyl-8-quinolinato)(p-phenylphenolato)aluminum (III) (BAlq) was vapor-deposited as a hole blocking layer in a thickness of 10 nm in the same manner for NPB, tris(8-hydroxyquinoline)aluminum (III) (Alq) was vapor-deposited as an electron transport layer (6) in a thickness of 20 nm, and then lithium quinolate (Liq) was vapor-deposited as an electron injecting layer (7) in a thickness of 1 to 2 nm. Thereafter, an Al cathode (8) was vapor-deposited in a thickness of 150 nm by using another vacuum vapor-deposit device to manufacture an OLED.

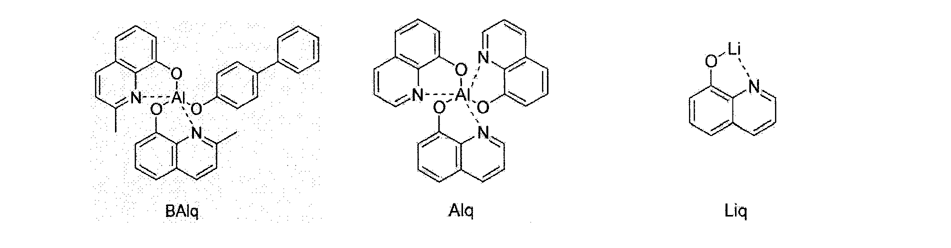

### [Example 2] Manufacture of an OLED (2)

A hole injecting layer and a hole transport layer were formed according to the procedure of Example 1, and an electroluminescent layer was vapor-deposited as follows. In another cell of said vacuum vapor-deposit device, charged was H-5 as an electroluminescent host material, and a phosphorescent compound (Compound 61) according to the present invention was charged to still another cell. The two materials were evaporated at different rates to carry out doping to vapor-deposit an electroluminescent layer (5) having 30 nm of thickness on the hole transport layer. The suitable doping concentration is 4 to 10 mol% on the basis of the host. Then, a hole blocking layer, an electron transport layer and an electron injecting layer were vapor-deposited according to the same procedure as in Example 1, and then Al cathode was vapor-deposited in a thickness of 150 nm by using another vacuum vapor-deposit device to manufacture an OLED.

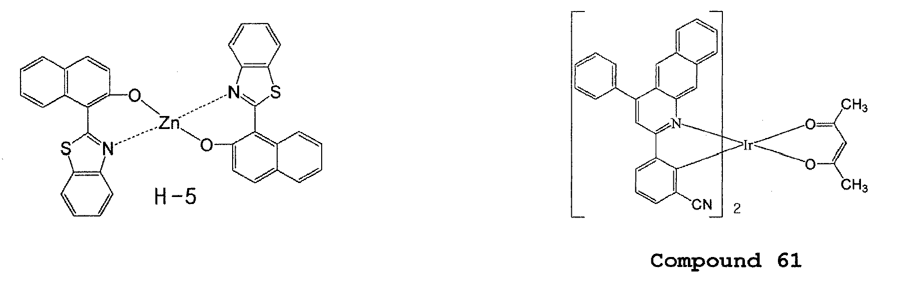

### [Example 3] Manufacture of an OLED (3)

A hole injecting layer, a hole transport layer and an electroluminescent layer were formed according to the same procedure as in Example 2, and then an electron transport layer and an electron injecting layer were vapor-deposited. Thereafter, Al cathode was vapor-deposited in a thickness of 150 nm by using another vacuum vapor-deposit device to manufacture an OLED.

### [Example 4] Evaluation of optical properties of electroluminescent materials

The complexes having high synthetic yield were purified by vacuum sublimation at 10⁻⁶ torr and used as a dopant for an electroluminescent layer of an OLED. In order to examine the performances of OLED's manufactured from Examples 1 to 3, luminous efficiency of OLED's was measured at 10 mA/cm². Properties of various electroluminescent compounds according to the invention are shown in Table 3.

**[Table 3]**

| | Material | Host | Hole blocking layer | EL color | Operation voltage | Max. luminous efficiency (cd/A) |
|---|---|---|---|---|---|---|
| Ex. 1 | Compound 1 | CBP | BAlq | Red | 8.2 | 6.8 |
| | Compound 14 | CBP | BAlq | Red | 8.1 | 7.7 |
| | Compound 49 | CBP | BAlq | Red | 7.9 | 8.2 |
| | Compound 96 | CBP | BAlq | Red | 7.7 | 7.1 |
| | Compound 142 | CBP | BAlq | Red | 7.9 | 7.9 |
| | Compound 177 | CBP | BAlq | Red | 7.7 | 7.8 |
| | Compound 191 | CBP | BAlq | Red | 8.2 | 5.8 |
| | Compound 198 | CBP | BAlq | Red | 7.9 | 6.1 |
| | Compound 288 | CBP | BAlq | Red | 8.0 | 5.9 |
| | Compound 356 | CBP | BAlq | Red | 8.3 | 6.4 |
| | Compound 390 | CBP | BAlq | Red | 7.7 | 6.0 |
| | Compound 400 | CBP | BAlq | Red | 8.2 | 5.6 |
| | Compound 450 | CBP | BAlq | Red | 7.7 | 6.4 |
| | Compound 467 | CBP | BAlq | Red | 7.9 | 6.1 |
| | Compound 586 | CBP | BAlq | Red | 8.0 | 5.8 |
| | Compound 671 | CBP | BAlq | Red | 8.0 | 6.9 |
| | Compound 800 | CBP | BAlq | Red | 7.6 | 6.7 |
| Ex. 2 | Compound 61 | H-5 | BAlq | Red | 7.4 | 6.8 |
| | Compound 217 | H-33 | BAlq | Red | 7.8 | 5.9 |
| | Compound 929 | H-10 | BAlq | Red | 8.0 | 6.3 |
| Ex. 3 | Compound 467 | H-77 | - | Red | 7.0 | 6.3 |
| | Compound 597 | H-5 | - | Red | 6.9 | 6.7 |
| | Compound 997 | H-64 | - | Red | 6.6 | 7.4 |

The compounds according to the present invention, to which benzo-quinoline was incorporated, exhibit improved red color coordinates as compared to the conventional compounds employing quinoline, iso-quinoline or pyridine, thereby having advantageous color reproductivity. Compounds (such as Compounds 177 and 997) to which ppy or (6-(4-tert-butylphenyl)pyridin-3-yl)(phenyl)methanone was incorporated as a subsidiary ligand, are good dopants with excellent color coordinate and high efficiency.

With identical device structure, using the host according to the present invention instead of CBP in an EL device did not provide significant change in efficiency, color coordinate and operation voltage. Thus it is anticipated that those hosts can be employed as a phosphorescent host, when being used with dopants according to the invention, instead of CBP as a conventional electroluminescent host. When the host according to the invention is employed without using a hole blocking layer, the device exhibits comparable or higher luminous efficiency as compared to that using conventional host, and provides decreased power consumption of the OLED due to lowered operation voltage by at least 0.7∼1.7 V. If the invention is applied to mass production of OLEDs, the time required for mass production can be also reduced to give great benefit on the commercialization.

## Claims

1. An organic electroluminescent compound represented by Chemical Formula (1): 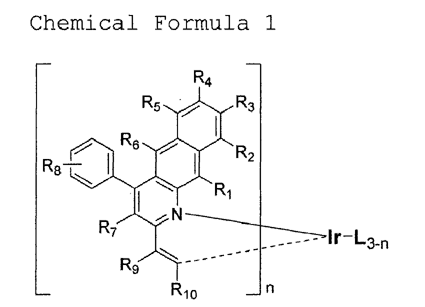 wherein, L is an organic ligand;
R₁ through R₇ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁ through R₇ may be linked to an adjacent substituent via (C3-C60)alkylene or (C3-C60)alkenylene with or without a fused ring to form an alicyclic ring, or a monocyclic or polycyclic aromatic ring;
R₈ represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
R₉ and R₁₀ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉ and R₁₀ may be linked to an adjacent substituent via (C3-C60)alkylene or (C3-C60)alkenylene with or without a fused ring to form an alicyclic ring, or a monocyclic or polycyclic aromatic ring;
the alkyl, alkenyl, alkynyl, cycloalkyl, heterocycloalkyl, aryl, heteroaryl, arylsilyl, alkylsilyl, alkylamino, arylamino of R₁ through R₁₀, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and
n is an integer from 1 to 3.

2. The organic electroluminescent compound according to claim 1, which is selected from the compounds represented by one of Chemical Formulas (2) to (7): 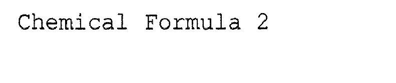 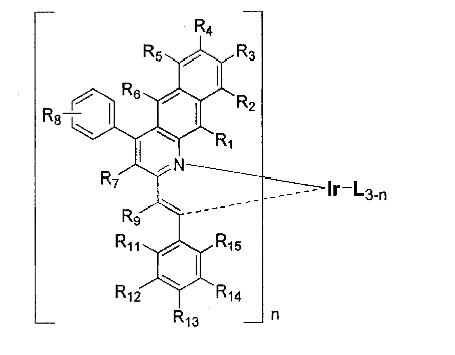 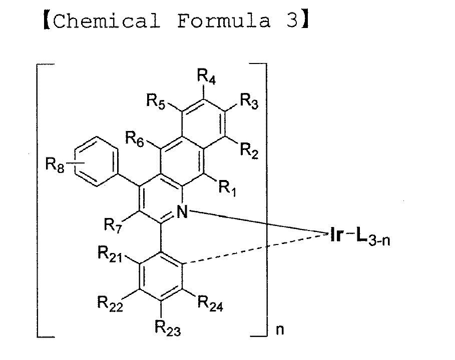 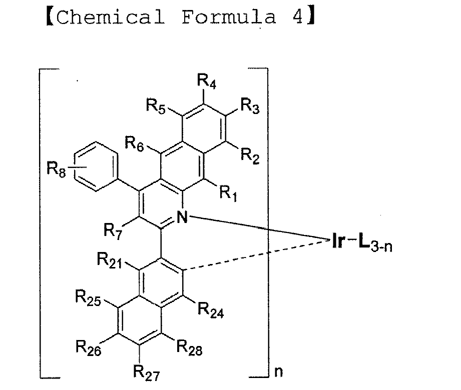 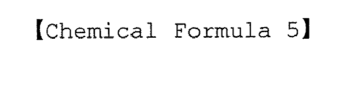 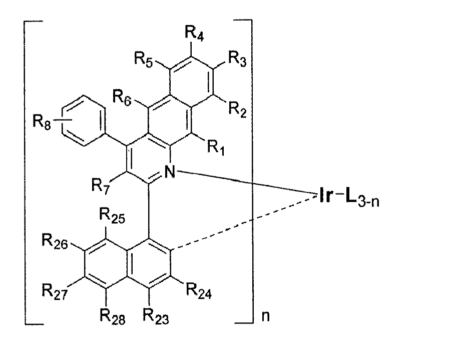 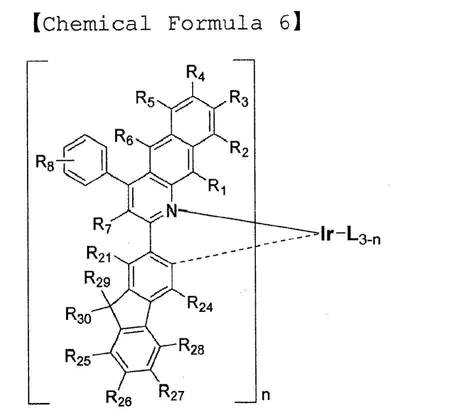 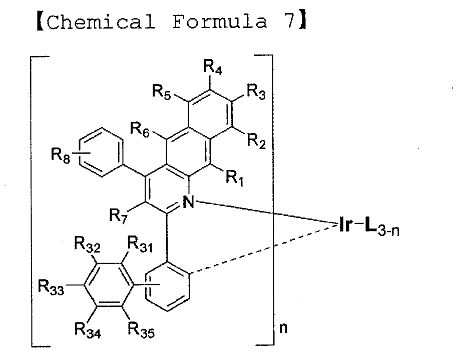 wherein, L, R₁, R₂, R₃, R₄, R₅, R₆, R₇, R₈, R₉ and n are defined as in Chemical Formula (1) of claim 1;
R₁₁ through R₂₈ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alky (C6-C60)aryloxy, (C6-C60)arylthio, (C1-C60)alkoxycarbonyl, carboxyl, nitro or hydroxyl, or each of R₁₁ through R₂₈ may be linked to an adjacent substituent via (C3-C60)alkylene or (C3-C60)alkenylene with or without a fused ring to form an alicyclic ring, or a monocyclic or polycyclic aromatic ring;
R₂₉ and R₃₀ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₂₉ and R₃₀ may be linked via (C3-C60)alkylene or (C3-C60)alkenylene with or without a fused ring to form an alicyclic ring, or a monocyclic or polycyclic aromatic ring;
R₃₁ through R₃₅ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-G60)arylthio, (C1-C60)alkoxycarbonyl, carboxyl, nitro or hydroxyl, or each of R₃₁ through R₃₅ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkylamino, arylamino of R₁₁ through R₃₅, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60) aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

3. The organic electroluminescent compound according to claim 1, wherein the ligand (L) has a structure represented by one of the following chemical formulas: 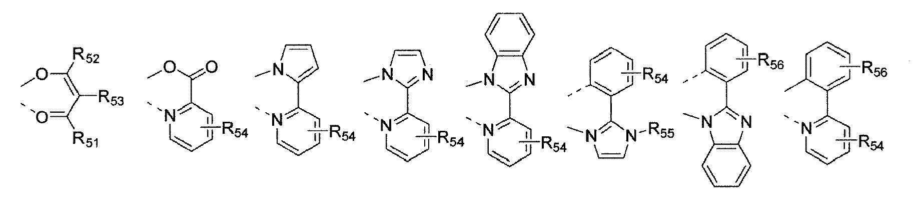 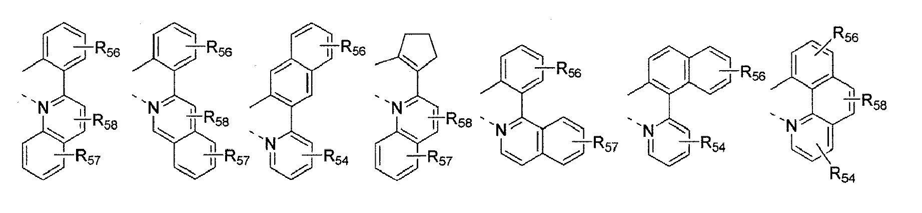 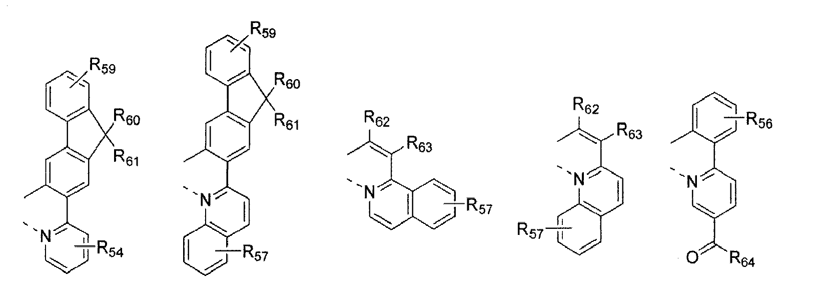 wherein, R₅₁ through R₆₄ independently represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alky (C6-C60)aryloxy, (C6-C60)arylthio, (C1-C60)alkoxycarbonyl, carboxyl, nitro or hydroxyl, or each of R₅₁ through R₆₄ may be linked to an adjacent substituent via (C3-C60)alkylene or (C3-C60)alkenylene with or without a fused ring to form an alicyclic ring, or a monocyclic or polycyclic aromatic ring; and the alkyl, alkenyl, alkynyl, cycloalkyl, heterocycloalkyl, aryl, heteroaryl, arylsilyl, alkylsilyl, alkylamino, arylamino of R₅₁ through R₆₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

4. An organic electroluminescent device which is comprised of a first electrode; a second electrode; and at least one organic layer(s) interposed between the first electrode and the second electrode; wherein the organic layer comprises an electroluminescent region comprising a compound represented by Chemical Formula (1): 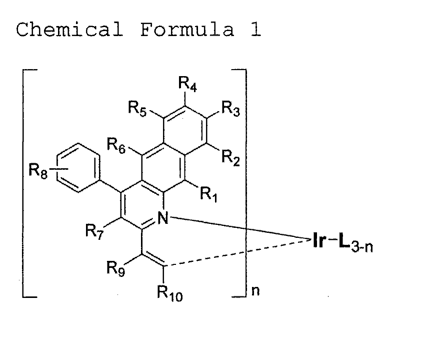 wherein, L is an organic ligand;
R₁ through R₇ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁ through R₇ may be linked to an adjacent substituent via (C3-C60)alkylene or (C3-C60)alkenylene with or without a fused ring to form an alicyclic ring, or a monocyclic or polycyclic aromatic ring;
R₈ represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
R₉ and R₁₀ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉ and R₁₀ may be linked to an adjacent substituent via (C3-C60)alkylene or (C3-C60)alkenylene with or without a fused ring to form an alicyclic ring, or a monocyclic or polycyclic aromatic ring;
the alkyl, alkenyl, alkynyl, cycloalkyl, heterocycloalkyl, aryl, heteroaryl, arylsilyl, alkylsilyl, alkylamino, arylamino of R₁ through R₁₀, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and
n is an integer from 1 to 3, and one or more host(s) selected from 1,3,5-tricarbazolylbenzene, polyvinylcarbazole, m-biscarbazolylphenyl, 4,4'4"-tri(N-carbazolyl)triphenylamine, 1,3,5-tri(2-carbazolylphenyl)benzene, 1,3,5-tris(2-carbazolyl-5-methoxyphenyl)benzene, bis(4-carbazolylphenyl)silane and compounds represented by one of Chemical Formulas (8) to (11): 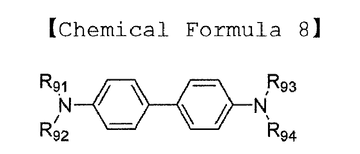
In Chemical Formula (8), R₉₁ through R₉₄ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉₁ through R₉₄ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkylamino, or arylamino of R₉₁ through R₉₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60) aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl. 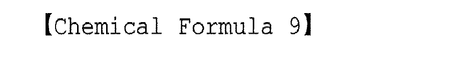 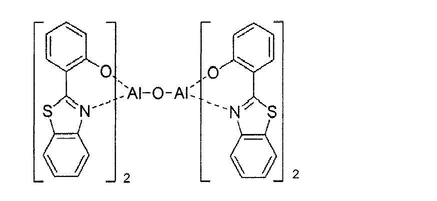 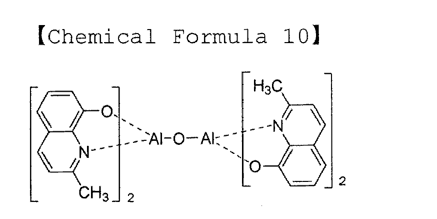
[Chemical Formula 11] L¹L²M¹(Q)_{y}
In Chemical Formula (11), the ligands, L¹ and L² are independently selected from the following structures: 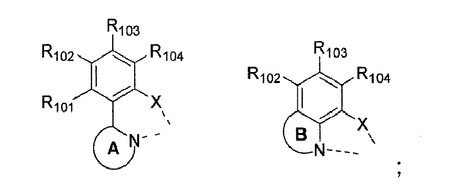
M¹ is a bivalent or trivalent metal;
y is 0 when M¹ is a bivalent metal, while y is 1 when M¹ is a trivalent metal;
Q represents (C6-C60)aryloxy or tri(C6-C60)arylsilyl, and the aryloxy and triarylsilyl of Q may be further substituted by (C1-C60)alkyl or (C6-C60)aryl;
X represents O, S or Se;
ring A represents oxazole, thiazole, imidazole, oxadiazole, thiadiazole, benzoxazole, benzothiazole, benzimidazole, pyridine or quinoline;
ring B represents pyridine or quinoline, and ring B may be further substituted by (C1-C60)alkyl, or phenyl or naphthyl with or without (C1-C60)alkyl substituent(s);
R₁₀₁ through R₁₀₄ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁₀₁ through R₁₀₄ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkylamino, or arylamino of ring A and R₁₀₁ through R₁₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

5. The organic electroluminescent device according to claim 4, wherein the organic layer comprises one or more compound(s) selected from a group consisting of arylamine compounds and styrylarylamine compounds.

6. The organic electroluminescent device according to claim 4, wherein the organic layer comprises one or more metal(s) selected from a group consisting of organic metals of Group 1, Group 2, 4^{th} period and 5^{th} period transition metals, lanthanide metals and d-transition elements.

7. The organic electroluminescent device according to claim 4, which has a pixel structure of independent light-emitting mode, which comprises an organic electroluminescent device containing said organic layer as a sub-pixel, and one or more sub-pixel(s) comprising one or more compound(s) selected from a group consisting of arylamine compounds and styrylarylamine compounds, patterned in parallel at the same time.

8. The organic electroluminescent device according to claim 4, which is an organic electroluminescent display comprising, in the organic layer, an organic electroluminescent compound according to any one of claims 1 to 3, and organic compounds or organometallic compounds having the electroluminescent peak with wavelength of green and blue, at the same time.

9. The organic electroluminescent device according to claim 4, wherein a mixed region of reductive dopant and organic substance, or a mixed region of oxidative dopant and organic substance is placed on the inner surface of one or both electrode(s) among the pair of electrodes.

10. An organic solar cell which comprises an organic electroluminescent compound represented by Chemical Formula (1): 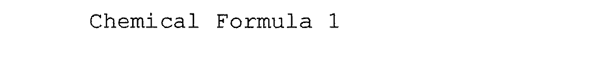 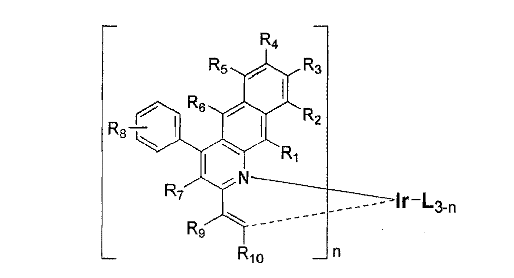 wherein, L is an organic ligand;
R₁ through R₇ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁ through R₇ may be linked to an adjacent substituent via (C3-C60)alkylene or (C3-C60)alkenylene with or without a fused ring to form an alicyclic ring, or a monocyclic or polycyclic aromatic ring;
R₈ represent hydrogen, deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
R₉ and R₁₀ independently represent hydrogen, deuterium, halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉ and R₁₀ may be linked to an adjacent substituent via (C3-C60)alkylene or (C3-C60)alkenylene with or without a fused ring to form an alicyclic ring, or a monocyclic or polycyclic aromatic ring;
the alkyl, alkenyl, alkynyl, cycloalkyl, heterocycloalkyl, aryl, heteroaryl, arylsilyl, alkylsilyl, alkylamino, arylamino of R₁ through R₁₀, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and n is an integer from 1 to 3.
